# EUROPEAN PATENT SPECIFICATION

(11) **EP 1 572 234 B1**
(45) Date of publication and mention of the grant of the patent: **22.02.2012**
(21) Application number: 03812906.0
(22) Date of filing: 10.12.2003
(51) Int. Cl.: A61K 39/29, A61K 39/00, A61K 39/385, A61K 39/295, C07K 17/00

(54) **STABILIZED IMMUNOGENIC HBc CHIMER PARTICLES**
STABILISIERTE IMMUNOGENE CHIMÄRE HBC-PARTIKEL
PARTICULES STABILISEES DE CHIMERES DE HBC IMMUNOGENES

(30) Priority: 10.12.2002 US 432123 P
(43) Date of publication of application: 14.09.2005
(73) Proprietor: Sanofi Pasteur Biologics Co., Cambridge, MA 02139 (US)
(72) Inventor: LYONS, Katelynne, San Diego, CA 92117 (US); BIRKETT, Ashley, J., Boston, MA 02111 (US); HARON, Jay, A., Jamul, CA 91935 (US)
(74) Representative: Fisher, Adrian John
(86) International application number: PCT/US2003/039164
(87) International publication number: WO 2004/053091

(56) References cited:
- WO-A-2004/075836
- WO-A2-02/14478
- US-A1- 2004 156 863
- US-B2- 7 361 352
- SCHODEL FLORIAN ET AL: "Avirulent Salmonella expressing hybrid hepatitis B virus core/pre-S genes for oral vaccination" VACCINE, vol. 11, no. 2, 1993, pages 143-148, XP002470452 & INTERNATIONAL CONFERENCE ON VACCINES FOR ENTERIC DISEASES; CAMBRIDGE, ENGLAND, UK; APRIL 13-15, 1992 ISSN: 0264-410X
- PUMPENS P ET AL: "Hepatitis B virus core particles as epitope carriers" INTERVIROLOGY, vol. 38, 1995, pages 63-74, XP002903139 ISSN: 0300-5526
- SCHODEL F ET AL: "STRUCTURE OF HEPATITIS B VIRUS CORE AND E-ANTIGEN A SINGLE PRECORE AMINO ACID PREVENTS NUCLEOCAPSID ASSEMBLY" JOURNAL OF BIOLOGICAL CHEMISTRY, AMERICAN SOCIETY OF BIOLOCHEMICAL BIOLOGISTS, BIRMINGHAM,, US, vol. 268, no. 2, 15 January 1993 (1993-01-15), pages 1332-1337, XP002928662 ISSN: 0021-9258
- ZHENG J ET AL: "THE STRUCTURE OF HEPADNAVIRAL CORE ANTIGENS" JOURNAL OF BIOLOGICAL CHEMISTRY, THE AMERICAN SOCIETY OF BIOLOGICAL CHEMISTS, INC.,, US, vol. 267, no. 13, May 1992 (1992-05), pages 9422-9429, XP008064651 ISSN: 0021-9258
- BIRNBAUM F ET AL: "Hepatitis B virus nucleocapsid assembly: primary structure requirements in the core protein" JOURNAL OF VIROLOGY, THE AMERICAN SOCIETY FOR MICROBIOLOGY, US, vol. 64, no. 7, July 1990 (1990-07), pages 3319-3330, XP002378507 ISSN: 0022-538X
- NASSAL M ET AL: "TOPOLOGICAL ANALYSIS OF THE HEPATITIS B VIRUS CORE PARTICLE BY CYSTEINE-CYSTEINE CROSS-LINKING" JOURNAL OF MOLECULAR BIOLOGY, LONDON, GB, vol. 225, no. 4, 1992, pages 1013-1025, XP009096430 ISSN: 0022-2836
- ZLOTNICK, A. ET AL.: 'Localization of the C terminus of the assembly domain of hepatitis B virus capsid protein: Implications for morphogenesis and organization of encapsidated RNA' PROC.NATL.ACAD.SCI. vol. 94, September 1997, pages 9556 - 9561, XP002982400
- ULRICH, R. ET AL.: 'Core particles of hepatitis B virus as carrier for foreign epitopes' ADVANCES IN VIRUS RESEARCH vol. 50, 1998, pages 141 - 182, XP000856161
- JEGERLEHNER, A. ET AL.: 'A molecular assembly system that renders antigens of choice highly repetitive for induction of protective B cell responses' VACCINE vol. 20, August 2002, pages 3104 - 3112, XP004374549
- KRATZ, P.A. ET AL.: 'Native display of complete foreign protein domainson the surface of hepatitis B virus capsids' PROC. NATL. ACAD. SCI. vol. 96, March 1999, pages 1915 - 1920, XP000910194
- NEIRYNCK, S. ET AL.: 'A universal influenza A vaccine based on the extracellular domain of the M2 protein' NATURE MEDICINE vol. 5, no. 10, pages 1157 - 1163, XP002196652
- PUMPENS, P. ET AL.: 'Hepatitis B core particles as a universal display model: a structure-function basis for development' FEBS LETTERS vol. 442, pages 1 - 6, XP002937462

### SEQUENCE LISTING

<110> Lyons, Katelynne J.
   Ashley, Birkett J.
   Haron, Jay A.
<120> STABILIZED IMMUNOGENIC HBc CHIMER PARTICLES
<130> ICC-136.0PCT (4564-91156)
<150> US 60/432,123
   <151> 2002-12-10
<150> US 10/274,616
   <151> 2002-10-21
<150> US 10/080,299
   <151> 2002-02-21
<150> US 10/082,014
   <151> 2002-02-22
<160> 455
<170> PatentIn version 3.2
<210> 1
   <211> 183
   <212> PRT
   <213> Hepatitis B virus
<400> 1
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Val Asn Leu Glu Asp Pro Ala
   65                  70                  75                  80
   Ser Arg Asp Leu Val Val Ser Tyr Val Asn Thr Asn Met Gly Leu Lys
                   85                  90                  95
   Phe Arg Gln Leu Leu Trp Phe His Ile Ser Cys Leu Thr Phe Gly Arg
               100                 105                 110
   Glu Thr Val Ile Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Gly Arg Ser Pro Arg Arg Arg Thr
   145                 150                 155                 160
   Pro Ser Pro Arg Arg Arg Arg Ser Gln Ser Pro Arg Arg Arg Arg Ser
                   165                 170                 175
   Gln Ser Arg Glu Ser Gln Cys
               180
<210> 2
   <211> 185
   <212> PRT
   <213> Hepatitis B virus
<400> 2
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Asn Asn Leu Gln Asp Pro Ala
   65                  70                  75                  80
   Ser Arg Asp Leu Val Val Asn Tyr Val Asn Thr Asn Met Gly Leu Lys
                   85                  90                  95
   Ile Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Asp Arg Gly Arg Ser Pro Arg Arg
   145                 150                 155                 160
   Arg Thr Pro Ser Pro Arg Arg Arg Arg Ser Gln Ser Pro Arg Arg Arg
                   165                 170                 175
   Arg Ser Gln Ser Arg Glu Ser Gln Cys
               180                 185
<210> 3
   <211> 185
   <212> PRT
   <213> Hepatitis B virus

<400> 3
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Asn Asn Leu Glu Asp Pro Ala
   65                  70                  75                  80
   Ser Arg Asp Leu Val Val Asn Tyr Val Asn Thr Asn Val Gly Leu Lys
                   85                  90                  95
   Ile Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Asp Arg Gly Arg Ser Pro Arg Arg
   145                 150                 155                 160
   Arg Thr Pro Ser Pro Arg Arg Arg Pro Ser Gln Ser Pro Arg Arg Arg
                   165                 170                 175
   Arg Ser Gln Ser Arg Glu Ser Gln Cys
               180                 185
<210> 4
   <211> 183
   <212> PRT
   <213> Hepatitis B virus
<400> 4
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ala Ala Leu Tyr Arg Asp Ala Leu Glu Ser Pro Glu His Cys
           35                  40                  45
   Ser Pro His His Thr Ala Leu Arg Gln Ala Ile Leu Cys Trp Gly Asp
       50                  55                  60
   Leu Met Thr Leu Ala Thr Trp Val Gly Thr Asn Leu Glu Asp Pro Ala
   65                  70                  75                  80
   Ser Arg Asp Leu Val Val Ser Tyr Val Asn Thr Asn Val Gly Leu Lys
                   85                  90                  95
   Phe Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Gly Arg Ser Pro Arg Arg Arg Thr
   145                 150                 155                 160
   Pro Ser Pro Arg Arg Arg Arg Ser Gln Ser Pro Arg Arg Arg Arg Ser
                   165                 170                 175
   Gln Ser Arg Glu Ser Gln Cys
               180
<210> 5
   <211> 183
   <212> PRT
   <213> woodchuck
<400> 5
   Met Asp Ile Asp Pro Tyr Lys Glu Phe Gly Ser Ser Tyr Gln Leu Leu
   1               5                   10                  15
   Asn Phe Leu Pro Leu Asp Phe Phe Pro Asp Leu Asn Ala Leu Val Asp
               20                  25                  30
   Thr Ala Thr Ala Leu Tyr Glu Glu Glu Leu Thr Gly Arg Glu His Cys
           35                  40                  45
   Ser Pro His His Thr Ala Ile Arg Gln Ala Leu Val Cys Trp Asp Glu
       50                  55                  60
   Leu Thr Lys Leu Ile Ala Trp Met Ser Ser Asn Ile Thr Ser Glu Gln
   65                  70                  75                  80
   Val Arg Thr Ile Ile Val Asn His Val Asn Asp Thr Trp Gly Leu Lys
                   85                  90                  95
   Val Arg Gln Ser Leu Trp Phe His Leu Ser Cys Leu Thr Phe Gly Gln
               100                 105                 110
   His Thr Val Gln Glu Phe Leu Val Ser Phe Gly Val Trp Ile Arg Thr
           115                 120                 125
   Pro Ala Pro Tyr Arg Pro Pro Asn Ala Pro Ile Leu Ser Thr Leu Pro
       130                 135                 140
   Glu His Thr Val Ile Arg Arg Arg Gly Gly Ala Arg Ala Ser Arg Ser
   145                 150                 155                 160
   Pro Arg Arg Arg Thr Pro Ser Pro Arg Arg Arg Arg Ser Gln Ser Pro
                   165                 170                 175
   Arg Arg Arg Arg Ser Gln Cys
               180
<210> 6
   <211> 217
   <212> PRT
   <213> ground squirrel
<400> 6
   Met Tyr Leu Phe His Leu Cys Leu Val Phe Ala Cys Val Pro Cys Pro
   1               5                   10                  15
   Thr Val Gln Ala Ser Lys Leu Cys Leu Gly Trp Leu Trp Asp Met Asp
               20                  25                  30
   Ile Asp Pro Tyr Lys Glu Phe Gly Ser Ser Tyr Gln Leu Leu Asn Phe
           35                  40                  45
   Leu Pro Leu Asp Phe Phe Pro Asp Leu Asn Ala Leu Val Asp Thr Ala
       50                  55                  60
   Ala Ala Leu Tyr Glu Glu Glu Leu Thr Gly Arg Glu His Cys Ser Pro
   65                  70                  75                  80
   His His Thr Ala Ile Arg Gln Ala Leu Val Cys Trp Glu Glu Leu Thr
                   85                  90                  95
   Arg Leu Ile Thr Trp Met Ser Glu Asn Thr Thr Glu Glu Val Arg Arg
               100                 105                 110
   Ile Ile Val Asp His Val Asn Asn Thr Trp Gly Leu Lys Val Arg Gln
           115                 120                 125
   Thr Leu Trp Phe His Leu Ser Cys Leu Thr Phe Gly Gly His Thr Val
       130                 135                 140
   Gln Glu Phe Leu Val Ser Phe Gly Val Trp Ile Arg Thr Pro Ala Pro
   145                 150                 155                 160
   Tyr Arg Pro Pro Asn Ala Pro Ile Leu Ser Thr Leu Pro Glu His Thr
                   165                 170                 175
   Val Ile Arg Arg Arg Gly Gly Ser Arg Ala Ala Arg Ser Pro Arg Arg
               180                 185                 190
   Arg Thr Pro Ser Pro Arg Arg Arg Arg Ser Gln Ser Pro Arg Arg Arg
           195                 200                 205
   Arg Ser Gln Ser Pro Ala Ser Asn Cys
       210                 215
<210> 7
   <211> 51
   <212> DNA
   <213> Artificial Sequence
<220>
   <223> modified plasmid pkk223
<400> 7
   ttcacacagg aaacagaatt cccggggatc cgtcgacctg cagccaagct t 51
<210> 8
   <211> 38
   <212> DNA
   <213> Artificial Sequence
<220>
   <223> plasmid pkk223
<400> 8
   ttcacataag gaggaaaaaa ccatgggatc cgaagctt 38
<210> 9
   <211> 19
   <212> PRT
   <213> Artficial sequence

<400> 9
   Ile Asn Ala Asn Pro Asn Ala Asn Pro Asn Ala Asn Pro Asn Ala Asn
   1               5                   10                  15
   Pro Glu Leu
<210> 10
   <211> 21
   <212> PRT
   <213> Artificial sequence
<220>
   <223> Chimera of malarial T cell epitope and hepatitis B
<400> 10
   Ile Glu Tyr Leu Asn Lys Ile Gln Asn Ser Leu Ser Thr Glu Trp Ser
   1               5                   10                  15
   Pro Cys Ser Val Thr
               20
<210> 11
   <211> 15
   <212> PRT
   <213> Streptococcus pneumoniae
<400> 11
   Lys Leu Glu Glu Leu Ser Asp Lys Ile Asp Glu Leu Asp Ala Glu
   1               5                   10                  15
<210> 12
   <211> 35
   <212> PRT
   <213> Streptococcus pneumoniae
<400> 12
   Gln Lys Lys Tyr Asp Glu Asp Gln Lys Lys Thr Glu Glu Lys Ala Ala
   1 5 10 15
   Leu Glu Lys Ala Ala Ser Glu Glu Met Asp Lys Ala Val Ala Ala Val
   20 25 30
   Gln Gln Ala
   35
<210> 13
   <211> 27
   <212> PRT
   <213> Cryptosporidium parvum
<400> 13
   Gln Asp Lys Pro Ala Asp Ala Pro Ala Ala Glu Ala Pro Ala Ala Glu
   1 5 10 15
   Pro Ala Ala Gln Gln Asp Lys Pro Ala Asp Ala
   20 25
<210> 14
   <211> 17
   <212> PRT
   <213> Human immunodeficiency virus
<400> 14
   Arg Lys Arg Ile His Ile Gly Pro Gly Arg Ala Phe Tyr Ile Thr Lys
   1 5 10 15
   Asn
<210> 15
   <211> 31
   <212> PRT
   <213> Foot-and-mouth disease virus
<400> 15
   Tyr Asn Gly Glu Cys Arg Tyr Asn Arg Asn Ala Val Pro Asn Leu Arg
   1 5 10 15
   Gly Asp Leu Gln Val Leu Ala Gln Lys Val Ala Arg Thr Leu Pro
   20 25 30
<210> 16
   <211> 10
   <212> PRT
   <213> Influenza A virus
<400> 16
   Tyr Arg Asn Leu Leu Trp Leu Thr Glu Lys
   1 5 10
<210> 17
   <211> 23
   <212> PRT
   <213> Influenza A virus
<400> 17
   Ser Leu Leu Thr Glu Val Glu Thr Pro Ile Arg Asn Glu Trp Gly Cys
   1 5 10 15
   Arg Cys Asn Gly Ser Ser Asp
   20
<210> 18
   <211> 23
   <212> PRT
   <213> Influenza A virus
<400> 18
   Ser Leu Leu Thr Glu Val Glu Thr Pro Ile Arg Asn Glu Trp Gly Cys
   1 5 10 15
   Arg Cys Asn Asp Ser Ser Asp
   20
<210> 19
   <211> 23
   <212> PRT
   <213> Influenza A virus
<400> 19
   Ser Leu Leu Thr Glu Val Glu Thr Pro Ile Arg Asn Glu Trp Gly Ala
   1 5 10 15
   Arg Ala Asn Asp Ser Ser Asp
   20
<210> 20
   <211> 19
   <212> PRT
   <213> Influenza A virus

<400> 20
   Glu Gln Gln Ser Ala Val Asp Ala Asp Asp Ser His Phe Val Ser Ile
   1 5 10 15
   Glu Leu Glu
<210> 21
   <211> 23
   <212> PRT
   <213> Influenza A virus
<400> 21
   Ser Leu Leu Thr Glu Val Glu Thr Pro Ile Arg Asn Glu Trp Gly Ser
   1 5 10 15
   Arg Ser Asn Asp Ser Ser Asp
   20
<210> 22
   <211> 23
   <212> PRT
   <213> Influenza A virus
<400> 22
   Ser Leu Leu Thr Glu Val Glu Thr Pro Ile Arg Asn Glu Trp Gly Ser
   1 5 10 15
   Arg Cys Asn Asp Ser Ser Asp
   20
<210> 23
   <211> 23
   <212> PRT
   <213> Influenza A virus
<400> 23
   Ser Leu Leu Thr Glu Val Glu Thr Pro Ile Arg Asn Glu Trp Gly Cys
   1               5                   10                  15
   Arg Ser Asn Asp Ser Ser Asp
               20
<210> 24
   <211> 23
   <212> PRT
   <213> Influenza A virus
<400> 24
   Ser Leu Leu Thr Glu Val Glu Thr Pro Ile Arg Asn Glu Trp Gly Cys
   1               5                   10                  15
   Arg Ala Asn Asp Ser Ser Asp
               20
<210> 25
   <211> 23
   <212> PRT
   <213> Influenza A virus
<400> 25
   Ser Leu Leu Thr Glu Val Glu Thr Pro Ile Arg Asn Glu Trp Gly Ala
   1               5                   10                  15
   Arg Cys Asn Asp Ser Ser Asp
               20
<210> 26
   <211> 24
   <212> PRT
   <213> Influenza A virus
<400> 26
   Met Ser Leu Leu Thr Glu Val Glu Thr Pro Ile Arg Asn Glu Trp Gly
   1               5                   10                  15
   Cys Arg Cys Asn Asp Ser Ser Asp
               20
<210> 27
   <211> 24
   <212> PRT
   <213> Influenza A virus
<400> 27
   Met Ser Leu Leu Thr Glu Val Glu Thr Pro Ile Arg Asn Glu Trp Gly
   1 5 10 15
   Ser Arg Ser Asn Asp Ser Ser Asp
   20
<210> 28
   <211> 35
   <212> PRT
   <213> Influenza A virus
<400> 28
   Met Gly Ile Ser Leu Leu Thr Glu Val Glu Thr Pro Ile Arg Asn Glu
   1 5 10 15
   Trp Gly Cys Arg Cys Asn Asp Ser Ser Asp Glu Leu Leu Gly Trp Leu
   20 25 30
   Trp Gly Ile
   35
<210> 29
   <211> 24
   <212> PRT
   <213> Influenza A virus
<400> 29
   Met Ser Leu Leu Thr Glu Val Glu Thr Pro Ile Arg Asn Glu Trp Gly
   1 5 10 15
   Ala Arg Ala Asn Asp Ser Ser Asp
   20
<210> 30
   <211> 24
   <212> PRT
   <213> Influenza A virus
<400> 30
   Met Ser Leu Leu Thr Glu Val Glu Thr Pro Ile Arg Asn Glu Trp Gly
   1 5 10 15
   Cys Arg Ala Asn Asp Ser Ser Asp
   20
<210> 31
   <211> 24
   <212> PRT
   <213> Influenza A virus

<400> 31
   Met Ser Leu Leu Thr Glu Val Glu Thr Pro Ile Arg Asn Glu Trp Gly
   1               5                   10                  15
   Ala Arg Cys Asn Asp Ser Ser Asp
               20
<210> 32
   <211> 24
   <212> PRT
   <213> Influenza A virus
<400> 32
   Met Ser Leu Leu Thr Glu Val Glu Thr Pro Ile Arg Asn Glu Trp Gly
   1               5                   10                  15
   Cys Arg Ser Asn Asp Ser Ser Asp
               20
<210> 33
   <211> 24
   <212> PRT
   <213> Influenza A virus
<400> 33
   Met Ser Leu Leu Thr Glu Val Glu Thr Pro Ile Arg Asn Glu Trp Gly
   1               5                   10                  15
   Ser Arg Cys Asn Asp Ser Ser Asp
               20
<210> 34
   <211> 46
   <212> PRT
   <213> Influenza A virus
<400> 34
   Ser Leu Leu Thr Glu Val Glu Thr Pro Ile Arg Asn Glu Trp Gly Ser
   1 5 10 15
   Arg Ser Asn Asp Ser Ser Asp Ser Leu Leu Thr Glu Val Glu Thr Pro
   20 25 30
   Ile Arg Asn Glu Trp Gly Ser Arg Ser Asn Asp Ser Ser Asp
   35 40 45
<210> 35
   <211> 69
   <212> PRT
   <213> Influenza A virus
<400> 35
   Ser Leu Leu Thr Glu Val Glu Thr Pro Ile Arg Asn Glu Trp Gly Ser
   1 5 10 15
   Arg Ser Asn Asp Ser Ser Asp Ser Leu Leu Thr Glu Val Glu Thr Pro
   20 25 30
   Ile Arg Asn Glu Trp Gly Ser Arg Ser Asn Asp Ser Ser Asp Ser Leu
   35 40 45
   Leu Thr Glu Val Glu Thr Pro Ile Arg Asn Glu Trp Gly Ser Arg Ser
   50 55 60
   Asn Asp Ser Ser Asp
   65
<210> 36
   <211> 46
   <212> PRT
   <213> Influenza A virus
<400> 36
   Ser Leu Leu Thr Glu Val Glu Thr Pro Ile Arg Asn Glu Trp Gly Ala
   1 5 10 15
   Arg Ala Asn Asp Ser Ser Asp Ser Leu Leu Thr Glu Val Glu Thr Pro
   20 25 30
   Ile Arg Asn Glu Trp Gly Ala Arg Ala Asn Asp Ser Ser Asp
   35 40 45
<210> 37
   <211> 69
   <212> PRT
   <213> Influenza A virus
<400> 37
   Ser Leu Leu Thr Glu Val Glu Thr Pro Ile Arg Asn Glu Trp Gly Ala
   1               5                   10                  15
   Arg Ala Asn Asp Ser Ser Asp Ser Leu Leu Thr Glu Val Glu Thr Pro
               20                  25                  30
   Ile Arg Asn Glu Trp Gly Ala Arg Ala Asn Asp Ser Ser Asp Ser Leu
           35                  40                  45
   Leu Thr Glu Val Glu Thr Pro Ile Arg Asn Glu Trp Gly Ala Arg Ala
       50                  55                  60
   Asn Asp Ser Ser Asp
    65
<210> 38
   <211> 19
   <212> PRT
   <213> Influenza A virus
<400> 38
   Glu Val Glu Thr Pro Ile Arg Asn Glu Trp Gly Ser Arg Cys Asn Asp
   1               5                   10                  15
   Ser Ser Asp
<210> 39
   <211> 38
   <212> PRT
   <213> Influenza A virus
<400> 39
   Glu Val Glu Thr Pro Ile Arg Asn Glu Trp Gly Ser Arg Cys Asn Asp
   1               5                   10                  15
   Ser Ser Asp Glu Val Glu Thr Pro Ile Arg Asn Glu Trp Gly Ser Arg
               20                  25                  30
   Cys Asn Asp Ser Ser Asp
           35
<210> 40
   <211> 57
   <212> PRT
   <213> Influenza A virus
<400> 40
   Glu Val Glu Thr Pro Ile Arg Asn Glu Trp Gly Ser Arg Cys Asn Asp
   1 5 10 15
   Ser Ser Asp Glu Val Glu Thr Pro Ile Arg Asn Glu Trp Gly Ser Arg
   20 25 30
   Cys Asn Asp Ser Ser Asp Glu Val Glu Thr Pro Ile Arg Asn Glu Trp
   35 40 45
   Gly Ser Arg Cys Asn Asp Ser Ser Asp
   50 55
<210> 41
   <211> 23
   <212> PRT
   <213> Influenza A virus
<220>
   <221> misc_feature
   <222> (1)..(8)
   <223> Xaa can be any naturally occurring amino acid
<220>
   <221> misc_feature
   <222> (15)..(23)
   <223> Xaa can be any naturally occurring amino acid

<400> 41
   Xaa Xaa Xaa Xaa Xaa Xaa Xaa Xaa Thr Pro Ile Arg Asn Glu Xaa Xaa
   1 5 10 15
   Xaa Xaa Xaa Xaa Xaa Xaa Xaa
   20
<210> 42
   <211> 47
   <212> PRT
   <213> Influenza A virus
<220>
   <221> misc_feature
   <222> (1)..(8)
   <223> Xaa can be any naturally occurring amino acid
<220>
   <221> misc_feature
   <222> (15)..(31)
   <223> Xaa can be any naturally occurring amino acid
<220>
   <221> misc_feature
   <222> (38)..(47)
   <223> Xaa can be any naturally occurring amino acid
<400> 42
   Xaa Xaa Xaa Xaa Xaa Xaa Xaa Xaa Thr Pro Ile Arg Asn Glu Xaa Xaa
   1               5                   10                  15
   Xaa Xaa Xaa Xaa Xaa Xaa Xaa Xaa Xaa Xaa Xaa Xaa Xaa Xaa Xaa Thr
               20                  25                  30
   Pro Ile Arg Asn Glu Xaa Xaa Xaa Xaa Xaa Xaa Xaa Xaa Xaa Xaa
           35                  40                  45
<210> 43
   <211> 70
   <212> PRT
   <213> Influenza A virus
<220>
   <221> misc_feature
   <222> (1)..(8)
   <223> Xaa can be any naturally occurring amino acid
<220>
   <221> misc_feature
   <222> (15).. (31)
   <223> Xaa can be any naturally occurring amino acid
<220>
   <221> misc_feature
   <222> (38)..(54)
   <223> Xaa can be any naturally occurring amino acid
<220>
   <221> misc_feature
   <222> (61)..(70)
   <223> Xaa can be any naturally occurring amino acid
<400> 43
   Xaa Xaa Xaa Xaa Xaa Xaa Xaa Xaa Thr Pro Ile Arg Asn Glu Xaa Xaa
   1               5                   10                  15
   Xaa Xaa Xaa Xaa Xaa Xaa Xaa Xaa Xaa Xaa Xaa Xaa Xaa Xaa Xaa Thr
               20                  25                  30
   Pro Ile Arg Asn Glu Xaa Xaa Xaa Xaa Xaa Xaa Xaa Xaa Xaa Xaa Xaa
           35                  40                  45
   Xaa Xaa Xaa Xaa Xaa Xaa Thr Pro Ile Arg Asn Glu Xaa Xaa Xaa Xaa
       50                  55                  60
   Xaa Xaa Xaa Xaa Xaa Xaa
   65                  70
<210> 44
   <211> 24
   <212> PRT
   <213> Influenza A virus
<220>
   <221> misc_feature
   <222> (1)..(8)
   <223> Xaa can be any naturally occurring amino acid
<220>
   <221> misc_feature
   <222> (10)..(11)
   <223> Xaa can be any naturally occurring amino acid
<220>
   <221> misc_feature
   <222> (13)..(24)
   <223> Xaa can be any naturally occurring amino acid
<400> 44
   Xaa Xaa Xaa Xaa Xaa Xaa Xaa Xaa Thr Xaa Xaa Arg Xaa Xaa Xaa Xaa
   1               5                   10                  15
   Xaa Xaa Xaa Xaa Xaa Xaa Xaa Xaa
               20
<210> 45
   <211> 47
   <212> PRT
   <213> Influenza A virus
<220>
   <221> misc_feature
   <222> (1)..(8)
   <223> Xaa can be any naturally occurring amino acid
<220>
   <221> misc_feature
   <222> (10)..(11)
   <223> Xaa can be any naturally occurring amino acid
<220>
   <221> misc_feature
   <222> (13)..(31)
   <223> Xaa can be any naturally occurring amino acid

<220>
   <221> misc_feature
   <222> (33)..(34)
   <223> Xaa can be any naturally occurring amino acid
<220>
   <221> misc_feature
   <222> (36)..(47)
   <223> Xaa can be any naturally occurring amino acid
<400> 45
   Xaa Xaa Xaa Xaa Xaa Xaa Xaa Xaa Thr Xaa Xaa Arg Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Thr
               20                  25                  30
   Xaa Xaa Arg Xaa Xaa Xaa Xaa Xaa Xaa Xaa Xaa Xaa Xaa Xaa Xaa
           35                  40                  45
<210> 46
   <211> 70
   <212> PRT
   <213> Influenza A virus
<220>
   <221> misc_feature
   <222> (1)..(8)
   <223> Xaa can be any naturally occurring amino acid
<220>
   <221> misc_feature
   <222> (10)..(11)
   <223> Xaa can be any naturally occurring amino acid
<220>
   <221> misc_feature
   <222> (13)..(31)
   <223> Xaa can be any naturally occurring amino acid
<220>
   <221> misc_feature
   <222> (33)..(34)
   <223> Xaa can be any naturally occurring amino acid
<220>
   <221> misc_feature
   <222> (36)..(54)
   <223> Xaa can be any naturally occurring amino acid
<220>
   <221> misc_feature
   <222> (56)..(57)
   <223> Xaa can be any naturally occurring amino acid
<220>
   <221> misc_feature
   <222> (59)..(70)
   <223> Xaa can be any naturally occurring amino acid
<400> 46
   Xaa Xaa Xaa Xaa Xaa Xaa Xaa Xaa Thr Xaa Xaa Arg Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Thr
               20                  25                  30
   Xaa Xaa Arg Xaa Xaa Xaa Xaa Xaa Xaa Xaa Xaa Xaa Xaa Xaa Xaa Xaa
           35                  40                  45
   Xaa Xaa Xaa Xaa Xaa Xaa Thr Xaa Xaa Arg Xaa Xaa Xaa Xaa Xaa Xaa
       50                  55                  60
   Xaa Xaa Xaa Xaa Xaa Xaa
   65                  70
<210> 47
   <211> 17
   <212> PRT
   <213> Influenza B virus
<400> 47
   Asn Asn Ala Thr Phe Asn Tyr Thr Asn Val Asn Pro Ile Ser His Ile
   1               5                   10                  15
   Arg
<210> 48
   <211> 142
   <212> PRT
   <213> Yersinia pestis
<400> 48
   Asp Ile Leu Lys Val Ile Val Asp Ser Met Asn His His Gly Asp Ala
   1               5                   10                  15
   Arg Ser Lys Leu Arg Glu Glu Leu Ala Glu Leu Thr Ala Glu Leu Lys
               20                  25                  30
   Ile Tyr Ser Val Ile Gln Ala Glu Ile Asn Lys His Leu Ser Ser Ser
           35                  40                  45
   Gly Thr Ile Asn Ile His Asp Lys Ser Ile Asn Leu Met Asp Lys Asn
       50                  55                  60
   Leu Tyr Gly Tyr Thr Asp Glu Glu Ile Phe Lys Ala Ser Ala Glu Tyr
   65                  70                  75                  80
   Lys Ile Leu Glu Lys Met Pro Gln Thr Thr Ile Gln Val Asp Gly Ser
                   85                  90                  95
   Glu Lys Lys Ile Val Ser Ile Lys Asp Phe Leu Gly Ser Glu Asn Lys
               100                 105                 110
   Arg Thr Gly Ala Leu Gly Asn Leu Lys Asn Ser Tyr Ser Tyr Asn Lys
           115                 120                 125
   Asp Asn Asn Glu Leu Ser His Phe Ala Thr Thr Cys Ser Asp
       130                 135                 140
<210> 49
   <211> 19
   <212> PRT
   <213> Haemophilus influenzae
<400> 49
   Cys Ser Ser Ser Asn Asn Asp Ala Ala Gly Asn Gly Ala Ala Gln Phe
   1               5                   10                  15
   Gly Gly Tyr
<210> 50
   <211> 11
   <212> PRT
   <213> Haemophilus influenzae
<400> 50
   Asn Lys Leu Gly Thr Val Ser Tyr Gly Glu Glu
   1               5                   10
<210> 51
   <211> 16
   <212> PRT
   <213> Haemophilus influenzae

<400> 51
   Asn Asp Glu Ala Ala Tyr Ser Lys Asn Arg Arg Ala Val Leu Ala Tyr
   1 5 10 15
<210> 52
   <211> 28
   <212> PRT
   <213> Moraxella catarrhalis
<400> 52
   Leu Asp Ile Glu Lys Asp Lys Lys Lys Arg Thr Asp Glu Gln Leu Gln
   1 5 10 15
   Ala Glu Leu Asp Asp Lys Tyr Ala Gly Lys Gly Tyr
   20 25
<210> 53
   <211> 28
   <212> PRT
   <213> Moraxella catarrhalis
<400> 53
   Leu Asp Ile Glu Lys Asn Lys Lys Lys Arg Thr Glu Ala Glu Leu Gln
   1 5 10 15
   Ala Glu Leu Asp Asp Lys Tyr Ala Gly Lys Gly Tyr
   20 25
<210> 54
   <211> 28
   <212> PRT
   <213> Moraxella catarrhalis
<400> 54
   Ile Asp Ile Glu Lys Lys Gly Lys Ile Arg Thr Glu Ala Glu Leu Leu
   1 5 10 15
   Ala Glu Leu Asn Lys Asp Tyr Pro Gly Gln Gly Tyr
   20 25
<210> 55
   <211> 25
   <212> PRT
   <213> Porphyromonas gingivalis
<400> 55
   Gly Val Ser Pro Lys Val Cys Lys Asp Val Thr Val Glu Gly Ser Asn
   1               5                   10                  15
   Glu Phe Ala Pro Val Gln Asn Leu Thr
               20                  25
<210> 56
   <211> 20
   <212> PRT
   <213> Porphyromonas gingivalis
<400> 56
   Arg Ile Gln Ser Thr Trp Arg Gln Lys Thr Val Asp Leu Pro Ala Gly
   1               5                   10                  15
   Thr Lys Tyr Val
               20
<210> 57
   <211> 21
   <212> PRT
   <213> Trypanosoma cruzi
<400> 57
   Lys Ala Ala Ile Ala Pro Ala Lys Ala Ala Ala Ala Pro Ala Lys Ala
   1               5                   10                  15
   Ala Thr Ala Pro Ala
               20
<210> 58
   <211> 24
   <212> PRT
   <213> Plasmodium falciparum
<400> 58
   Asn Ala Asn Pro Asn Val Asp Pro Asn Ala Asn Pro Asn Ala Asn Pro
   1               5                   10                  15
   Asn Ala Asn Pro Asn Val Asp Pro
               20
<210> 59
   <211> 20
   <212> PRT
   <213> Plasmodium falciparum
<400> 59
   Asn Ala Asn Pro Asn Val Asp Pro Asn Ala Asn Pro Asn Ala Asn Pro
   1               5                   10                  15
   Asn Ala Asn Pro
               20
<210> 60
   <211> 20
   <212> PRT
   <213> Plasmodium falciparum
<400> 60
   Asn Ala Asn Pro Asn Ala Asn Pro Asn Ala Asn Pro Asn Val Asp Pro
   1               5                   10                  15
   Asn Ala Asn Pro
               20
<210> 61
   <211> 28
   <212> PRT
   <213> Plasmodium falciparum
<400> 61
   Asn Ala Asn Pro Asn Val Asp Pro Asn Ala Asn Pro Asn Ala Asn Pro
   1               5                   10                  15
   Asn Ala Asn Pro Asn Val Asp Pro Asn Ala Asn Pro
               20                  25
<210> 62
   <211> 20
   <212> PRT
   <213> Plasmodium falciparum
<400> 62
   Asn Pro Asn Val Asp Pro Asn Ala Asn Pro Asn Ala Asn Pro Asn Ala
   1               5                   10                  15
   Asn Pro Asn Val
               20
<210> 63
   <211> 22
   <212> PRT
   <213> Plasmodium falciparum
<400> 63
   Asn Pro Asn Val Asp Pro Asn Ala Asn Pro Asn Ala Asn Pro Asn Ala
   1               5                   10                  15
   Asn Pro Asn Val Asp Pro
               20
<210> 64
   <211> 24
   <212> PRT
   <213> Plasmodium falciparum

<400> 64
   Asn Pro Asn Val Asp Pro Asn Ala Asn Pro Asn Ala Asn Pro Asn Ala
   1               5                   10                  15
   Asn Pro Asn Val Asp Pro Asn Ala
               20
<210> 65
   <211> 18
   <212> PRT
   <213> Plasmodium falciparum
<400> 65
   Asn Val Asp Pro Asn Ala Asn Pro Asn Ala Asn Pro Asn Ala Asn Pro
   1               5                   10                  15
   Asn Val
<210> 66
   <211> 20
   <212> PRT
   <213> Plasmodium falciparum
<400> 66
   Asn Val Asp Pro Asn Ala Asn Pro Asn Ala Asn Pro Asn Ala Asn Pro
   1 5 10 15
   Asn Val Asp Pro
   20
<210> 67
   <211> 22
   <212> PRT
   <213> Plasmodium falciparum
<400> 67
   Asn Val Asp Pro Asn Ala Asn Pro Asn Ala Asn Pro Asn Ala Asn Pro
   1 5 10 15
   Asn Val Asp Pro Asn Ala
   20
<210> 68
   <211> 16
   <212> PRT
   <213> Plasmodium falciparum
<400> 68
   Asp Pro Asn Ala Asn Pro Asn Ala Asn Pro Asn Ala Asn Pro Asn Val
   1 5 10 15
<210> 69
   <211> 18
   <212> PRT
   <213> Plasmodium falciparum
<400> 69
   Asp Pro Asn Ala Asn Pro Asn Ala Asn Pro Asn Ala Asn Pro Asn Val
   1 5 10 15
   Asp Pro
<210> 70
   <211> 20
   <212> PRT
   <213> Plasmodium falciparum
<400> 70
   Asp Pro Asn Ala Asn Pro Asn Ala Asn Pro Asn Ala Asn Pro Asn Val
   1 5 10 15
   Asp Pro Asn Ala
   20
<210> 71
   <211> 19
   <212> PRT
   <213> Plasmodium vivax
<400> 71
   Gly Asp Arg Ala Asp Gly Gln Pro Ala Gly Asp Arg Ala Asp Gly Gln
   1 5 10 15
   Pro Ala Gly
<210> 72
   <211> 18
   <212> PRT
   <213> Plasmodium vivax
<400> 72
   Arg Ala Asp Asp Arg Ala Ala Gly Gln Pro Ala Gly Asp Gly Gln Pro
   1 5 10 15
   Ala Gly
<210> 73
   <211> 18
   <212> PRT
   <213> Plasmodium vivax
<400> 73
   Ala Asn Gly Ala Gly Asn Gln Pro Gly Ala Asn Gly Ala Gly Asp Gln
   1 5 10 15
   Pro Gly
<210> 74
   <211> 18
   <212> PRT
   <213> Plasmodium vivax
<400> 74
   Ala Asn Gly Ala Asp Asn Gln Pro Gly Ala Asn Gly Ala Asp Asp Gln
   1 5 10 15
   Pro Gly
<210> 75
   <211> 18
   <212> PRT
   <213> Plasmodium vivax
<400> 75
   Ala Asn Gly Ala Gly Asn Gln Pro Gly Ala Asn Gly Ala Asp Asn Gln
   1 5 10 15
   Pro Gly
<210> 76
   <211> 18
   <212> PRT
   <213> Plasmodium vivax

<400> 76
   Ala Asn Gly Ala Gly Asn Gln Pro Gly Ala Asn Gly Ala Asp Asp Gln
   1 5 10 15
   Pro Gly
<210> 77
   <211> 22
   <212> PRT
   <213> Plasmodium vivax
<400> 77
   Ala Pro Gly Ala Asn Gln Glu Gly Gly Ala Ala Ala Pro Gly Ala Asn
   1 5 10 15
   Gln Glu Gly Gly Ala Ala
   20
<210> 78
   <211> 36
   <212> PRT
   <213> Plasmodium vivax
<400> 78
   Ala Asn Gly Ala Gly Asn Gln Pro Gly Ala Asn Gly Ala Gly Asp Gln
   1 5 10 15
   Pro Gly Ala Asn Gly Ala Asp Asn Gln Pro Gly Ala Asn Gly Ala Asp
   20 25 30
   Asp Gln Pro Gly
   35
<210> 79
   <211> 16
   <212> PRT
   <213> Plasmodium berghei
<400> 79
   Asp Pro Pro Pro Pro Asn Pro Asn Asp Pro Pro Pro Pro Asn Pro Asn
   1 5 10 15
<210> 80
   <211> 24
   <212> PRT
   <213> Plasmodium yoelii
<400> 80
   Gln Gly Pro Gly Ala Pro Gln Gly Pro Gly Ala Pro Gln Gly Pro Gly
   1 5 10 15
   Ala Pro Gln Gly Pro Gly Ala Pro
   20
<210> 81
   <211> 15
   <212> PRT
   <213> Streptococcus sobrinus
<400> 81
   Lys Pro Arg Pro Ile Tyr Glu Ala Lys Leu Ala Gln Asn Gln Lys
   1 5 10 15
<210> 82
   <211> 16
   <212> PRT
   <213> Streptococcus sobrinus
<400> 82
   Ala Lys Ala Asp Tyr Glu Ala Lys Leu Ala Gln Tyr Glu Lys Asp Leu
   1 5 10 15
<210> 83
   <211> 9
   <212> PRT
   <213> Shigella flexneri
<400> 83
   Lys Asp Arg Thr Leu Ile Glu Gln Lys
   1 5
<210> 84
   <211> 15
   <212> PRT
   <213> respiratory syncytial virus
<400> 84
   Cys Ser Ile Cys Ser Asn Asn Pro Thr Cys Trp Ala Ile Cys Lys
   1 5 10 15
<210> 85
   <211> 25
   <212> PRT
   <213> Entamoeba histolytica
<400> 85
   Val Glu Cys Ala Ser Thr Val Cys Gln Asn Asp Asn Ser Cys Pro Ile
   1 5 10 15
   Ile Ala Asp Val Glu Lys Cys Asn Gln
   20 25
<210> 86
   <211> 34
   <212> PRT
   <213> Schistosoma japonicum
<400> 86
   Asp Leu Gln Ser Glu Ile Ser Leu Ser Leu Glu Asn Gly Glu Leu Ile
   1 5 10 15
   Arg Arg Ala Lys Ser Ala Glu Ser Leu Ala Ser Glu Leu Gln Arg Arg
   20 25 30
   Val Asp
<210> 87
   <211> 34
   <212> PRT
   <213> Schistosoma mansoni
<400> 87
   Asp Leu Gln Ser Glu Ile Ser Leu Ser Leu Glu Asn Ser Glu Leu Ile
   1 5 10 15
   Arg Arg Ala Lys Ala Ala Glu Ser Leu Ala Ser Asp Leu Gln Arg Arg
   20 25 30
   Val Asp
<210> 88
   <211> 26
   <212> PRT
   <213> Bovine Inhibin
<400> 88
   Ser Thr Pro Pro Leu Pro Trp Pro Trp Ser Pro Ala Ala Leu Arg Leu
   1 5 10 15
   Leu Gln Arg Pro Pro Glu Glu Pro Ala Ala
   20 25
<210> 89
   <211> 17
   <212> PRT
   <213> Ebola virus
<400> 89
   Ala Thr Gln Val Glu Gln His His Arg Arg Thr Asp Asn Asp Ser Thr
   1 5 10 15
   Ala
<210> 90
   <211> 17
   <212> PRT
   <213> Ebola virus

<400> 90
   His Asn Thr Pro Val Tyr Lys Leu Asp Ile Ser Glu Ala Thr Gln Val
   1 5 10 15
   Glu
<210> 91
   <211> 17
   <212> PRT
   <213> Ebola virus
<400> 91
   Gly Lys Leu Gly Leu Ile Thr Asn Thr Ile Ala Gly Val Ala Val Leu
   1 5 10 15
   Ile
<210> 92
   <211> 14
   <212> PRT
   <213> Escherichia coli
<400> 92
   Cys Cys Glu Leu Cys Cys Tyr Pro Ala Cys Ala Gly Cys Asn
   1 5 10
<210> 93
   <211> 18
   <212> PRT
   <213> Escherichia coli
<400> 93
   Asn Thr Phe Tyr Cys Cys Glu Leu Cys Cys Tyr Pro Ala Cys Ala Gly
   1 5 10 15
   Cys Asn
<210> 94
   <211> 18
   <212> PRT
   <213> Escherichia coli
<400> 94
   Ser Ser Asn Tyr Cys Cys Glu Leu Cys Cys Tyr Pro Ala Cys Ala Gly
   1 5 10 15
   Cys Asn
<210> 95
   <211> 42
   <212> PRT
   <213> Alzheimer's disease b-Amyloid
<400> 95
<210> 96
   <211> 17
   <212> PRT
   <213> Alzheimer's disease b-Amyloid
<400> 96
   Asp Ala Glu Phe Arg His Asp Ser Gly Tyr Glu Val His His Gln Lys
   1 5 10 15
   Leu
<210> 97
   <211> 11
   <212> PRT
   <213> Alzheimer's disease b-Amyloid
<400> 97
   Glu Asp Val Gly Ser Asn Lys Gly Ala Ile Ile
   1 5 10
<210> 98
   <211> 33
   <212> PRT
   <213> Alzheimer's disease b-Amyloid
<400> 98
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly
<210> 99
   <211> 32
   <212> PRT
   <213> alzheimer's disease b-amplyoid
<400> 99
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
<210> 100
   <211> 13
   <212> PRT
   <213> Neisseria meningitidis
<400> 100
   Tyr Val Ala Val Glu Asn Gly Val Ala Lys Lys Val Ala
   1 5 10
<210> 101
   <211> 15
   <212> PRT
   <213> Neisseria meningitidis
<400> 101
   His Phe Val Gln Gln Thr Pro Lys Ser Gln Pro Thr Leu Val Pro
   1 5 10 15
<210> 102
   <211> 13
   <212> PRT
   <213> Neisseria meningitidis
<400> 102
   His Val Val Val Asn Asn Lys Val Ala Thr His Val Pro
   1 5 10
<210> 103
   <211> 12
   <212> PRT
   <213> Neisseria meningitidis

<400> 103
   Pro Leu Gln Asn Ile Gln Pro Gln Val Thr Lys Arg
   1 5 10
<210> 104
   <211> 21
   <212> PRT
   <213> Neisseria meningitidis
<400> 104
   Ala Gln Ala Ala Asn Gly Gly Ala Ala Ser Gly Gln Val Lys Val Thr
   1 5 10 15
   Lys Val Thr Lys Ala
   20
<210> 105
   <211> 10
   <212> PRT
   <213> Neisseria meningitidis
<400> 105
   Tyr Val Asp Glu Gln Ser Lys Tyr His Ala
   1 5 10
<210> 106
   <211> 15
   <212> PRT
   <213> Neisseria meningitidis
<400> 106
   His Phe Val Gln Asn Lys Gln Asn Gln Pro Pro Thr Leu Val Pro
   1 5 10 15
<210> 107
   <211> 18
   <212> PRT
   <213> Neisseria meningitidis
<400> 107
   Lys Pro Ser Ser Thr Asn Ala Lys Thr Gly Asn Lys Val Glu Val Thr
   1 5 10 15
   Lys Ala
<210> 108
   <211> 17
   <212> PRT
   <213> Neisseria meningitidis
<400> 108
   Tyr Trp Thr Thr Val Asn Thr Gly Ser Ala Thr Thr Thr Thr Phe Val
   1 5 10 15
   Pro
<210> 109
   <211> 11
   <212> PRT
   <213> Neisseria meningitidis
<400> 109
   Tyr Val Asp Glu Lys Lys Lys Met Val His Ala
   1 5 10
<210> 110
   <211> 13
   <212> PRT
   <213> Neisseria meningitidis
<400> 110
   His Tyr Thr Arg Gln Asn Asn Ala Asp Val Phe Val Pro
   1 5 10
<210> 111
   <211> 14
   <212> PRT
   <213> Neisseria meningitidis
<400> 111
   Tyr Tyr Thr Lys Asp Thr Asn Asn Asn Leu Thr Leu Val Pro
   1 5 10
<210> 112
   <211> 14
   <212> PRT
   <213> Neisseria meningitidis
<400> 112
   Pro Pro Gln Lys Asn Gln Ser Gln Pro Val Val Thr Lys Ala
   1 5 10
<210> 113
   <211> 14
   <212> PRT
   <213> Neisseria meningitidis
<400> 113
   Pro Pro Ser Lys Gly Gln Thr Gly Asn Lys Val Thr Lys Gly
   1 5 10
<210> 114
   <211> 14
   <212> PRT
   <213> Neisseria meningitidis
<400> 114
   Pro Pro Ser Lys Ser Gln Pro Gln Val Lys Val Thr Lys Ala
   1 5 10
<210> 115
   <211> 18
   <212> PRT
   <213> Neisseria meningitidis
<400> 115
   Gln Pro Gln Thr Ala Asn Thr Gln Gln Gly Gly Lys Val Lys Val Thr
   1 5 10 15
   Lys Ala
<210> 116
   <211> 18
   <212> PRT
   <213> Neisseria meningitidis
<400> 116
   Gln Pro Gln Val Thr Asn Gly Val Gln Gly Asn Gln Val Lys Val Thr
   1 5 10 15
   Lys Ala
<210> 117
   <211> 18
   <212> PRT
   <213> Neisseria meningitidis
<400> 117
   Gln Pro Ser Lys Ala Gln Gly Gln Thr Asn Asn Gln Val Lys Val Thr
   1 5 10 15
   Lys Ala
<210> 118
   <211> 20
   <212> PRT
   <213> Neisseria meningitidis
<400> 118
   Pro Pro Ser Ser Asn Gln Gly Lys Asn Gln Ala Gln Thr Gly Asn Thr
   1 5 10 15
   Val Thr Lys Ala
   20
<210> 119
   <211> 18
   <212> PRT
   <213> Neisseria meningitidis
<400> 119
   Pro Pro Ser Lys Ser Gln Gly Lys Thr Gly Asn Gln Val Lys Val Thr
   1 5 10 15
   Lys Ala
<210> 120
   <211> 18
   <212> PRT
   <213> Neisseria meningitidis

<400> 120
   Pro Pro Ser Lys Ser Gln Gly Thr Asn Asn Asn Gln Val Lys Val Thr
   1               5                   10                  15
   Lys Ala
<210> 121
   <211> 18
   <212> PRT
   <213> Neisseria meningitidis
<400> 121
   Pro Pro Ser Lys Ser Gln Pro Gly Gln Val Lys Val Thr Lys Val Thr
   1               5                   10                  15
   Lys Ala
<210> 122
   <211> 24
   <212> PRT
   <213> Neisseria meningitidis
<400> 122
   Gln Leu Gln Leu Thr Glu Gln Pro Ser Ser Thr Asn Gly Gln Thr Gly
   1               5                   10                  15
   Asn Gln Val Lys Val Thr Lys Ala
               20
<210> 123
   <211> 24
   <212> PRT
   <213> Neisseria meningitidis
<400> 123
   Gln Leu Gln Leu Thr Glu Ala Pro Ser Lys Ser Gln Gly Ala Ala Ser
   1 5 10 15
   Asn Gln Val Lys Val Thr Lys Ala
   20
<210> 124
   <211> 19
   <212> PRT
   <213> Neisseria meningitidis
<400> 124
   Ser Ala Tyr Thr Pro Ala His Val Tyr Val Asp Asn Lys Val Ala Lys
   1 5 10 15
   His Val Ala
<210> 125
   <211> 21
   <212> PRT
   <213> Neisseria meningitidis
<400> 125
   Ser Ala Tyr Thr Pro Ala His Phe Val Gln Asn Lys Gln Asn Asn Asn
   1 5 10 15
   Pro Thr Leu Val Pro
   20
<210> 126
   <211> 12
   <212> PRT
   <213> Neisseria meningitidis
<400> 126
   Val Glu Gly Arg Asn Tyr Gln Leu Gln Leu Thr Glu
   1 5 10
<210> 127
   <211> 12
   <212> PRT
   <213> Neisseria meningitidis
<400> 127
   Pro Ala Gln Asn Ser Lys Ser Ala Tyr Thr Pro Ala
   1 5 10
<210> 128
   <211> 22
   <212> PRT
   <213> Neisseria meningitidis
<400> 128
   Gln Leu Gln Leu Thr Glu Pro Pro Ser Lys Asn Gln Ala Gln Thr Gln
   1 5 10 15
   Asn Lys Val Thr Lys Ala
   20
<210> 129
   <211> 16
   <212> PRT
   <213> Neisseria meningitidis
<400> 129
   Gly Arg Asp Ala Phe Glu Leu Phe Leu Leu Gly Ser Gly Ser Asp Glu
   1 5 10 15
<210> 130
   <211> 31
   <212> PRT
   <213> Neisseria meningitidis
<400> 130
   Arg His Ala Asn Val Gly Arg Asp Ala Phe Glu Leu Phe Leu Leu Gly
   1 5 10 15
   Ser Gly Ser Asp Glu Ala Lys Gly Thr Asp Pro Leu Lys Asn His
   20 25 30
<210> 131
   <211> 18
   <212> PRT
   <213> Neisseria meningitidis
<400> 131
   Gly Arg Asp Ala Phe Asn Leu Phe Leu Leu Gly Arg Ile Gly Asp Asp
   1 5 10 15
   Asp Glu
<210> 132
   <211> 17
   <212> PRT
   <213> Neisseria meningitidis
<400> 132
   Gly Arg Asn Ala Phe Glu Leu Phe Leu Ile Gly Ser Ala Thr Ser Asp
   1 5 10 15
   Gln
<210> 133
   <211> 15
   <212> PRT
   <213> Neisseria meningitidis
<400> 133
   Gln Val Lys Val Thr Lys Ala Lys Ser Arg Ile Arg Thr Lys Ile
   1 5 10 15
<210> 134
   <211> 13
   <212> PRT
   <213> Neisseria meningitidis
<400> 134
   Thr Leu Val Pro Ala Val Val Gly Lys Pro Gly Ser Asp
   1 5 10
<210> 135
   <211> 17
   <212> PRT
   <213> Neisseria meningitidis

<400> 135
   His Ala Lys Ala Ser Ser Ser Leu Gly Ser Ala Lys Gly Phe Ser Pro
   1 5 10 15
   Arg
<210> 136
   <211> 15
   <212> PRT
   <213> Neisseria meningitidis
<400> 136
   Thr Arg Tyr Lys Asn Tyr Lys Ala Pro Ser Thr Asp Phe Lys Leu
   1               5                   10                  15
<210> 137
   <211> 18
   <212> PRT
   <213> Neisseria meningitidis
<400> 137
   Ser Leu Asn Arg Ala Ser Val Asp Leu Gly Gly Ser Asp Ser Phe Ser
   1               5                   10                  15
   Gln Thr
<210> 138
   <211> 21
   <212> PRT
   <213> Neisseria meningitidis
<400> 138
   Gly Lys Val Asn Thr Val Lys Asn Val Arg Ser Gly Glu Leu Ser Ala
   1               5                   10                  15
   Gly Val Arg Val Lys
               20
<210> 139
   <211> 21
   <212> PRT
   <213> Neisseria meningitidis
<400> 139
   Gly Lys Val Asn Thr Val Lys Asn Val Arg Ser Gly Glu Leu Ser Val
   1               5                   10                  15
   Gly Val Arg Val Lys
               20
<210> 140
   <211> 13
   <212> PRT
   <213> Homo sapiens
<400> 140
   Ala Pro Glu Trp Pro Gly Ser Arg Asp Lys Arg Thr Leu
   1 5 10
<210> 141
   <211> 9
   <212> PRT
   <213> Homo sapiens
<400> 141
   Glu Asp Gly Gln Val Met Asp Val Asp
   1 5
<210> 142
   <211> 8
   <212> PRT
   <213> Homo sapiens
<400> 142
   Ser Thr Thr Gln Glu Gly Glu Leu
   1 5
<210> 143
   <211> 10
   <212> PRT
   <213> Homo sapiens
<400> 143
   Gly His Thr Phe Glu Asp Ser Thr Lys Lys
   1 5 10
<210> 144
   <211> 8
   <212> PRT
   <213> Homo sapiens
<400> 144
   Gly Gly Gly His Phe Pro Pro Thr
   1 5
<210> 145
   <211> 6
   <212> PRT
   <213> Homo sapiens
<400> 145
   Pro Gly Thr Ile Asn Ile
   1 5
<210> 146
   <211> 5
   <212> PRT
   <213> Homo sapiens
<400> 146
   Phe Thr Pro Pro Thr
   1 5
<210> 147
   <211> 8
   <212> PRT
   <213> Homo sapiens
<400> 147
   Ile Asn His Arg Gly Tyr Trp Val
   1 5
<210> 148
   <211> 17
   <212> PRT
   <213> Homo sapiens
<400> 148
   Gly Glu Phe Cys Ile Asn His Arg Gly Tyr Trp Val Cys Gly Asp Pro
   1 5 10 15
   Ala
<210> 149
   <211> 14
   <212> PRT
   <213> Homo sapiens
<400> 149
   Met Ala Pro Glu Trp Pro Gly Ser Arg Asp Lys Arg Thr Leu
   1 5 10
<210> 150
   <211> 10
   <212> PRT
   <213> Homo sapiens
<400> 150
   Met Glu Asp Gly Gln Val Met Asp Val Asp
   1 5 10
<210> 151
   <211> 9
   <212> PRT
   <213> Homo sapiens
<400> 151
   Met Ser Thr Thr Gln Glu Gly Glu Leu
   1 5
<210> 152
   <211> 11
   <212> PRT
   <213> Homo sapiens

<400> 152
   Met Gly His Thr Phe Glu Asp Ser Thr Lys Lys
   1 5 10
<210> 153
   <211> 9
   <212> PRT
   <213> Homo sapiens
<400> 153
   Met Gly Gly Gly His Phe Pro Pro Thr
   1 5
<210> 154
   <211> 7
   <212> PRT
   <213> Homo sapiens
<400> 154
   Met Pro Gly Thr Ile Asn Ile
   1 5
<210> 155
   <211> 6
   <212> PRT
   <213> Homo sapiens
<400> 155
   Met Phe Thr Pro Pro Thr
   1 5
<210> 156
   <211> 9
   <212> PRT
   <213> Homo sapiens
<400> 156
   Met Ile Asn His Arg Gly Tyr Trp Val
   1 5
<210> 157
   <211> 18
   <212> PRT
   <213> Homo sapiens
<400> 157
   Met Gly Glu Phe Cys Ile Asn His Arg Gly Tyr Trp Val Cys Gly Asp
   1 5 10 15
   Pro Ala
<210> 158
   <211> 21
   <212> PRT
   <213> Hepatitis B virus
<400> 158
   Met Gly Thr Asn Leu Ser Val Pro Asn Pro Leu Gly Phe Phe Pro Asp
   1 5 10 15
   His Gln Leu Asp Pro
   20
<210> 159
   <211> 8
   <212> PRT
   <213> Hepatitis B virus
<400> 159
   Pro Leu Gly Phe Phe Pro Asp His
   1 5
<210> 160
   <211> 10
   <212> PRT
   <213> Hepatitis B virus
<400> 160
   Pro Leu Gly Phe Phe Pro Asp His Gln Leu
   1 5 10
<210> 161
   <211> 26
   <212> PRT
   <213> Hepatitis B virus
<400> 161
   Met Gln Trp Asn Ser Thr Ala Phe His Gln Thr Leu Gln Asp Pro Arg
   1 5 10 15
   Val Arg Gly Leu Tyr Leu Pro Ala Gly Gly
   20 25
<210> 162
   <211> 14
   <212> PRT
   <213> Hepatitis B
<400> 162
   Met Gln Trp Ser Thr Ala Phe His Gln Thr Leu Gln Asp Pro
   1 5 10
<210> 163
   <211> 14
   <212> PRT
   <213> Hepatitis B virus
<400> 163
   Met Gln Trp Ser Thr Ala Leu His Gln Ala Leu Gln Asp Pro
   1 5 10
<210> 164
   <211> 6
   <212> PRT
   <213> Hepatitis B virus
<400> 164
   Gln Asp Pro Arg Val Arg
   1 5
<210> 165
   <211> 13
   <212> PRT
   <213> Hepatitis B virus
<400> 165
   Asp Pro Arg Val Arg Gly Leu Tyr Leu Pro Ala Gly Gly
   1 5 10
<210> 166
   <211> 13
   <212> PRT
   <213> Hepatitis B virus
<400> 166
   Asp Pro Arg Val Arg Gly Leu Tyr Phe Pro Ala Gly Gly
   1 5 10
<210> 167
   <211> 24
   <212> PRT
   <213> B. anthracis
<400> 167
   Ile Val Thr Lys Glu Asn Thr Ile Ile Asn Pro Ser Glu Asn Gly Asp
   1 5 10 15
   Thr Ser Thr Asn Gly Ile Glu Leu
   20
<210> 168
   <211> 15
   <212> PRT
   <213> Hookworm

<400> 168
   Ile Val Tyr Gln His Ser His Gly Glu Asp Arg Pro Gly Glu Leu
   1 5 10 15
<210> 169
   <211> 8
   <212> PRT
   <213> Artificial Sequence
<220>
   <223> linker peptide
<400> 169
   Gly Ser Gly Asp Gly Glu Gly Gly
   1               5
<210> 170
   <211> 10
   <212> PRT
   <213> Artificial Sequence
<220>
   <223> flexible linker arm
<400> 170
   Gly Gly Gly Gly Ser Gly Gly Gly Gly Thr
   1               5                   10
<210> 171
   <211> 9
   <212> PRT
   <213> Artificial Sequence
<220>
   <223> Flexible linker arm sequence
<400> 171
   Gly Gly Gly Gly Ser Gly Gly Gly Gly
   1               5
<210> 172
   <211> 7
   <212> PRT
   <213> Artificial sequence
<220>
   <223> Flexible linker arm
<400> 172
   Gly Ser Gly Asp Glu Gly Gly
   1               5
<210> 173
   <211> 8
   <212> PRT
   <213> Artificial sequence
<220>
   <223> Flexible linker arm
<400> 173
   Gly Gly Gly Gly Ser Gly Gly Gly
   1 5
<210> 174
   <211> 16
   <212> PRT
   <213> HIV
<400> 174
   Gly Pro Lys Glu Pro Phe Arg Asp Tyr Val Asp Arg Phe Tyr Lys Cys
   1 5 10 15
<210> 175
   <211> 17
   <212> PRT
   <213> Corynebacterium diphtheriae
<400> 175
   Phe Gln Val Val His Asn Ser Tyr Asn Arg Pro Ala Tyr Ser Pro Gly
   1 5 10 15
   Cys
<210> 176
   <211> 25
   <212> PRT
   <213> Borrelia burgdorferi
<400> 176
   Val Glu Ile Lys Glu Gly Thr Val Thr Leu Lys Arg Glu Ile Asp Lys
   1 5 10 15
   Asn Gly Lys Val Thr Val Ser Leu Cys
   20 25
<210> 177
   <211> 19
   <212> PRT
   <213> Borrelia burgdorferi
<400> 177
   Thr Leu Ser Lys Asn Ile Ser Lys Ser Gly Glu Val Ser Val Glu Leu
   1 5 10 15
   Asn Asp Cys
<210> 178
   <211> 11
   <212> PRT
   <213> Influenza A virus
<400> 178
   Ser Ser Val Ser Ser Phe Glu Arg Phe Glu Cys
   1 5 10
<210> 179
   <211> 10
   <212> PRT
   <213> Influenza A virus
<400> 179
   Leu Ile Asp Ala Leu Leu Gly Asp Pro Cys
   1 5 10
<210> 180
   <211> 9
   <212> PRT
   <213> Influenza A virus
<400> 180
   Thr Leu Ile Asp Ala Leu Leu Gly Cys
   1 5
<210> 181
   <211> 24
   <212> PRT
   <213> Influenza A virus
<400> 181
   Phe Trp Arg Gly Glu Asn Gly Arg Lys Thr Arg Ser Ala Tyr Glu Arg
   1 5 10 15
   Met Cys Asn Ile Leu Lys Gly Lys
   20
<210> 182
   <211> 22
   <212> PRT
   <213> Influenza A virus
<400> 182
   Leu Arg Val Leu Ser Phe Ile Arg Gly Thr Lys Val Ser Pro Arg Gly
   1 5 10 15
   Lys Leu Ser Thr Arg Gly
   20
<210> 183
   <211> 22
   <212> PRT
   <213> Influenza A virus

<400> 183
   Ser Leu Val Gly Ile Asp Pro Phe Lys Leu Leu Gln Asn Ser Gln Val
   1 5 10 15
   Tyr Ser Leu Ile Arg Pro
   20
<210> 184
   <211> 24
   <212> PRT
   <213> Influenza A virus
<400> 184
   Ala Val Lys Gly Val Gly Thr Met Val Met Glu Leu Ile Arg Met Ile
   1 5 10 15
   Lys Arg Gly Ile Asn Asp Arg Asn
   20
<210> 185
   <211> 21
   <212> PRT
   <213> Trypanosoma cruzi
<400> 185
   Ser His Asn Phe Thr Leu Val Ala Ser Val Ile Ile Glu Glu Ala Pro
   1 5 10 15
   Ser Gly Asn Thr Cys
   20
<210> 186
   <211> 16
   <212> PRT
   <213> Plasmodium falciparum
<400> 186
   Ser Val Gln Ile Pro Lys Val Pro Tyr Pro Asn Gly Ile Val Tyr Cys
   1 5 10 15
<210> 187
   <211> 16
   <212> PRT
   <213> Plasmodium falciparum
<400> 187
   Asp Phe Asn His Tyr Tyr Thr Leu Lys Thr Gly Leu Glu Ala Asp Cys
   1 5 10 15
<210> 188
   <211> 18
   <212> PRT
   <213> Plasmodium falciparum
<400> 188
   Pro Ser Asp Lys His Ile Glu Gln Tyr Lys Lys Ile Lys Asn Ser Ile
   1 5 10 15
   Ser Cys
<210> 189
   <211> 20
   <212> PRT
   <213> Plasmodium falciparum
<400> 189
   Glu Tyr Leu Asn Lys Ile Gln Asn Ser Leu Ser Thr Glu Trp Ser Pro
   1 5 10 15
   Cys Ser Val Thr
   20
<210> 190
   <211> 19
   <212> PRT
   <213> Plasmodium vivax
<400> 190
   Tyr Leu Asp Lys Val Arg Ala Thr Val Gly Thr Glu Trp Thr Pro Cys
   1 5 10 15
   Ser Val Thr
<210> 191
   <211> 20
   <212> PRT
   <213> Plasmodium yoelii
<400> 191
   Glu Phe Val Lys Gln Ile Ser Ser Gln Leu Thr Glu Glu Trp Ser Gln
   1 5 10 15
   Cys Ser Val Thr
   20
<210> 192
   <211> 16
   <212> PRT
   <213> Streptococcus sobrinus
<400> 192
   Lys Pro Arg Pro Ile Tyr Glu Ala Lys Leu Ala Gln Asn Gln Lys Cys
   1 5 10 15
<210> 193
   <211> 17
   <212> PRT
   <213> Streptococcus sobrinus
<400> 193
   Ala Lys Ala Asp Tyr Glu Ala Lys Leu Ala Gln Tyr Glu Lys Asp Leu
   1 5 10 15
   Cys
<210> 194
   <211> 16
   <212> PRT
   <213> Lymphocytic choriomeningitis virus
<400> 194
   Arg Pro Gln Ala Ser Gly Val Tyr Met Gly Asn Leu Thr Ala Gln Cys
   1 5 10 15
<210> 195
   <211> 16
   <212> PRT
   <213> Clostridium tetani
<400> 195
   Gln Tyr Ile Lys Ala Asn Ser Lys Phe Ile Gly Ile Thr Glu Leu Cys
   1 5 10 15
<210> 196
   <211> 19
   <212> PRT
   <213> Neisseria meningitidis
<400> 196
   Ala Ile Trp Gln Val Glu Gln Lys Ala Ser Ile Ala Gly Thr Asp Ser
   1 5 10 15
   Gly Trp Cys
<210> 197
   <211> 19
   <212> PRT
   <213> Neisseria meningitidis
<400> 197
   Asn Tyr Lys Asn Gly Gly Phe Phe Val Gln Tyr Gly Gly Ala Tyr Lys
   1 5 10 15
   Arg His Cys
<210> 198
   <211> 19
   <212> PRT
   <213> Neisseria meningitidis

<400> 198
   His Asn Ser Gln Thr Glu Val Ala Ala Thr Leu Ala Tyr Arg Phe Gly
   1               5                   10                  15
   Asn Val Cys
<210> 199
   <211> 19
   <212> PRT
   <213> Neisseria meningitidis
<400> 199
   Thr Pro Arg Val Ser Tyr Ala His Gly Phe Lys Gly Leu Val Asp Asp
   1               5                   10                  15
   Ala Asp Cys
<210> 200
   <211> 19
   <212> PRT
   <213> Neisseria meningitidis
<400> 200
   Arg Phe Gly Asn Ala Val Pro Arg Ile Ser Tyr Ala His Gly Phe Asp
   1               5                   10                  15
   Phe Ile Cys
<210> 201
   <211> 19
   <212> PRT
   <213> Neisseria meningitidis
<400> 201
   Ala Phe Lys Tyr Ala Arg His Ala Asn Val Gly Arg Asn Ala Phe Glu
   1               5                   10                  15
   Leu Phe Cys
<210> 202
   <211> 20
   <212> PRT
   <213> Neisseria meningitidis
<400> 202
   Ser Gly Ala Trp Leu Lys Arg Asn Thr Gly Ile Gly Asn Tyr Thr Gln
   1 5 10 15
   Ile Asn Ala Cys
   20
<210> 203
   <211> 16
   <212> PRT
   <213> Neisseria meningitidis
<400> 203
   Ala Gly Glu Phe Gly Thr Leu Arg Ala Gly Arg Val Ala Asn Gln Cys
   1 5 10 15
<210> 204
   <211> 16
   <212> PRT
   <213> Neisseria meningitidis
<400> 204
   Ile Gly Asn Tyr Thr Gln Ile Asn Ala Ala Ser Val Gly Leu Arg Cys
   1 5 10 15
<210> 205
   <211> 16
   <212> PRT
   <213> Neisseria meningitidis
<400> 205
   Gly Arg Asn Tyr Gln Leu Gln Leu Thr Glu Gln Pro Ser Arg Thr Cys
   1 5 10 15
<210> 206
   <211> 16
   <212> PRT
   <213> Neisseria meningitidis
<400> 206
   Ser Gly Ser Val Gln Phe Val Pro Ala Gln Asn Ser Lys Ser Ala Cys
   1 5 10 15
<210> 207
   <211> 16
   <212> PRT
   <213> Neisseria meningitidis
<400> 207
   His Ala Asn Val Gly Arg Asp Ala Phe Asn Leu Phe Leu Leu Gly Cys
   1 5 10 15
<210> 208
   <211> 16
   <212> PRT
   <213> Neisseria meningitidis
<400> 208
   Leu Gly Arg Ile Gly Asp Asp Asp Glu Ala Lys Gly Thr Asp Pro Cys
   1 5 10 15
<210> 209
   <211> 16
   <212> PRT
   <213> Neisseria meningitidis
<400> 209
   Ser Val Gln Phe Val Pro Ala Gln Asn Ser Lys Ser Ala Tyr Lys Cys
   1 5 10 15
<210> 210
   <211> 16
   <212> PRT
   <213> Neisseria meningitidis
<400> 210
   Asn Tyr Ala Phe Lys Tyr Ala Lys His Ala Asn Val Gly Arg Asp Cys
   1 5 10 15
<210> 211
   <211> 16
   <212> PRT
   <213> Neisseria meningitidis
<400> 211
   Ala His Gly Phe Asp Phe Ile Glu Arg Gly Lys Lys Gly Glu Asn Cys
   1 5 10 15
<210> 212
   <211> 16
   <212> PRT
   <213> Neisseria meningitidis
<400> 212
   Gly Val Asp Tyr Asp Phe Ser Lys Arg Thr Ser Ala Ile Val Ser Cys
   1 5 10 15
<210> 213
   <211> 16
   <212> PRT
   <213> Neisseria meningitidis
<400> 213
   His Asp Asp Met Pro Val Ser Val Arg Tyr Asp Ser Pro Asp Phe Cys
   1 5 10 15
<210> 214
   <211> 27
   <212> PRT
   <213> Neisseria meningitidis

<400> 214
   Arg Phe Gly Asn Ala Val Pro Arg Ile Ser Tyr Ala His Gly Phe Asp
   1 5 10 15
   Phe Ile Glu Arg Gly Lys Lys Gly Glu Asn Cys
   20 25
<210> 215
   <211> 24
   <212> PRT
   <213> Neisseria meningitidis
<400> 215
   Asn Tyr Ala Phe Lys Tyr Ala Lys His Ala Asn Val Gly Arg Asp Ala
   1 5 10 15
   Phe Asn Leu Phe Leu Leu Gly Cys
   20
<210> 216
   <211> 26
   <212> PRT
   <213> Neisseria meningitidis
<400> 216
   Ser Gly Ala Trp Leu Lys Arg Asn Thr Gly Ile Gly Asn Tyr Thr Gln
   1 5 10 15
   Ile Asn Ala Ala Ser Val Gly Leu Arg Cys
   20 25
<210> 217
   <211> 20
   <212> PRT
   <213> Neisseria meningitidis
<400> 217
   Ser Gly Ser Val Gln Phe Val Pro Ala Gln Asn Ser Lys Ser Ala Tyr
   1 5 10 15
   Thr Pro Ala Cys
   20
<210> 218
   <211> 19
   <212> PRT
   <213> Neisseria meningitidis
<400> 218
   Thr Gly Ala Asn Asn Thr Ser Thr Val Ser Asp Tyr Phe Arg Asn Arg
   1 5 10 15
   Ile Thr Cys
<210> 219
   <211> 19
   <212> PRT
   <213> Neisseria meningitidis
<400> 219
   Ile Tyr Asp Phe Lys Leu Asn Asp Lys Phe Asp Lys Phe Lys Pro Tyr
   1 5 10 15
   Ile Gly Cys
<210> 220
   <211> 19
   <212> PRT
   <213> Neisseria meningitidis
<400> 220
   Leu Ser Ala Ile Tyr Asp Phe Lys Leu Asn Asp Lys Phe Lys Pro Tyr
   1               5                   10                  15
   Ile Gly Cys
<210> 221
   <211> 19
   <212> PRT
   <213> Neisseria meningitidis
<400> 221
   Asn Gly Trp Tyr Ile Asn Pro Trp Ser Glu Val Lys Phe Asp Leu Asn
   1               5                   10                  15
   Ser Arg Cys
<210> 222
   <211> 20
   <212> PRT
   <213> Hepatitis B virus
<400> 222
   Met Asp Ile Asp Pro Tyr Lys Glu Phe Gly Ala Thr Val Glu Leu Leu
   1               5                   10                  15
   Ser Phe Leu Pro
               20
<210> 223
   <211> 24
   <212> PRT
   <213> Hepatitis B virus
<400> 223
   Arg Asp Leu Leu Asp Ala Ser Ala Leu Tyr Arg Glu Ala Leu Glu Ser
   1               5                   10                  15
   Pro Glu His Cys Ser Pro His His
               20
<210> 224
   <211> 25
   <212> PRT
   <213> Hepatitis B virus
<400> 224
   Thr Trp Val Gly Val Asn Leu Glu Asp Pro Ala Ser Arg Asp Leu Val
   1 5 10 15
   Val Ser Tyr Val Asn Thr Asn Met Gly
   20 25
<210> 225
   <211> 16
   <212> PRT
   <213> Hepatitis B virus
<400> 225
   Val Val Ser Tyr Val Asn Thr Asn Met Gly Leu Lys Phe Arg Gln Leu
   1 5 10 15
<210> 226
   <211> 21
   <212> PRT
   <213> Hepatitis B virus
<400> 226
   Leu Leu Trp Phe His Ile Ser Cys Leu Thr Phe Gly Arg Glu Thr Val
   1 5 10 15
   Ile Glu Tyr Leu Val
   20
<210> 227
   <211> 32
   <212> PRT
   <213> Hepatitis B virus

<400> 227
   Leu Leu Trp Phe His Ile Ser Cys Leu Thr Phe Val Ser Phe Gly Val
   1 5 10 15
   Trp Ile Arg Thr Pro Pro Ala Tyr Arg Pro Pro Asn Ala Pro Ile Leu
   20 25 30
<210> 228
   <211> 21
   <212> PRT
   <213> Hepatitis B virus
<400> 228
   Val Ser Phe Gly Val Trp Ile Arg Thr Pro Pro Ala Tyr Arg Pro Pro
   1               5                   10                  15
   Asn Ala Pro Ile Leu
               20
<210> 229
   <211> 12
   <212> PRT
   <213> Hepatitis B virus
<400> 229
   Val Ser Phe Gly Val Trp Ile Arg Thr Pro Pro Ala
   1               5                   10
<210> 230
   <211> 12
   <212> PRT
   <213> Hepatitis B virus
<400> 230
   Pro Pro Ala Tyr Arg Pro Pro Asn Ala Pro Ile Leu
   1               5                   10
<210> 231
   <211> 12
   <212> PRT
   <213> Hepatitis B virus
<400> 231
   Trp Ile Arg Thr Pro Pro Ala Tyr Arg Pro Pro Asn
   1               5                   10
<210> 232
   <211> 20
   <212> PRT
   <213> Hepatitis B virus
<400> 232
   Pro His His Thr Ala Leu Arg Gln Ala Ile Leu Cys Trp Gly Glu Leu
   1 5 10 15
   Met Thr Leu Ala
   20
<210> 233
   <211> 13
   <212> PRT
   <213> Artificial sequence
<220>
   <223> PADRE eptiope
<400> 233
   Ala Lys Phe Val Ala Ala Trp Thr Leu Lys Ala Ala Ala
   1 5 10
<210> 234
   <211> 549
   <212> DNA
   <213> Hepatitis B virus
<400> 234
   atggacatcg acccttataa agaatttgga gctactgtgg agttactctc gtttttgcct
   60
   tctgacttct ttccttcagt acgagatctt ctagataccg cctcagctct gtatcgggaa
   120
   gccttagagt ctcctgagca ttgttcacct caccatactg cactcaggca agcaattctt
   180
   tgctgggggg aactaatgac tctagctacc tgggtgggtg ttaatttgga agatccagcg
   240
   tctagagacc tagtagtcag ttatgtcaac actaatatgg gcctaaagtt caggcaactc
   300
   ttgtggtttc acatttcttg tctcactttt ggaagagaaa cagttataga gtatttggtg
   360
   tctttcggag tgtggattcg cactcctcca gcttatagac caccaaatgc ccctatccta
   420
   tcaacacttc cggagactac tgttgttaga cgacgaggca ggtcccctag aagaagaact
   480
   ccctcgcctc gcagacgaag gtctcaatcg ccgcgtcgca gaagatctca atctcgggaa
   540
   tctcaatgt
   549
<210> 235
   <211> 555
   <212> DNA
   <213> Hepatitis B virus
<400> 235
   atggacattg acccttataa agaatttgga gctactgtgg agttactctc gtttttgcct
   60
   tctgacttct ttccttccgt acgagatctc ctagacaccg cctcagctct gtatcgagaa
   120
   gccttagagt ctcctgagca ttgctcacct caccatactg cactcaggca agccattctc
   180
   tgctgggggg aattgatgac tctagctacc tgggtgggta ataatttgca agatccagca
   240
   tccagagatc tagtagtcaa ttatgttaat actaacatgg gtttaaagat caggcaacta
   300
   ttgtggtttc atatatcttg ccttactttt ggaagagaga ctgtacttga atatttggtc
   360
   tctttcggag tgtggattcg cactcctcca gcctatagac caccaaatgc ccctatctta
   420
   tcaacacttc cggaaactac tgttgttaga cgacgggacc gaggcaggtc ccctagaaga
   480
   agaactccct cgcctcgcag acgcagatct caatcgccgc gtcgcagaag atctcaatct
   540
   cgggaatctc aatgt
   555
<210> 236
   <211> 555
   <212> DNA
   <213> Hepatitis B virus
<400> 236
   atggacattg acccttataa agaatttgga gctactgtgg agttactctc gtttttgcct 60
   tctgacttct ttccttccgt cagagatctc ctagacaccg cctcagctct gtatcgagaa 120
   gccttagagt ctcctgagca ttgctcacct caccatactg cactcaggca agccattctc 180
   tgctgggggg aattgatgac tctagctacc tgggtgggta ataatttgga agatccagca 240
   tctagggatc ttgtagtaaa ttatgttaat actaacgtgg gtttaaagat caggcaacta 300
   ttgtggtttc atatatcttg ccttactttt ggaagagaga ctgtacttga atatttggtc 360
   tctttcggag tgtggattcg cactcctcca gcctatagac caccaaatgc ccctatctta 420
   tcaacacttc cggaaactac tgttgttaga cgacgggacc gaggcaggtc ccctagaaga 480
   agaactccct cgcctcgcag acgcagatct ccatcgccgc gtcgcagaag atctcaatct 540
   cgggaatctc aatgt 555
<210> 237
   <211> 549
   <212> DNA
   <213> Hepatitis B virus
<400> 237
   atggacattg acccttataa agaatttgga gctactgtgg agttactctc gtttttgcct 60
   tctgacttct ttccttccgt acgagatctt ctagataccg ccgcagctct gtatcgggat 120
   gccttagagt ctcctgagca ttgttcacct caccatactg cactcaggca agcaattctt 180
   tgctggggag acttaatgac tctagctacc tgggtgggta ctaatttaga agatccagca 240
   tctagggacc tagtagtcag ttatgtcaac actaatgtgg gcctaaagtt cagacaatta 300
   ttgtggtttc acatttcttg tctcactttt ggaagagaaa cggttctaga gtatttggtg 360
   tcttttggag tgtggattcg cactcctcca gcttatagac caccaaatgc ccctatccta 420
   tcaacgcttc cggagactac tgttgttaga cgacgaggca ggtcccctag aagaagaact 480
   ccctcgcctc gcagacgaag atctcaatcg ccgcgtcgca gaagatctca atctcgggaa 540
   tctcaatgt 549
<210> 238
   <211> 549
   <212> DNA
   <213> Woodchuck

<400> 238
   atggctttgg ggcatggaca tagatcctta taaagaattt ggttcatctt atcagttgtt 60
   gaattttctt cctttggact tctttcctga tcttaatgct ttggtggaca ctgctactgc 120
   cttgtatgaa gaagaactaa caggtaggga acattgctct ccgcaccata cagctattag 180
   acaagcttta gtatgctggg atgaattaac taaattgata gcttggatga gctctaacat 240
   aacttctgaa caagtaagaa caatcattgt aaatcatgtc aatgatacct ggggacttaa 300
   ggtgagacaa agtttatggt ttcatttgtc atgtctcact ttcggacaac atacagttca 360
   agaattttta gtaagttttg gagtatggat caggactcca gctccatata gacctcctaa 420
   tgcacccatt ctctcgactc ttccggaaca tacagtcatt aggagaagag gaggtgcaag 480
   agcttctagg tcccccagaa gacgcactcc ctctcctcgc aggagaagat ctcaatcacc 540
   gcgtcgcag 549
<210> 239
   <211> 651
   <212> DNA
   <213> Ground squirrel
<400> 239
   atgtatcttt ttcacctgtg ccttgttttt gcctgtgttc catgtcctac tgttcaagcc
   60
   tccaagctgt gccttggatg gctttgggac atggacatag atccctataa agaatttggt
   120
   tcttcttatc agttgttgaa ttttcttcct ttggactttt ttcctgatct caatgcattg
   180
   gtggacactg ctgctgctct ttatgaagaa gaattaacag gtagggagca ttgttctcct
   240
   catcatactg ctattagaca ggccttagtg tgttgggaag aattaactag attaattaca
   300
   tggatgagtg aaaatacaac agaagaagtt agaagaatta ttgttgatca tgtcaataat
   360
   acttggggac ttaaagtaag acagacttta tggtttcatt tatcatgtct tacttttgga
   420
   caacacacag ttcaagaatt tttggttagt tttggagtat ggattagaac tccagctcct
   480
   tatagaccac ctaatgcacc cattttatca actcttccgg aacatacagt cattaggaga
   540
   agaggaggtt caagagctgc taggtccccc cgaagacgca ctccctctcc tcgcaggaga
   600
   aggtctcaat caccgcgtcg cagacgctct caatctccag cttccaactg c
   651
<210> 240
   <211> 18
   <212> DNA
   <213> Artificial sequence
<220>
   <223> primer
<400> 240
   ggtgcatgca aggagatg
   18
<210> 241
   <211> 55
   <212> DNA
   <213> Artificial sequence
<220>
   <223> primer
<400> 241
   gcgaagcttc ggatcccatg gttttttcct ccttatgtga aattgttatc cgctc
   55
<210> 242
   <211> 24
   <212> DNA
   <213> Artificial sequence
<220>
   <223> primer
<400> 242
   ttgggccatg gacatcgacc ctta
   24
<210> 243
   <211> 31
   <212> DNA
   <213> Artificial sequence
<220>
   <223> primer
<400> 243
   cgcaagctta aacaacagta gtctccggaa g
   31
<210> 244
   <211> 43
   <212> DNA
   <213> Artificial sequence
<220>
   <223> primer
<400> 244
   gtttctcttc caaaagtgag gctagaaatg tgaaaccaca aga
   43
<210> 245
   <211> 20
   <212> DNA
   <213> Artificial sequence
<220>
   <223> primer
<400> 245
   ctcacttttg gaagagaaac
   20
<210> 246
   <211> 39
   <212> DNA
   <213> Artificial sequence
<220>
   <223> primer
<400> 246
   gagcgcagta tggtgaggtg agctatgctc aggagactc
   39
<210> 247
   <211> 25
   <212> DNA
   <213> Artificial sequence
<220>
   <223> primer
<400> 247
   gaggcgctca ggcaagcaat tcttt
   25
<210> 248
   <211> 35
   <212> DNA
   <213> Artificial sequence

<220>
   <223> primer
<400> 248
   cgcaagctta ctagcaaaca acagtagtct cggaa
   35
<210> 249
   <211> 49
   <212> DNA
   <213> Artificial sequence
<220>
   <223> primer
<400> 249
   gcgctcttcc aaaagtgagg ctagaaatgc aaaaccacaa gagttgcct
   49
<210> 250
   <211> 42
   <212> DNA
   <213> Artificial sequence
<220>
   <223> primer
<400> 250
   gcgggcccat attagtgttg caataactga ctactaggtc tc
   42
<210> 251
   <211> 50
   <212> DNA
   <213> Artificial sequence
<220>
   <223> primer
<400> 251
   gcggctcgcc ccagcaaaga attgcttgtc tacacgcagt atggtgaggt
   50
<210> 252
   <211> 27
   <212> DNA
   <213> Artificial sequence
<220>
   <223> primer
<400> 252
   ggggcgagct aatgactcta gctacct
   27
<210> 253
   <211> 43
   <212> DNA
   <213> Artificial sequence
<220>
   <223> primer
<400> 253
   gcggctcgcc ccagcaaaga atgcattgcc tgagcgcagt atg
   43
<210> 254
   <211> 20
   <212> DNA
   <213> Artificial sequence
<220>
   <223> primer
<400> 254
   ttaggcccat attagtgttg
   20
<210> 255
   <211> 44
   <212> DNA
   <213> Artificial sequence
<220>
   <223> primer
<400> 255
   gcggggccta aagttcaggc aatgcttgtg gtttcacatt tcta
   44
<210> 256
   <211> 31
   <212> DNA
   <213> Artificial sequence
<220>
   <223> primer
<400> 256
   gcgccaggtg catagagtca ttagttcccc c
   31
<210> 257
   <211> 17
   <212> DNA
   <213> Artificial sequence
<220>
   <223> primer
<400> 257
   ctacctgggt gggtgtt
   17
<210> 258
   <211> 19
   <212> DNA
   <213> Artificial sequence
<220>
   <223> primer
<400> 258
   tatgggccta aagttcagg
   19
<210> 259
   <211> 49
   <212> DNA
   <213> Artificial sequence
<220>
   <223> primer
<400> 259
   gcggctcgcc gcagcaaaga attgcttgtc tgagcgcagt atggtgagg
   49
<210> 260
   <211> 33
   <212> DNA.
   <213> Artificial sequence
<220>
   <223> primer
<400> 260
   gcggggccta aagtgcaggc aactcttgtg gtt
   33
<210> 261
   <211> 29
   <212> DNA
   <213> Artificial sequence
<220>
   <223> primer

<400> 261
   gcggctagct ggatcttcgc aattaacac
   29
<210> 262
   <211> 28
   <212> DNA
   <213> Artificial sequence
<220>
   <223> primer
<400> 262
   gcggctagct gcgacctagt agtcagtt
   28
<210> 263
   <211> 24
   <212> DNA
   <213> Hepatitis B virus
<400> 263
   ttgggccatg gacatcgacc ctta
   24
<210> 264
   <211> 31
   <212> DNA
   <213> Hepatitis B virus
<400> 264
   gcggaattcc atcttccaaa ttaacaccca c
   31
<210> 265
   <211> 39
   <212> DNA
   <213> Hepatitis B virus
<400> 265
   cgcgaattca aaaagagctc ccagcgtcta gagacctag
   39
<210> 266
   <211> 31
   <212> DNA
   <213> Hepatitis B virus
<400> 266
   cgcaagctta aacaacagta gtctccggaa g
   31
<210> 267
   <211> 37
   <212> DNA
   <213> Artificial Sequence
<220>
   <223> Amplification primer containing a restriction endonuclease site
<400> 267
   cgcaagctta ctagcaaaca acagtagtct ccggaag
   37
<210> 268
   <211> 28
   <212> DNA
   <213> Artificial Sequence
<220>
   <223> Amplification primer containing a restriction endonuclease site
<400> 268
   ggaaagctta ctaacattga gattcccg
   28
<210> 269
   <211> 31
   <212> DNA
   <213> Artificial sequence
<220>
   <223> primer
<400> 269
   gcggaattcc atcttccaaa ttaacaccca c
   31
<210> 270
   <211> 30
   <212> DNA
   <213> Artificial sequence
<220>
   <223> primer
<400> 270
   gcggaattcc atcttcgcaa ttaacaccca
   30
<210> 271
   <211> 39
   <212> DNA
   <213> Artificial sequence
<220>
   <223> primer
<400> 271
   cgggaattca aaaagagctc ccagcgtcta gagacctag
   39
<210> 272
   <211> 37
   <212> DNA
   <213> Artificial sequence
<220>
   <223> primer
<400> 272
   gcggaattca aaaagagctc ccagctagct gcgacct
   37
<210> 273
   <211> 108
   <212> DNA
   <213> Artificial sequence
<220>
   <223> primer
<400> 273
   aattctggat gcggaatttc gtcatgacag cggctatgag gtgcaccatc agaaactggt
   60
   tttctttgcc gaagatgtcg gttctaacaa gggggcaatt atcgagct
   108
<210> 274
   <211> 72
   <212> DNA
   <213> Artificial sequence
<220>
   <223> primer

<400> 274
   aattgtcacg aaagaaaata ctataattaa cccttctgag aatggtgaca cctccacgaa
   60
   cgggatcgag ct
   72
<210> 275
   <211> 45
   <212> DNA
   <213> Artificial sequence
<220>
   <223> primer
<400> 275
   aattgtttat cagcattctc acggcgaaga tcgtccaggt gagct
   45
<210> 276
   <211> 78
   <212> DNA
   <213> Artificial sequence
<220>
   <223> primer
<400> 276
   aatttctctg ttaaccgaag tggagacgcc gattcgtaac gaatggggta gccgctctaa
   60
   tgatagctct gacgagct
   78
<210> 277
   <211> 12
   <212> PRT
   <213> Hepatitis B virus
<400> 277
   Met Gly Cys Glu Leu Asp Pro Tyr Lys Glu Phe Gly
   1 5 10
<210> 278
   <211> 40
   <212> DNA
   <213> Artificial sequence
<220>
   <223> oligonucleotide
<400> 278
   gcgccatggg gtgtgagctc gacccttata aagaatttgg
   40
<210> 279
   <211> 12
   <212> PRT
   <213> Hepatitis B virus
<400> 279
   Met Gly Cys Asp Ile Asp Pro Tyr Lys Glu Phe Gly
   1 5 10
<210> 280
   <211> 40
   <212> DNA
   <213> Artificial sequence
<220>
   <223> oligonucleotide
<400> 280
   gcgccatggg gtgtgacatc gacccttata aagaatttgg
   40
<210> 281
   <211> 42
   <212> DNA
   <213> Artificial Sequence
<220>
   <223> Amplification primer containing a restriction endonuclease site
<400> 281
   cgcaagctta gagctcttga attccaacaa cagtagtctc cg
   42
<210> 282
   <211> 28
   <212> DNA
   <213> Artificial Sequence
<220>
   <223> Amplification primer containing a restriction endonuclease site
<400> 282
   cgcgagctcc cagcgtctag agacctag
   28
<210> 283
   <211> 17
   <212> DNA
   <213> Artificial Sequence
<220>
   <223> Amplification primer containing a restriction endonuclease site
<400> 283
   gtatcaggct gaaaatc
   17
<210> 284
   <211> 19
   <212> PRT
   <213> Plasmodium falciparum
<400> 284
   Ile Asn Ala Asn Pro Asn Ala Asn Pro Asn Ala Asn Pro Asn Ala Asn
   1 5 10 15
   Pro Glu Leu
<210> 285
   <211> 57
   <212> DNA
   <213>. Plasmodium falciparum
<400> 285
   aattaacgct aatccgaacg ctaatccgaa cgctaatccg aacgctaatc cggagct
   57
<210> 286
   <211> 49
   <212> DNA
   <213> Plasmodium falciparum

<400> 286
   ccggattagc gttcggatta gcgttcggat tagcgttcgg attagcgtt
   49
<210> 287
   <211> 31
   <212> PRT
   <213> Plasmodium falciparum
<400> 287
   Ile Asn Ala Asn Pro Asn Val Asp Pro Asn Ala Asn Pro Asn Ala Asn
   1 5 10 15
   Pro Asn Ala Asn Pro Asn Val Asp Pro Asn Ala Asn Pro Glu Leu
   20 25 30
<210> 288
   <211> 93
   <212> DNA
   <213> Plasmodium falciparum
<400> 288
   aattaacgct aatccgaacg ttgacccgaa cgctaatccg aacgctaatc cgaacgctaa
   60
   tccgaacgtt gacccgaacg ctaatccgga gct
   93
<210> 289
   <211> 91
   <212> DNA
   <213> Plasmodium falciparum
<400> 289
   ggagctccgg attagcgttc gggtcaacgt tcggattagc gttcggatta gcgttcggat
   60
   tagcgttcgg gtcaacgttc ggattagcgt t
   91
<210> 290
   <211> 23
   <212> PRT
   <213> Plasmodium falciparum
<400> 290
   Ile Asn Ala Asn Pro Asn Val Asp Pro Asn Ala Asn Pro Asn Ala Asn
   1               5                   10                  15
   Pro Asn Ala Asn Pro Glu Leu
               20
<210> 291
   <211> 69
   <212> DNA
   <213> Plasmodium falciparum
<400> 291
   aattaacgcg aatccgaacg tggatccgaa tgccaaccct aacgccaacc caaatgcgaa
   60
   cccagagct
   69
<210> 292
   <211> 61
   <212> DNA
   <213> Plasmodium falciparum
<400> 292
   ctgggttcgc atttgggttg gcgttagggt tggcattcgg atccacgttc ggattcgcgt 60
   t 61
<210> 293
   <211> 23
   <212> PRT
   <213> Plasmodium falciparum
<400> 293
   Ile Asn Ala Asn Pro Asn Ala Asn Pro Asn Ala Asn Pro Asn Val Asp
   1 5 10 15
   Pro Asn Ala Asn Pro Glu Leu
   20
<210> 294
   <211> 69
   <212> DNA
   <213> Plasmodium falciparum
<400> 294
   aattaacgcg aatccgaatg ccaaccctaa cgccaaccca aacgtggatc cgaatgcgaa 60
   cccagagct 69
<210> 295
   <211> 61
   <212> DNA
   <213> Plasmodium falciparum
<400> 295
   ctgggttcgc attcggatcc acgtttgggt tggcgttagg gttggcattc ggattcgcgt 60
   t 61
<210> 296
   <211> 31
   <212> PRT
   <213> Plasmodium falciparum
<400> 296
   Ile Asn Ala Asn Pro Asn Val Asp Pro Asn Ala Asn Pro Asn Ala Asn
   1 5 10 15
   Pro Asn Ala Asn Pro Asn Val Asp Pro Asn Ala Asn Pro Glu Leu
   20 25 30
<210> 297
   <211> 93
   <212> DNA
   <213> Plasmodium falciparum
<400> 297
   aattaacgcg aatccgaacg tggatccaaa tgccaaccct aacgctaatc caaacgccaa
   60
   cccgaatgtt gaccccaatg ccaatccgga gct
   93
<210> 298
   <211> 85
   <212> DNA
   <213> Plasmodium falciparum
<400> 298
   ccggattggc attggggtca acattcgggt tggcgtttgg attagcgtta gggttggcat
   60
   ttggatccac gttcggattc gcgtt
   85
<210> 299
   <211> 23
   <212> PRT
   <213> Plasmodium falciparum
<400> 299
   Ile Asn Pro Asn Val Asp Pro Asn Ala Asn Pro Asn Ala Asn Pro Asn
   1 5 10 15
   Ala Asn Pro Asn Val Glu Leu
   20
<210> 300
   <211> 69
   <212> DNA
   <213> Plasmodium falciparum
<400> 300
   aattaatccg aacgtggatc caaatgccaa ccctaacgct aatccaaacg ccaacccgaa
   60
   tgttgagct
   69
<210> 301
   <211> 61
   <212> DNA
   <213> Plasmodium falciparum

<400> 301
   caacattcgg gttggcgttt ggattagcgt tagggttggc atttggatcc acgttcggat
   60
   t
   61
<210> 302
   <211> 25
   <212> PRT
   <213> Plasmodium falciparum
<400> 302
   Ile Asn Pro Asn Val Asp Pro Asn Ala Asn Pro Asn Ala Asn Pro Asn
   1 5 10 15
   Ala Asn Pro Asn Val Asp Pro Glu Leu
   20 25
<210> 303
   <211> 75
   <212> DNA
   <213> Plasmodium falciparum
<400> 303
   aattaatccg aacgtggatc caaatgccaa ccctaacgct aatccaaacg ccaacccgaa
   60
   tgttgaccct gagct
   75
<210> 304
   <211> 67
   <212> DNA
   <213> Plasmodium falciparum
<400> 304
   cagggtcaac attcgggttg gcgtttggat tagcgttagg gttggcattt ggatccacgt
   60
   tcggatt
   67
<210> 305
   <211> 27
   <212> PRT
   <213> Plasmodium falciparum
<400> 305
   Ile Asn Pro Asn Val Asp Pro Asn Ala Asn Pro Asn Ala Asn Pro Asn
   1 5 10 15
   Ala Asn Pro Asn Val Asp Pro Asn Ala Glu Leu
   20 25
<210> 306
   <211> 81
   <212> DNA
   <213> Plasmodium falciparum
<400> 306
   aattaatccg aacgtggatc caaatgccaa ccctaacgct aatccaaacg ccaacccgaa
   60
   tgttgaccct aatgctgagc t
   81
<210> 307
   <211> 73
   <212> DNA
   <213> Plasmodium falciparum
<400> 307
   cagcattagg gtcaacattc gggttggcgt ttggattagc gttagggttg gcatttggat
   60
   ccacgttcgg att
   73
<210> 308
   <211> 21
   <212> PRT
   <213> Plasmodium falciparum
<400> 308
   Ile Asn Val Asp Pro Asn Ala Asn Pro Asn Ala Asn Pro Asn Ala Asn
   1               5                   10                  15
   Pro Asn Val Glu Leu
               20
<210> 309
   <211> 63
   <212> DNA
   <213> Plasmodium falciparum
<400> 309
   aattaacgtg gatccaaatg ccaaccctaa cgctaatcca aacgccaacc cgaatgttga
   60
   gct
   63
<210> 310
   <211> 55
   <212> DNA
   <213> Plasmodium falciparum
<400> 310
   caacattcgg gttggcgttt ggattagcgt tagggttggc atttggatcc acgtt
   55
<210> 311
   <211> 23
   <212> PRT
   <213> Plasmodium falciparum
<400> 311
   Ile Asn Val Asp Pro Asn Ala Asn Pro Asn Ala Asn Pro Asn Ala Asn
   1               5                   10                  15
   Pro Asn Val Asp Pro Glu Leu
               20
<210> 312
   <211> 69
   <212> DNA
   <213> Plasmodium falciparum
<400> 312
   aattaacgtg gatccaaatg ccaaccctaa cgctaatcca aacgccaacc cgaatgttga 60
   ccctgagct 69
<210> 313
   <211> 61
   <212> DNA
   <213> Plasmodium falciparum
<400> 313
   cagggtcaac attcgggttg gcgtttggat tagcgttagg gttggcattt ggatccacgt 60
   t 61
<210> 314
   <211> 25
   <212> PRT
   <213> Plasmodium falciparum
<400> 314
   Ile Asn Val Asp Pro Asn Ala Asn Pro Asn Ala Asn Pro Asn Ala Asn
   1               5                   10                  15
   Pro Asn Val Asp Pro Asn Ala Glu Leu
               20                  25
<210> 315
   <211> 75
   <212> DNA
   <213> Plasmodium falciparum

<400> 315
   aattaacgtg gatccaaatg ccaaccctaa cgctaatcca aacgccaacc cgaatgttga 60
   ccctaatgct gagct 75
<210> 316
   <211> 67
   <212> DNA
   <213> Plasmodium falciparum
<400> 316
   cagcattagg gtcaacattc gggttggcgt ttggattagc gttagggttg gcatttggat
   60
   ccacgtt
   67
<210> 317
   <211> 19
   <212> PRT
   <213> Plasmodium falciparum
<400> 317
   Ile Asp Pro Asn Ala Asn Pro Asn Ala Asn Pro Asn Ala Asn Pro Asn
   1 5 10 15
   Val Glu Leu
<210> 318
   <211> 57
   <212> DNA
   <213> Plasmodium falciparum
<400> 318
   aattgatcca aatgccaacc ctaacgctaa tccaaacgcc aacccgaatg ttgagct
   57
<210> 319
   <211> 49
   <212> DNA
   <213> Plasmodium falciparum
<400> 319
   caacattcgg gttggcgttt ggattagcgt tagggttggc atttggatc
   49
<210> 320
   <211> 21
   <212> PRT
   <213> Plasmodium falciparum
<400> 320
   Ile Asp Pro Asn Ala Asn Pro Asn Ala Asn Pro Asn Ala Asn Pro Asn
   1 5 10 15
   Val Asp Pro Glu Leu
   20
<210> 321
   <211> 63
   <212> DNA
   <213> Plasmodium falciparum
<400> 321
   aattgatcca aatgccaacc ctaacgctaa tccaaacgcc aacccgaatg ttgaccctga
   60
   gct
   63
<210> 322
   <211> 55
   <212> DNA
   <213> Plasmodium falciparum
<400> 322
   cagggtcaac attcgggttg gcgtttggat tagcgttagg gttggcattt ggatc
   55
<210> 323
   <211> 23
   <212> PRT
   <213> Plasmodium falciparum
<400> 323
   Ile Asp Pro Asn Ala Asn Pro Asn Ala Asn Pro Asn Ala Asn Pro Asn
   1 5 10 15
   Val Asp Pro Asn Ala Glu Leu
   20
<210> 324
   <211> 69
   <212> DNA
   <213> Plasmodium falciparum
<400> 324
   aattgatcca aatgccaacc ctaacgctaa tccaaacgcc aacccgaatg ttgaccctaa
   60
   tgccgagct
   69
<210> 325
   <211> 61
   <212> DNA
   <213> Plasmodium falciparum
<400> 325
   cggcattagg gtcaacattc gggttggcgt ttggattagc gttagggttg gcatttggat
   60
   c
   61
<210> 326
   <211> 21
   <212> PRT
   <213> Plasmodium falciparum
<400> 326
   Ile Glu Tyr Leu Asn Lys Ile Gln Asn Ser Leu Ser Thr Glu Trp Ser
   1 5 10 15
   Pro Cys Ser Val Thr
   20
<210> 327
   <211> 69
   <212> DNA
   <213> Plasmodium falciparum
<400> 327
   aattgaatat ctgaacaaaa tccagaactc tctgtccacc gaatggtctc cgtgctccgt
   60
   tacctagta
   69
<210> 328
   <211> 69
   <212> DNA
   <213> Plasmodium falciparum
<400> 328
   agcttactag gtaacggagc acggagacaa ttcggtggac agagagttct ggattttgtt
   60
   cagatattc
   69
<210> 329
   <211> 24
   <212> PRT
   <213> Plasmodium vivax
<400> 329
   Ile Pro Ala Gly Asp Arg Ala Asp Gly Gln Pro Ala Gly Asp Arg Ala
   1 5 10 15
   Ala Gly Gln Pro Ala Gly Glu Leu
   20
<210> 330
   <211> 72
   <212> DNA
   <213> Plasmodium vivax

<400> 330
   aattccggct ggtgaccgtg cagatggcca gccagcgggt gaccgcgctg caggccagcc
   60
   ggctggcgag ct
   72
<210> 331
   <211> 64
   <212> DNA
   <213> Plasmodium vivax
<400> 331
   cgccagccgg ctggcctgca gcgcggtcac ccgctggctg gccatctgca cggtcaccag
   60
   ccgg
   64
<210> 332
   <211> 21
   <212> PRT
   <213> Plasmodium vivax
<400> 332
   Ile Asp Arg Ala Ala Gly Gln Pro Ala Gly Asp Arg Ala Asp Gly Gln
   1 5 10 15
   Pro Ala Gly Glu Leu
   20
<210> 333
   <211> 63
   <212> DNA
   <213> Plasmodium vivax
<400> 333
   aattgacaga gcagccggac aaccagcagg cgatcgagca gacggacagc ccgcagggga
   60
   gct
   63
<210> 334
   <211> 55
   <212> DNA
   <213> Plasmodium vivax
<400> 334
   cccctgcggg ctgtccgtct gctcgatcgc ctgctggttg tccggctgct ctgtc
   55
<210> 335
   <211> 21
   <212> PRT
   <213> Plasmodium vivax
<400> 335
   Ile Ala Asn Gly Ala Gly Asn Gln Pro Gly Ala Asn Gly Ala Gly Asp
   1 5 10 15
   Gln Pro Gly Glu Leu
   20
<210> 336
   <211> 63
   <212> DNA
   <213> Plasmodium vivax
<400> 336
   aattgcgaac ggcgccggta atcagccggg ggcaaacggc gcgggtgatc aaccagggga
   60
   gct
   63
<210> 337
   <211> 55
   <212> DNA
   <213> Plasmodium vivax
<400> 337
   cccctggttg atcacccgcg ccgtttgccc ccggctgatt accggcgccg ttcgc
   55
<210> 338
   <211> 21
   <212> PRT
   <213> Plasmodium vivax
<400> 338
   Ile Ala Asn Gly Ala Asp Asn Gln Pro Gly Ala Asn Gly Ala Asp Asp
   1 5 10 15
   Gln Pro Gly Glu Leu
   20
<210> 339
   <211> 63
   <212> DNA
   <213> Plasmodium vivax
<400> 339
   aattgcgaac ggcgccgata atcagccggg tgcaaacggg gcggatgacc aaccaggcga
   60
   gct
   63
<210> 340
   <211> 55
   <212> DNA
   <213> Plasmodium vivax
<400> 340
   cgcctggttg gtcatccgcc ccgtttgcac ccggctgatt atcggcgccg ttcgc
   55
<210> 341
   <211> 39
   <212> PRT
   <213> Plasmodium vivax
<400> 341
   Ile Ala Asn Gly Ala Gly Asn Gln Pro Gly Ala Asn Gly Ala Gly Asp
   1 5 10 15
   Gln Pro Gly Ala Asn Gly Ala Asp Asn Gln Pro Gly Ala Asn Gly Ala
   20 25 30
   Asp Asp Gln Pro Gly Glu Leu
   35
<210> 342
   <211> 117
   <212> DNA
   <213> Plasmodium vivax
<400> 342
   aattgcgaac ggcgccggta atcagccggg agcaaacggc gcgggggatc aaccaggcgc 60
   caatggtgca gacaaccagc ctggggcgaa tggagccgat gaccaacccg gcgagct 117
<210> 343
   <211> 109
   <212> DNA
   <213> Plasmodium vivax
<400> 343
   cgccgggttg gtcatcggct ccattcgccc caggctggtt gtctgcacca ttggcgcctg 60
   gttgatcccc cgcgccgttt gctcccggct gattaccggc gccgttcgc 109
<210> 344
   <211> 25
   <212> PRT
   <213> Plasmodium vivax
<400> 344
   Ile Ala Pro Gly Ala Asn Gln Glu Gly Gly Ala Ala Ala Pro Gly Ala
   1 5 10 15
   Asn Gln Glu Gly Gly Ala Ala Glu Leu
   20 25
<210> 345
   <211> 75
   <212> DNA
   <213> Plasmodium vivax
<400> 345
   aattgcgccg ggcgccaacc aggaaggtgg ggctgcagcg ccaggagcca atcaagaagg 60
   cggtgcagcg gagct 75
<210> 346
   <211> 67
   <212> DNA
   <213> Plasmodium vivax

<400> 346
   ccgctgcacc gccttcttga ttggctcctg gcgctgcagc cccaccttcc tggttggcgc 60
   ccggcgc 67
<210> 347
   <211> 26
   <212> PRT
   <213> Artificial Sequence
<220>
   <223> Amplification primer containing a restriction endonuclease site
<400> 347
   Ile Ser Leu Leu Thr Glu Val Glu Thr Pro Ile Arg Asn Glu Trp Gly
   1 5 10 15
   Cys Arg Cys Asn Asp Ser Ser Asp Glu Leu
   20 25
<210> 348
   <211> 78
   <212> DNA
   <213> Artificial Sequence
<220>
   <223> Amplification primer containing a restriction endonuclease site
<400> 348
   aattagcctg ttaaccgaag tggagacgcc gatccgtaac gaatggggct gccgctgtaa 60
   tgattcttcc gacgagct 78
<210> 349
   <211> 70
   <212> DNA
   <213> Artificial Sequence
<220>
   <223> Amplification primer containing a restriction endonuclease site
<400> 349
   cgtcggaaga atcattacag cggcagcccc attcgttacg gatcggcgtc tccacttcgg 60
   ttaacaggct 70
<210> 350
   <211> 26
   <212> PRT
   <213> Artificial Sequence
<220>
   <223> Amplification primer containing a restriction endonuclease site
<400> 350
   Met Ser Leu Leu Thr Glu Val Glu Thr Pro Ile Arg Asn Glu Trp Gly
   1               5                   10                  15
   Cys Arg Cys Asn Asp Ser Ser Asp Glu Leu
               20                  25
<210> 351
   <211> 78
   <212> DNA
   <213> Artificial Sequence
<220>
   <223> Amplification primer containing a restriction endonuclease site
<400> 351
   catgtctctg ctgaccgaag ttgaaacccc tatcagaaac gaatgggggt gcagatgtaa
   60
   cgattcaagt gatgagct
   78
<210> 352
   <211> 70
   <212> DNA
   <213> Artificial Sequence
<220>
   <223> Amplification primer containing a restriction endonuclease site
<400> 352
   catcacttga atcgttacat ctgcaccccc attcgtttct gataggggtt tcaacttcgg
   60
   tcagcagaga
   70
<210> 353
   <211> 26
   <212> PRT
   <213> Artificial Sequence
<220>
   <223> Amplification primer containing a restriction endonuclease site
<400> 353
   Met Ser Leu Leu Thr Glu Val Glu Thr Pro Ile Arg Asn Glu Trp Gly
   1 5 10 15
   Ser Arg Cys Asn Asp Ser Ser Asp Glu Leu
   20 25
<210> 354
   <211> 78
   <212> DNA
   <213> Artificial Sequence
<220>
   <223> Amplification primer containing a restriction endonuclease site
<400> 354
   catgtctctg ctgaccgaag ttgaaacccc tatcagaaac gaatgggggt ctagatgtaa
   60
   cgattcaagt gatgagct
   78
<210> 355
   <211> 70
   <212> DNA
   <213> Artificial Sequence
<220>
   <223> Amplification primer containing a restriction endonuclease site
<400> 355
   catcacttga atcgttacat ctagaccccc attcgtttct gataggggtt tcaacttcgg
   60
   tcagcagaga
   70
<210> 356
   <211> 26
   <212> PRT
   <213> Artificial Sequence
<220>
   <223> Amplification primer containing a restriction endonuclease site
<400> 356
   Met Ser Leu Leu Thr Glu Val Glu Thr Pro Ile Arg Asn Glu Trp Gly
   1 5 10 15
   Cys Arg Ser Asn Asp Ser Ser Asp Glu Leu
   20 25
<210> 357
   <211> 78
   <212> DNA
   <213> Artificial Sequence
<220>
   <223> Amplification primer containing a restriction endonuclease site
<400> 357
   catgtctctg ctgaccgaag ttgaaacccc tatcagaaac gaatgggggt gcagatcgaa
   60
   cgattcaagt gatgagct
   78
<210> 358
   <211> 70
   <212> DNA
   <213> Artificial Sequence

<220>
   <223> Amplification primer containing a restriction endonuclease site
<400> 358
   catcacttga atcgttcgat ctgcaccccc attcgtttct gataggggtt tcaacttcgg
   60
   tcagcagaga
   70
<210> 359
   <211> 26
   <212> PRT
   <213> Influenza A virus
<400> 359
   Ile Ser Leu Leu Thr Glu Val Glu Thr Pro Ile Arg Asn Glu Trp Gly
   1 5 10 15
   Ser Arg Ser Asn Asp Ser Ser Asp Glu Leu
   20 25
<210> 360
   <211> 78
   <212> DNA
   <213> Influenza A virus
<400> 360
   aatttctctg ttaaccgaag tggagacgcc gattcgtaac gaatggggta gccgctctaa 60
   tgatagctct gacgagct 78
<210> 361
   <211> 70
   <212> DNA
   <213> Influenza A virus
<400> 361
   cgtcagagct atcattagag cggctacccc attcgttacg aatcggcgtc tccacttcgg 60
   ttaacagaga 70
<210> 362
   <211> 26
   <212> PRT
   <213> Artificial Sequence
<220>
   <223> Amplification primer containing a restriction endonuclease site
<400> 362
   Met Ser Leu Leu Thr Glu Val Glu Thr Pro Ile Arg Asn Glu Trp Gly
   1 5 10 15
   Ser Arg Ser Asn Asp Ser Ser Asp Glu Leu
   20 25
<210> 363
   <211> 78
   <212> DNA
   <213> Artificial Sequence
<220>
   <223> Amplification primer containing a restriction endonuclease site
<400> 363
   catgtctctg ctgaccgaag ttgaaacccc tatcagaaac gaatgggggt ctagatcgaa
   60
   cgattcaagt gatgagct
   78
<210> 364
   <211> 70
   <212> DNA
   <213> Artificial Sequence
<220>
   <223> Amplification primer containing a restriction endonuclease site
<400> 364
   catcacttga atcgttcgat ctagaccccc attcgtttct gataggggtt tcaacttcgg
   60
   tcagcagaga
   70
<210> 365
   <211> 21
   <212> DNA
   <213> Artificial Sequence
<220>
   <223> Amplification primer containing a restriction endonuclease site
<400> 365
   gcgggatccg gagcttatcg a
   21
<210> 366
   <211> 24
   <212> PRT
   <213> Artificial sequence
<220>
   <223> Amplification primer containing a restirction site
<400> 366
   Gly Cys Gly Cys Thr Cys Gly Ala Gly Ala Thr Cys Ala Cys Thr Thr
   1 5 10 15
   Gly Ala Ala Thr Cys Gly Thr Thr
   20
<210> 367
   <211> 33
   <212> DNA
   <213> Artificial sequence
<220>
   <223> Amplification primer containing a restriction site
<400> 367
   gcgctcgaga gcttattgac cgaagttgaa acc
   33
<210> 368
   <211> 24
   <212> DNA
   <213> Artificial sequence
<220>
   <223> Amplification primer containing restriction site
<400> 368
   gcgctgcaga tcacttgaat cgtt
   24
<210> 369
   <211> 25
   <212> DNA
   <213> Artificial sequence
<220>
   <223> Amplification primer containing a restriction site
<400> 369
   gcgctgcagt ctctgctgac cgaag
   25
<210> 370
   <211> 22
   <212> DNA
   <213> Artificial Sequence
<220>
   <223> Amplification primer containing a restriction endonuclease site
<400> 370
   cgcgacatgt ctctgctgac cg
   22
<210> 371
   <211> 31
   <212> DNA
   <213> Artificial sequence

<220>
   <223> oligonucleotide
<400> 371
   cgcaagctta aacaacagta gtctccggaa g
   31
<210> 372
   <211> 52
   <212> DNA
   <213> Artificial Sequence
<220>
   <223> Amplification primer containing a restriction endonuclease site
<400> 372
   gcgaagctta ctaaggggag cggcctcgtc gacgaacaac agtagtctcc gg
   52
<210> 373
   <211> 55
   <212> DNA
   <213> Artificial Sequence
<220>
   <223> Amplification primer containing a restriction endonuclease site
<400> 373
   gcgaagctta ctaacaaggg gagcggcctc gtcgacgaac aacagtagtc tccgg
   55
<210> 374
   <211> 49
   <212> DNA
   <213> Artificial Sequence
<220>
   <223> Amplification primer containing a restriction endonuclease site
<400> 374
   gcgaagctta ctaaggcgag ggagtgcgcc gacgagggga gcggcctcg
   49
<210> 375
   <211> 52
   <212> DNA
   <213> Artificial Sequence
<220>
   <223> Amplification primer containing a restriction endonuclease site
<400> 375
   gcgaagctta ctaacaaggc gagggagtgc gccgacgagg ggagcggcct cg
   52
<210> 376
   <211> 49
   <212> DNA
   <213> Artificial Sequence
<220>
   <223> Amplification primer containing a restriction endonuclease site
<400> 376
   gcgaagctta ctacggcgat tgagagcgtc gacggcgagg cgagggagt
   49
<210> 377
   <211> 52
   <212> DNA
   <213> Artificial Sequence
<220>
   <223> Amplification primer containing a restriction endonuclease site
<400> 377
   gcgaagctta ctaacacggc gattgagagc gtcgacggcg aggcgaggga gt
   52
<210> 378
   <211> 66
   <212> DNA
   <213> Artificial Sequence
<220>
   <223> Amplification primer containing a restriction endonuclease site
<400> 378
   gcgaagctta ctaacattga gattcccgag attgagatcg ccggcgacgc ggcgattgag
   60
   agcgtc
   66
<210> 379
   <211> 32
   <212> DNA
   <213> Artificial Sequence
<220>
   <223> Amplification primer containing a restriction endonuclease site
<400> 379
   gcgaagctta ctattgagat tcccgagatt ga
   32
<210> 380
   <211> 28
   <212> DNA
   <213> Artificial Sequence
<220>
   <223> Amplification primer containing a restriction endonuclease site
<400> 380
   ggaaagctta ctaacattga gattcccg
   28
<210> 381
   <211> 35
   <212> PRT
   <213> Hepatitis B virus
<400> 381
   Val Arg Arg Arg Gly Arg Ser Pro Arg Arg Arg Thr Pro Ser Pro Arg
   1 5 10 15
   Arg Arg Arg Ser Gln Ser Pro Arg Arg Arg Arg Ser Gln Ser Arg Glu
   20 25 30
   Ser Gln Cys
   35
<210> 382
   <211> 34
   <212> PRT
   <213> Hepatitis B virus
<400> 382
   Val Arg Arg Arg Gly Arg Ser Pro Arg Arg Arg Thr Pro Ser Pro Arg
   1 5 10 15
   Arg Arg Arg Ser Gln Ser Pro Arg Arg Arg Arg Ser Gln Ser Arg Glu
   20 25 30
   Ser Gln
<210> 383
   <211> 24
   <212> PRT
   . <213> Hepatitis B virus
<400> 383
   Val Arg Arg Arg Gly Arg Ser Pro Arg Arg Arg Thr Pro Ser Pro Arg
   1 5 10 15
   Arg Arg Arg Ser Gln Ser Pro Cys
   20
<210> 384
   <211> 23
   <212> PRT
   <213> Hepatitis B virus

<400> 384
   Val Arg Arg Arg Gly Arg Ser Pro Arg Arg Arg Thr Pro Ser Pro Arg
   1 5 10 15
   Arg Arg Arg Ser Gln Ser Pro
   20
<210> 385
   <211> 16
   <212> PRT
   <213> Hepatitis B virus
<400> 385
   Val Arg Arg Arg Gly Arg Ser Pro Arg Arg Arg Thr Pro Ser Pro Cys
   1 5 10 15
<210> 386
   <211> 15
   <212> PRT
   <213> Hepatitis B virus
<400> 386
   Val Arg Arg Arg Gly Arg Ser Pro Arg Arg Arg Thr Pro Ser Pro
   1 5 10 15
<210> 387
   <211> 9
   <212> PRT
   <213> Hepatitis B virus
<400> 387
   Val Arg Arg Arg Gly Arg Ser Pro Cys
   1               5
<210> 388
   <211> 8
   <212> PRT
   <213> Hepatitis B virus
<400> 388
   Val Arg Arg Arg Gly Arg Ser Pro
   1               5
<210> 389
   <211> 203
   <212> PRT
   <213> Hepatitis B virus
<400> 389
   Met Ser Leu Leu Thr Glu Val Glu Thr Pro Ile Arg Asn Glu Trp Gly
   1               5                   10                  15
   Cys Arg Cys Asn Asp Ser Ser Asp Pro Tyr Lys Glu Phe Gly Ala Thr
               20                  25                  30
   Val Glu Leu Leu Ser Phe Leu Pro Ser Asp Phe Phe Pro Ser Val Arg
   35 40 45
   Asp Leu Leu Asp Thr Ala Ser Ala Leu Tyr Arg Glu Ala Leu Glu Ser
   50 55 60
   Pro Glu His Cys Ser Pro His His Thr Ala Leu Arg Gln Ala Ile Leu
   65 70 75 80
   Cys Trp Gly Glu Leu Met Thr Leu Ala Thr Trp Val Gly Val Asn Leu
   85 90 95
   Glu Asp Pro Ala Ser Arg Asp Leu Val Val Ser Tyr Val Asn Thr Asn
   100 105 110
   Met Gly Leu Lys Phe Arg Gln Leu Leu Trp Phe His Ile Ser Cys Leu
   115 120 125
   Thr Phe Gly Arg Glu Thr Val Ile Glu Tyr Leu Val Ser Phe Gly Val
   130 135 140
   Trp Ile Arg Thr Pro Pro Ala Tyr Arg Pro Pro Asn Ala Pro Ile Leu
   145 150 155 160
   Ser Thr Leu Pro Glu Thr Thr Val Val Arg Arg Arg Gly Arg Ser Pro
   165 170 175
   Arg Arg Arg Thr Pro Ser Pro Arg Arg Arg Arg Ser Gln Ser Pro Arg
   180 185 190
   Arg Arg Arg Ser Gln Ser Arg Glu Ser Gln Cys
   195 200
<210> 390
   <211> 176
   <212> PRT
   <213> Artificial sequence
<220>
   <223> Influenza-Hepatitis B chimera
<400> 390
   Met Asp Ile Asp Pro Tyr Lys Glu Phe Gly Ala Thr Val Glu Leu Leu
   1 5 10 15
   Ser Phe Leu Pro Ser Asp Phe Phe Pro Ser Val Arg Asp Leu Leu Asp
   20 25 30
   Thr Ala Ser Ala Leu Tyr Arg Glu Ala Leu Glu Ser Pro Glu His Cys
   35 40 45
   Ser Pro His His Thr Ala Leu Arg Gln Ala Ile Leu Cys Trp Gly Glu
   50 55 60
   Leu Met Thr Leu Ala Thr Trp Val Gly Val Asn Leu Glu Asp Gly Ile
   65 70 75 80
   Ser Leu Leu Thr Glu Val Glu Thr Pro Ile Arg Asn Glu Trp Gly Cys
   85 90 95
   Arg Cys Asn Asp Ser Ser Asp Glu Leu Pro Ala Ser Arg Asp Leu Val
   100 105 110
   Val Ser Tyr Val Asn Thr Asn Met Gly Leu Lys Phe Arg Gln Leu Leu
   115 120 125
   Trp Phe His Ile Ser Cys Leu Thr Phe Gly Arg Glu Thr Val Ile Glu
   130 135 140
   Tyr Leu Val Ser Phe Gly Val Trp Ile Arg Thr Pro Pro Ala Tyr Arg
   145 150 155 160
   Pro Pro Asn Ala Pro Ile Leu Ser Thr Leu Pro Glu Thr Thr Val Val
   165 170 175
<210> 391
   <211> 177
   <212> PRT
   <213> Artificial sequence
<220>
   <223> Influenza-Heoatitis B mutant chimera
<400> 391
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Val Asn Leu Glu Asp Gly Ile
   65                  70                  75                  80
   Ser Leu Leu Thr Glu Val Glu Thr Pro Ile Arg Asn Glu Trp Gly Ala
                   85                  90                  95
   Arg Ala Asn Asp Ser Ser Asp Glu Leu Pro Ala Ser Arg Asp Leu Val
               100                 105                 110
   Val Ser Tyr Val Asn Thr Asn Met Gly Leu Lys Phe Arg Gln Leu Leu
           115                 120                 125
   Trp Phe His Ile Ser Cys Leu Thr Phe Gly Arg Glu Thr Val Ile Glu
       130                 135                 140
   Tyr Leu Val Ser Phe Gly Val Trp Ile Arg Thr Pro Pro Ala Tyr Arg
   145                 150                 155                 160
   Pro Pro Asn Ala Pro Ile Leu Ser Thr Leu Pro Glu Thr Thr Val Val
                   165                 170                 175
   Cys
<210> 392
   <211> 183
   <212> PRT
   <213> Hepatitis B virus
<400> 392
   Met Gly Ile Ser Leu Leu Thr Glu Val Glu Thr Pro Ile Arg Asn Glu
   1 5 10 15
   Trp Gly Cys Arg Cys Asn Asp Ser Ser Asp Glu Leu Leu Gly Trp Leu
   20 25 30
   Trp Gly Ile Asp Ile Asp Pro Tyr Lys Glu Phe Gly Ala Thr Val Glu
   35 40 45
   Leu Leu Ser Phe Leu Pro Ser Asp Phe Phe Pro Ser Val Arg Asp Leu
   50 55 60
   Leu Asp Thr Ala Ser Ala Leu Tyr Arg Glu Ala Leu Glu Ser Pro Glu
   65 70 75 80
   His Cys Ser Pro His His Thr Ala Leu Arg Gln Ala Ile Leu Cys Trp
   85 90 95
   Gly Glu Leu Met Thr Leu Ala Thr Trp Val Gly Val Asn Leu Glu Asp
   100 105 110
   Pro Ala Ser Arg Asp Leu Val Val Ser Tyr Val Asn Thr Asn Met Gly
   115 120 125
   Leu Lys Phe Arg Gln Leu Leu Trp Phe His Ile Ser Cys Leu Thr Phe
   130 135 140
   Gly Arg Glu Thr Val Ile Glu Tyr Leu Val Ser Phe Gly Val Trp Ile
   145 150 155 160
   Arg Thr Pro Pro Ala Tyr Arg Pro Pro Asn Ala Pro Ile Leu Ser Thr
   165 170 175
   Leu Pro Glu Thr Thr Val Val
   180
<210> 393
   <211> 184
   <212> PRT
   <213> Hepatitis B virus
<400> 393
   Met Gly Ile Ser Leu Leu Thr Glu Val Glu Thr Pro Ile Arg Asn Glu
   1               5                   10                  15
   Trp Gly Cys Arg Cys Asn Asp Ser Ser Asp Glu Leu Leu Gly Trp Leu
               20                  25                  30
   Trp Gly Ile Asp Ile Asp Pro Tyr Lys Glu Phe Gly Ala Thr Val Glu
           35                  40                  45
   Leu Leu Ser Phe Leu Pro Ser Asp Phe Phe Pro Ser Val Arg Asp Leu
       50                  55                  60
   Leu Asp Thr Ala Ser Ala Leu Tyr Arg Glu Ala Leu Glu Ser Pro Glu
   65                  70                  75                  80
   His Cys Ser Pro His His Thr Ala Leu Arg Gln Ala Ile Leu Cys Trp
                   85                  90                  95
   Gly Glu Leu Met Thr Leu Ala Thr Trp Val Gly Val Asn Leu Glu Asp
               100                 105                 110
   Pro Ala Ser Arg Asp Leu Val Val Ser Tyr Val Asn Thr Asn Met Gly
           115                 120                 125
   Leu Lys Phe Arg Gln Leu Leu Trp Phe His Ile Ser Cys Leu Thr Phe
       130                 135                 140
   Gly Arg Glu Thr Val Ile Glu Tyr Leu Val Ser Phe Gly Val Trp Ile
   145                 150                 155                 160
   Arg Thr Pro Pro Ala Tyr Arg Pro Pro Asn Ala Pro Ile Leu Ser Thr
                   165                 170                 175
   Leu Pro Glu Thr Thr Val Val Cys
               180
<210> 394
   <211> 18
   <212> PRT
   <213> Artificial Sequence

<220>
   <223> Amplification primer containing a restriction endonuclease site.
<400> 394
   Met Gly Ser Arg Cys Asn Asp Ser Ser Asp Ile Asp Pro Tyr Lys Glu
   1 5 10 15
   Phe Gly
<210> 395
   <211> 59
   <212> DNA
   <213> Artificial Sequence
<220>
   <223> Amplification primer containing a restriction endonuclease site.
<400> 395
   ggcgccatgg ggtctagatg taacgattca agtgacatcg acccttataa agaatttcg
   59
<210> 396
   <211> 16
   <212> PRT
   <213> Artificial Sequence
<220>
   <223> Amplification primer containing a restriction endonuclease site.
<400> 396
   Met Gly Cys Asn Asp Ser Ser Asp Ile Asp Pro Tyr Lys Glu Phe Gly
   1 5 10 15
<210> 397
   <211> 52
   <212> DNA
   <213> Artificial Sequence
<220>
   <223> Amplification primer containing a restriction endonuclease site.
<400> 397
   gcgccatggg gtgtaacgat tcaagtgaca tcgaccctta taaagaattt gg
   52
<210> 398
   <211> 11
   <212> PRT
   <213> Artificial sequence
<220>
   <223> Hbc precore alternative linker
<400> 398
   Glu Leu Leu Gly Trp Leu Trp Gly Ile Asp Ile
   1 5 10
<210> 399
   <211> 14
   <212> PRT
   <213> Hepatitis B virus
<400> 399
   Ser Lys Leu Cys Leu Gly Trp Leu Trp Gly Met Asp Ile Asp
   1 5 10
<210> 400
   <211> 38
   <212> PRT
   <213> Hepatitis B virus
<400> 400
   Met Gly Ile Ser Leu Leu Thr Glu Val Glu Thr Pro Ile Arg Asn Glu
   1 5 10 15
   Trp Gly Cys Arg Cys Asn Asp Ser Ser Asp Glu Leu Leu Gly Trp Leu
   20 25 30
   Trp Gly Ile Asp Ile Asp
   35
<210> 401
   <211> 24
   <212> PRT
   <213> Hepatitis B virus
<400> 401
   Met Ser Leu Leu Thr Glu Val Glu Thr Pro Ile Arg Asn Glu Trp Gly
   1 5 10 15
   Cys Arg Cys Asn Asp Ser Ser Asp
   20
<210> 402
   <211> 27
   <212> PRT
   <213> Hepatitis B virus
<400> 402
   Met Ser Leu Leu Thr Glu Val Glu Thr Pro Ile Arg Asn Glu Trp Gly
   1 5 10 15
   Cys Arg Cys Asn Asp Ser Ser Asp Glu Leu Asp
   20 25
<210> 403
   <211> 27
   <212> PRT
   <213> Hepatitis B virus
<400> 403
   Met Ser Leu Leu Thr Glu Val Glu Thr Pro Ile Arg Asn Glu Trp Gly
   1 5 10 15
   Ser Arg Ser Asn Asp Ser Ser Asp Glu Leu Asp
   20 25
<210> 404
   <211> 27
   <212> PRT
   <213> Artificial sequence
<220>
   <223> Chimera of Hepatitis B virus and Influenza A virus
<400> 404
   Met Ser Leu Leu Thr Glu Val Glu Thr Pro Ile Arg Asn Glu Trp Gly
   1 5 10 15
   Ser Arg Cys Asn Asp Ser Ser Asp Glu Leu Asp
   20 25
<210> 405
   <211> 27
   <212> PRT
   <213> Artificial sequence
<220>
   <223> Chimera of Hepatitis B and Influenza A viruses
<400> 405
   Met Ser Leu Leu Thr Glu Val Glu Thr Pro Ile Arg Asn Glu Trp Gly
   1 5 10 15
   Cys Arg Ser Asn Asp Ser Ser Asp Glu Leu Asp
   20 25
<210> 406
   <211> 52
   <212> PRT
   <213> Artificial sequence

<220>
   <223> Chimera of Hepatitis B and Influenza A viruses
<400> 406
   Met Ser Leu Leu Thr Glu Val Glu Thr Pro Ile Arg Asn Glu Trp Gly
   1               5                   10                  15
   Cys Arg Cys Asn Asp Ser Ser Asp Leu Glu Ser Leu Leu Thr Glu Val
               20                  25                  30
   Glu Thr Pro Ile Arg Asn Glu Trp Gly Cys Arg Cys Asn Asp Ser Ser
           35                  40                  45
   Asp Glu Leu Asp
       50
<210> 407
   <211> 52
   <212> PRT
   <213> Artificial sequence
<220>
   <223> Chimera of Hepatitis B and Influenza A viruses
<400> 407
   Met Ser Leu Leu Thr Glu Val Glu Thr Pro Ile Arg Asn Glu Trp Gly
   1               5                   10                  15
   Ser Arg Ser Asn Asp Ser Ser Asp Leu Glu Ser Leu Leu Thr Glu Val
               20                  25                  30
   Glu Thr Pro Ile Arg Asn Glu Trp Gly Cys Arg Cys Asn Asp Ser Ser
           35                  40                  45
   Asp Glu Leu Asp
       50
<210> 408
   <211> 77
   <212> PRT
   <213> Artificial sequence
<220>
   <223> Chimera of Hepatitis B and Influenza A viruses
<400> 408
   Met Ser Leu Leu Thr Glu Val Glu Thr Pro Ile Arg Asn Glu Trp Gly
   1               5                   10                  15
   Ser Arg Ser Asn Asp Ser Ser Asp Leu Gln Ser Leu Leu Thr Glu Val
               20                  25                  30
   Glu Thr Pro Ile Arg Asn Glu Trp Gly Ser Arg Ser Asn Asp Ser Ser
           35                  40                  45
   Asp Leu Glu Ser Leu Leu Thr Glu Val Glu Thr Pro Ile Arg Asn Glu
       50                  55                  60
   Trp Gly Cys Arg Cys Asn Asp Ser Ser Asp Glu Leu Asp
   65                  70                  75
<210> 409
   <211> 6
   <212> PRT
   <213> Influenza virus
<400> 409
   Met Leu Glu Pro Phe Gln
   1               5
<210> 410
   <211> 6
   <212> PRT
   <213> Influenza virus
<400> 410
   Met Leu Glu Pro Leu Gln
   1               5
<210> 411
   <211> 6
   <212> PRT
   <213> Artificial sequence
<220>
   <223> primer protein seuqence
<400> 411
   Met Asp Ile Asp Pro Tyr
   1               5
<210> 412
   <211> 7
   <212> PRT
   <213> Artificail sequence
<400> 412
   Val Val Thr Thr Glu Pro Leu
   1               5
<210> 413
   <211> 15
   <212> PRT
   <213> Artificial sequence
<220>
   <223> primer protein sequence
<400> 413
   Thr Glu Arg Gly Phe Thr Leu Ser Ser Ile His Phe Trp Leu Leu
   1               5                   10                  15
<210> 414
   <211> 7
   <212> PRT
   <213> Artificial sequence
<220>
   <223> protein primer
<400> 414
   Leu Thr Phe Gly Arg Glu Thr
   1               5
<210> 415
   <211> 13
   <212> PRT
   <213> Artificial sequence
<220>
   <223> protein primer
<400> 415
   Leu Ala Thr His His Pro Ser Ser His Glu Pro Ser Glu
   1               5                   10
<210> 416
   <211> 7
   <212> PRT
   <213> Artificial sequence
<220>
   <223> protein
<400> 416
   Ala Leu Arg Gln Ala Ile Leu
   1 5
<210> 417
   <211> 7
   <212> PRT
   <213> Artificial sequence

<220>
   <223> protein sequence
<400> 417
   Cys Val Val Thr Thr Glu Pro
   1 5
<210> 418
   <211> 15
   <212> PRT
   <213> Artificial sequence
<220>
   <223> protein
<400> 418
   Arg Gly Phe Thr Leu Ser Ser Ile Cys Phe Trp Leu Leu Gln Arg
   1 5 10 15
<210> 419
   <211> 12
   <212> PRT
   <213> Artificial sequence
<220>
   <223> protein primer
<400> 419
   Gly Met Asn Thr Asn Cys Tyr Ser Val Val Leu Asp
   1 5 10
<210> 420
   <211> 15
   <212> PRT
   <213> Artificial sequence
<220>
   <223> protein primer
<400> 420
   Glu Gly Trp Cys Leu Ile Ala Gln Arg Cys Ala Thr His His Pro
   1 5 10 15
<210> 421
   <211> 9
   <212> PRT
   <213> Artificial sequence
<220>
   <223> protein primer
<400> 421
   Trp Gly Glu Leu Met Thr Leu Ala Thr
   1 5
<210> 422
   <211> 13
   <212> PRT
   <213> Artificial sequence
<220>
   <223> protein primer
<400> 422
   Glu Gly Trp Cys Leu Ile Cys Gln Arg Leu Ala Thr His
   1 5 10
<210> 423
   <211> 6
   <212> PRT
   <213> Artificial sequence
<220>
   <223> protein primer
<400> 423
   Leu Gly Met Asn Thr Asn
   1 5
<210> 424
   <211> 13
   <212> PRT
   <213> Artificial sequence
<220>
   <223> protein primer
<400> 424
   Gly Leu Lys Phe Arg Gln Cys Leu Trp Phe His Ile Ser
   1               5                   10
<210> 425
   <211> 9
   <212> PRT
   <213> Artificial sequence
<220>
   <223> protein primer
<400> 425
   Trp Thr Cys Leu Thr Met Leu Glu Gly
   1               5
<210> 426
   <211> 6
   <212> PRT
   <213> Artificial sequence
<220>
   <223> primer protein sequence
<400> 426
   Ala Thr Trp Val Gly Val
   1               5
<210> 427
   <211> 6
   <212> PRT
   <213> Artificial sequence
<220>
   <223> primer protein sequence

<400> 427
   Met Gly Leu Lys Phe Arg
   1               5
<210> 428
   <211> 15
   <212> PRT
   <213> Artificial sequence
<220>
   <223> primer protein sequence
<400> 428
   Glu Gly Cys Cys Leu Ile Ala Gln Arg Leu Ala Thr His His Pro
   1 5 10 15
<210> 429
   <211> 14
   <212> PRT
   <213> Artificial sequence
<220>
   <223> protein primer sequence
<400> 429
   Gly Leu Lys Cys Arg Gln Leu Leu Trp Phe Ser Ala Pro Asp
   1 5 10
<210> 430
   <211> 10
   <212> PRT
   <213> Artificial sequence
<220>
   <223> protein primer sequence
<400> 430
   Ser Ala Pro Asp Asp Glu Cys Asn Val Gly
   1 5 10
<210> 431
   <211> 8
   <212> PRT
   <213> Artificial sequence
<220>
   <223> protein primer sequence
<400> 431
   Ala Ser Cys Asp Leu Val Val Ser
   1 5
<210> 432
   <211> 9
   <212> PRT
   <213> Artificial sequence
<220>
   <223> protein primer sequence
<400> 432
   Ile Gly Asp Glu Leu Asn Val Gly Val
   1 5
<210> 433
   <211> 9
   <212> PRT
   <213> Artificial sequence
<220>
   <223> protein primer sequence
<400> 433
   Ile Gly Asp Glu Cys Asn Val Gly Val
   1 5
<210> 434
   <211> 12
   <212> PRT
   <213> Artificial sequence
<220>
   <223> protein primer sequence
<400> 434
   Gly Ile Gln Lys Glu Leu Pro Ala Ser Arg Asp Leu
   1 5 10
<210> 435
   <211> 12
   <212> PRT
   <213> Artificial sequence
<220>
   <223> protein primer sequence
<400> 435
   Gly Ile Gln Lys Glu Leu Pro Ala Ser Cys Asp Leu
   1 5 10
<210> 436
   <211> 36
   <212> PRT
   <213> Artificial sequence
<220>
   <223> primer protein sequence
<400> 436
   Ile Leu Asp Ala Glu Phe Arg His Asp Ser Gly Tyr Glu Val His His
   1 5 10 15
   Gln Lys Leu Val Phe Phe Ala Glu Asp Val Gly Ser Asn Lys Gly Ala
   20 25 30
   Ile Ile Glu Leu
   35
<210> 437
   <211> 24
   <212> PRT
   <213> Artificial sequence
<220>
   <223> primer protein sequence
<400> 437
   Ile Val Thr Lys Glu Asn Thr Ile Ile Asn Pro Ser Glu Asn Gly Asp
   1 5 10 15
   Thr Ser Thr Asn Gly Ile Glu Leu
   20
<210> 438
   <211> 15
   <212> PRT
   <213> Artificial sequence
<220>
   <223> protein primer sequence

<400> 438
   Ile Val Tyr Gln His Ser His Gly Glu Asp Arg Pro Gly Glu Leu
   1 5 10 15
<210> 439
   <211> 26
   <212> PRT
   <213> Artificial sequence
<220>
   <223> protein primer sequence
<400> 439
   Ile Ser Leu Leu Thr Glu Val Glu Thr Pro Ile Arg Asn Glu Trp Gly
   1 5 10 15
   Ser Arg Ser Asn Asp Ser Ser Asp Glu Leu
   20 25
<210> 440
   <211> 43
   <212> DNA
   <213> Artificial sequence
<220>
   <223> primer
<400> 440
   ccatggacat cgacccttat cgcaatttgg agctactgtg gag
   43
<210> 441
   <211> 44
   <212> DNA
   <213> Artificial sequence
<220>
   <223> primer
<400> 441
   ctccacagta gctccaaatt cgcgataagg gtcgatgtcc atgg
   44
<210> 442
   <211> 37
   <212> DNA
   <213> Artificial sequence
<220>
   <223> primer
<400> 442
   cactaatatg ggcctaaggt tcaggcaact cttgtgg
   37
<210> 443
   <211> 37
   <212> DNA
   <213> Artificial sequence
<220>
   <223> primer
<400> 443
   ccacaagagt tgcctgaacc ttaggcccat attagtg
   37
<210> 444
   <211> 41
   <212> DNA
   <213> Artificial sequence
<220>
   <223> primer
<400> 444
   gccttagagt ctcctgagca ttgttcacct caccatactg c
   41
<210> 445
   <211> 41
   <212> DNA
   <213> Artificial sequence
<220>
   <223> primer
<400> 445
   gcagtatggt gaggtgaaga atgctcagga gactctaagg c
   41
<210> 446
   <211> 44
   <212> DNA
   <213> Artificial sequence
<220>
   <223> primer
<400> 446
   ggcaactctt gtggtttcac atttcttgtc tcattttgga agag
   44
<210> 447
   <211> 45
   <212> DNA
   <213> Artificial sequence
<220>
   <223> primer
<400> 447
   ctcttccaaa agtgagagaa gaaatgtgaa accacaagag ttgcc
   45
<210> 448
   <211> 42
   <212> DNA
   <213> Artificial sequence
<220>
   <223> primer
<400> 448
   cctgggtggg tgttaatttg aaagaagatc cagcgtctag ag
   42
<210> 449
   <211> 42
   <212> DNA
   <213> Artificial sequence
<220>
   <223> primer
<400> 449
   ctctagacgc tggatcttct ttcaaattaa cacccaccca gg
   42
<210> 450
   <211> 24
   <212> PRT
   <213> Artificial sequence
<220>
   <223> peptide conjugate sequence

<400> 450
   Met Ser Leu Leu Thr Glu Val Glu Thr Pro Ile Arg Asn Glu Trp Gly
   1 5 10 15
   Cys Arg Cys Asn Asp Ser Ser Asp
   20
<210> 451
   <211> 20
   <212> PRT
   <213> Yersinia pestis
<400> 451
   Gly Asp Ile Pro Tyr Leu Gly Ala Leu Phe Arg Arg Lys Ser Glu Leu
   1 5 10 15
   Thr Arg Arg Thr
   20
<210> 452
   <211> 24
   <212> PRT
   <213> Influenza
<400> 452
   Met Ser Leu Leu Thr Glu Val Glu Thr Pro Ile Arg Asn Glu Trp Gly
   1 5 10 15
   Cys Arg Cys Asn Asp Ser Ser Asp
   20
<210> 453
   <211> 23
   <212> PRT <213> Influenza
<400> 453
   Ser Leu Leu Thr Glu Val Glu Thr Pro Ile Arg Asn Glu Trp Gly Cys
   1 5 10 15
   Arg Cys Asn Asp Ser Ser Asp
   20
<210> 454
   <211> 24
   <212> PRT
   <213> Influenza
<400> 454
   Met Ser Leu Leu Thr Glu Val Glu Thr Pro Ile Arg Asn Glu Trp Gly
   1               5                   10                  15
   Cys Arg Cys Asn Asp Ser Ser Asp
               20
<210> 455
   <211> 23
   <212> PRT
   <213> Inlfuenza
<400> 455
   Ser Leu Leu Thr Glu Val Glu Thr Pro Ile Arg Asn Glu Trp Gly Cys
   1               5                   10                  15
   Arg Cys Asn Asp Ser Ser Asp
               20

## Description

### TECHNICAL FIELD

The present invention relates to the intersection of the fields of immunology and protein engineering, and particularly to a chimeric hepatitis B virus (HBV) nucleocapsid protein (HBc) that is useful as the immunogen in a vaccine and contains an N-terminal cysteine residue and the replacement of one or both of the cysteine residues that are present in the native sequence at HBc positions 48 and 107.

### BACKGROUND OF THE INVENTION

The family hepadnaviridae are enveloped DNA-containing animal viruses that can cause hepatitis B in humans (HBV). The hepadnavirus family includes hepatitis B viruses of other mammals, e.g., woodchuck (WHV), and ground squirrel (GSHV), and avian viruses found in ducks (DHV) and herons (HeHV). Hepatitis B virus (HBV) used herein refers to a member of the family hepadnaviridae that infects mammals, as compared to a virus that infects an avian host, unless the discussion refers to a specific example of a non-mammalian virus.

The nucleocapsid or core of the mammalian hepatitis B virus (HBV or hepadnavirus) contains a sequence of 183 or 185 amino acid residues, depending on viral subtype, whereas the duck virus capsid contains 262 amino acid residues. Hepatitis B core protein monomers of the several hepadnaviridae self-assemble in infected cells into stable aggregates known as hepatitis B core protein particles (HBc particles). Two three-dimensional structures are reported for HBc particles. A first that comprises a minor population contains 90 copies of the HBc subunit protein as dimers or 180 individual monomeric proteins, and a second, major population that contains 120 copies of the HBc subunit protein as dimers or 240 individual monomeric proteins. These particles are referred to as T = 3 or T = 4 particles, respectively, wherein "T" is the triangulation number. These HBc particles of the human-infecting virus (human virus) are about 30 or 34 nm in diameter, respectively. Pumpens et al. (1995) Intervirology, 38:63-74; and Metzger et al. (1998) J. Gen. Viol., 79:587-590.

Conway et al., (1997) Nature, 386:91-94, describe the structure of human HBc particles at 9 Ångstrom resolution, as determined from cryo-electron micrographs. Bottcher et al. (1997), Nature, 386:88-91, describe the polypeptide folding for the human HBc monomers, and provide an approximate numbering scheme for the amino acid residues at which alpha-helical regions and their linking loop regions form. Zheng et al. (1992), J. Biol. Chem., 267(13):9422-9429 report that core particle formation is not dependent upon the arginine-rich C-terminal domain, the binding of nucleic acids or the formation of disulfide bonds based on their study of mutant proteins lacking one or more cysteines and others' work with C-terminal-truncated proteins [Birnbaum et al., (1990) J. Virol. 64:3319-3330].

The hepatitis B nucleocapsid or viral core protein (HBc) has been disclosed as an immunogenic carrier moiety that stimulates the T cell response of an immunized host animal. See, for example, U.S. Patents No. 4,818,527, No 4,882,145 and No. 5,143,726. A particularly useful application of this carrier is its ability to present foreign or heterologous B cell epitopes at the site of the immunodominant loop that is present at about residue positions 70-90, and more usually recited as about positions 75 through 85 from the amino-terminus (N-terminus) of the protein. Clarke et al. (1991) F. Brown et al. eds., Vaccines 91, Cold Spring Harbor Laboratory, Cold Spring Harbor, New York, pp.313-318.

During viral replication, HBV nucleocapsids associate with the viral RNA pre-genome, the viral reverse transcriptase (Pol), and the terminal protein (derived from Pol) to form replication competent cores. The association between the nucleocapsid and the viral RNA pre-genome is mediated by an arginine-rich domain at the carboxyl-terminus (C-terminus). When expressed in heterologous expression systems, such as *E.coli* where viral RNA pre-genome is absent, the protamine-like C-terminus; i.e., residues at positions 150 through 183, can bind *E.coli* RNA. Zheng et al. (1992) JBC, 267(13): 9422-9429.

HBcAg is a particulate protein derived from the hepatitis B virus that has been proposed as a carrier for heterologous epitopes. The relative immunogenicity of HBsAg (HBs) has been compared with HBcAg (HBc), and the ability of each to evoke immune responses in different genetic backgrounds [Milich et al., Science, (1986) 234(4782): 1398-1401]. These data emphasize the higher immunogenicity of HBc relative to HBs, and the universal responsiveness to HBc, irrespective of genetic background.

For example, HBc is more than 300 times more immunogenic than HBs in BALB/c mice; and, although both B10.S and B10.M mice are non-responders to HBs, every strain tested is responsive to HBc. These results re-emphasize the suitability of HBc as a vaccine carrier and specifically, its superiority over HBs, hence the selection of HBc as opposed to HBs to carry heterologous epitopes.

Another advantage of the HBc carrier is the fact that it may not require potent adjuvants for efficacy. This is due to the high inherent immunogenicity of the particle. A comparison of the immunogenicity of HBc-*P. berghei* particles showed that alum, which is approved for human use, was more effective than either IFA or CFA [Schodel et al., J. Exp. Med., (1994) 180(3): p. 1037-46]. The importance of this observation is highlighted by toxicity problems associated with newer, more complex adjuvants as was recently noted in clinical trials of SKB's candidate malaria vaccine [Stoute et al., N. Engl. J. Med., [1997] 336(2): p. 86-91].

In an application as a vaccine carrier moiety, it is preferable that the HBV nucleocapsids not bind nucleic acid derived from the host. Birnbaum et al. (1990) J. Virol., 64:3319-3330 showed that the protamine-like C-terminal domain of HBV nucleocapsids could be deleted without interfering with the protein's ability to assemble into virus-like particles. It is thus reported that proteins truncated to about position 144, i.e., containing the HBc sequence from position one through about 144, can self-assemble, whereas deletions beyond residue 139 abrogate capsid assembly [Birnbaum et al., (1990) J. Virl., 64: 3319-3330; Seifer et al., (1995) Intervirology, 38:47-62].

Zlotnick et al., (1997) Proc. Natl. Acad. Sci., USA, 94:9556-9561 studied the assembly of full length and truncated HBc proteins in to particles. In addition to discussing full length molecules, those authors reported the preparation of a truncated protein that contained the HBc sequence from position 1 through 149 in which the cysteines at positions 48, 61 and 107 were each replaced by alanines and in which a cysteine residue was added at the C-terminus (position 150). That C-terminal mercaptan was used for linkage to a gold atom cluster for labeling in electron microscopy.

More recently, Metzger et al. (1998) J. Gen. Viol., 79:587-590 reported that the proline at position 138 (Pro-138 or P138) of the human viral sequence is required for particle formation. Those authors also reported that assembly capability of particles truncated at the carboxy-terminus to lengths of 142 and 140 residues was affected, with assembly capability being completely lost with truncations resulting in lengths of 139 and 137 residues.

Several groups have shown that truncated particles exhibit reduced stability relative to standard hepatitis B core particles [Galena et al. (1989) J. Virol., 63:4645-4652; Inada, et al. (1989) Virus Res., 14:27-48], evident by variability in particle sizes and the presence of particle fragments in purified preparations [Maassen et al., (1994) Arch. Virol., 135:131-142]. Thus, prior to the report of Metzger et al., above, Pumpens et al., (1995) Intervirology, 38:63-74 summarized the literature reports by stating that the carboxy-terminal border for HBc sequences required for self-assembly was located between amino acid residues 139 and 144, and that the first two or three amino-terminal residues could be replaced by other sequences, but elimination of four or eleven amino-terminal residues resulted in the complete disappearance of chimeric protein in transformed *E. coli* cells.

Recombinantly-produced hybrid HBc particles bearing internal insertions (referred to in the art as HBc chimeric particles or HBc chimers) of various polypeptide sequences have been prepared by heterologous expression in a wide variety of organisms, including *E. coli, B. subtilis, Vaccinia, Salmonella typhimurium*, *Saccharomyces cerevisiae.* See, for example Pumpens et al. (1995) Intervirology, 38:63-74, and the citations therein that note the work of several research groups.

Such HBc chimers often appear to have a less ordered structure, when analyzed by electron microscopy, compared to particles that lack heterologous epitopes [Schodel et al., (1994) J. Exp. Med., 180:1037-1046]. In some cases the insertion of heterologous epitopes into C-terminally truncated HBc particles has such a dramatic destabilizing affect that hybrid particles cannot be recovered following heterologous expression [Schodel et al. (1994) Infect. Immunol., 62 : 1669-1676]. Thus, many chimeric HBc particles are so unstable that they fall apart during purification to such an extent that they are unrecoverable or they show very poor stability characteristics, making them problematic for vaccine development.

The above Pumpens et al. (1995) *Intervirology,* **38**:63-74 report lists particle-forming chimers in which the inserted polypeptide sequence is at the N-terminus, the C-terminus and between the termini. Insert lengths reported in that article are 24 to 50 residues at the N-terminus, 7 to 43 residues internally, and 11 to 741 residues at the C-terminus.

Kratz et al., (1999) Proc. Natl. Acad. Sci., U.S.A., 96:1915-1920 recently described the *E. coli* expression of chimeric HBc particles comprised of a truncated HBc sequence internally fused to the 238-residue green fluorescent protein (GFP). This chimer contained the inserted GFP sequence flanked by a pair of glycine-rich flexible linker arms replacing amino acid residues 79 and 80 of HBc. Those particles were said to effectively elicit antibodies against native GFP in rabbits as host animals.

U.S. Patent No. 5,990,085 describes two fusion proteins formed from an antigenic bovine inhibin peptide fused into (i) the immunodominant loop between residues 78 and 79 and (ii) after residue 144 of carboxy-terminal truncated HBc. Expressed fusion proteins were said to induce the production of anti-inhibin antibodies when administered in a host animal. The titers thirty days after immunization reported in that patent are relatively low, being 1:3000-15,000 for the fusion protein with the loop insertion and 1:100-125 for the insertion after residue 144.

U.S. Patent No. 6,231,864 teaches the preparation and use of a strategically modified hepatitis B core protein that is linked to a hapten. The modified core protein contains an insert of one to about 40 residues in length that contains a chemically-reactive amino acid residue to which the hapten is pendently linked.

Recently published WO 01/27281 teaches that the immune response to HBc can be changed from a Th1 response to a Th2 response by the presence or absence, respectively, of the C-terminal cysteine-containing sequence of the native molecule. That disclosure also opines that disulfide formation by C-terminal cysteines could help to stabilize the particles. The presence of several residues of the native HBc sequence immediately upstream of the C-terminal cysteine was said to be preferred, but not required. One such alternative that might be used to replace a truncated C-terminal HBc sequence was said to include a C-terminal cysteine and an optional sequence that defines an epitope from a protein other than HBc.

Published PCT application WO 01/98333 teaches the deletion of one or more of the four arginine repeats present at the C-terminus of native HBc, while maintaining the C-terminal cysteine residue. That application also teaches that the deleted region can be replaced by an epitope from a protein other than HBc so that the HBc portion of the molecule so formed acts as a carrier for the added epitope.

Published PCT applications corresponding to WO 02/13765 A2 and WO 02/14478 A2 of one of the present inventors teach that stabilization of C-terminally truncated HBc particles can be achieved through the use of one or more added cysteine residues in the chimer proteins from which the particles are assembled. Those added cysteine residues are taught to be at or near the C-terminus of the chimeric protein.

A structural feature whereby the stability of full-length HBc particles could be retained, while abrogating the nucleic acid binding ability of full-length HBc particles, would be highly beneficial in vaccine development using the hepadnaviral nucleocapsid delivery system. Indeed, Ulrich et al. in their recent review of the use of HBc chimers as carriers for foreign epitopes *[*Adv. Virus Res., 50: 141-182 (1998) Academic Press] note three potential problems to be solved for use of those chimers in human vaccines. A first potential problem is the inadvertent transfer of nucleic acids in a chimer vaccine to an immunized host. A second potential problem is interference from preexisting immunity to HBc. A third possible problem relates to the requirement of reproducible preparation of intact chimer particles that can also withstand long-term storage.

The above four published PCT applications appear to contain teachings that can be used to overcome the potential problems disclosed by Ulrich et al. As disclosed hereinafter, the present invention provides another HBc chimer that provides unexpectedly high titers of antibodies, and in one aspect also provides a solution to the problems of HBc chimer stability as well as the substantial absence of nucleic acid binding ability of the construct. In addition, a contemplated recombinant chimer exhibits minimal, if any, antigenicity toward preexisting anti-HBc antibodies.

The above particle instability findings related to N-terminal truncated HBc chimer molecules notwithstanding, Neirynck et al., (October 1999) Nature Med., 5(10):1157-1163 reported that particle formation occurred in E. coli expression of a HBc chimer that contained the N-terminal 24-residue portion of the influenza M2 protein fused at residue 5 to full length HBc. It is noted that residue 4 of HBc is identical to residue 24 of the M2 sequence so it can also be said that those workers deleted residues 1-3.

The previously discussed use of hybrid HBc proteins with truncated C-termini for vaccine applications offers several advantages over their full-length counterparts, including enhanced expression levels and lack of bound *E.coli* RNA. However, C-terminally truncated particles engineered to display heterologous epitopes are often unstable, resulting in particles that either fail to associate into stable particulate structures following expression, or that readily dissociate into.non-particulate structures during and/or following purification. Such a lack of stability is exhibited by particles comprised of chimeric HBc molecules that are C-terminally truncated to HBc position 149 and also contain residues inserted into the immunogenic loop at residue positions 70 through 80.

Others have reported that in wild type hepadnaviral core antigens a cysteine residue upstream of the HBcAg start codon is directly involved in the prevention of particle formation [Schodel et al. (Jan. 15, 1993) J. Biol. Chem., 268(2):1332-1337; Wasenauer et al. (Mar. 1993) J. Virol., 67(3):1315-1322; and Nassal et al. (Jul. 1993) J. Virol., 67(7):4307-4315]. All three groups reported that in wild type HBeAg, the cysteine residue at position -7 of the pre-core sequence, which is present when the core gene is translated from an upstream initiator methionine at position -30, is responsible for preventing particle formation and therefore facilitating the transition from particulate HBcAg to secreted, non-particulate HBeAg.

Bachmann and co-workers [Jegerlehner et al., (2002) Vaccine, 20:3104-3112] compared a fusion construct substantially identical to that of Neirynck et al. above, with a coupled construct similar to that disclosed in U.S. No. 6,231,864 to one o the present inventors in which the external 23-residues of the M2 protein of influenza A was coupled via a linker to a lysine residue engineered into the loop of a C-terminally truncated HBc (1-149). In that conjugate, the residues of HBc loop positions 79 and 80 (proline and alanine) were replaced by a pentapeptide containing two glycine residues on either side of a lysine residue. That chimer of Bachmann et al. also had the cysteine residues at positions 48 and 107 replaced by serine residues. The authors' results after immunizations indicated an increase in anti-M2 titers and enhanced survival (6/6 vs. 0/3) for the coupled construct over the N-terminal fusion protein.

Those chimer proteins were said to form particles and to be capable of being linked to a polypeptide or protein hapten. The *E. coli*-expressed particles were purified by gel filtration and hydroxyapatite column. Correct assembly of the particles was said to be evidenced by the presence of single bands on agarose gel electrophoresis using ethidium bromide or Coomassie blue staining. However, examination of the provided figures appears to show at least two smears for the modified HBc chimers as compared to the native HBC particles. Each of the SDS-PAGE gels shown in the article was conducted under reducing conditions so that only substituted and unsubstituted chimer monomers were visible, as compared to assembled particles and monomers.

As disclosed hereinafter, the present invention provides one solution to the problems of HBC chimer stability as well as the substantial absence of nucleic acid binding ability of the construct, while providing powerfully immunogenic materials.

### BRIEF SUMMARY OF THE INVENTION

The present Invention contemplates an immunogen for a vaccine or inoculum comprised of a recombinantly engineered hepadnavirus nucleocapsid protein; i.e., a hepatitis B core (HBc) chimeric protein [also referred to herein as a recombinant chimer HBc protein molecule, a chimer hepatitis B core protein molecule, a HBc chimer molecule or just a chimer] that self-assembles into particles after expression by a host cell, as defined in claim 1. A contemplated chimer molecule can be truncated at least at the C-terminus relative to a native core molecule that usually terminates at about residue position 183.

The chimer of the invention is a recombinant HBc protein molecule up to 380 amino acid residues in length. That chimer contains the HBc sequence of residue positions 4 through 75 and 85 through 140 in which one or preferably both cysteine residues at positions 48 and 107 is replaced by another residue such as serine. The chimer includes up to all of the residues of the sequence of positions 76 through 05 present and peptide-bonded to up to 75, and preferably one to 45, amino acid residues that are heterologous to HBc and constitute an immunogen, an anti-antigen or a chemically-reactive linker residue for a conjugated hapten that is present in a sequence of one to 40 amino acid residues.

Additionally, the chimer molecule contains (b) one to three cysteine residues present (i) at an amino acid position of the chimer molecule corresponding to amino acid position -20 to about +1, from the N-terminus of the HBc sequence of SEQ ID NO:1 [N-terminal cysteine residue (s)] in a sequence other than that of the HBo precore sequence, and, optionaly (ii) toward the C-terminus of the molecule from the C-terminal residue of the HBc sequence and within about 30 residues from the C-terminus of the chimer molecule. [C-terminal cysteine residue(s)].

Such a contemplated chimer molecule
(c) contains up to about 20 percent substitute amino acid residues in the HBc sequence, and
(d) self-assembles on expression by a host cell into particles that upon collection, purification and dissolution, exhibit a ratio of absorbance at 280 nm to 260 nm of 0.9 to about 1.7, and more preferably about 1.2 to about 1.7, as discussed hereinafter.

Those self-assembled particles, are typically more stable than are particles formed from otherwise identical HBc chimer molecules that contain both cysteine residues at positions 48 and 107 after storage at 37° C in a 20 mM sodium phosphate buffer at pH 6.8 for a time period of one month. Thus, the absence of one or, more preferably, both cysteines at residue positions 48 and 107 can enhance the storage stability of a particle that is otherwise stabilized by the presence of an N- or C-terminal cysteine or both. The replacement of one or both of those cysteine residues can also enhance Expression of the chimer molecule particles.

A recombinant hepatitis B virus core (HBc) protein chimer molecule contains four peptide-linked amino acid residue sequence domains from the N-terminus that are denominated Domains I, II, III and IV, discussed further below.

Looking generally, usually present HBc cysteine residues at positions 48 and 107 are replaced by other residues such as serine in all contemplated chimer molecules and all contemplated chimer molecules contain at least one N- or C-terminal cysteine residue that is not native to the HBc sequence. Thus, in some embodiments, it is preferred that the BBc sequence of Domain T include the residues of position 4 through position 75 alone plus at least an N-terminal Cysteine residue, In other embodiments, it is preferred that a contemplated chimer molecule contain not only an N-terminal cysteine residue, but also contain one cysteine residue within Domain IV as noted above that is alone; i.e., added to the HBc sequence or within another amino acid residue sequence.

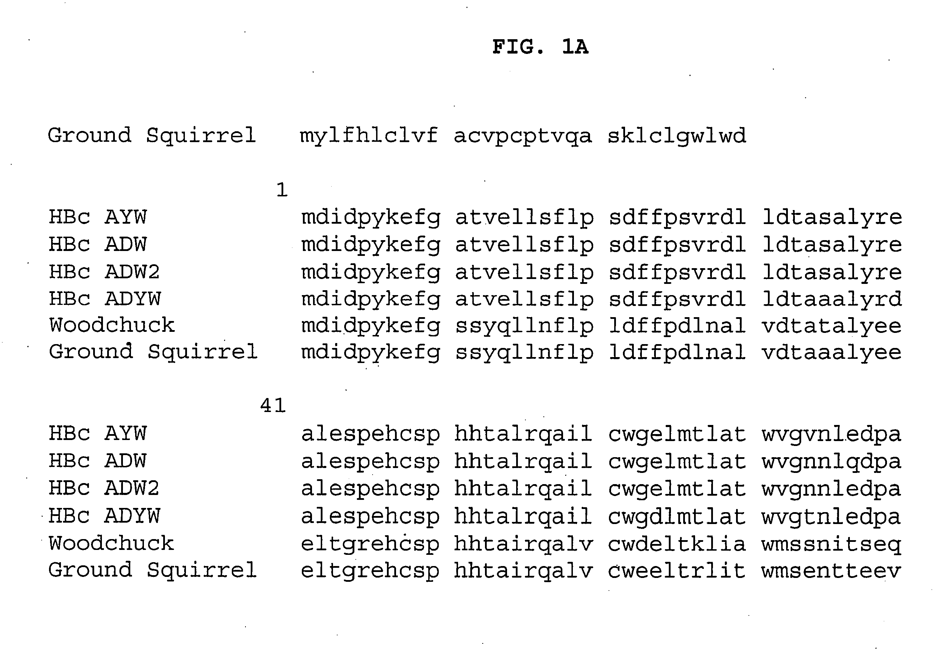

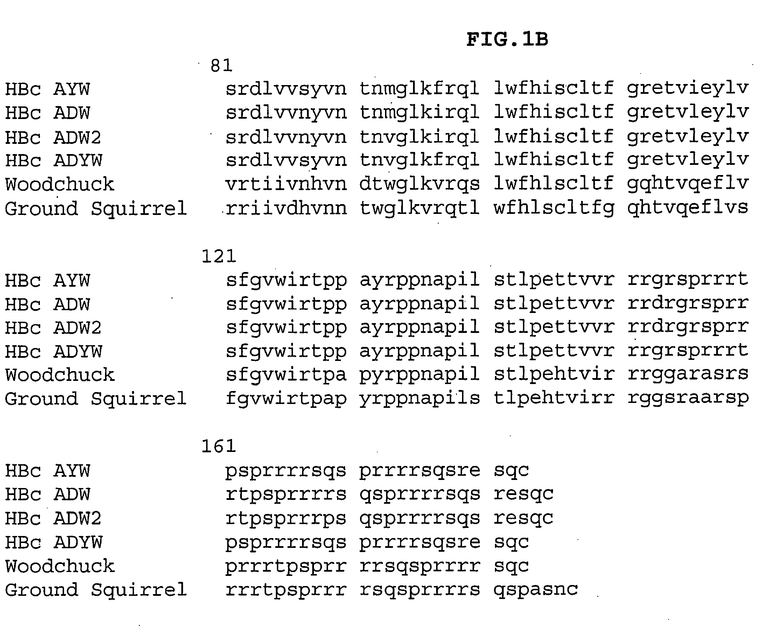

An HBc cysteine residue is present at about position 61 in each of the HBc sequences of Fig. 1.

A contemplated vaccine or inoculum comprises an immune response-inducing amount of before-mentioned self-assembled chimer molecule particles dissolved or dispersed in a pharmaceutically acceptable diluent composition that typically also contains water. A particularly preferred non-HBc epitope present in a contemplated chimer molecule at one or more of Domains I, II and IV is an immunogenic sequence from the malaria CS protein, a sequence from the amino-terminal 24 residues of the influenza A M2 protein, a sequence from the preS1 or preS2 regions of the hepatitis B surface protein (HBs) or an additional T cell-stimulating sequence from HBc as is disclosed in U.S. Patent No. 5,124,726 (and its parent No. 4,882,145) to Thornton et al. such as the sequence of positions about 85 to about 100 or the narrower range of about position 93 to about position 100. A particularly preferred linker residue is a lysine residue.

The present invention has several benefits and advantages.

A particular benefit of the invention is that a preferred chimer particle immunogen typically exhibits greater stability upon preparation than do otherwise identical HBc chimer particles that include the cysteine residues replaced at positions 48 and/or 107, while being substantially free of nucleic acids.

An advantage of the invention is that particles formed from chimers containing residues other than cysteine at positions 48 and 107 form disulfide-containing particles more rapidly than do particles assembled from chimer proteins containing cysteines at positions 48 and 107.

Another benefit of the invention is that the recombinant immunogen is prepared easily and using well-known cell culture techniques.

Another advantage of the invention is that the immunogen is easily prepared using well-known recombinant techniques.

A further benefit of the invention is that the expression of particles is enhanced as compared to similar chimer molecules that include both cysteines at positions 48 and 107.

A further advantage of the invention is that the expressed particles are more readily characterized such as for quality assurance purposes than are particles that contain cysteines at positions 48 and 107.

Still further benefits and advantages will be apparent to the worker of ordinary skill from the disclosure that follows.

### BRIEF DESCRIPTION OF THE DRAWINGS

In the drawings forming a portion of this disclosure
Fig.1, shown in two panels as Fig. 1A and Fig. 1B, provides an alignment of six published sequences for mammalian HBc proteins from six viruses. The first (SEQ ID NO:1), human viral sequence is of the ayw subtype and was published in Galibert et al. (1983) Nature, 281:646-650; the second human viral sequence (SEQ ID NO:2), of the *adw* subtype, was published by Ono et al. (1983) Nucleic Acids Res., 11(6): 1747-1757; the third human viral sequence (SEQ ID NO:3), is of the *adw2* subtype and was published by Valenzuela et al., Animal Virus Genetics, Field et al. eds., Academic Press, New York (1980)pages 57-70; the fourth human viral sequence (SEQ ID NO:4), is of the *adyw* subtype that was published by Pasek et al. (1979) Nature, 282:575-579; the fifth sequence (SEQ ID NO:5), is that of the woodchuck virus that was published by Galibert et al. (1982) J. Virol., 41:51-65; and the sixth mammalian sequence, (SEQ ID NO:6), is that of the ground squirrel that was published by Seeger et al. (1984) J. Virol., 51:367-375.

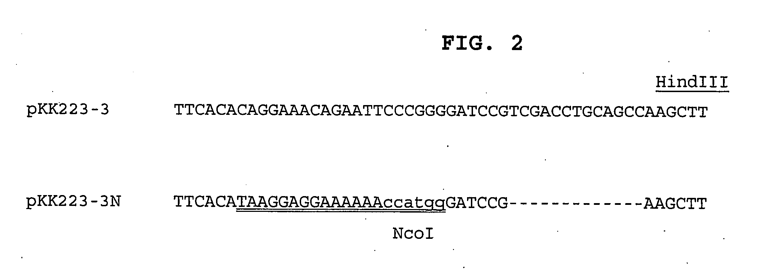

Fig. 2 shows the modifications made to commercial plasmid vector pKK223-3 in the preparation of plasmid vector pKK223-3N used herein for preparation of recombinant HBc chimers. The modified sequence (SEQ ID NO:7) is shown below the sequence of the commercially available vector (SEQ ID NO:8). The bases of the added NcoI site are shown in lower case letters and the added bases are shown with double underlines, whereas the deleted bases are shown as dashes. The two restriction sites present in this segment of the sequence (NcoI and HindIII) are indicated.

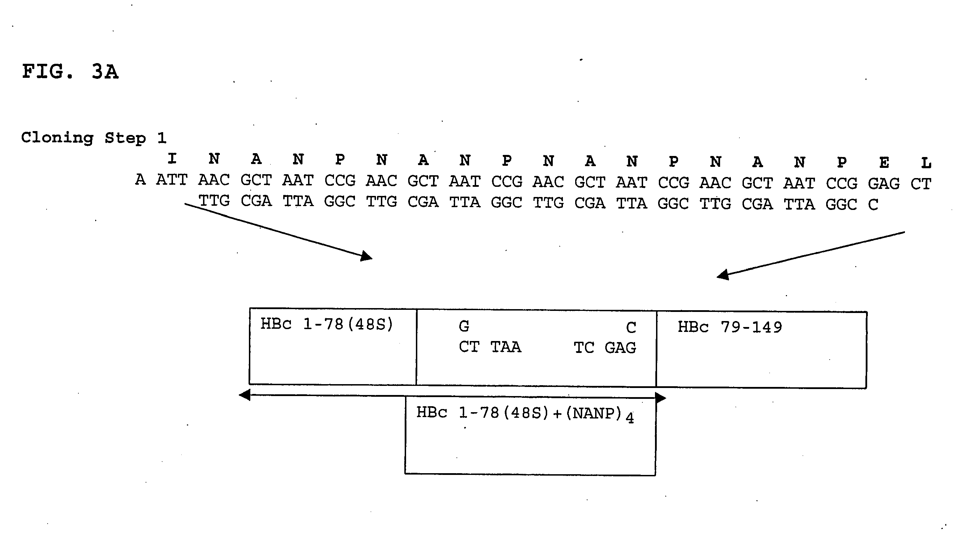

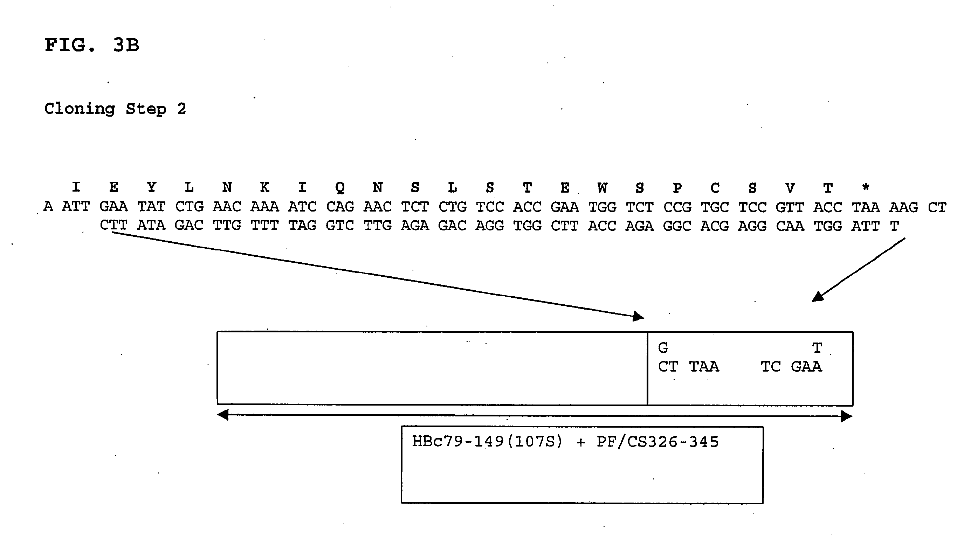

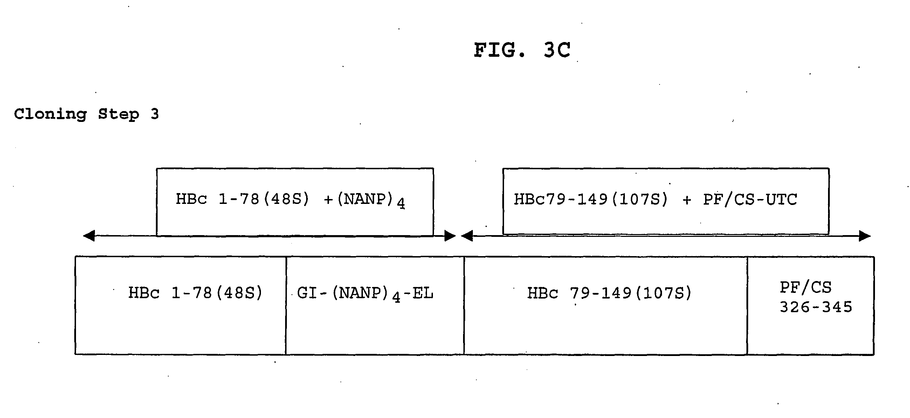

Fig. 3, shown in three panels as Figs. 3A, 3B and 3C, schematically illustrates a preferred cloning strategy in which a malarial B cell epitope such as (NANP)₄ (SEQ ID NO:9) is cloned into the EcoRI and SacI sites between positions 78 and 79 of an engineered HBc gene in which cysteine residue 48 is replaced by a serine residue [Fig. 3A, HBc 1-78(48S)], which destroys the EcoRI site, while preserving the SacI site. Fig. 3B shows DNA that encodes a T cell epitope such as that referred to as Pf/CS-UTC and a stop codon (SEQ ID NO:10) cloned into the EcoRI and HindIII sites at the C-terminus of an engineered, truncated HBc gene that encodes HBc residues 79-149 with the cysteine at residue position 107 replaced by a serine residue [HBc 79-149(107S) + PF/CS-UTC]. PCR amplification of the construct of Fig. 3B using a primer having a 5'-terminal SacI restriction site adjacent to a HBc-encoding sequence beginning at residue position 79 digestion of the amplified sequence and reaction of the construct of Fig. 3A with SacI, followed by ligation of the appropriate portions is shown in Fig. 3C to form a single gene construct that encodes B cell- and T cell-containing epitopes of an immunogen for a vaccine against *P*. *falciparum.*

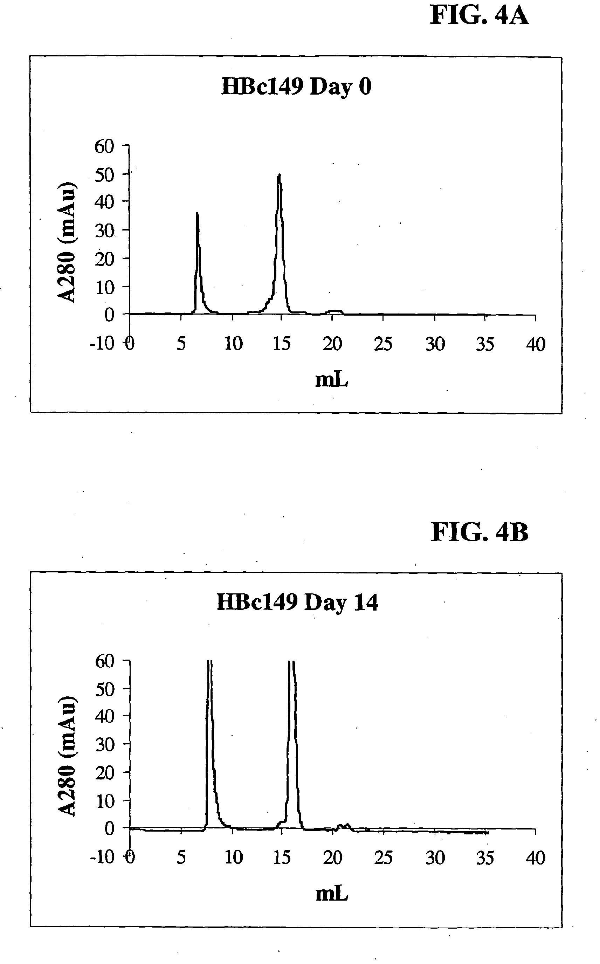

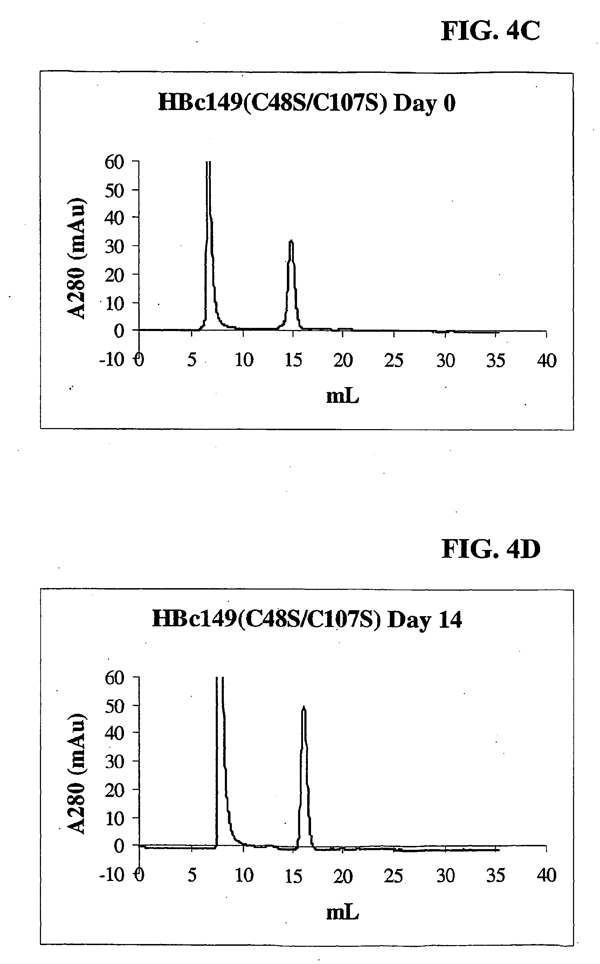

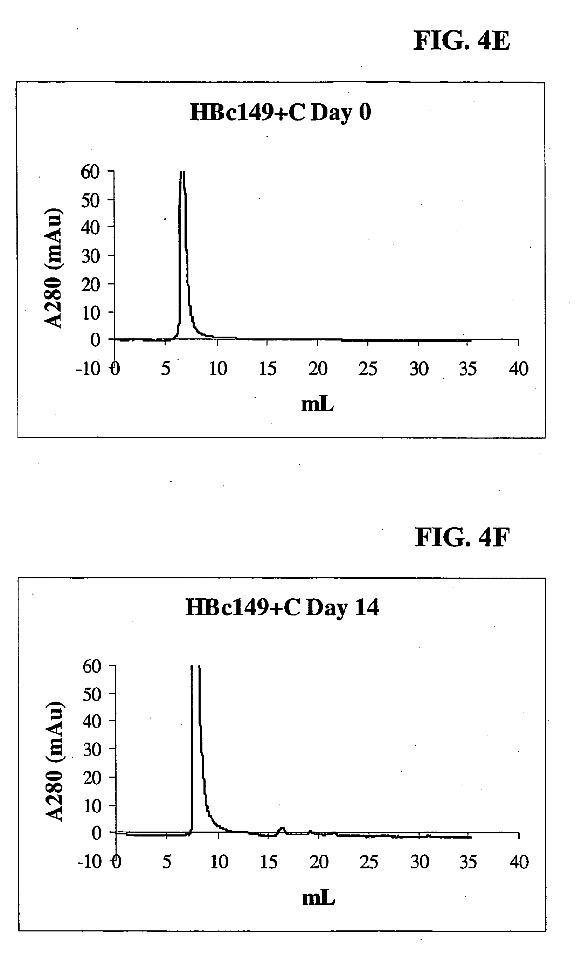

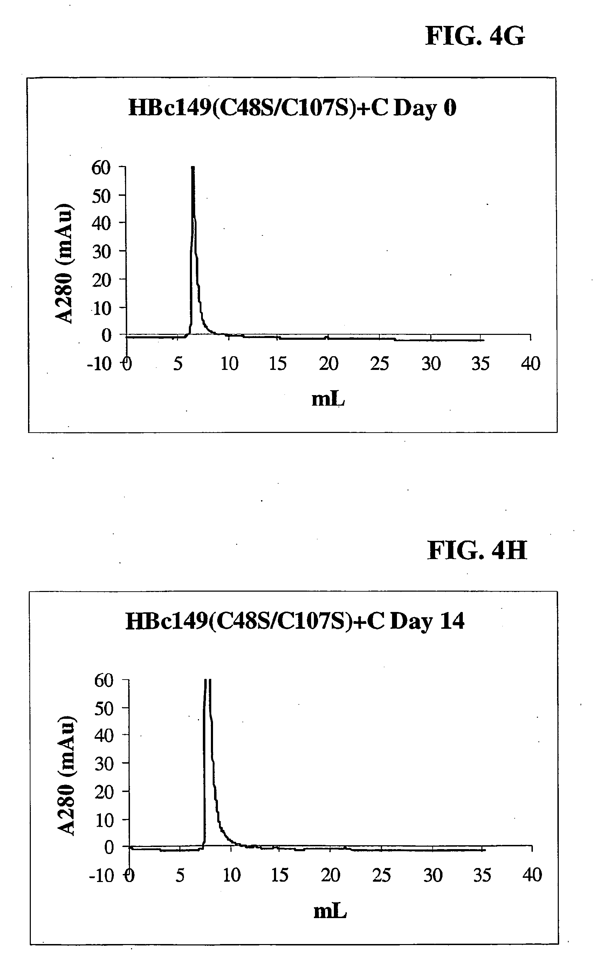

Fig. 4, in eight panels as Figs. 4A-4H, are analytical size exclusion chromatography elution profiles for four particles designated HBc149; HBc149(C48S/C107S); HBc149+C and HBc149(C48S/C107S)+C at days zero (4A, 4C, 4E and 4G) and 14 after incubation at 37°C (4B, 4D, 4F, and 4H), respectively. Assembled particles elute at 7 mL, whereas lower order structures elute in subsequent peaks. Samples were run in 20 mM sodium phosphate, pH 6.8 and 0.02% sodium azide, and absorbance at 280 nm is shown on the ordinate in milliabsorption units (mAu) and volume in milliliters (mL) is shown on the abscissa.

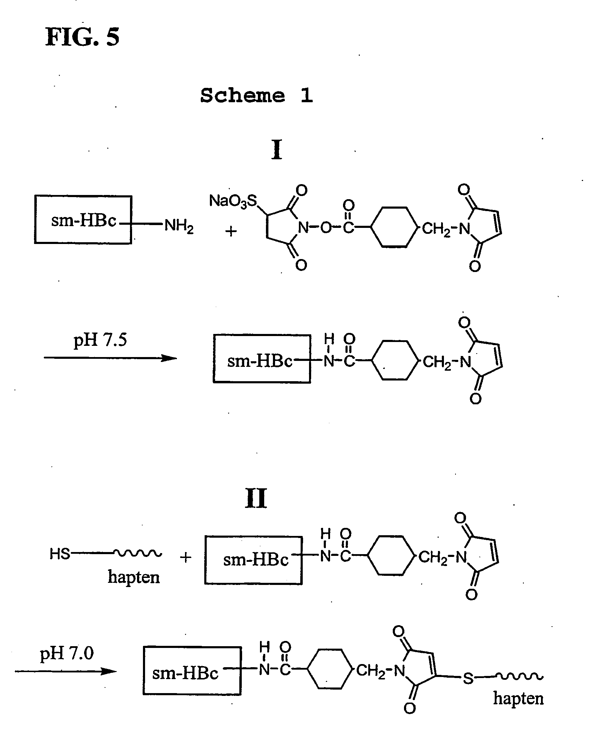

Fig. 5, taken from U.S. Patent No. 6,231,864 illustrates a reaction scheme (Scheme 1) that shows two reaction sequences for (I) forming an activated carrier for pendently linking a hapten to a chimeric hepatitis B core protein (sm-HBc) particle using sulpho-succinimidyl 4-(N-maleimidomethyl)-cyclohexane 1-carboxylate (sulpho-SMCC), and then (II) linking a sulfhydryl-terminated (cysteine-terminated) hapten to the activated carrier to form a conjugate particle. The sm-HBc particle is depicted as a box having a single pendent amino group (for purposes of clarity of the figure), whereas the sulfhydryl-terminated hapten is depicted as a line terminated with an SH group.

### Definitions

Numerals utilized in conjunction with HBc chimers indicate the position in the HBc ayw amino acid residue sequence of SEQ ID NO:1 at which one or more residues has been added to or deleted from the sequence, regardless of whether additions or deletions to the amino acid residue sequence are present. Thus, HBc149 indicates that the chimer ends at residue 149, whereas HBc149 + C150 indicates that that same chimer contains a cysteine residue at HBc position 150 relative to the sequence numbers of SEQ ID NO:1.

The term "antibody" refers to a molecule that is a member of a family of glycosylated proteins called immunoglobulins, which can specifically bind to an antigen.

The word "antigen" has been used historically to designate an entity that is bound by an antibody or receptor, and also to designate the entity that induces the production of the antibody. More current usage limits the meaning of antigen to that entity bound by an antibody or receptor, whereas the word "immunogen" is used for the entity that induces antibody production or binds to the receptor. Where an entity discussed herein is both immunogenic and antigenic, reference to it as either an immunogen or antigen is typically made according to its intended utility.

"Antigenic determinant" refers to the actual structural portion of the antigen that is immunologically bound by an antibody combining site or T-cell receptor. The term is also used interchangeably with "epitope". An antigenic determinant is thus a structure that stimulates antibody production or T cell activation, and the presence of such a structure can be ascertained by determining which structure is bound by antibodies or induces T cell activation.

The word "conjugate" as used herein refers to a hapten operatively linked to a carrier protein, as through an amino acid residue side chain.

The term "conservative substitution" as used herein denotes that one amino acid residue has been replaced by another, biologically similar residue. Examples of conservative substitutions include the substitution of one hydrophobic residue such as isoleucine, valine, leucine or methionine for another, or the substitution of one polar residue for another such as between arginine and lysine, between glutamic and aspartic acids or between glutamine and asparagine and the like. Also included as contemplated "conservative substitutions" are those in which a residue from one mammalian HBc sequence such as those in FIG. 1 is used to replace a residue in the same position in another mammalian HBc sequence.

The term "corresponds" in its various grammatical forms as used in relation to peptide sequences means the peptide sequence described plus or minus up to about three amino acid residues at either or both of the amino- and carboxy-termini and containing only conservative substitutions in particular amino acid residues along the polypeptide sequence.

The term "Domain" is used herein to mean a portion of a recombinant HBc chimer molecule that is identified by (i) residue position numbering relative to the position numbers of HBcAg subtype ayw as reported by Galibert et al., (1979) Nature, 281:646-650 (SEQ ID NO: 1). The polypeptide portions of at least chimer Domains I, II and III are believed to exist in a similar tertiary form to the corresponding sequences of naturally occurring HBcAg.

As used herein, the term "fusion protein" designates a polypeptide that contains at least two amino acid residue sequences not normally found linked together in nature that are operatively linked together end-to-end (head-to-tail) by a peptide bond between their respective carboxy- and amino-terminal amino acid residues. The fusion proteins of the present invention are HBc chimer molecules that induce the production of antibodies that immunoreact with a polypeptide that corresponds in amino acid residue sequence to the polypeptide portion of the fusion protein.

The phrase "hepatitis B" as used here refers in its broadest context to any member of the family of mammalian hepadnaviridae, as discussed before.

The words "polypeptide" and "peptide" are used interchangeably throughout the specification and designate a linear series of amino acid residues connected one to the other by peptide bonds between the alpha-amino and carboxy groups of adjacent amino acids. Polypeptides can be a variety of lengths, either in their neutral (uncharged) forms or in forms that are salts. It is well understood in the art that amino acid residue sequences contain acidic and basic groups, and that the particular ionization state exhibited by the peptide is dependent on the pH value of the surrounding medium when the peptide is in solution, or that of the medium from which it was obtained if the peptide is in solid form. Thus, "polypeptide" or its equivalent terms is intended to include the appropriate amino acid residue sequence referenced. A peptide or polypeptide is always shown herein from left to right and in the direction from amino-terminus (N-terminus) to carboxy-terminus (C-terminus) .

The term "residue" is used interchangeably with the phrase amino acid residue. All amino acid residues identified herein are in the natural or L-configuration. In keeping with standard polypeptide nomenclature, [J. Biol. Chem., 243:3557-59 (1969)], abbreviations for amino acid residues are as shown in the following Table of Correspondence.

| TABLE OF CORRESPONDENCE | | |
|---|---|---|
| 1-Letter | 3-Letter | AMINO ACID |
| Y | Tyr | L-tyrosine |
| G | Gly | glycine |
| F | Phe | L-phenylalanine |
| M | Met | L-methionine |
| A | Ala | L-alanine |
| S | Ser | L-serine |
| I | Ile | L-isoleucine |
| L | Leu | L-leucine |
| T | Thr | L-threonine |
| V | Val | L-valine |
| P | Pro | L-proline |
| K | Lys | L-lysine, |
| H | His | L-histidine |
| Q | Gln | L-glutamine |
| B | Glu | L-glutamic acid |
| Z | Glx | L-glutamic acid or L-glutamine |
| W | Trp | L-tryptophan |
| R | Arg | L-arginine |
| D | Asp | L-aepartic acid |
| N | Asn | L-asparagine |
| B | Asx | L-aspartic acid or L-asparagine |
| C | Cys | L-cysteine |

### DETAILED DESCRIPTION OP THE INVENTION

The present invention contemplates an immunogenic HBc protein, as defined in claim 1. A contemplated chimer molecule is preferably truncated at least at the C-terminus relative to a native core molecule whose C-terminus in normally at residue position 183 for the native ayw subtype of Fig. 1. Particles containing a contemplated chimer molecule are stabilized by replacement of one or both of the cysteine residues that are present in the native HBc protein (Fig. 1) at residue positions 48 and 107 and by the presence of an added cysteine residue that is located at or near one or both of the C- and N-termini. Such particles are preferably substantially free of binding to nucleic acids as is discussed hereinafter.

More particularly, a contemplated chimer molecule has a length of up to 380 residues, and most preferably up to 200 residues. Such a contemplated chimer molecule preferably contains at least 135, of the N-terminal 183 amino acid residues, preferably 163 amino acid residues of HBc, and more preferably of the N-terminal 156 amino acid residues, and most preferably of the N-terminal 149 amino acid residues. The HBc sequence present includes the HBc sequence of residue positions 4 through 75 and 85 through 140 in which one or preferably both cysteine resides at positions 48 and 107 is (are) replaced by another residue.

A Contemplated chimer molecule also contains (a) one to three cysteine residues at an amino acid position of the chimer molecule corresponding to amino acid position -20 to about +1 from the N-terminus of the HBc sequence of SEQ CD NO:1 [N-terminal cysteine residue(s)] in a sequence other than that of the HBc.precore sequence, and, optionally, (b) one to about three cysteine residues toward the C-terminus of the molecule from the C-terminal residue of the HBc sequence and within about 30 residues from the C-terminus of the chimer molecule [C-terminal cysteine residue(s)]. The chimer, molecule (i) contains up to about 20 percent substituted amino acid residues in the HBc sequence, and (ii) self-assembles into particles that are preferably substantially free of binding to nucleic acids on expression by a host cell.

The formed particles exhibit a ratio of absorbance at 280 nm to 260 nm in phosphate buffered saline (PBS), pH 7.4, as discussed hereinafter, of 0.9 to about 1.7, and evidence the substantial freedom from nucleic acid binding by exhibiting a ratio of absorbance at 280 nm to 260 nm in phosphate buffered saline (PBS), pH 7.4 and more preferably of about 1.2 to about 1.7. The formed particles are typically and preferably more stable [after isolation] than are particles formed from otherwise identical HBc chimer molecules that contain both cysteine residues at positions 48 and 107 after storage at 37° C in a 20 mM sodium phosphate buffer at pH 6.8 for a time period of one month. In addition, the chimer optionally contains a peptide-bonded heterologous amino acid residue Sequence at in the HBc immunodominant loop (i.e., between residue positions about 76 through about 85; HBc loop or simply loop), and optionally at the N- and/or C-terminus of the chimer. A heterologous sequence present at the N-terminus of the molecule can have a length of up to 75 amino acid residues, whereas a heterologous sequence present at the C-terminus of the molecule can have a length of up to 100 amino acid residues. When present at either of the N-terminus or C-terminus of the molecule, the heterologous sequence usually contains a B cell or T cell immunogenic epitope, or both, and preferably contains up to 75 amino acid residues.

The HBc immunodominant loop sequence can contain (i) up to all residues in a sequence of HBc positions about 76 through about 85 present and peptide-bonded to one to 75 amino acid residues that are heterologous to the HBc loop and constitute an epitope-containing immunogen, an anti-antigen or a chemically-reactive linker residue for a conjugated hapten present in a sequence of up to 40 residues.

A heterologous amino acid residue sequence of up to 40 residues that includes a linker residue for a conjugated hapten can thus be present in the HBc loop, as can an anti-antigenic residue or sequence. The anti-antigenic residue or sequence, or residue or sequence containing a chemically-reactive linker residue for a conjugated hapten each preferably contain one to 40 amino acid residues, more preferably contain one to five residues, and most preferably contain a single residue. The heterologous epitope-containing immunogen sequence present in the HBc loop preferably contains up to 75 residues and more preferably up to 50 residues, and most preferably up to 25 residues.

The term "anti-antigen" as used herein is intended to mean a residue or sequence that disrupts the antigenicity of a particular sequence; i.e., ability of that sequence to be bound by an antibody. Thus, an anti-antigen can be present in the immunogenic loop, and when present, that residue or sequence disrupts the binding of antibodies to the loop- Those antibodies can be monoclonal or polyclonal, but monoclonal antibodies such as that designated 3105 that is available commercially from the Institute of Immunology Co., Ltd., Tokyo, Japan. That monoclonal antibody is understood to bind to the amino acid residues at the tip of the HBc immunogenic loop. A chemically reactive linker residue for a conjugated hapten can function as an anti-antigen in disrupting antibody binding to the loop. However, for convenience, an anti-antigenic residue or sequence will be deemed herein to be a residue or sequence that lacks a chemically reactive residue such as a lysine or cysteine and functions as an anti-antigen.

In another embodiment, the leucine and arginine residues of HBc positions 76 and 82 are both replaced by cysteine residues and zero to all of the remaining residues in the sequence of HBc positions about 76 through about 85 are present, peptide-bonded to an optionally present sequence of one to about 245 amino acid residues that constitute a heterologous immunogen, an anti-antigenic sequence or a chemically-reactive linker residue for a conjugated hapten present in a sequence of up to about 40 amino acid residues.

In addition to serine, other contemplated replacements for the cysteines at HBc positions 48 and 107 include any residue other than cysteine, including particularly glutamine, asparagine, serine, alanine, threonine and lysine.

The HBc chimer molecule sequence optionally includes (a') a peptide-bonded heterologous amino acid sequence containing an immunogenic sequence at one or more of the N-terminus, and the C-terminus of the chimer,

The chimeric protein molecule also contains (a') one to three cysteine residues at an amino acid position of the chimer molecule corresponding to amino acid position -20 to about +1 from the N-terminus of the HBc sequence of SEQ ID NO:1 [N-terminal cysteine residue(s)] in a sequence other than that of the HBc precore sequence and optionally, (b') one to about three cysteine residues toward the C-terminus of the molecule from the C-terminal residue of the HBc sequence and within about 30 residues from the C-terminus of the chimer molecule [C-terminal cysteine residue(s)].

A chimeric protein molecule contains up to about 20 percent substituted amino acid residues in the HBc sequence, and self-assembles into particles that are preferably substantially free of binding to nucleic acids (exhibit a ratio of absorbance at 280 nm to 260 nm of about 0.9 to about 1.7, and more preferably about 1.2 to about 1.7, as discussed hereinafter) on expression by a host cell. The particles are typically and preferably more stable after storage at 37° C in a 20 mM sodium phosphate buffer at pH 6.8 for a time period of one month than are particles formed from otherwise identical HBc chimer molecules that contain both cysteine residues at positions 48 and 107. This enhanced stability can measured by comparison of areas under peaks observed in analytical size exclusion chromatography or by comparison of non-reducing SDS gel band sizes or protein densities.

The chimeric protein contains one or more N-terminal cysteine residue(s) that can confer further enhanced stability on formation to the self-assembled particles. In another embodiment, the chimeric protein also contains one or more C-terminal cysteine residue (s) that also can confer further enhanced stability on formation to the self-assembled particles. A contemplated chimeric protein molecule can thus contain a cysteine residue at or near both of the N- and C-termini, that is, a chimeric protein molecule can contain both an N-terminal cysteine residue and a C-terminal cysteine residue, as defined previously.

A contemplated chimeric protein can contain the residues from position 150 through 183 of HBc, but is preferably sufficiently free of arginine and or lysine residues downstream of (toward the carboxyl-terminus from) HBc residue position 149 so that the self-assembled particles are substantially free of nucleic acid binding. In some embodiments, the HBc sequence from position 149 through about position 163 that includes two of the arginine-rich repeat sequences is present (See, Fig. 1). In other embodiments, the HBc sequence through about position 156 that contains one arginine-rich sequence is present. In still other embodiments, the C-terminal HBc sequence ends between HBc positions 140 and 149 and the chimer molecule is free of the arginine repeats present in a native HBc sequence of Fig. 1 from position 150 through the C-terminus or a similar sequence containing lysine residues in place of one or more of the arginine residues. Substantial freedom from nucleic acid binding is discussed hereinafter and is readily determined.

For ease of discussion, contemplated chimer sequences and sequence position numbers referred to herein are based on the sequence and position numbering of the human hepatitis B core protein of subtype *ayw* [Galibert et al., (1979) Nature, 281:646-650] that is shown in SEQ ID NO: 1. It is to be understood, however, that in view of the great similarity between the mammalian hepadnavirus capsid protein sequences and similar particle formation exhibited by those proteins, which are well-known to skilled workers, a discussion regarding human HBc subtype *ayw* is also applicable to subtype *adw*, as well as the woodchuck and ground squirrel proteins. As a consequence of those great similarities, HBc sequences are recited generally herein as a "HBc," sequence, unless otherwise stated.

That chimer molecule contains up to about 20 percent substituted amino acid residues in the HBc sequence, and self-assembles into particles that are substantially free of binding to nucleic acids on expression by a host cell. The particles are preferably more stable after storage at 37° C in a 20 mM sodium phosphate buffer at pH 6.8 for a time period of one month than are particles formed from otherwise identical HBc chimer molecules that contain both cysteine residues at positions 48 and 107. A contemplated chimer molecule also preferably exhibits an enhanced amount of expression

A chimeric protein of this embodiment contains a peptide-bonded heterologous amino acid residue sequence in the HBc immunodominant loop (i.e., between residue positions 76 through 85) and optionally at the N- and/or C-terminus of the chimer as discussed before. The HBc immunogenic loop sequence can contain (i) up to all residues in a sequence of HBc positions 76 through 85 present, and peptide-bonded to one to 75, amino acid residues that are heterologous to the HBc loop and constitute an immunogen, or a sequence of up to 40 residues that constitutes a chemically-reactive linker residue for a conjugated hapten.

As noted before, a contemplated chimer molecule contains at least one cysteine residue that is located (i) at a position of about -20 to about +1 relative to the N-terminus of HBc as is illustrated in Fig. 1 and SEQ ID NO: 1 and, optionally, (ii) toward the C-terminus of the molecule from the C-terminal residue of the HBc sequence and within about 30 residues from the C-terminus of the chimer molecule. The concept of a negative amino acid position is usually associated with a leader sequence such as the precore sequence of HBc. That concept is used similarly here in that one can simply align a given chimer molecule sequence with that of SEQ ID NO: 1 to determine the position of the chimer that corresponds to that of the starting methionine residue of position +1 of HBc. Inasmuch as amino acid residue sequences are normally shown from left to right and in the direction from N-terminus to C-terminus, any aligned chimer molecule residue to the left of the position that can be occupied by the HBc start methionine has a negative position. A contemplated cysteine residue can occur at a position about twenty residues to the left of the aligned start methionine of HBc to the position corresponding to that start methionine.

In one preferred embodiment, the sequence of 10 residues of positions 76 through 85 (position 76-85 sequence) is present, but interrupted by one to about 50 residues of the immunogen-, or 1 to about 40 residues of an anti-antigen- or linker-containing sequence. In another embodiment, the 10-mer sequence of HBc positions 76-85 is present with two replacement cysteine residues at HBc positions 76 and 82, and includes an interrupting sequence of up to 50 residues that can be as above.

In one aspect, a contemplated chimer molecule contains a sequence comprising an epitope at the N-terminus peptide-bonded to one of HBc residues 2-4, in addition to an immunogen-, or a linker residue-containing sequence peptide-bonded near the middle of the molecule located between HBc residues 76 and 85 in the immunodominant loop. In a further aspect, an immunogen-containing sequence is located at the C-terminal portion of the chimer molecule peptide-bonded to one of HBc residues 136-149 or one of residues 140-156, or one of residues 140-163.

In yet other aspects, two or three immunogen-containing sequences are present at the above locations, or one or two immunogen-containing sequences are present along with a linker residue for an epitope. The multiple immunogens can be the same at two or three positions, or dimers or trimers of a particular immunogen can be present in one or more positions. Each of those chimer molecules also contains one or both of an N-terminal or C-terminal cysteine residue(s), as discussed before. Specific examples of several of these immunogens, the chimer molecules and their self-assembled particles are discussed hereinafter.

One preferred HBc chimer molecule can contain about 130 to about 355 amino acid residues, contains a substitute residue for either or both of cysteine residues at positions 48 and 107, and at least one added N- or C-terminal stabilizing cysteine residue that is not present in a naturally occurring HBc molecule. In some preferred embodiments, HBc residue 4 is present, whereas residues 2-4 are present in other preferred embodiments, so that Domain I can begin at HBc residue 4, 3 or 2 and continue through residue 75; i.e., the HBc residue at HBc position 75. Residue 1 is methionine, the amino acid of the DNA start codon. Where the chimer contains an N-terminal immunogenic sequence or cysteine residue(s), it is preferred that the native methionine that is normally present at position 1 of HBc be absent so that only one start signal is present in the encoding DNA or RNA.

The heterologous immunogenic immunogen that can be present in Domain I or in the immunodominant loop of Domain II preferably contains about 15 to about 50 residues, although an epitope as short as about 6 amino acid residues can induce and be recognized by antibodies and is therefore useful, and an about 75-mer sequence can be present at the N-terminus in Domain I.

It is preferred that all of the HBc residues of Domain II from position 76 through position 85 are present, although interrupted by one or more other residues. Domain II preferably contains at least four residues, that can have any sequence that does not interfere witch expression or use, but those residues are preferably part of the sequence between the residues of positions 75 and 85. In one embodiment, the residues of loop positions 76 and 82 are mutated and replaced by cysteine residues so that further stabilization of the loop can be achieved.

A Domain IV immunogenic sequence is preferably heterologous to the sequence of HBc from about position 163 to the HBc C-terminus. The immunogenic sequence, when present in Domain IV, is preferably a T cell epitope, but can also be a B cell epitope as are usually present in one or the other of Domains I and II. Illustrative B cell and T cell epitopes from the HBc sequence, from the preS1 and preS2 regions of HBs and other sources are provided in Tables A and B, hereinafter.

Domains IV can also contain zero to three cysteine residues and those Cys residues are present within about 30 residues of the carboxy-terminus (C-terminus) of the chimer molecule. Preferably, one cysteine (Cys) residue is present, and that Cys is preferably present as the carboxy-terminal (c-terminal) residue, unless a T cell epitope is present as part of Domain IV. When such a T cell epitope is present, the preferred Cys is preferably within the C-terminal last five residues of the HBc chimer.

In one embodiment, a particularly preferred chimer contains two immunogenic epitope sequences. Those two immunogenic epitope sequences are present in Domains I and II, or III and IV. One of the two immunogenic epitope sequences is preferably a B cell epitope in some embodiments. In other embodiments, one of the two immunogenic epitope sequences is a T cell epitope. More preferably the two immunogenic epitopes are different B cell and T cell epitopes. In addition, a plurality of B cell epitopes can be present at a B cell epitope location, as can a plurality of T cell epitopes be present at a T cell epitope location.

In the embodiments in which the chimer molecule contains an immunogenic epitope sequence in Domain 11, it is preferred that that the sequence contain one or more B cell epitopes, that the HBc sequence between amino acid residues 76 and 85 be present, but interrupted by the immunogenic epitope(s), and that the chimer further include one or more T cell epitopes in Domain IV peptide-bonded to one of HBc residues 140-156.

This same preference holds for those chimer molecules in which the heterologous linker residue for a conjugated epitope is present in Domain II, thereby providing one or more immunogenic epitopes in Domain II, with residues 76 and 85 present, but interrupted by the heterologous linker residue, with a T cell epitope being present peptide-bonded to one of HBc residues 140-156. The particles formed from such chimer molecules typically contain a ratio of conjugated epitope to C-terminal peptide-bonded T cell epitope of about 1:4 to 1:1, with a ratio of about 1:2 being common.

In an illustrative structure of an above-described chimer molecule, a heterologous linker residue for a conjugated epitope is present in Domain II and a T cell epitope is present in Domain IV, with no additional B cell epitope being present in Domain II. Such a chimer exhibits immunogenicity of the T cell epitope, while exhibiting minimal, HBc antigenicity as measured by binding of anti-loop monoclonal antibodies in an ELISA assay as discussed hereinafter.

A preferred contemplated HBc chimer molecule contains a sequence of about 135 to about 360 residues. A preferred HBc chimer molecule that can contain one or two immunogenic epitopes of preferred lengths of about 15 to about 75 residues each and a preferred HBc portion length of about 140 to about 156 residues has a sequence length of about 170 to about 280 amino acid residues. Particularly preferred chimer molecules that contain one or two immunogenic epitopes have a length of about 190 to about 225 residues. A particularly preferred chimer molecule that is free of added immunogenic epitopes can have a length of about 140 to about 156 residues. It is to be understood that a wide range of chimer molecule lengths is contemplated in view of the variations in length of the N- and C-terminal HBc portions and differing lengths of the several contemplated epitopes that can be inserted in the immunogenic loop.

A contemplated recombinant protein, after Expression by a host cell, self-assembles to form particles that are substantially free of binding to nucleic acids. The contemplated HBc chimer particles are generally spherical in shape and are usually homogeneous in size for a given preparation. These chimeric particles thus resemble native HBc particles that have a similar shape and size and can be recovered from infected persons.

A contemplated chimer particle comprises previously discussed chimer molecule.

A contemplated particle is preferably sufficiently free of arginine and/or lysine residues in Domain IV so that the self-aesembled particles are substantially free of nucleic acid binding and exhibits a 260/260 absorbance ratio of about 0.9 to about 1.7 and more preferably about 1.2 to about 1.7, as discussed hereinafter. Thus, a preferred chimeric protein is free of the HBc sequence between positions 163 and 183, and a more preferred chimer protein is free of the HBc sequence between positions 156 and 183. A particularly preferred HBc chimer molecule contains fewer than nine arginine and lysine residues and mixtures thereof from HBc position 149 through the C-terminus of the chimer molecule.

The substantial freedom of nucleic acid binding exhibited by contemplated particles can be readily determined by a comparison of the absorbance of the particles in aqueous solution measured at both 280 and 260 nm; i.e., a 280/260 absorbance ratio.

The contemplated particles do not bind substantially to nucleic acids that are oligomeric and/or polymeric DNA and RNA species originally present in the cells of the organism used to express the protein. Such nucleic acids exhibit an absorbance at 260 nm and relatively less absorbance at 280 nm, whereas a protein such as a contemplated chimer absorbs relatively less at 260 nm and has a greater absorbance at 280 nm.

Thus, recombinantly expressed full length HBc particles or chimeric HBc particles that contain the arginine-and lysine-rich sequence at residue positions 150-183 (or 150-185) sometimes referred to in the art as the protamine region exhibit a ratio of absorbance at 280 nm to absorbance at 260 nm (280/260 absorbance ratio) of about 0.8. Particles that include the HBc sequence through position 163 exhibit a 280/260 ratio of about 0.9 to about 1.0, and stain positively for nucleic acid with ethidium bromide.

On the other hand, particles sufficiently free of arginine and lysine residues in Domain IV so that the self-assembled particles are substantially free of nucleic acid binding such as particles that are free of the arginine-rich nucleic acid binding region of naturally occurring HBc like as those that contain fewer than three arginine or lysine residues or mixtures thereof adjacent to each other, or those having a native or chimeric sequence that ends at about HBc residue position 140 to position 149, exhibit a 280/260 absorbance ratio of about 1.2 to about 1.7. A more typical 280/260 absorbance ratio is about 1.4 to about 1.7. This range is due in large part to the number of aromatic amino acid residues present in Domains II and IV of a given chimeric HBc particle.

The presence of the above-discussed N-terminal and optional C-terminal cysteine residue (a) provides an enhancement of the ability of the chimer molecules to form stable immunogenic particles (discussed hereinafter). In addition, the replacement of the native cysteines at one or both of positions 48 and 107 enhances the stability of the formed particles in addition to that provided by the N- and/or C-terminal cysteine residue(s). Thus, a contemplated HBc chimer particle immunogen tends to form particles that stay together upon collection and initial purification as measured by analytical size exclusion chromatography and upon analysis by non-reducing SDS-PAGE, whose details are discussed hereinafter.

Contemplated particles are typically and preferably more stable after storage at 37° C in a 20 mM sodium phosphate buffer at pH 6.8 for a time period of one month than are particles formed from otherwise identical HBc Chimer molecules that contain both cysteine residues at positions 48 and 107. Examples of enhanced stabilities are discussed in greater detail in the Examples that follow.

Domain I of a preferred contemplated chimeric HBc protein constitutes an amino acid residue sequence of HBc beginning with at least amino acid residue position 4 through position 75, and Domain III constitutes a HBc sequence from position 86 through position 135. Those Domains include at least one replacement of another residue for a cysteine residue present in a native sequence shown in Fig. 1 at one or both of positions 48 and 107. The sequences from any of the mammalian hepadnaviruses can be used for either of Domains I and III, and sequences from two or more viruses can be used in one chimer, but the noted replacement of one or both cysteine residues at positions 48 and 107 relates to any sequence used. Preferably, and for ease of construction, the human ayw sequence is used through out the chimer.

HBc chimers having a Domain I that contains more than a deletion of the first three amino-terminal (N-terminal) residues have been reported to result in the complete disappearance of HBc chimer protein in E. coli cells. Pumpens et al., (1995) Intervirology, 38:63-74. On the other hand, a recent study in which an immunogenic 23-mer polypeptide from the influenza M2 protein was fused to the HBc N-terminal sequence reported that the resultant fusion protein formed particles when residues 1-3 of the native HBc sequence were replaced. Neirynck et al. (October 1999) Nature Med., 5(10):1157-1163. Thus, the art teaches that particles can form when an added amino acid sequence is present peptide-bonded to one of residues 2-4 of HBc, whereas particles do not form if no additional sequence is present and more than residues 1-3 are deleted from the N-terminus of HBV.

An N-terminal epitope sequence peptide-bonded to one of the first five N-terminal residues of HBc can contain a single cysteine residue or a sequence of up to about 30 residues that comprise an immunogenic sequence. The one to three cysteine residues can be present at a convenient location in the sequence, but are typically near the C-terminus of the added sequence so that the added N-terminal cysteine residue(s) are at a position of about -20 to about +1, and more preferably at a position of about -14 to about +1, relative to the HBc N-terminus as shown in SEQ ID NO: 1. Exemplary sequences include a B cell or T cell epitope such as those discussed and illustrated hereinafter (Tables A and B, respectively), the 23-mer polypeptide from the influenza M2 protein of Neirynck et al., above, that includes two cysteine residues, and variants of that sequence contain at least about 6 residues, a sequence of another (heterologous) protein such as β-galactosidase as can occur in fusion proteins as a result of the expression system used, or another hepatitis B-related sequence such as that from the PreS1 or PreS2 regions or the major HBsAg immunogenic sequence.

The heterologous sequence present in the immunodominant loop can constitute (i) a sequence of up to about 40 residues that contains heterologous linker residue for a epitope such as a B cell or T cell epitope or an anti-antigenic residue or sequence, or (ii) an immunogenic B or T cell epitope that preferably contains 6 to about 50, more preferably about 15 to about 50, and most preferably about 20 to about 30 amino acid residues,. Those one or more residues are positioned so that they are peptide-bonded between zero, or preferably at least 4 and more preferably at least 8 residues, or all of the residues of about positions 76 through 85 of the HBc sequence. Immunogenic B cell epitope sequences are preferably peptide-bonded into the HBc sequence or linked at this position by the linker residue, and use of a B cell epitope is discussed illustratively hereinafter.

Those preferred at least 4 HBc residues that are preferably present in the loop (Domain II) can be all in one sequence such as residues 82-85, or can be split on either side of (flank) the heterologous linker residue(s) as where residues 76-77 and 84-85 are present or where residues 76 and 83-85 are present. More preferably, Domain II contains at least 8 residues of the HBc sequence from residue 76 through 85. Most preferably, the sequence of all 10 residues of positions 76 through 85 is present in the chimer.

The residues added to the HBc loop sequence can be heterologous to a HBc sequence or can correspond to one or more immunogenic portions of the HBc sequence that are other than those of HBc at about positions 76-85. Such a sequence is therefore heterologous to the position of insertion in HBc. A single added heterologous, residue can be a heterologous linker residue for a B cell epitope or an anti-antigen as discussed before. The longer sequences, typically at least 6 amino acid residues long to about 50 amino acid residues long and more preferably about 15 to about 50 residues in length, as noted before, are in a sequence that comprises an immunogen such as a B cell or T cell epitope, except for heterologous residues encoded by restriction sites.

Exemplary peptide B cell epitopes useful for both linkage to the linker residue after expression of a contemplated chimer and for expression within a HBc chimer at one or more of the N-terminus, within the immunogenic loop or at the C-terminus of the chimer are illustrated in Table A, below, along with the common name given to the gene from which the sequence is obtained, the literature or patent citation for published epitopes, and SEQ ID NO.

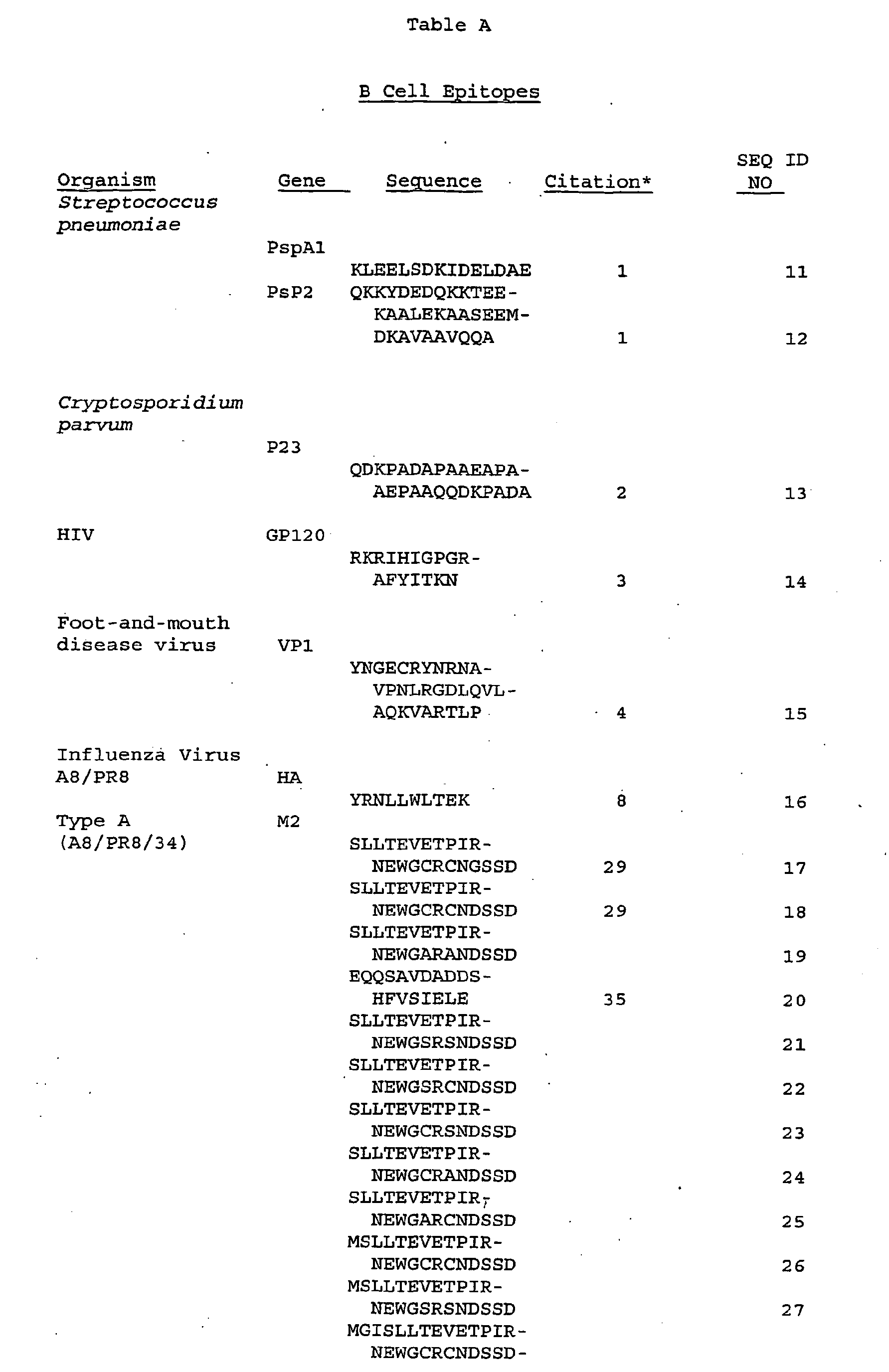

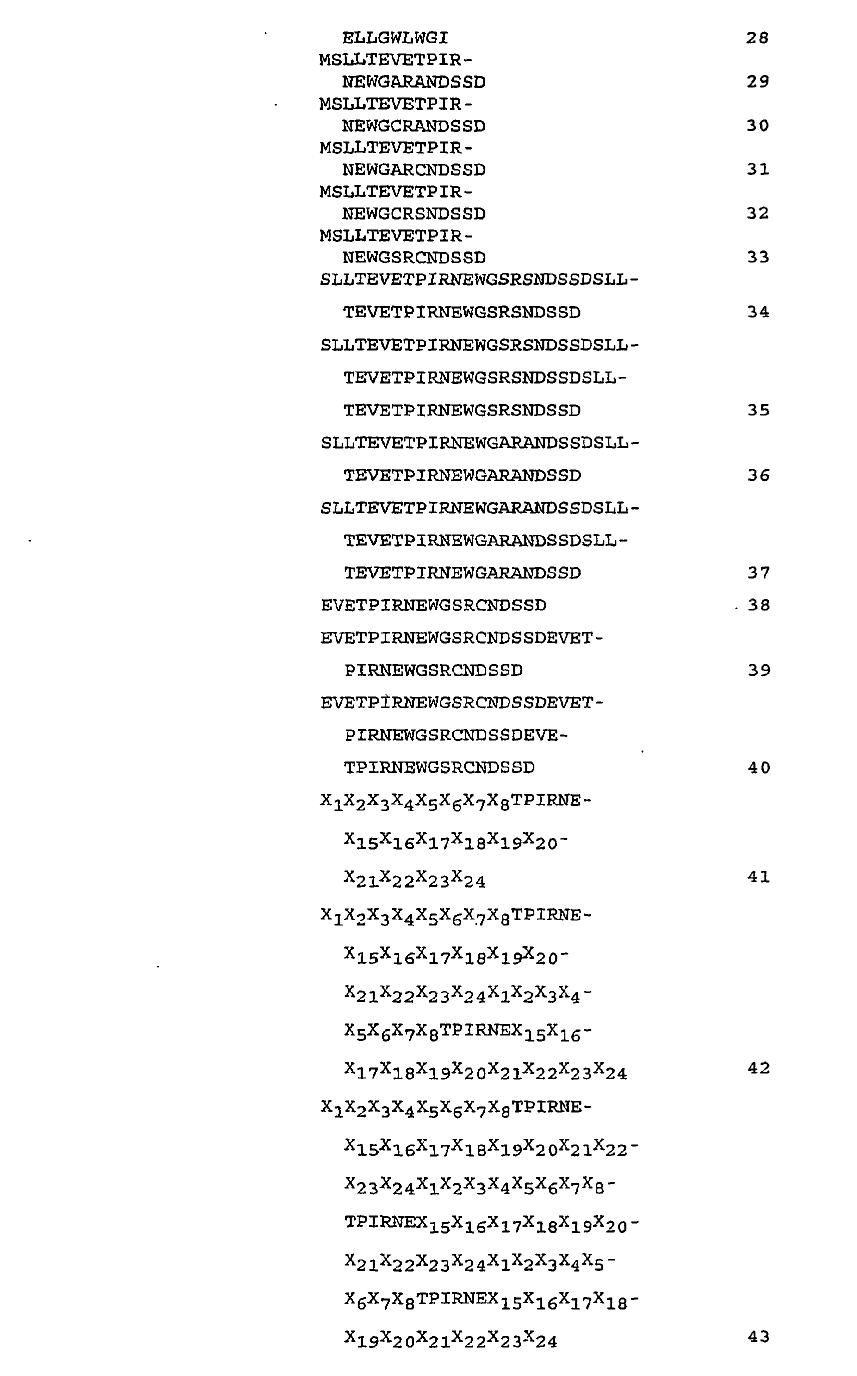

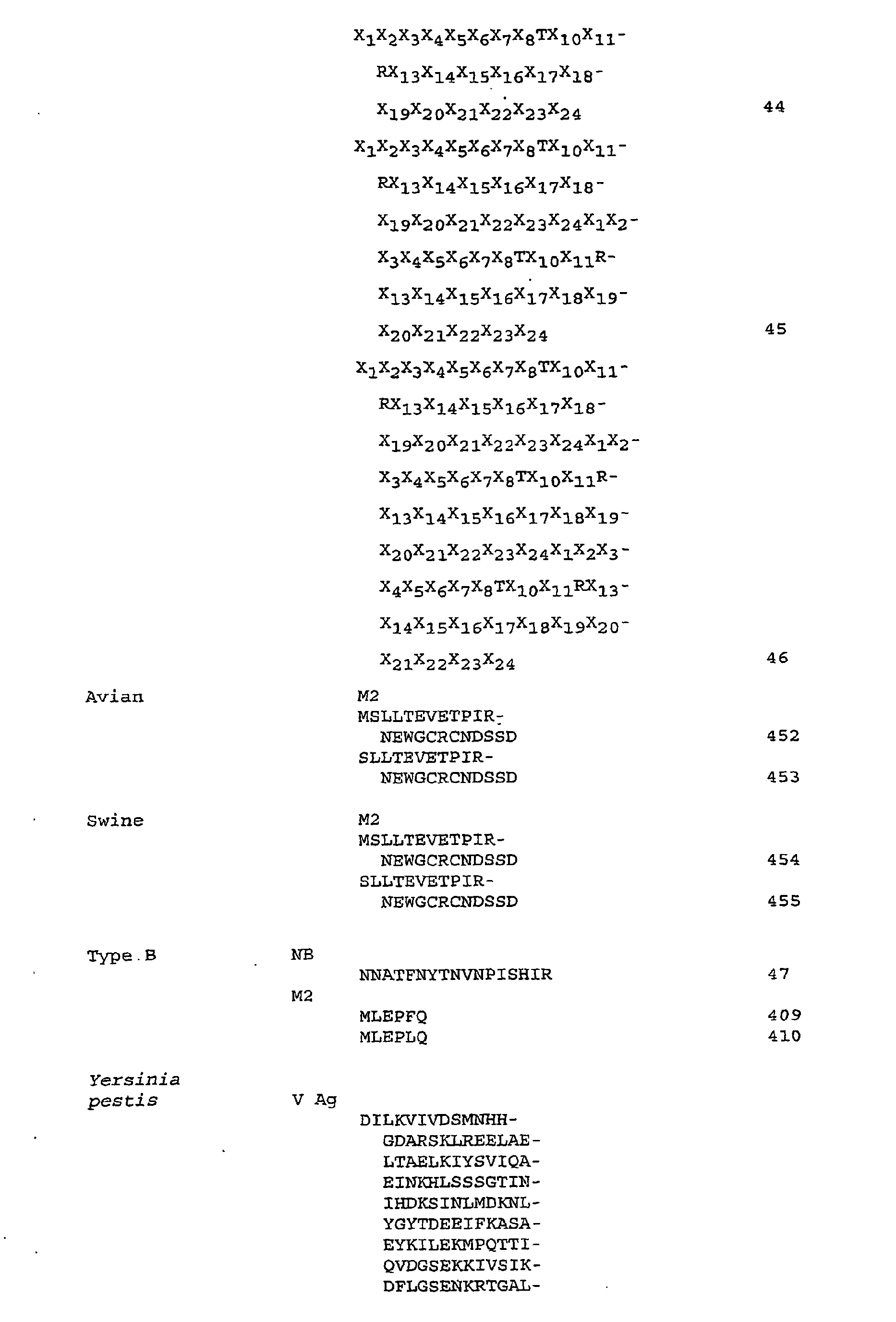

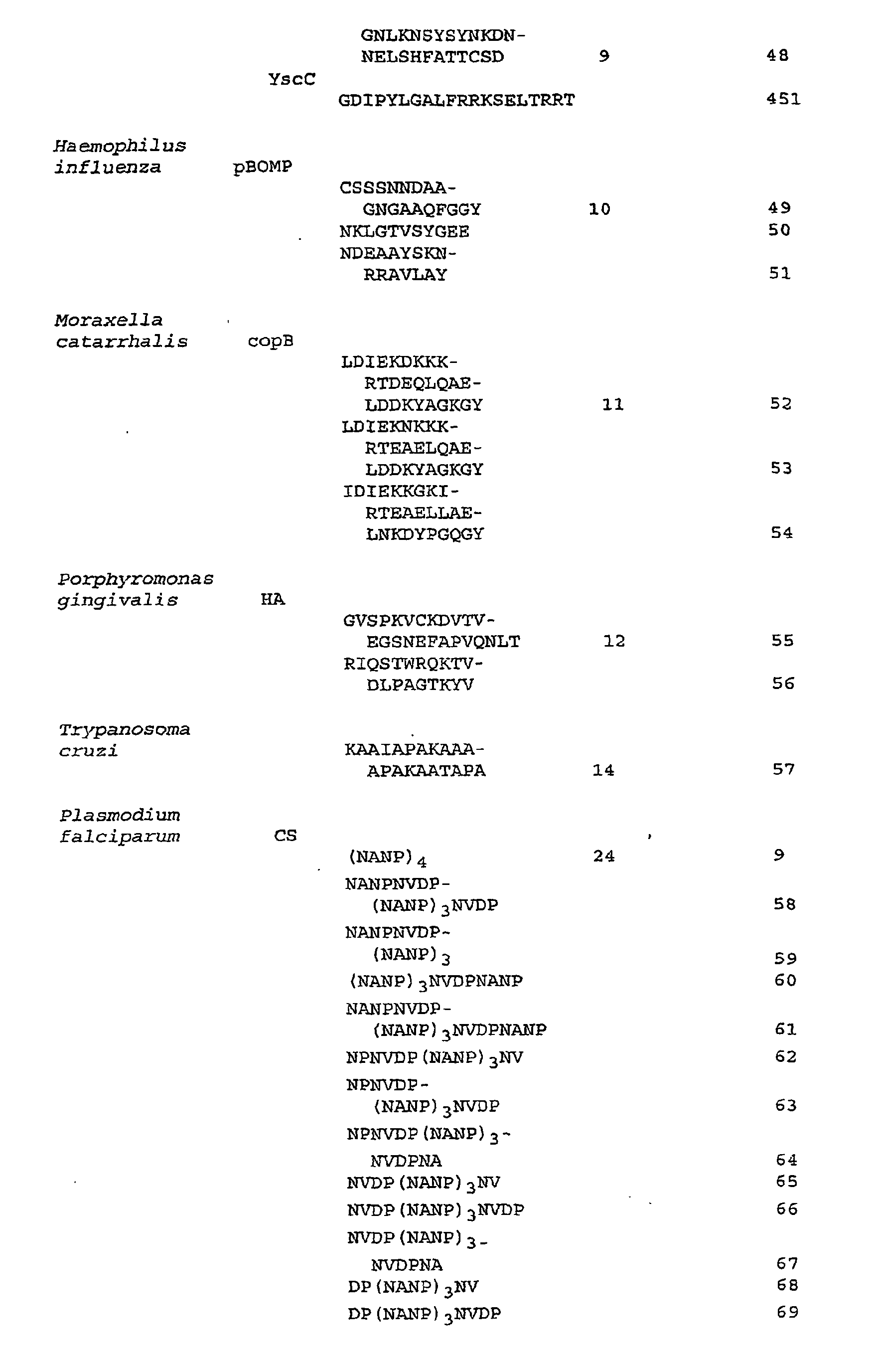

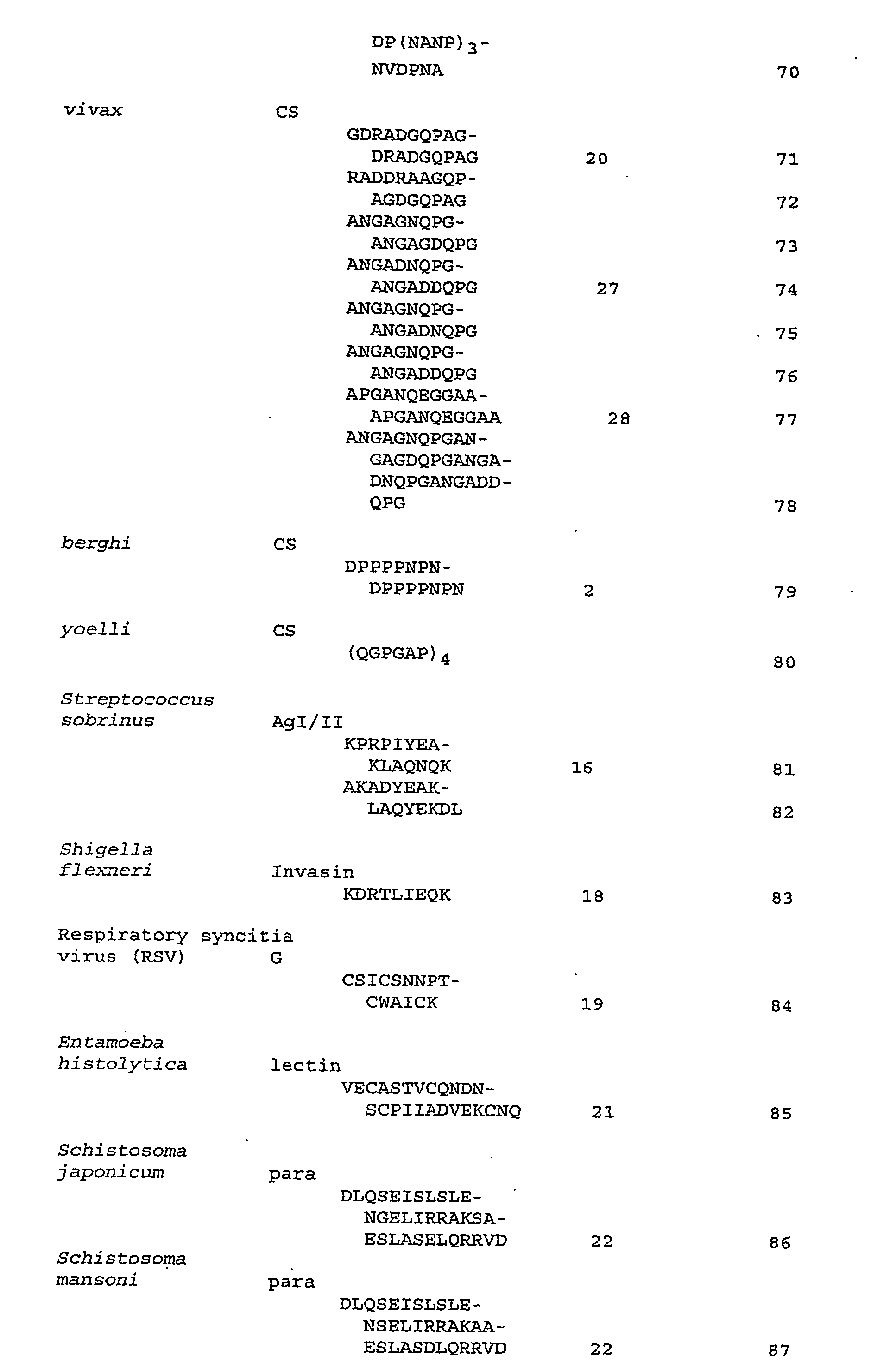

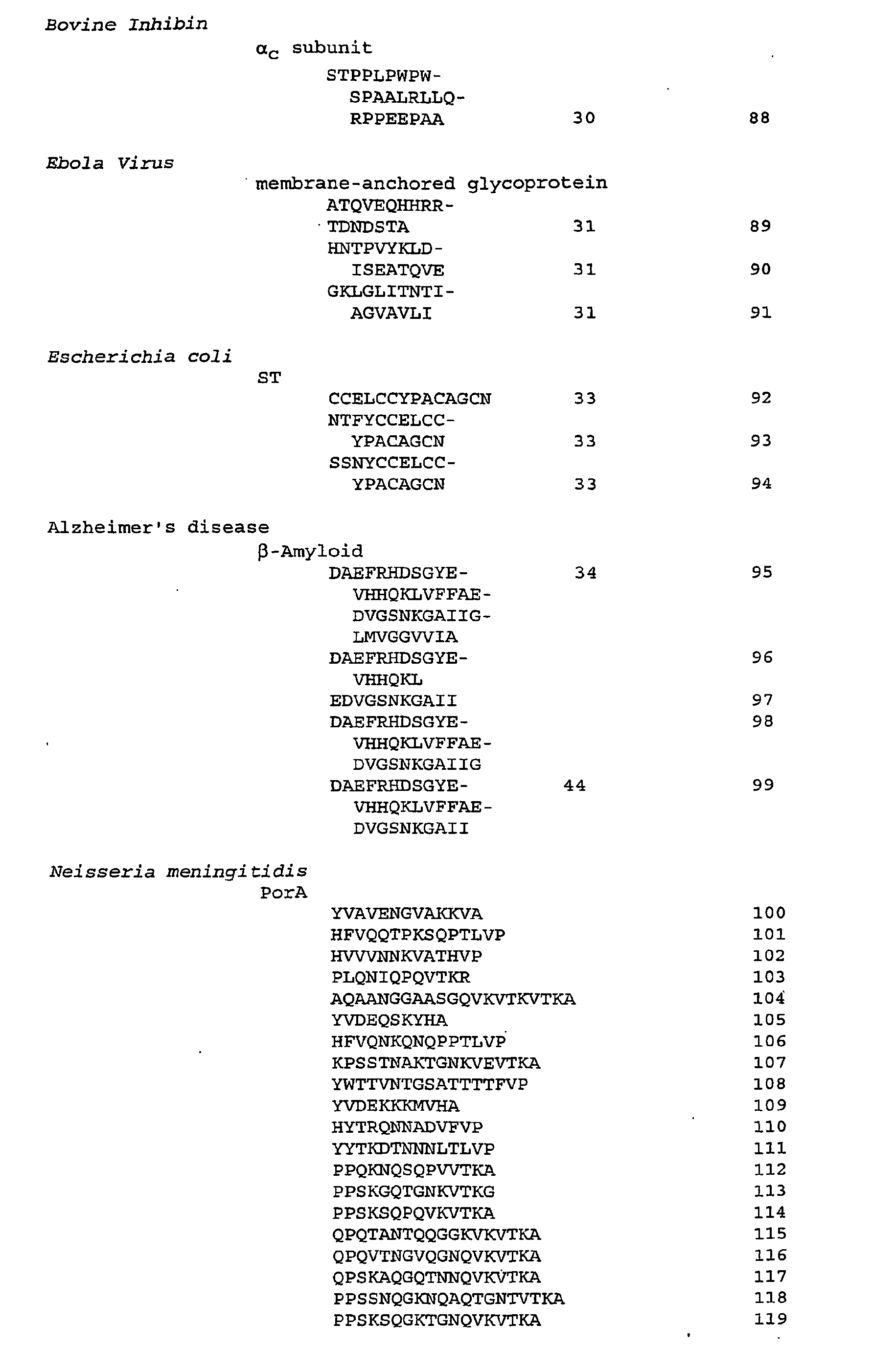

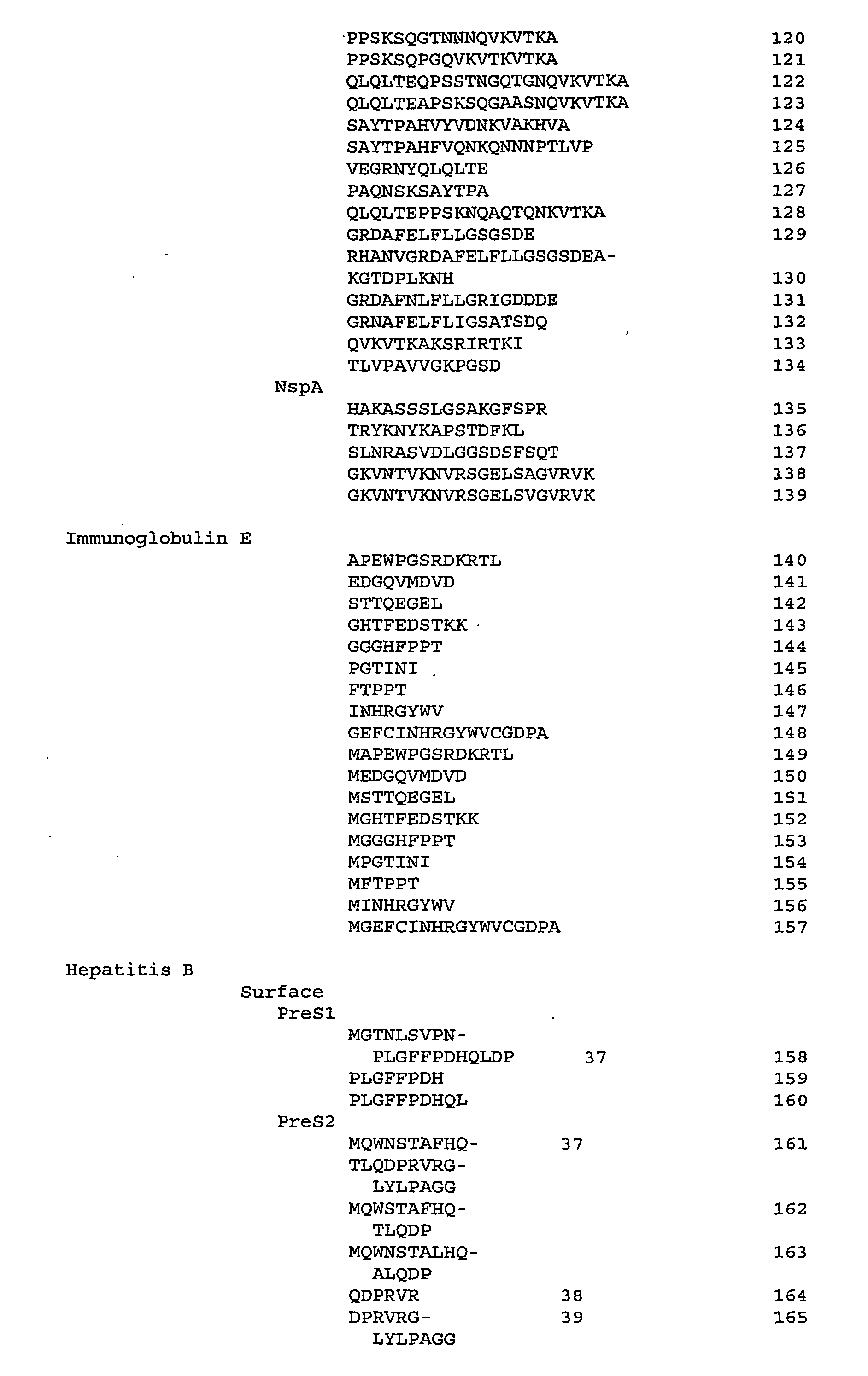

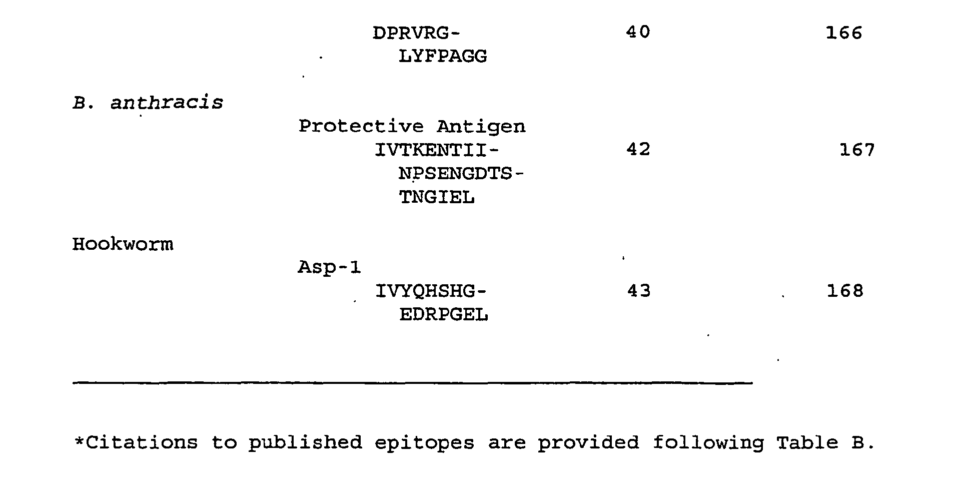

For influenza A M2 polypeptide sequence X₁X₂X₃X₄X₅X₆X₇X₈TX₁₀X₁₁RX₁₃X₁₄X₁₅X₁₆X₁₇X₁₈X₁₉X20X21-X₂₂X₂₃X₂₄ of SEQ ID NO:44:
residues X₁ through X₈ are absent or present, and when present are the residues naturally present in the M2 protein sequence that are methionine, serine, leucine, leucine, threonine or proline, glutamic acid, valine, and glutamic acid, respectively, with the proviso that when one subscripted X residue is present, any remaining subscripted X with a higher subscript number up to 8 is also present,
X₁₀ is present and is proline, leucine or histidine,
X₁₁ is present and is isoleucine or threonine,
X₁₃ is present and is asparagine or serine,
X₁₄ is present and is glutamic acid or glycine,
residues X₁₅ and X₁₆ are present or absent, and when present are tryptophan and glycine or glutamic acid, respectively,
residues X₁₇ and X₁₉ are present or absent, and when present are independently cysteine, serine, or alanine,
residue X₁₈ is present or absent, and when present is arginine or lysine, and
residues X₂₀ through X₂₄ are present or absent, and when present are the residues naturally present in the M2 protein sequence that are asparagine or serine, aspartic acid or glycine, serine, serine and aspartic acid respectively, with the proviso that when one subscripted X residue is present, any remaining subscripted X residue with a lower subscript number down to 15 is also present.

Similarly, in the preferred above influenza A M2 sequence of SEQ ID NO: 39:
residues X₁ through X₈ are absent or present, and when present are the residues naturally present in the M2 protein sequence that are methionine, serine, leucine, leucine, threonine, glutamic acid, valine, and glutamic acid, respectively, with the proviso that when one subscripted X residue is present, any remaining subscripted X with a higher subscript number up to 8 is also present,
residues X₁₅ and X₁₆ are present or absent, and when present are tryptophan and glycine, respectively,
residues X₁₇ and X₁₉ are present or absent, and when present are independently cysteine, serine, or alanine,
residue X₁₈ is present or absent, and when present is arginine, and
residues X₂₀ through X₂₄ are present or absent, and when present are the residues naturally present in the M2 protein sequence that are asparagine, aspartic acid, serine, serine and aspartic acid respectively, with the proviso that when one subscripted X residue is present; any remaining subscripted X residue with a lower subscript number down to 15 is also present.

The remaining residues of Domain II that are present on either side of the heterologous residue or sequence are preferably residues of HBc position 76 through position 85. Thus, in an illustrative example, where residues 78 through 82 have been replaced, the chimer sequence in Domain II is 76 through 77, followed by restriction site-encoded residues, the immunogenic (epitope) sequence, further restriction site-encoded residues, and then HBc sequence 84 through 85. A typical exemplary sequence of a chimer prepared by an insertion strategy between residues 78 and 79 is that of HBc from position 2 through 78, followed by restriction site-encoded residues, the immunogenic sequence, further restriction site-encoded residues and HBc sequence 79 through 85. The sequence of other contemplated chimers through Domains I and II should be apparent from these illustrations and those that follow and need not be enumerated.

It has been found that a short hydrophilic peptide containing a plurality of glycine residues and having a length of about 5 to about 9 residues peptide-bonded at the C-terminus of an above-noted *Neisseria meningitidis* B cell epitope sequence can assist in the expression of a chimeric particle containing that sequence. One useful short peptide is that disclosed in Karpenko et al., (2000) Amino Acids 18:329-337, having the sequence GSGDGEGG of SEQ ID NO: 169.

As already noted, a heterologous linker for a conjugated epitope is peptide-bonded at a position in the HBc sequence between amino acid residues 76 and 85. As was the case for the immunogenic epitope, the HBc sequence of residues 76 through 85 is preferably present, but interrupted by the heterologous linker for a conjugated epitope. This chimer preferably includes the HBc sequence of position 4 through at least position 140, plus a cysteine residue near the N-terminus or the C-terminus of the chimer protein. More preferably, the HBc sequence of positions 2 through 149 are present, but interrupted between residues 76 and 85 by the heterologous linker for a conjugated epitope, and the chimer molecule contains a C-terminal cysteine.

The heterologous linker for a conjugated epitope is most preferably a lysine (K) residue. Glutamic or aspartic acid, tyrosine and cysteine residues can also be used as linker residues, as can tyrosine and cysteine residues. It is noted that more than one linker can be present such as a sequence of three lysines, but such use is not preferred because heterogeneous conjugates can be formed from such use in which the conjugated hapten is bonded to one linker in a first chimer and to a different linker in a second chimer molecule. U.S. Patent No. 6,231,864 B1 discloses HBc chimer molecules containing one or more linking residues, but lacking a stabilizing N-terminal cysteine residue.

It is also noted that an immunogenic epitope-containing sequence present in a contemplated HBc chimer can also be separated from the HBc sequence residues by a "flexible linker arm" on one or both sides of (flanking) the immunogenic (epitope) sequence. This is particularly the case where the immunogenic sequence is greater than about 30 amino acid residues long. Exemplary flexible linker arm sequences typically contain about 4 to about 10 glycine residues that are thought to permit the inserted sequence to "bulge" outwardly from the otherwise bulging loop sequence and add further stability to the construct. These flexible linker arms are similar to those discussed before in relation to a *Neisseria meningitidis* B cell epitope sequence such as the peptide of SEQ ID NO: 125. Illustrative other flexible linker arm sequences are disclosed in Kratz et al. (March 1999) Proc. Natl. Acad. Sci., U.S.A., 96:1915-1920 and are exemplified by the amino acid residue sequences:
GGGGSGGGGT SEQ ID NO: 170
GGGGSGGGG SEQ ID NO: 171.

The sequence immediately below is utilized at the C-terminus of an inserted epitope-containing sequence, whereas the sequence thereafter is used at each of the N- and C-termini of inserted immunogenic sequences
GSGDEGG SEQ ID NO: 172
GGGGSGGG SEQ ID NO: 173.

As was noted previously, Domain III constitutes the sequence of HBc from position 86 through position 135. Consequently, the sequence of the illustrative chimers discussed above for Domains I and II, can be extended so that the first-discussed chimer has the sequence of HBc from position 84 through position 140, and the second-discussed chimer has the sequence of HBc from position 79 through position 140.

Domain IV contains a sequence that (i) includes a HBV sequence from about position 136 through 140 and optionally through position 149, position 156 or less preferably position 163, or even through position 183, (ii) contains zero up to about three cysteine (Cys) residues, and (iii) up to about 75 amino acid residues in an immunogenic sequence that is preferably heterologous to HBc at position 163, and more preferably position 156, trough the C-terminus, with the proviso that Domain IV contains at least 5 amino acid residues of the HBc sequence from position 136 through 140. The Domain IV immunogenic sequence more preferably contains up to about 50 amino acid residues, and most preferably contains up to about 25 residues. The Domain IV sequence can thus be substantially any sequence, except the C-terminal HBc sequence from about position 163 to the C-terminus.

The length of the Domain IV sequence can be five residues; i.e., the residue of position 136 through 140, up to about 125 amino acid residues (up to about HBc position 183 plus up to about 75 immunogenic residues of an immunogenic sequence) including up to a total of three cysteines.

Where an epitope peptide-bonded to one or both of Domains I or II contains up to about 30 or about 50 residues, respectively, as is preferred for those epitopes, more preferred lengths of the chimer molecule, including the Domain IV epitope, are about 150 to about 280 residues. Particularly preferred chimer molecules containing two immunogenic epitopes have a length of about 190 to about 210 residues. Freedom of the resulting particle from nucleic acid binding is determined by measuring the 280/250 absorbance ratio, as discussed previously.

The Domain IV sequence can include zero up to three Cys residues. When present, it is preferred that the one or more Cys residues be within about 30 residues from the C-terminus of the chimer molecule, and preferably at or within about five amino acid residues of the c-terminus of the chimeric protein molecule. In addition, when more than one Cys residue is present in a Domain IV sequence, it is preferred that those Cys residues be adjacent to each other.

It is preferred that the Domain IV sequence include a T cell epitope, a plurality of T cell epitopes that are the same or different or an additional B cell epitope for the organism against which a contemplated chimer is intended to be used as an immunogen. Exemplary Domain IV T cell epitope sequences are provided in Table B, below, as in Table A, with illustrative added C-terminal cysteine residues underlined (C).

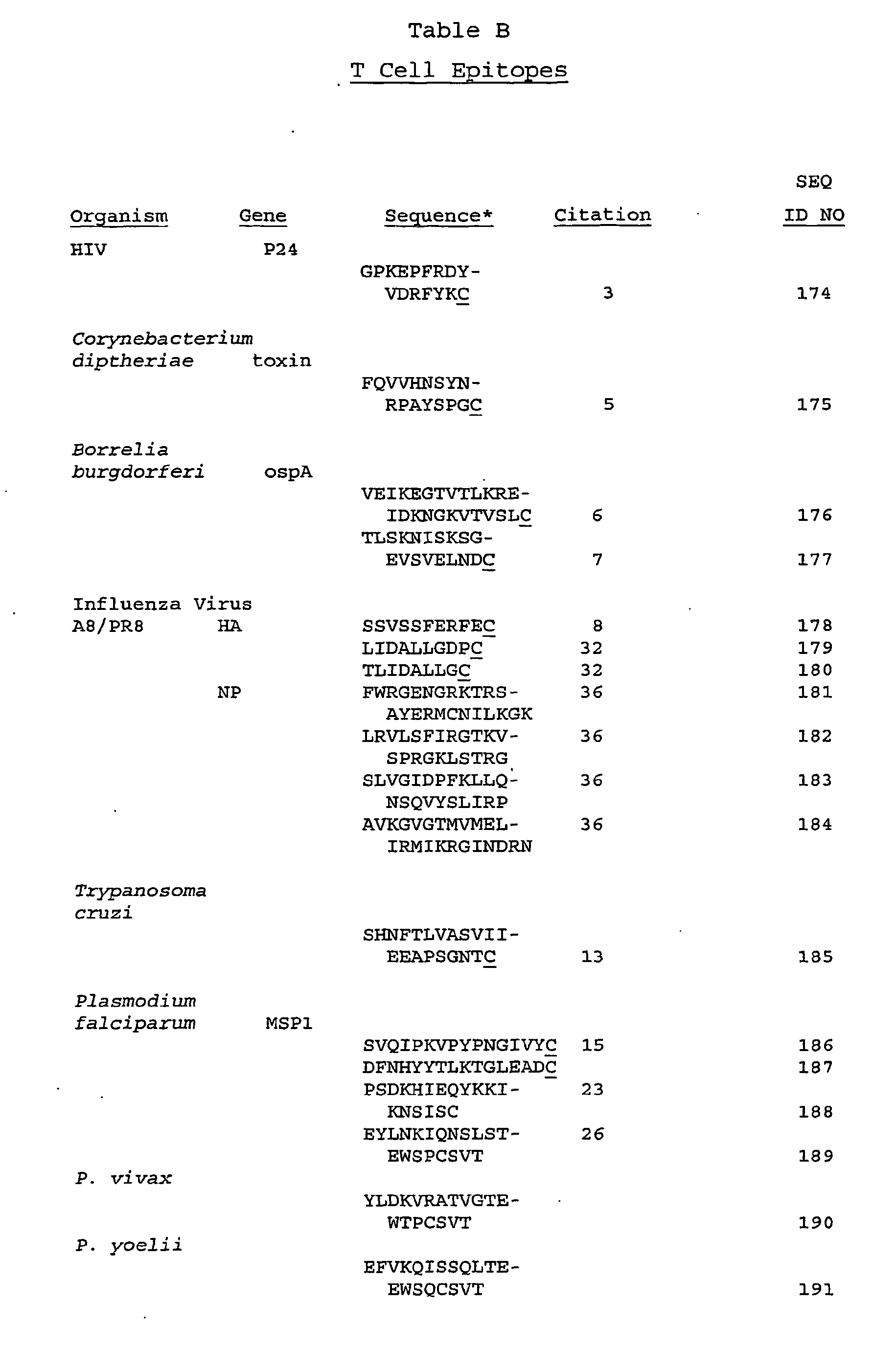

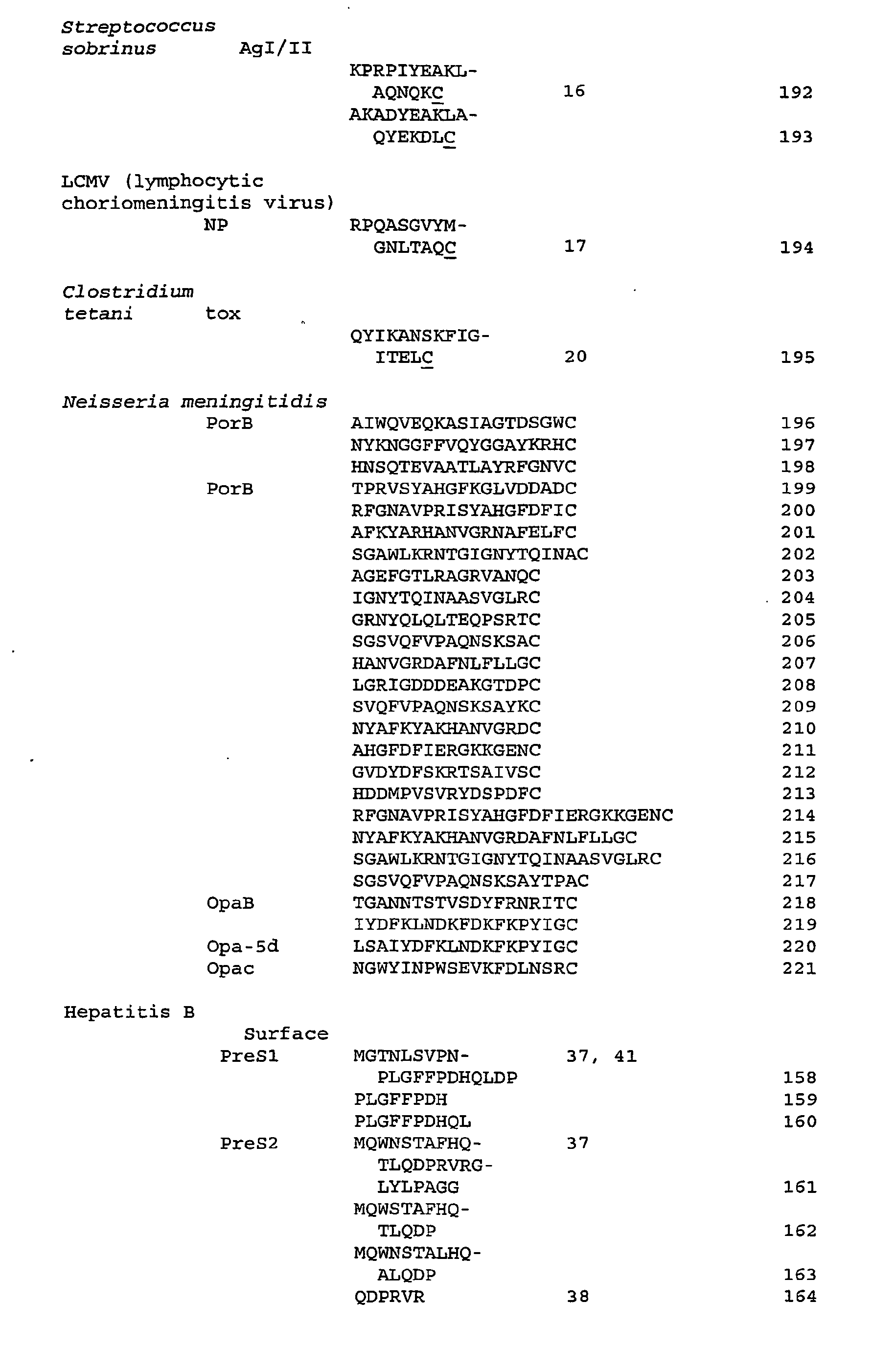

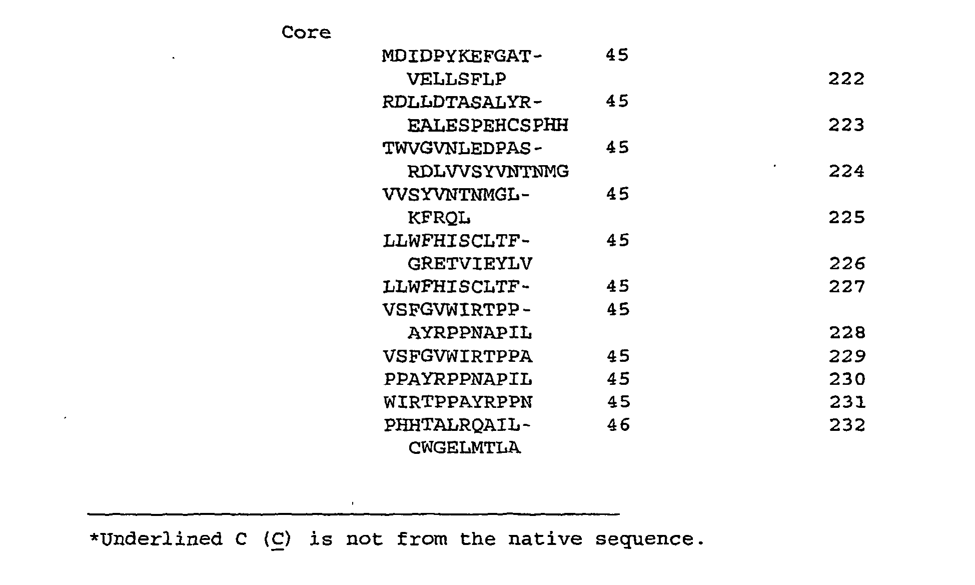

Citations:
1. EPO 786 521A.
2. WO 98/07320.
3. US No. 5,639,854.
4. US No. 4,544,500.
5. EPO 399001 B1.
6. Bockenstedt et al. (1996) J. Immunol., 157, 12:5496.
7. Zhong et al. (1996) Eur. J. Immunol., 26, 11:2749.
8. Brumeanu et al. (1996) Immunotechnology, 2, 2:85.
9. Hill et al. (1997) Infect. Immun., 65, 11:4476.
10. EPO 432 220 B1.
11. WO 98/06851.
12. Kelly et al. (1997) Clin. Exp. Immunol., 110, 2:285.
13. Kahn et al. (1997) J. Immunol., 159, 9:4444.
14. WO 97/18475.
15. Ohta et al. (1997) Int. Arch. Allergy Immunol., 114,1:15.
16. Staffileno et al. (1990) Arch. Oral Biol., 35: Suppl. 47S.
17. Saron et al. (1997) Proc. Natl. Acad. Sci. USA ,94,7:3314.
18. Corthesy et al. (1996) J. Biol. Chem., 271, 52:33670.
19. Bastien et al. (1997) Virol., 234, 1:118.
20. Yang et al. (1997) Vaccine, 15, 4:377.
21. Lotter et al. (1997) J. Exp. Med., 185, 10:1793.
22. Nara et al. (1997) Vaccine 15, 1:79.
23. U.S. No. 4,886,782.
24. Zavala et al. (1985) Science, 228:1436.
25. Schodel et al. (1994) J. Exper. Med., 180:1037.
26. Calvo-Calle et al. (1997) J. Immunol. 159, 3:1362.
27. Qari et al. (1992) Mol. Biochem. Parasitol., 55(1-2):105.
28. Qari et al. (1993) Lancet, 341(8848):780.
29. Neirynck et al. (Oct 1999) Nature Med., 5(10):1157-1163.
30. Thompson et al. (1994) Eur.J. Biochem., 226(3):751-764.
31. Wilson et al. (2000) Science, 287:1664-1666.
32. Brown et al. (1993) J. Virol., 67 (5):2887-2893.
33. U.S. No. 4,886,663.
34. Schenk et al. (Jul 8, 1999) Nature, 400(6740):116-117.
35. Slepushkin et al. (1995) Vaccine, 13(15):1399-1402.
36. Brett et al., (1991) J. Immunol., 147(3):984-991.
37. Neurath et al., (1986) F. Brown et al. eds., Vaccines 85, Cold Spring Harbor Laboratory, Cold Spring Harbor, New York, pp.185-189.
38. Kent et al., (1987) F. Brown et al. eds., Vaccines 86, Cold Spring Harbor Laboratory, Cold Spring Harbor, New York, pp.365-369.
39. Milich et al., (1987) F. Brown et al. eds., Vaccines 86, Cold Spring Harbor Laboratory, Cold Spring Harbor, New York, pp.377-382.
40. Thornton et al., (1987) F. Brown et al. eds., Vaccines 87, Cold Spring Harbor Laboratory, Cold Spring Harbor, New York, pp.77-80.
41. Milich et al., (1987) F. Brown et al. eds., Vaccines 87, Cold Spring Harbor Laboratory, Cold Spring Harbor, New York, pp.50-55.
42. Little et al., (1996) Microbiology 142:707-715.
43. Provided by Dr. Peter Hotez, George Washington University.
44. Morgan et al., (2000) Nature 408:982-985.
45. U.S. Patent No. 4,882,145.
46. Alexander et al., (1994) Immunity 1:751-761.
47. Muyanga et al., (2001) Arch. Virol. 146(9):1667-1679.

Another useful T cell epitope is a synthetic sequence referred to as a PADRE epitope (See, U.S. Patent No. 6,413,517 to Sette et al.) One exemplary epitope is that disclosed by Alexander et al., (1994) Immunity, 5:751-761 that has the sequence AKFVAAWTLKAAA (SEQ ID NO: 233).

The amino acid sequence of HBc from residue position 4 through at least position 140 is preferably present in a contemplated chimer molecule and particle. The sequence from position 2 through position 1.49 and up to about position 163 is more preferably present. A B cell epitope, when present, is preferably present between residues 76 and 85. One or both of the cysteine residues normally present at HBc positions 48 and 107 are replaced (substituted) with another residue. At least a single cysteine residue is present at or near the N-terminus in Domain I as already noted and optionally near the C-terminus, as discussed before. One or more T cell epitopes can also be present as an N-terminal or c-terminal addition to the HBc sequence. A contemplated recombinant HBc chimer is substantially free of bound nucleic acid. A contemplated chimer particle in which one or both of the cysteine residues normally present at HBc positions 48 and 107 is more stable than is a similar particle that does not contain those replacements; i.e., a chimer that contains the cysteines at positions 48 and 107.

A contemplated recombinant HBc chimer molecule is typically present and is used as a self-assembled particle. These particles are comprised of 180 to 240 chimer molecules (90 or 120 dimer pairs), usually 240 chimer molecules, that separate into protein molecules in the presence of disulfide reducing agents such as 2-mercaptoethanol, and the individual molecules are therefore thought to be bound together in the particle primarily by disulfide bonds. It is believed that the observed enhanced stability is due to the absence of one or both of the cysteines at positions 48 and 107.

These particles are similar to the particles observed in patients infected with HBV, but these particles are non-infectious. Upon expression by various prokaryotic and eukaryotic hosts, the individual recombinant HBc chimer molecules assemble into particles that can be readily harvested from the host cells, nutrient solution of both, and purified, if desired.

As noted before, the HBc immunodominant loop is usually recited as being located at about positions 75 through about 85 from the amino-terminus (N-terminus) of the intact protein. An immunogenic epitope-containing sequence of Domain II is placed into that immunodominant loop sequence. That placement can substantially eliminate the HBc immunogenicity and antigenicity of the HBc loop sequence, while presenting the immunogenic sequence or linker residue in an extremely immunogenic position in the assembled chimer particles.

In addition to the before-discussed N- and C-truncations, insertion of various epitopes and spacers, a contemplated chimer molecule can also contain conservative substitutions in the amino acid residues that constitute HBc Domains I, II, III and IV. Conservative substitutions are as defined before. An illustrative conservative substitution is seen in the replacement of residues at positions 2 and 3 (aspartic acid and isoleucine; DI) by glutamic acid and leucine (EL) residues that are encoded by an EcoRI restriction site used to add nucleic acids that code for a desired N-terminal epitope, including an N-terminal cysteine residue. Further illustrative examples are the replacement of lysine residues at positions 7 and 97 by arginines, and the replacement of cysteines at positions 48 and 107 by serine residues.

More rarely, a "nonconservative" change, e.g., replacement of a glycine with a tryptophan, or of a cysteine with an alanine residue is contemplated. Analogous minor variations can also include amino acid deletions or insertions, or both. Guidance in determining which amino acid residues can be substituted, inserted, or deleted without abolishing biological activity or particle formation can be found using computer programs well known in the art, for example LASERGENE software (DNASTAR Inc., Madison, Wis.)

The HBc portion of a chimer molecule of the present invention; i.e., the portion having the HBc sequence that has other than a sequence or residue of an added immunogen, anti-antigen, linker, flexible linker arm or heterologous residue(s) that are a restriction enzyme artifact, most preferably has the amino acid residue sequence of subtype ayw that is shown in Fig. 1 (SEQ ID NO: 1), less any portion or portions of the subtype ayw sequence that are absent because of truncation at one or both termini. Typically, that sequence is that of HBc positions 2 through 149 or position 156. Somewhat less preferred are the corresponding amino acid residue sequences of subtypes *adw, adw2* and adyw that are also shown in Fig. 1 (SEQ ID NOs: 2, 3 and 4). Less preferred still are the sequences of woodchuck and ground squirrel at aligned positions 2 through 149 or 2 through 156 that are the last two sequences of Fig 1 (SEQ ID NOs: 5 and 6). As noted elsewhere, portions of different sequences from different mammalian HBc proteins can be used together in a single chimer.

When the HBc portion of a chimer molecule of the present invention as above described has other than a sequence of a mammalian HBc molecule corresponding to positions 2 through about 183, up to about 20 percent of the amino acid residues are substituted as compared to SEQ ID NO: 1 from position 2 through 183, and preferably position 2 through 163. It is preferred that up to about 10 percent, and more preferably up to about 5 percent, and most preferably up to about 3 percent of the amino acid residues are substituted as compared to SEQ ID NO: 1 from position 2 through 163.

A contemplated chimer of 183 HBc residues can therefore contain up to about 36 residues that are different from those of SEQ ID NO:1 at positions 2 through 183, and preferably about 18 residues. More preferably, about 9 residues are different from the ayw sequence (SEQ ID NO: 1) at residue positions 2-183, and most preferably about 5 residues are different Differences in shorter sequences, e.g., 2-149, or 2-156, or 2-163 are proportional to those discussed before based on percentage. Substitutions, other than in the immunodominant loop of Domain II or at the termini, are preferably in the non-helical portions of the chimer molecule and are typically between residues 2 to about 15 and residues 24 to about 50 to help assure particle formation. See Koschel et al., (1999) J. Virol., 73(3):2153-2160.

Where a HBc sequence is truncated at the C-terminus beyond position 163 or at the N-terminus, or contains one or more deletions in the immunodominant loop, the number of substituted residues is proportionally fewer because the total length of the sequence is less than 163 residues. Deletions elsewhere in the molecule are considered conservative substitutions for purposes of calculation so that if, for example, Domain III were to have a C-terminus at position 133 instead of 135, two residues (134 and 135) would be presumed to be present for purposes of calculation.

### Chimer Preparation

A contemplated chimeric HBc immunogen is typically prepared using the well-known techniques of recombinant DNA technology.' Thus, sequences of nucleic acids that encode particular polypeptide sequences are added to and deleted from the precursor sequence that encodes HBc to form a nucleic acid that encodes a contemplated chimer.

An illustrative contemplated chimeric immunogen typically utilizes a cysteine residue present in the influenza A M2 sequence as the N-terminal cysteine. Primers for the preparation of such chimer molecules by *in vitro* mutagenesis of a polynucleotide encoding an HBc molecule are discussed hereinafter. When a cysteine-containing M2 polypeptide epitope is not present at the N-terminus, the N-terminal cysteine can be provided by in vitro mutagenesis using a primer that encodes just a cysteine-containing portion of the M2 polypeptide or a simple N-terminal start sequence such as Met-Cys- or Met-Gly-Cys-.

As was noted previously, the HBc immunodominant loop is usually recited as being located at about positions 75 through 85 from the amino-terminus (N-terminus) of the intact protein. The illustrative influenza A M2 B cell epitope-containing sequence can be placed into that immunodominant loop sequence of Domain II. That placement substantially diminishes the HBc immunogenicity and antigenicity of the HBc loop sequence, while presenting the influenza A M2 B cell epitope in an extremely immunogenic position in the assembled chimer particles.

One of two strategies is preferred for placing the heterologous epitope sequence into the loop sequence. The first strategy is referred to as replacement, in which DNA that codes for a portion of the immunodominant loop is excised and replaced with DNA that encodes a heterologous epitope such as a B cell sequence. The second strategy is referred to as insertion, in which a heterologous epitope is inserted between adjacent residues in the loop.

Site-directed mutagenesis using the polymerase chain reaction (PCR) is used in one exemplary replacement approach to provide a chimeric HBc DNA sequence that encodes a pair of different restriction sites, e.g. EcoRI and SacI, one near each end of the immunodominant loop-encoding DNA. Exemplary residues replaced are 76 through 81. The loop-encoding section is excised, a desired sequence that encodes the heterologous B cell epitope is ligated into the restriction sites and the resulting DNA is used to express the HBc chimer. See, for example, Table 2 of Pumpens et al., (1995) Intervirology, 38:63-74 for exemplary uses of this technique.

Alternatively, a single restriction site or two sites can be introduced into the region, the DNA cut with a restriction enzyme(s) to provide "sticky" or blunt ends, and an appropriate sticky- or blunt-ended heterologous DNA segment ligated into the cut region. Examples of this type of sequence replacement into HBc can be found in the work reported in Schodel et al., (1991) F. Brown et al. eds., Vaccines 91, Cold Spring Harbor Laboratory, Cold Spring Harbor, New York, pp.319-325, Schodel et al., Behring Inst. Mitt., 1997(98): p. 114-119 and Schodel et al., J. Exp. Med., (1994) 180(3): p. 1037-4, the latter two papers discussing the preparation of vaccines against malarial pathogens P. yoelii and *P. berghei,* respectively. A replacement strategy that results in a net removal of residues from the immunodominant loop is usually not used herein.

The insertion position within the HBc immunogenic loop and the presence of loop residues can be of import to the activity of the immunogen. Thus, as is illustrated in published PCT applications PCT/US01/25625 and PCT/US01/41759, placement of a malarial B cell epitope between HBc residues 78 and 79 provides a particulate immunogen that is ten to one thousand times more immunogenic than placement of the same immunogen in an excised and replaced region between residues 76 and 81. In addition, placement of the same malarial immunogen between residues 78 and 79 as compared to between residues 77 and 78 provided an unexpected enhancement in immunogenicity of about 15-fold.

Insertion is therefore generally preferred. In an illustrative example of the insertion strategy, site-directed mutagenesis is used to create two restriction sites adjacent to each other and between codons encoding adjacent amino acid residues, such as those at residue positions 78 and 79. This technique adds twelve base pairs that encode four amino acid residues (two for each restriction site) between formerly adjacent residues in the HBc loop.

Upon cleavage with the restriction enzymes, ligation of the DNA coding for the heterologous B cell epitope sequence and expression of the DNA to form HBc chimers, the HBc loop amino acid sequence is seen to be interrupted on its N-terminal side by the two residues encoded by the 5' restriction site, followed toward the C-terminus by the heterologous B-cell epitope sequence, followed by two more heterologous, non-loop residues encoded by the 3' restriction site and then the rest of the loop sequence. This same strategy can be used for insertion into Domain I of an N-terminal cysteine or N-terminal sequence as was reported in Neirynck et al., (1999) Nature Med., 5(10):1157-1163 or for insertion into Domain IV of a T cell epitope or one or more cysteine residues. A similar strategy using an insertion between residues 82 and 83 is reported in Schodel et al., (1990) F. Brown et al. eds., Vaccines 90, Cold Spring Harbor Laboratory, Cold Spring Harbor, New York, pp.193-198.

More specifically, a DNA sequence that encodes a C-terminal truncated HBc sequence (HBc149) is engineered to contain adjacent EcoRI and SacI sites between residues 78 and 79. Cleavage of that DNA with both enzymes provides one fragment that encodes HBc positions 1-78 3'-terminated with an EcoRI sticky end, whereas the other fragment has a 5'-terminal SacI sticky end and encodes residues of positions 79-149. Ligation of a synthetic nucleic acid having a 5' AATT overhang followed by a sequence that encodes a desired B cell epitope and a AGCT 3'overhang provides a HBc chimer sequence that encodes that B cell epitope flanked on each side by two heterologous residues [GlyIle (GI) and GluLeu (EL), respectively] between residues 78 and 79, while usually destroying the EcoRI site and preserving the SacI site.

A similar strategy can be used for insertion of a cysteine-containing sequence in Domain IV, such as a malarial T cell epitope that contains the *P*. *falciparum* CS protein sequence from position 326 through position 345 and is referred to herein as PF/CS326-345 (Pf-UTC). Here, EcoRI and HindIII restriction sites are engineered into the HBc DNA sequence after amino acid residue position 149. After digestion with EcoRI and HindIII, a synthetic DNA having the above AATT 5'overhang followed by a T cell epitope-encoding sequence, one or more stop codons and a 3' AGCT overhang were ligated into the digested sequence to form a sequence that encoded HBc residues 1-149 followed by two heterologous residues (GI), the heterologous T cell epitope, the stop codon and the HindIII site.

PCR amplification using a forward primer having a SacI restriction site followed by a sequence encoding HBc beginning at residue position 79, followed by digestion with SacI and HindIII provided a sequence encoding HBc positions 79-149 plus the two added residues and the T cell epitope at the C-terminus. Digestion of the construct with SacI and HindIII and subsequent ligation with a B cell-containing construct provides the complete gene encoding a desired recombinant HBc chimer immunogen having the sequence, from the N-terminus, of HBc positions 1-78, two added residues, the B cell epitope (e.g. malarial), two added residues, HBc positions 79-149, two added residues, and the T cell epitope that is shown in Fig. 2C.

Similar techniques can be used to place a heterologous linker residue for conjugation of a B cell epitope onto the loop region sequence. Contemplated linker residues include lysine (Lys), which is particularly preferred, aspartic acid (Asp), glutamic acid (Glu), cysteine (Cys) and tyrosine (Tyr).

It is noted that the amino acid residue sequence shown in SEQ ID NO:1 contains a Glu and an Asp residue at positions 77 and 78. Nonetheless, introduction of an additional, heterologous, carboxyl-containing residue is still contemplated. The chemical reactivity of the existing glutamic and aspartic acids may be reduced by other factors. For example, it is known in the art that a neighboring proline, such as that found at position 79, can neutralize and thereby reduce the chemical reactivity of a proximal carboxyl group.

Here, using the first noted insertion strategy, five heterologous residues are placed into the loop sequence; one that is the heterologous linker residue for conjugating a B cell epitope and two residues adjacent on either side of that one residue that are themselves also adjacent to loop sequence residues and are an expression product of the inserted restriction sites (restriction enzyme artifacts). It is noted that one can also use site-directed mutagenesis to add a single codon into the HBc loop sequence that encodes the heterologous linker residue for a B cell epitope.

It is noted that the preferred use of two heterologous residues on either side of (flanking) a B cell or T cell epitope is a matter of convenience. As a consequence, one can also use zero to three or more added residues that are not part of the HBc sequence on either or both sides of an inserted sequence. One or both ends of the insert and HBc nucleic acid can be "chewed back" with an appropriate nuclease (e.g. S1 nuclease) to provide blunt ends that can be ligated together. Added heterologous residues that are neither part of the inserted B cell or T cell epitopes nor a part of the HBc sequence are not counted in the number of residues present in a recited Domain, unless those residues are conservative replacements for residues already present, as where the residues GluLeu replace AspIle in some of the constructs discussed hereinafter.

It is also noted that one can also synthesize all or a part of a desired recombinant HBc chimer nucleic acid using well-known synthetic methods as is discussed and illustrated in U. S. Patent No. 5,656,472 for the synthesis of the 177 base pair DNA that encodes the 59 residue ribulose bis-phosphate carboxylase-oxygenase signal peptide of *Nicotiana tabacum.* For example, one can synthesize Domains I and II with a blunt or a "sticky end" that can be ligated to Domains III and IV to provide a construct that expresses a contemplated HBc chimer that contains zero added residues to the N-terminal side of the B cell epitope and zero to three added residues on the C-terminal side or at the Domain II/III junction or at some other desired location.

An alternative insertion technique was reported in Clarke et al. (1991) F. Brown et al. eds., Vaccines 91, Cold Spring Harbor Laboratory, Cold Spring Harbor, New York, pp.313-318. Here, taking advantage of the degeneracy of the genetic code, those workers engineered a single restriction site corresponding to residues 80 and 81 that encoded the original residues present at those positions. Their expressed HBc chimers thereby contained no restriction site-encoded residues, and contained the residues of the HBc loop immediately adjacent to the inserted sequence.

A nucleic acid sequence (segment) that encodes a previously described HBc chimer molecule or a complement of that coding sequence is also contemplated herein. Such a nucleic acid segment is present in isolated and purified form in some preferred embodiments.

In living organisms, the amino acid residue sequence of a protein or polypeptide is directly related via the genetic code to the deoxyribonucleic acid (DNA) sequence of the gene that codes for the protein. Thus, through the well-known degeneracy of the genetic code, additional DNAs and corresponding RNA sequences (nucleic acids) can be prepared as desired that encode the same chimer amino acid residue sequences, but are sufficiently different from a before-discussed gene sequence that the two sequences do not hybridize at high stringency, but do hybridize at moderate stringency.

High stringency conditions can be defined as comprising hybridization at a temperature of about 50°-55°C in 6XSSC and a final wash at a temperature of 68°C in 1-3XSSC. Moderate stringency conditions comprise hybridization at a temperature of about 50°C to about 65°C in 0.2 to 0.3 M NaCl, followed by washing at about 50°C to about 55°C in 0.2X SSC, 0.1% SDS (sodium dodecyl sulfate).

A nucleic sequence (DNA sequence or an RNA sequence) that (1) itself encodes, or its complement encodes, a chimer molecule whose HBc portion from residue position 4 through 136, when present, is that of SEQ ID NOs: 1, 2, 3, 4, 5 or 6 and (2) hybridizes with a DNA sequence of SEQ ID NOs: 234, 235, 236, 237, 238 or 239 at least at moderate stringency (see below); and (3) whose HBc sequence shares at least 80 percent, and more preferably at least 90 percent, and even more preferably at least 95 percent, and most preferably 100 percent identity with a DNA sequence of SEQ ID NOs: 234, 235, 236, 237, 238 and 239, is defined as a DNA variant sequence. As is well-known, a nucleic acid sequence such as a contemplated nucleic acid sequence is expressed when operatively linked to an appropriate promoter in an appropriate expression system as discussed elsewhere herein.
HBC AYW DNA; SEQ ID NO:234
HBc ADW DNA; SEQ ID NO:235
HBc ADW2 DNA; SEQ ID NO:236
HBc ADYW DNA; SEQ ID NO:237
Woodchuck DNA; SEQ ID NO:238
Ground squirrel DNA; SEQ ID NO:239

An analog or analogous nucleic acid (DNA or RNA) sequence that encodes a contemplated chimer molecule is also contemplated as part of this invention. A chimer analog nucleic acid sequence or its complementary nucleic acid sequence encodes a HBc amino acid residue sequence that is at least 80 percent, and more preferably at least 90 percent, and most preferably is at least 95 percent identical to the HBc sequence portion from residue position 4 through residue position 140 shown in SEQ ID NOs: 1, 2, 3, 4, 5 or 6. This DNA or RNA is referred to herein as an "analog of" or "analogous to" a sequence of a nucleic acid of SEQ ID NOs: 234, 235, 236, 237, 238 and 239, and hybridizes with the nucleic acid sequence of SEQ ID NOs: 234, 235, 236, 237, 238 and 239 or their complements herein under moderate stringency hybridization conditions. A nucleic acid that encodes an analogous sequence, upon suitable transfection and expression, also produces a contemplated chimer.

Different hosts often have preferences for a particular codon to be used for encoding a particular amino acid residue. Such codon preferences are well known and a DNA sequence encoding a desired chimer sequence.can be altered using in vitro mutagenesis, for example, so that host-preferred codons are utilized for a particular host in which the enzyme is to be expressed. In addition, one can also use the degeneracy of the genetic code to encode the HBc portion of a sequence of SEQ ID NOs: 234, 235, 236, 237, 238 and 239 that avoids substantial identity with a DNA of SEQ ID Nos: 1, 2, 3, 4, 5 or 6 or their complements. Thus, a useful analogous DNA sequence need not hybridize with the nucleotide sequences of SEQ ID Nos 234, 235, 236, 237, 238 and 239 or a complement under conditions of moderate stringency, but can still provide a contemplated chimer molecule.

A recombinant nucleic acid molecule such as a DNA molecule, comprising a vector operatively linked to an exogenous nucleic acid segment (e.g., a DNA segment or sequence) that defines a gene that encodes a contemplated chimer, as discussed above, and a promoter suitable for driving the expression of the gene in a compatible host organism, is also contemplated in this invention. More particularly, also contemplated is a recombinant DNA molecule that comprises a vector containing a promoter for driving the expression of the chimer in host organism cells operatively linked to a DNA segment that defines a gene for the HBc portion of a chimer or a DNA variant that has at least 90 percent identity to the chimer gene of SEQ ID NOs: 234, 235, 236, 237, 238 and 239 and hybridizes with that gene under moderate stringency conditions.

Further contemplated is a recombinant DNA molecule that comprises a vector containing a promoter for driving the expression of a chimer in host organism cells operatively'linked to a DNA segment that is an analog nucleic acid sequence that encodes an amino acid residue sequence of a HBc chimer portion that is at least 80 percent identical, more preferably 90 percent identical, and most preferably 95 percent identical to the HBc portion of a sequence of SEQ ID NOs: 1, 2, 3, 4, 5 or 6. That recombinant DNA molecule, upon suitable transfection and expression by a host cell, provides a contemplated chimer molecule.

It is noted that the 30 amino acid residue N-terminal sequence of ground squirrel HBc does not permit alignment with any of the other HBc sequences. That sequence and its encoding nucleic acid sequences and their complements are not included in the above percentages of identity, nor are the portions of nucleic acid that encode that 30-residue sequence or its complement used in hybridization determinations. Similarly, sequences that are truncated at either or both of the HBc N- and C-termini are not included in identity calculations, nor are those sequences in which residues of the immunodominant loop are removed for insertion of a heterologous epitope. Thus, only those HBc-encoding bases or HBc sequence residues that are present in a chimer molecule are included and compared to an aligned nucleic acid or amino acid residue sequence in the identity percentage calculations.

Inasmuch as the coding sequences for the gene disclosed herein is illustrated in SEQ ID NOs: 234, 235, 236, 237, 238 and 239 isolated nucleic acid segments, preferably DNA sequences, variants and analogs thereof can be prepared by in vitro mutagenesis, as is well known in the art and discussed in Current Protocols In Molecular Biology, Ausabel et al. eds., John Wiley & Sons (New York: 1987) p. 8.1.1-8.1.6, that begin at the initial ATG codon for a gene and end at or just downstream of the stop codon for each gene. Thus, a desired restriction site can be engineered at or upstream of the initiation codon, and at or downstream of the stop codon so that other genes can be prepared, excised and isolated.

As is well known in the art, so long as the required nucleic acid, illustratively DNA sequence, is present, (including start and stop signals), additional base pairs can usually be present at either end of the segment and that segment can still be utilized to express the protein. This, of course, presumes the absence in the segment of an operatively linked DNA sequence that represses expression, expresses a further product that consumes the enzyme desired to be expressed, expresses a product that consumes a wanted reaction product produced by that desired enzyme, or otherwise interferes with expression of the gene of the DNA segment.

Thus, so long as the DNA segment is free of such interfering DNA sequences, a DNA segment of the invention can be about 500 to about 15,000 base pairs in length. The maximum size of a recombinant DNA molecule, particularly an expression vector, is governed mostly by convenience and the vector size that can be accommodated by a host cell, once all of the minimal DNA sequences required for replication and expression, when desired, are present. Minimal vector sizes are well known. Such long DNA segments are not preferred, but can be used.

DNA segments that encode the before-described chimer can be synthesized by chemical techniques, for example, the phosphotriester method of Matteucci et al. (1981) J. Am. Chem. Soc., 103:3185. Of course, by chemically synthesizing the coding sequence, any desired modifications can be made simply by substituting the appropriate bases for those encoding the native amino acid residue sequence. However, DNA segments including sequences discussed previously are preferred.

A contemplated HBc chimer can be produced (expressed) in a number of transformed host systems, typically host cells, although expression in acellular, *in vitro,* systems is also contemplated. These host cellular systems include, but are not limited to, microorganisms such as bacteria transformed with recombinant bacteriophage, plasmid, or cosmid DNA expression vectors; yeast transformed with yeast expression vectors; insect cell systems infected with virus expression vectors (e.g. baculovirus); plant cell systems transformed with virus expression vectors (e.g. cauliflower mosaic virus; tobacco mosaic virus) or with bacterial expression vectors (e.g., Ti plasmid); or appropriately transformed animal cell systems such as CHO, VERO or COS cells. The invention is not limited by the host cell system employed.

DNA segments containing a gene encoding the HBc chimer are preferably obtained from recombinant DNA molecules (plasmid vectors) containing that gene. Vectors capable of directing the expression of a chimer gene into the protein of a HBc chimer is referred to herein as an "expression vector".

An expression vector contains expression control elements including the promoter. The chimer-coding gene is operatively linked to the expression vector to permit the promoter sequence to direct RNA polymerase binding and expression of the chimer-encoding gene. Useful in expressing the polypeptide coding gene are promoters that are inducible, viral, synthetic, constitutive as described by Poszkowski et al. (1989) EMBO J., 3:2719 and Odell et al. (1985) Nature, 313:810, as well as temporally regulated, spatially regulated, and spatiotemporally regulated as given in Chua et al. (1989) Science, 244:174-181.

One preferred promoter for use in prokaryotic cells such as *E*. *coli* is the Rec 7 promoter that is inducible by exogenously supplied nalidixic acid. A more preferred promoter is present in plasmid vector JHEX25 (available from Promega Corp., Madison WI) that is inducible by exogenously supplied isopropyl-β-D-thiogalacto-pyranoside (IPTG). A still more preferred promoter, the tac promoter, is present in plasmid vector pKK223-3 and is also inducible by exogenously supplied IPTG. The pKK223-3 plasmid can be successfully expressed in a number of *E. coli* strains, such as XL-1, TB1, BL21 and BLR, using about 25 to about 100 µM IPTG for induction. Surprisingly, concentrations of about 25 to about 50 µM IPTG have been found to provide optimal results in 2 L shaker flasks and fermentors.

Several strains of *Salmonella* such as *S*. *typhi* and *S. typhimurium and S. typhimurium-E. coli* hybrids have been used to express immunogenic transgenes including prior HBc chimer particles both as sources of the particles for use as immunogens and as live, attenuated whole cell vaccines and inocula, and those expression and vaccination systems can be used herein. See, U.S. Patent No. 6,024,961; U.S. Patent No. 5,888,799; U.S. Patent No. 5,387,744; U.S. Patent No. 5,297,441; Ulrich et al., (1998) Adv. Virus Res., 50:141-182; Tacket et al., (Aug 1997) Infect. Immun., 65(8):3381-3385; Schodel et al., (Feb 1997) Behring Inst. Mitt., 98:114-119; Nardelli-Haefliger et al., (Dec 1996) Infect. Immun., 64(12):5219-5224; Londono et al., (Apr 1996) Vaccine, 14(6):545-552, and the citations therein.

Expression vectors compatible with eukaryotic cells, such as those compatible with yeast cells or those compatible with cells of higher plants or mammals, are also contemplated herein. Such expression vectors can also be used to form the recombinant DNA molecules of the present invention. Vectors for use in yeasts such as *S*. *cerivisiae* or *Pichia pastoris* can be episomal or integrating, as is well known. Eukaryotic cell expression vectors are well known in the art and are available from several commercial sources. Normally, such vectors contain one or more convenient restriction sites for insertion of the desired DNA segment and promoter sequences. Optionally, such vectors contain a selectable marker specific for use in eukaryotic cells. Exemplary promoters for use in *S*. *cerevisiae* include the S. cerevisiae phosphoglyceric acid kinase (PGK) promoter and the divergent promoters GAL 10 and GAL 1, whereas the alcohol oxidase gene (AOX1) is a useful promoter for *Pichia pastoris.*

For example, to produce chimers in the methylotrophic yeast, *P. pastoris*, a gene that encodes a desired chimer is placed under the control of regulatory sequences that direct expression of structural genes in *Pichia.* The resultant expression-competent forms of those genes are introduced into *Pichia* cells.

More specifically, the transformation and expression system described by Cregg et al. (1987) Biotechnology, 5:479-485; (1987) Molecular and Cellular Biology, 12:3376-3385 can be used. A gene for a chimer V12.Pf3.1 is placed downstream from the - alcohol oxidase gene (AOX1) promoter and upstream from the transcription terminator sequence of the same AOX1 gene. The gene and its flanking regulatory regions are then introduced into a plasmid that carries both the *P*. *pastoris* HIS4 gene and a *P*. *pastoris* ARS sequence (Autonomously Replicating Sequence), which permit plasmid replication within *P*. *pastoris* cells [Cregg et al. (1987) Molecular and Cellular Biology, 12:3376-3385].

The vector also contains appropriate portions of a plasmid such as pBR322 to permit growth of the plasmid in *E. coli* cells. The resultant plasmid carrying a chimer gene, as well as the various additional elements described above, is illustratively transformed into a his4 mutant of *P*. *pastoris;* i.e. cells of a strain lacking a functional histidinol dehydrogenase gene.

After selecting transformant colonies on media lacking histidine, cells are grown on media lacking histidine, but containing methanol as described Cregg et al. (1987) Molecular and Cellular Biology, 12:3376-3385, to induce the AOX1 promoters. The induced AOX1 promoters cause expression of the chimer protein and the production of chimer particles in *P*. *pastoris.*

A contemplated chimer gene can also be introduced by integrative transformation, which does not require the use of an ARS sequence, as described by Cregg et al. (1987) Molecular and Cellular Biology, 12:3376-3385.

Production of chimer particles by recombinant DNA expression in mammalian cells is illustratively carried out using a recombinant DNA vector capable of expressing the chimer gene in Chinese hamster ovary (CHO) cells. This is accomplished using procedures that are well known in the art and are described in more detail in Sambrook et al., Molecular Cloning: A Laboratory Manual, 2nd ed., Cold Spring Harbor Laboratories (1989).

In one illustrative example, the simian virus (SV40) based expression vector, pKSV-10 (Pharmacia Fine Chemicals, Piscataway, NJ), is subjected to restriction endonuclease digestion by NcoI and HindIII. A NcoI/HindIII sequence fragment that encodes the desired HBc chimer prepared as described in Example 1 is ligated into the expression plasmid, which results in the formation of a circular recombinant expression plasmid denominated pSV-Pf.

The expression plasmid pSV-Pf contains an intact *E*. *coli* ampicillin resistance gene. *E*. *coli* RR101 (Bethesda Research Laboratories, Gaithersburg, MD), when transformed with pSV-Pf, can thus be selected on the basis of ampicillin resistance for those bacteria containing the plasmid. Plasmid-containing bacteria are then cloned and the clones are subsequently screened for the proper orientation of the inserted coding gene into the expression vector.

The above obtained plasmid, pSV-Pf, containing the gene that encodes a desired HBc chimer is propagated by culturing *E*. *coli* containing the plasmid. The plasmid DNA is isolated from *E*. *coli* cultures as described in Sambrook et al., above.

Expression of a chimer is accomplished by the introduction of pSV-Pf into the mammalian cell line, e.g., CHO cells, using the calcium phosphate-mediated transfection method of Graham et al.(1973) Virol., 52:456, or a similar technique.

To help ensure maximal efficiency in the introduction of pSV-Pf into CHO cells in culture, the transfection is carried out in the presence of a second plasmid, pSV2NEO (ATCC #37149) and the cytotoxic drug G418 (GIBCO Laboratories, Grand Island, N.Y.) as described by Southern et al. (1982) J. Mol. Appl. Genet., 1:327. Those CHO cells that are resistant to G418 are cultured, have acquired both plasmids, pSV2NEO and pSV-Pf, and are designated CHO/pSV-Pf cells. By virtue of the genetic architecture of the pSV-Pf expression vector, a chimer is expressed in the resulting CHO/pSV-Pf cells and can be detected in and purified from the cytoplasm of these cells. The resulting composition containing cellular protein is separated on a column as discussed elsewhere herein.

The choice of which expression vector and ultimately to which promoter a chimer-encoding gene is operatively linked depends directly on the functional properties desired, e.g. the location and timing of protein expression, and the host cell to be transformed. These are well known limitations inherent in the art of constructing recombinant DNA molecules. However, a vector useful in practicing the present invention can direct the replication, and preferably also the expression (for an expression vector) of the chimer gene included in the DNA segment to which it is operatively linked.

In one preferred embodiment, the host that expresses the chimer is the prokaryote, *E*. *coli,* and a preferred vector includes a prokaryotic replicon; i.e., a DNA sequence having the ability to direct autonomous replication and maintenance of the recombinant DNA molecule extrachromosomally in a prokaryotic host cell transformed therewith. Such replicons are well known in the art.

Those vectors that include a prokaryotic replicon can also include a prokaryotic promoter region capable of directing the expression of a contemplated HBc chimer gene in a host cell, such as *E. coli,* transformed therewith. Promoter sequences compatible with bacterial hosts are typically provided in plasmid vectors containing one or more convenient restriction sites for insertion of a contemplated DNA segment. Typical of such vector plasmids are pUC8, pUC9, and pBR329 available from Biorad Laboratories, (Richmond, CA) and pPL and pKK223-3 available from Pharmacia, Piscataway, NJ.

Typical vectors useful for expression of genes in cells from higher plants and mammals are well known in the art and include plant vectors derived from the tumor-inducing (Ti) plasmid of *Agrobacterium* tumefaciens described by Rogers et al. (1987) Meth. in Enzymol., 153:253-277 and mammalian expression vectors pKSV-10, above, and pCI-neo (Promega Corp., #E1841, Madison, WI). However, several other expression vector systems are known to function in plants including pCaMVCN transfer control vector described by Fromm et al. (1985) Proc. Natl. Acad. Sci. USA, 82:58-24. Plasmid pCaMVCN (available from Pharmacia, Piscataway, NJ) includes the cauliflower mosaic virus CaMV 35S promoter.

The above plant expression systems typically provide systemic or constitutive expression of an inserted transgene. Systemic expression can be useful where most or all of a plant is used as the source to a contemplated chimer molecule or resultant particles or where a large part of the plant is used to provide an oral vaccine. However, it can be more efficacious to express a chimer molecule or particles in a plant storage organ such as a root, seed or fruit from which the particles can be more readily isolated or ingested.

One manner of achieving storage organ expression is to use a promoter that expresses its controlled gene in one or more preselected or predetermined non-photosynthetic plant organs. Expression in one or more preselected storage organs with little or no expression in other organs such as roots, seed or fruit versus leaves or stems is referred to herein as enhanced or preferential expression. An exemplary promoter that directs expression in one or more preselected organs as compared to another organ at a ratio of at least 5:1 is defined herein as an organ-enhanced promoter. Expression in substantially only one storage organ and substantially no expression in other storage organs is referred to as organ-specific expression; i.e., a ratio of expression products in a storage organ relative to another of about 100:1 or greater indicates organ specificity. Storage organ-specific promoters are thus members of the class of storage organ-enhanced promoters.

Exemplary plant storage organs include the roots of carrots, taro or manioc, potato tubers, and the meat of fruit such as red guava, passion fruit, mango, papaya, tomato, avocado, cherry, tangerine, mandarin, palm, melons such cantaloupe and watermelons and other fleshy fruits such as squash, cucumbers, mangos, apricots, peaches, as well as the seeds of maize (corn), soybeans, rice, oil seed rape and the like.

The CaMV 35S promoter is normally deemed to be a constitutive promoter. However, recent research has shown that a 21-bp region of the CaMV 35S promoter, when operatively linked into another, heterologous usual green tissue promoter, the rbcS-3A promoter, can cause the resulting chimeric promoter to become a root-enhanced promoter. That 21-bp sequence is disclosed in U.S. Patent No. 5,023,179. The chimeric rbcS-3A promoter containing the 21-bp insert of U.S. Patent No. 5,023,179 is a useful root-enhanced promoter herein.

A similar root-enhanced promoter that includes the above 21-bp segment is the -90 to +8 region of the CAMV 35S promoter itself. U.S. Patent No. 5,110,732 discloses that that truncated CaMV 35S promoter provides enhanced expression in roots and the radical of seed, a tissue destined to become a root. That promoter is also useful herein.

Another useful root-enhanced promoter is the -1616 to -1 promoter of the oil seed rape (*Brassica napus L*.) gene disclosed in PCT/GB92/00416 (WO 91/13922 published Sep. 19, 1991). *E*. *coli* DH5.alpha. harboring plasmid pRlambdaS4 and bacteriophage lambda.beta.1 that contain this promoter were deposited at the National Collection of Industrial and Marine Bacteria, Aberdeen, GB on Mar. 8, 1990 and have accession numbers NCIMB40265 and NCIMB40266. A useful portion of this promoter can be obtained as a 1.0 kb fragment by cleavage of the plasmid with HaeIII.

A preferred root-enhanced promoter is the mannopine synthase (mas) promoter present in plasmid pKan2 described by DiRita and Gelvin (1987) Mol. Gen. Genet, 207:233-241. This promoter is removable from its plasmid pKan2 as a XbaI-XbalI fragment.

The preferred mannopine synthase root-enhanced promoter is comprised of the core mannopine synthase (mas) promoter region up to position -138 and the mannopine synthase activator from -318 to - 213, and is collectively referred to as AmasPmas. This promoter has been found to increase production in tobacco roots about 10- to about 100-fold compared to leaf expression levels.

Another root specific promoter is the about 500 bp 5' flanking sequence accompanying the hydroxyproline-rich glycopeprotein gene, HRGPnt3, expressed during lateral root initiation and reported by Keller et al. (1989) Genes Dev., 3:1639-1646. Another preferred root-specific promoter is present in the about -636 to -1 5' flanking region of the tobacco root-specific gene ToRBF reported by Yamamoto et al. (1991) Plant Cell, 3:371-381. The cis-acting elements regulating expression are more specifically located by those authors in the region from about -636 to about -299 5' from the transcription initiation site. Yamamoto et al. reported steady state mRNA production from the ToRBF gene in roots, but not in leaves, shoot meristems or stems.

Still another useful storage organ-specific promoter are the 5' and 3' flanking regions of the fruit-ripening gene E8 of the tomato, *Lycopersicon esculentum.* These regions and their cDNA sequences are illustrated and discussed in Deikman et al. (1988) EMBO J. , 7 (11) :3315-3320 and (1992) Plant Physiol., 100:2013-2017.

Three regions are located in the 2181 bp of the 5' flanking sequence of the gene and a 522 bp sequence 3' to the poly (A) addition site appeared to control expression of the E8 gene. One region from -2181 to -1088 is required for activation of E8 gene transcription in unripe fruit by ethylene and also contributes to transcription during ripening. Two further regions, -1088 to -863 and -409 to -263, are unable to confer ethylene responsiveness in unripe fruit but are sufficient for E8 gene expression during ripening.

The maize sucrose synthase-1 (Sh) promoter that in corn expresses its controlled enzyme at high levels in endosperm, at much reduced levels in roots and not in green tissues or pollen has been reported to express a chimeric reporter gene, β-glucuronidase (GUS), specifically in tobacco phloem cells that are abundant in stems and roots. Yang et al. (1990) Proc. Natl. Acad. Sci., U.S.A., 87:4144-4148. This promoter is thus useful for plant organs such as fleshy fruits like melons, e.g. cantaloupe, or seeds that contain endosperm and for roots that have high levels of phloem cells.

Another exemplary tissue-specific promoter is the lectin promoter, which is specific for seed tissue. The lectin protein in soybean seeds is encoded by a single gene (Le1) that is only expressed during seed maturation and accounts for about 2 to about 5 percent of total seed mRNA. The lectin gene and seed-specific promoter have been fully characterized and used to direct seed specific expression in transgenic tobacco plants. See, e.g., Vodkin et al. (1983) Cell, 34:1023 and Lindstrom et al. (1990) Developmental Genetics, 11:160.

A particularly preferred tuber-specific expression promoter is the 5' flanking region of the potato patatin gene. Use of this promoter is described in Twell et al. (1987) Plant Mol. Biol., 9:365-375. This promoter is present in an about 406 bp fragment of bacteriophage LPOTI. The LPOTI promoter has regions of over 90 percent homology with four other patatin promoters and about 95 percent homology over all 400 bases with patatin promoter PGT5. Each of these promoters is useful herein. See, also, Wenzler et al. (1989) Plant Mol. Biol., 12:41-50.

Still further organ-enhanced and organ-specific promoter are disclosed in Benfey et al. (1988) Science, 244:174-181.

Each of the promoter sequences utilized is substantially unaffected by the amount of chimer molecule or particles in the cell. As used herein, the term "substantially unaffected" means that the promoter is not responsive to direct feedback control (inhibition) by the chimer molecules or particles accumulated in transformed cells or transgenic plant.

Transfection of plant cells using *Agrobacterium tumefaciens* is typically best carried out on dicotyledonous plants. Monocots are usually most readily transformed by so-called direct gene transfer of protoplasts. Direct gene transfer is usually carried out by electroportation, by polyethyleneglycol-mediated transfer or bombardment of cells by microprojectiles carrying the needed DNA. These methods of transfection are well-known in the art and need not be further discussed herein. Methods of regenerating whole plants from transfected cells and protoplasts are also well-known, as are techniques for obtaining a desired protein from plant tissues. See, also, U.S. Patents No. 5,618,988 and 5,679,880 and the citations therein.

A transgenic plant formed using *Agrobacterium* transformation, electroportation or other methods typically contains a single gene on one chromosome. Such transgenic plants can be referred to as being heterozygous for the added gene. However, inasmuch as use of the word "heterozygous" usually implies the presence of a complementary gene at the same locus of the second chromosome of a pair of chromosomes, and there is no such gene in a plant containing one added gene as here, it is believed that a more accurate name for such a plant is an independent segregant, because the added, exogenous chimer molecule-encoding gene segregates independently during mitosis and meiosis. A transgenic plant containing an organ-enhanced promoter driving a single structural gene that encodes a contemplated HBc chimeric molecule; i.e., an independent segregant, is a preferred transgenic plant.

More preferred is a transgenic plant that is homozygous for the added structural gene; i.e., a transgenic plant that contains two added genes, one gene at the same locus on each chromosome of a chromosome pair. A homozygous transgenic plant can be obtained by sexually mating (selfing) an independent segregant transgenic plant that contains a single added gene, germinating some of the seed produced and analyzing the resulting plants produced for enhanced chimer particle accumulation relative to a control (native, non-transgenic) or an independent segregant transgenic plant. A homozygous transgenic plant exhibits enhanced chimer particle accumulation as compared to both a native, non-transgenic plant and an independent segregant transgenic plant.

It is to be understood that two different transgenic plants can also be mated to produce offspring that contain two independently segregating added, exogenous (heterologous) genes. Selfing of appropriate progeny can produce plants that are homozygous for both added, exogenous genes that encode a chimeric HBc molecule. Back-crossing to a parental plant and out-crossing with a non-transgenic plant are also contemplated.

A transgenic plant of this invention thus has a heterologous structural gene that encodes a contemplated chimeric HBc molecule. A preferred transgenic plant is an independent segregant for the added heterologous chimeric HBc structural gene and can transmit that gene to its progeny. A more preferred transgenic plant is homozygous for the heterologous gene, and transmits that gene to all of its offspring on sexual mating.

Inasmuch as a gene that encodes a chimeric HBc molecule does not occur naturally in plants, a contemplated transgenic plant accumulates chimeric HBc molecule particles in a greater amount than does a non-transformed plant of the same type or strain when both plants are grown under the same conditions.

The phrase "same type" or "same strain" is used herein to mean a plant of the same cross as or a clone of the untransformed plant. Where alleic variations among siblings of a cross are small, as with extensively inbred plant, comparisons between siblings can be used or an average arrived at using several siblings. Otherwise, clones are preferred for the comparison.

Seed from a transgenic plant is grown in the field greenhouse, window sill or the like, and resulting sexually mature transgenic plants are self-pollinated to generate true breeding plants. The progeny from these plants become true breeding lines that are evaluated for chimeric HBc molecule particle accumulation, preferably in the field, under a range of environmental conditions.

A transgenic plant homozygous for chimeric HBc molecule particle accumulation is crossed with a parent plant having other desired traits. The progeny, which are heterozygous or independently segregatable for chimeric HBc molecule particle accumulation, are backcrossed with one or the other parent to obtain transgenic plants that exhibit chimeric HBc molecule particle accumulation and the other desired traits. The backcrossing of progeny with the parent may have to be repeated more than once to obtain a transgenic plant that possesses a number of desirable traits.

An insect cell system can also be used to express a HBc chimer. For example, in one such .system *Autographa californica* nuclear polyhedrosis virus (AcNPV) or baculovirus is used as a vector to express foreign genes in *Spodoptera frugiperda* cells or in Trichoplusia larvae.

The sequences encoding a chimer can be cloned into a non-essential region of the virus, such as the polyhedrin gene, and placed under control of the polyhedrin promoter. Successful insertion of chimer sequence renders the polyhedrin gene inactive and produces recombinant virus lacking coat protein. The recombinant viruses can then be used to infect, for example, *S. Frugiperda* cells or *Trichoplusia* larvae in which the HBc chimer can be expressed. E. Engelhard et al. (1994) Proc. Natl. Acad. Sci., USA, 91:3224-3227; and V. Luckow, Insect Cell Expression Technology, pp. 183-218, in Protein Engineering: Principles and Practice, J.L. Cleland et al. eds., Wiley-Liss, Inc, 1996). Heterologous genes placed under the control of the polyhedrin promoter of the *Autographa californica* nuclear polyhedrosis virus (AcNPV) are.often expressed at high levels during the late stages of infection.

Recombinant baculoviruses containing the chimeric gene are constructed using the baculovirus shuttle vector system (Luckow et al. (1993) J. Virol., 67:4566-4579], sold commercially as the Bac-To-Bac™ baculovirus expression system (Life Technologies). Stocks of recombinant viruses are prepared and expression of the recombinant protein is monitored by standard protocols (O'Reilly et al., Baculovirus Expression Vectors: A Laboratory Manual, W.H. Freeman and Company, New York, 1992; and King et al., The Baculovirus Expression System: A Laboratory Guide, Chapman & Hall, London, 1992).

A variety of methods have been developed to operatively link DNA to vectors via complementary cohesive termini or blunt ends. For instance, complementary homopolymer tracts can be added to the DNA segment to be inserted into the vector DNA. The vector and DNA segment are then joined by hydrogen bonding between the complementary homopolymeric tails to form recombinant DNA molecules.

Alternatively, synthetic linkers containing one or more restriction endonuclease sites can be used to join the DNA segment to the expression vector, as noted before. The synthetic linkers are attached to blunt-ended DNA segments by incubating the blunt-ended DNA segments with a large excess of synthetic linker molecules in the presence of an enzyme that is able to catalyze the ligation of blunt-ended DNA molecules, such as bacteriophage T4 DNA ligase. Thus, the products of the reaction are DNA segments carrying synthetic linker sequences at their ends. These DNA segments are then cleaved with the appropriate restriction endonuclease and ligated into an expression vector that has been cleaved with an enzyme that produces termini compatible with those of the synthetic linker. Synthetic linkers containing a variety of restriction endonuclease sites are commercially available from a number of sources including New England BioLabs, Beverly, MA.

A desired DNA segment can also be obtained using PCR technology in which the forward and reverse primers contain desired restriction sites that can be cut after amplification so that the gene can be inserted into the vector. Alternatively PCR products can be directly cloned into vectors containing T-overhangs (Promega Corp., A3600, Madison, WI) as is well known in the art.

The expressed chimeric protein self-assembles into particles within the host cells, whether in single cells or in cells within a multicelled host. The particle-containing cells are harvested using standard procedures, and the cells are lysed using a French pressure cell, lysozyme, sonicator, bead beater or a microfluidizer (Microfluidics International Corp., Newton MA). After clarification of the lysate, particles are precipitated with 45 percent ammonium sulfate, resuspended in 20 mM sodium phosphate, pH 6.8 and dialyzed against the same buffer. The dialyzed material is clarified by brief centrifugation and the supernatant subjected to gel filtration chromatography using Sepharose^{®} CL-4B. Particle-containing fractions are identified, subjected to hydroxyapatite chromatography, and reprecipitated with ammonium sulfate prior to resuspension, dialysis and sterile filtration and storage at -70°C.

### HBc Chimer Conjugates

Any hapten (immunogen) to which a B cell or T cell response is desired can be linked to a contemplated HBc chimer or chimer particle such as a chimer particle containing a heterologous linker residue such as a lysine, glutamic or aspartic acid, cysteine or tyrosine in the loop region of Domain II and an added cysteine residue near the N-terminus in Domain I to form a HBc chimer conjugate. The hapten of interest typically is a B cell immunogen. The hapten can be a polypeptide, a protein, a carbohydrate (saccharide; i.e., oligo- or polysaccharide), or a non-polypeptide, non-carbohydrate chemical such as 2,4-dinitrobenzene or a medicament such as cocaine or nicotine. Illustrative saccharide compounds include lipooligosaccharides (LOS) of NTHi or *M. cat.* [Sun et. al. (2000) Vaccine 2000, 18(13):1264-1272; and Jiao et. al. (2002) Infect. Immun., 70(11):5982-5989]. LOS consist of a hydrophobic lipid A portion and a hydrophilic core oligosaccharide portion and are one of the major components in the outer membranes of gram-negative bacteria. Detoxified LOS (dLOS) molecules are de-O-acylated or de-N-acylated, or both, and exhibit about 100- to about 10,000-fold diminished endotoxicity compared with the starting LOS in the *Limulus* amoebocyte lysate (LAL) assay as described in Hochstein (1990) in Clinical application of the Limulus amoebocyte lysate test, R.B. Prior, ed. CRC Press, Inc., Boca Raton, FL, pages 38-49.

There are many methods known in the art to couple carrier proteins to polysaccharides. Aldehyde groups can be prepared on either the reducing end [Anderson (1983) Infect. Immun., 39:233-238; Jennings et al. (1981) J. Immunol., 127:1011-1018; Poren et al. (1985) Mol. Immunol., 22:907-919] or the terminal end [Anderson et al. (1986) J. Immunol., 137:1181-1186; Beuvery et al. (1986) Dev. Bio. Scand., 65:197-204] of an oligosaccharide or relatively small polysaccharide, which can be linked to the carrier protein via reductive amination. In addition, oxidation of adjacent hydroxyls by periodate ion followed by reductive amination of the resulting aldehyde using cyanoborohydride is particularly useful for those HBc chimer molecules having an added ε-amine-supplying lysine residue added into the immunogenic loop of the chimer.

Large polysaccharides can be conjugated by either terminal activation [Anderson et al.(1986) J. Immunol., 137:1181-1186] or by random activation of several functional groups along the polysaccharide chain [Chu et al. (1983) Inflect. Immun., 40:245-256; Gordon, U.S. Patent No. 4,619,828 (1986); Marburg, U.S. Patent No. 4,882,317 (1989)]. Random activation of several functional groups along the polysaccharide chain can lead to a conjugate that is highly cross-linked due to random linkages along the polysaccharide chain. The optimal ratio of polysaccharide to carrier protein depends on the particular polysaccharide, the carrier protein, and the conjugate used.

Detailed reviews of methods of conjugation of saccharide to carrier proteins can be found in Dick et al., in Contributions to Microbiology and Immunology, Vol. 10, Cruse et al., eds., (S. Karger: 1989), pages 48-114; Jennings et al., in Neoglycoconjugates: Preparation and Applications, Lee et al., eds., (Academic Press: 1994), pages 325-371; Aplin et al., (1981) CRC Crit. Rev. Biochem., 10:259-306; and Stowell et al.(1980) Adv. Carbohydr. Chem. Biochem., 37:225-281.

The carbohydrate itself can be synthesized by methods known in the art, for example by enzymatic glycoprotein synthesis as described by Witte et al. (1997) J. Am. Chem. Soc., 119:2114-2118.

Several oligosaccharides, synthetic and semi-synthetic, and natural, are discussed in the following paragraphs as examples of oligosaccharides that are contemplated haptens to be used in making a HBc conjugate of the present invention.

An oligosaccharide hapten suitable for preparing vaccines for the treatment of *Haemophilus influenza* type b (Hib) is made up of from 2 to 20 repeats of D-ribose-D-ribitol-phosphate (I, below), D-ribitol-phosphate-D-ribose (II, below), or phosphate-D-ribose-D-ribitol (III, below). Eduard C. Beuvery et al., EP-0 276 516-B1.

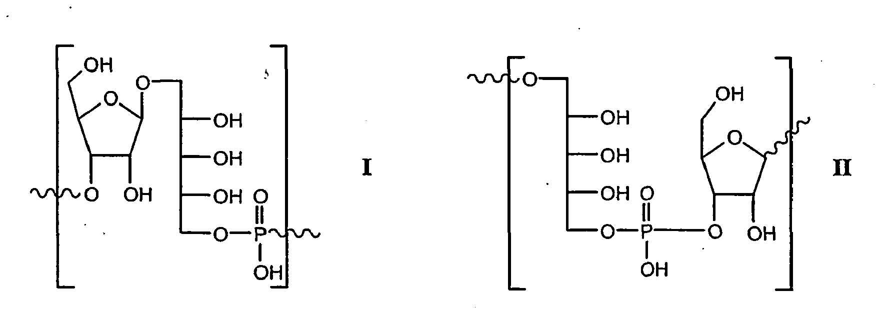

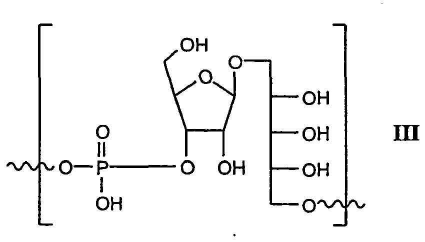

U.S. Patent No. 4,220,717 also discloses a polyribosyl ribitol phosphate (PRP) hapten for *Haemophilus influenzae* type b.

Peterson et al. (1998) Infect. Immun., 66(8):3848-3855, disclose a trisaccharide hapten, αKdo (2 8) αKdo (2 4) αKdo, that provides protection from *Chlamydia pneumoniae*. *Chlamydia pneumoniae* is a cause of human respiratory infections ranging from pharyngitis to fatal pneumonia. Kdo is 3-deoxy-D-manno-oct-2-ulosonic acid.

Andersson et al., EP-0 126 043-A1, disclose saccharides that can be used in the treatment, prophylaxis or diagnosis of bacterial infections caused by *Streptococci pneumoniae.* One class of useful saccharides is derived from the disaccharide GlcNAcβ1 3Gal. Andersson et al.,above, also reported neolactotetraosylceramide to be useful, which is Galβ1 4GlcNAcβ1 3Galβ1 4Glc-Cer.

McKenney et al. (1999) Science, 284:1523-1527, disclose a polysaccharide, poly-N-succinyl β1 6GlcN (PNSG) that provides protection from *Staphylococcus aureus. S. aureus* is a common cause of community-acquired infections, including endocarditis, osetemylitis, septic arthritis, pneumonia, and abscesses.

European Patent No. 0 157 899-B1 discloses the isolation of pneumococcal polysaccharides that are useful in the present invention. The following table lists the pneumococcal culture types that produce capsular polysaccharides useful as haptens in the present invention.

Polysaccharide Hapten Sources

| Danish Type Nomenclature | U.S. Nomenclature | 1978 ATCC Catalogue Number |
|---|---|---|
| 1 | 1 | 6301 |
| 2 | 2 | 6302 |
| 3 | 3 | 6303 |
| 4 | 4 | 6304 |
| 5 | 5 | |
| 6A | 6 | 6306 |
| 6B | 26 | 6326 |
| 7F | 51 | 10351 |
| 8 | 8 | 6308 |
| 9N | 9 | 6309 |
| 9V | 68 | |
| 10A | 34 | |
| 11A | 43 | |
| 12F | 12 | 6312 |
| 14 | 14 | 6314 |
| 15B | 54 | |
| 17F | 17 | |
| 18C | 56 | 10356 |
| 19A | 57 | |
| 19F | 19 | 6319 |
| 20 | 20 | 6320 |
| 22F | 22 | |
| 23F | 23 | 6323 |
| 25 | 25 | 6325 |
| 33F | 70 | |

*Moraxella (Branhamella) catarrhalis* is a reported cause of otitis media and sinusitis in children and lower respiratory tract infections in adults. The lipid A portion of the lipooligosaccharide (LOS) surface antigen of the bacterium is cleaved at the 3-deoxy-D-*manno*-octulosonic acid-glucosamine linkage. The cleavage product is treated with mild alkali or hydrazine to remove ester-linked fatty acids, while preserving amide-linked fatty acids to yield detoxified lipooligosaccharide (dLOS) from *M. catarrhalis.* The dLOS is not immunogenic until it is attached to a protein carrier. Xin-Xing Gu et al. (1998) Infect. Immun., 66(5):1891-1897.

Group B streptococci (GBS) cause of sepsis, meningitis, and related neurologic disorders in humans. The capsular polysaccharide-specific antibodies are known to protect human infants from infection. Jennings et al., U.S. Patent No. 5,795,580. The repeating unit of the GBS capsular polysaccharide type II is: 4) -(β-D-Glc*p*NAc-(1 3)-[β-D-Gal*p*(1 6)] - β-D-Gal*p* (1 4) - β-D-Glc*p*- (1 3) - β-D-Gl*cp-*(1 2) - [α-D-Neu*p*NAc (2 3) ] - β-D-Gal*p*- (1, where the bracketed portion is a branch connected to the unbracketed subunit immediately following. The repeating unit of GBS capsular polysaccharide type V is: 4)-[α-D-Neu*p*NAc-(2 3)-β-D-Gal*p*-(1 4)-β-D-Glc*p*NAc- (1 6) ] -α-D-Glc*p*- (1 4) - [β-D-Glc*p*-(1 3)] -β-D-Gal*p*- (1 4) -β-D-Glc*p*- (1.

European patent application No. EU-0 641 568-A1, Brade, discloses the method of obtaining ladder-like banding pattern antigen from *Chlamydia trachomatis, pneumoniae* and *psittaci.*

Slovin et al., (1999) Proc. Natl. Acad. Sci., U.S.A., 96(10):5710-5715 report use of a Synthetic oligosaccharide, globo H, linked to KLH as a carrier in the preparation of a vaccine used against prostate cancer. Similarly, Helling et al., (July 1995) Cancer Res., 55:2783-2788 report the use of KLH-linked G_{M2} in a vaccine for treating patients with melanoma. The latter vaccine was prepared by ozone cleavage of the ceramide double bond of G_{M2}, introduction of an aldehyde group and reductive amination onto KLH. A similar procedure can be utilized with a contemplated chimer particle.

Oligosaccharidal portions of sphingolipids such as globosides and gangliosides that are present on the surface of other tumor cells as well as normal cells such as melanoma, neuroblastoma and healthy brain cells, can similarly be used herein as haptens. The oligosaccharide portion of the globoside globo H has the structure Fucα-(1 2)-Galβ(1 3)-GalNAcβ-(1 3)-Gala-(1 4)-Galβ-(1 4)Glc, whereas the saccharide protions of gangliosides G_{M2}, G_{M1} and G_{D1a} have the following structures: GalNAcβ-(1 4 ) - [NeuAcα- (2 3)]-Galβ- (1 4) -Glc; Galβ- (1 3) -GalNAcβ- (1 4) - [NeuAcα-(2 3)] -Galβ- (1 4) -Glc; and NeuAc-(2 3) -Galβ- (1 3)-GalNAcβ- (1 4) - [NeuAcα- (2 3)]-galop-(1 4)-Glc, respectively.

U.S. Patent No. 4,356,170 discloses the preparation of useful polysaccharides that are reduced and then oxidized to form compounds having terminal aldehyde groups that can be reductively aminated onto free amine groups of carrier proteins such as tetanus toxoid and diphtheria toxoid with or without significant cross-linking. Exemplary useful bacterial polysaccharides include β-hemolytic streptococci, *Haemophilus influenza*, meningococci, pneumococci and *E. coli.* Rather than reductively aminating the particles, a linker arm such as that provided by an ε-amino C₂-C₈ alkylcarboxylic acid can be reductively aminated on to the polysaccharide, followed by linkage to the particles using a watersoluble carbodiimide.

It is thus seen that the word "hapten" is used herein somewhat more broadly than is usual, to include small molecules that do not themselves induce an immune response, as well as larger molecule such as proteins that often can themselves induce an immune response. An HBc chimer particle conjugate so formed is useful as an inoculum or vaccine, as is discussed hereinafter. Because the chimer protein self assembles after expression and a conjugate is formed after expression, conjugate formation is typically done using the assembled particles as compared to the free protein molecules.

Methods for operatively linking individual haptens (immunogens) to a protein or polypeptide through an amino acid residue side chain of the protein or polypeptide to form a pendently-linked immunogenic conjugate, e.g., a branched-chain polypeptide polymer, are well known in the art. Those methods include linking through one or more types of functional groups on various side chains and result in the carrier protein polypeptide backbone (here, a HBc chimer) within the particle being pendently linked--covalently linked (coupled)-- to the hapten but separated by at least one side chain.

Methods for linking carrier proteins to haptens using each of the above functional groups are described in Erlanger, (1980) Method of Enzymology, 70:85; Aurameas et al., (1978) Scand. J. Immunol., Vol. 8, Suppl. 7, 7-23 and U.S. Patent No. 4,493,795 to Nestor et al*.* In addition, a site-directed coupling reaction, as described in Rodwell et al. (1985) Biotech., 3:889-894 can be carried out so that the biological activity of the polypeptides is not substantially diminished.

Furthermore, as is well known in the art, both the HBc protein and a polypeptide hapten can be used in their native form or their functional group content can be modified by succinylation of lysine residues or reaction with cysteine-thiolactone. A sulfhydryl group can also be incorporated into either carrier protein or conjugate by reaction of amino functional groups with 2-iminothiolane, the N-hydroxysuccinimide ester of 3-(3-dithiopyridyl)-propionate, or other reagents known in the art.

The HBc chimer or hapten can also be modified to incorporate a spacer arm, such as hexamethylenediamine or another bifunctional molecule, to facilitate the pendent linking. Such a procedure is discussed below.

Methods for covalent bonding of a polypeptide hapten are extremely varied and are well known by workers skilled in the immunological arts. For example, following U.S. Patent No. 4,818,527, m-maleimidobenzoyl-N-hydroxysuccinimide ester (ICN Biochemicals, Inc., Costa Mesa, CA) or succinimidyl 4-(N-maleimidomethyl)cyclohexane-1-carboxylate (SMCC, Pierce Chemical Co., Rockford, IL) is reacted with an appropriate HBc chimer to form an activated carrier. That activated carrier is then reacted with a hapten such as a sulfhydryl-terminated hapten or a polypeptide that either contains a terminal cysteine or to which an additional amino- or carboxy-terminal cysteine residue has been added to form a covalently bonded HBc chimer conjugate. As an alternative example, the amino group of a polypeptide hapten can be first reacted with N-succinimidyl 3-(2-pyridylthio)propionate (SPDP, Pharmacia, Piscataway, NJ), and that thiol-containing polypeptide can be reacted with the activated carrier after reduction. Of course, the sulfur-containing moiety and double bond-containing Michael acceptor can be reversed. These reactions are described in the supplier's literature, and also in Kitagawa, et al. (1976) J. Biochem., 79:233 and in Lachmann et al., in 1986 Synthetic Peptides as Antigens, (Ciba Foundation Symposium 119), pp. 25-40 (Wiley, Chichester: 1986). See also, Fig. 5.

U.S. Patent No. 4,767,842 teaches several modes of covalent attachment between a carrier and polypeptide that are useful here. In one method, tolylene diisocyanate is reacted with the carrier in a dioxane-buffer solvent at zero degrees C to form an activated carrier. A polypeptide hapten is thereafter admixed and reacted with the activated carrier to form the covalently bonded HBc chimer conjugate.

Particularly useful are a large number of heterobifunctional agents that form a disulfide link at one functional group end and an amide link at the other, including N-succidimidyl-3-(2-pyridyldithio)-propionate (SPDP), discussed before that creates a disulfide linkage between itself and a thiol in either the HBc chimer or the hapten. Exemplary reagents include a cysteine residue in a polypeptide hapten and an amine on the coupling partner such as the ε-amine of a lysine or other free amino group in the carrier protein. A variety of such disulfide/amide forming agents are known. See for example Immun. Rev. (1982) 62:185.

Other bifunctional coupling agents form a thioether rather than a disulfide linkage. Many of these thioether-forming agents are commercially available and include reactive esters of 6-maleimidocaproic acid, 2-bromoacetic acid, 2-iodoacetic acid, 4-(N-maleimidomethyl)cyclohexane-1-carboxylic acid and the like. The carboxyl groups can be activated by combining them with succinimide or 1-hydroxy-2-nitro-4-sulfonic acid, sodium salt. The particularly preferred coupling agent for the method of this invention is succinimidyl 4-(maleimidomethyl)cyclohexane-1-carboxylate (SMCC) obtained from Pierce Chemical Co., Rockford, IL. Another heterobifunctional cross-linker is N-[γ-maleimidobutyroxy)succinimide ester (GMBS) and its sulfonate salt (sulfo-GMBS), and similar compounds as are available from Pierce Chemical Co. The foregoing list is not meant to be exhaustive, and modifications of the named compounds can clearly be used. Fig. 5 provides a schematic representation (Scheme 1) of the formation of a HBc activated carrier using SMCC (I) and the subsequent reaction of that activated carrier with a sulfhydryl-terminated hapten (II).

A polypeptide hapten can be obtained in a number of ways well known in the art. Usual peptide synthesis techniques can be readily utilized. For example, recombinant and PCR-based techniques to produce longer peptides are useful. Because the desired sequences are usually relatively short, solid phase chemical synthesis is useful.

Exemplary polypeptide haptens are shown in Tables A and B hereinbefore. Each of those polypeptides can be utilized via its N-terminal amino group, or by use of an additional N-terminal cysteine that is not shown in the table.

Related chemistry is used to couple what may be called "chemical compounds" to carrier proteins. Typically, an appropriate functional group for coupling is designed into the chemical compound. An exemplary chemical hapten to which induced antibodies protect against *Streptococcus pneumoniae* is 6-*O*-phosphocholine hydroxyhexanoate. Fischer et al- (1995) J. Immunol., 154:3373-3382. The table below provides further exemplary chemical haptens.

Further specifics concerning useful haptens can be found in before-mentioned, and now published, U.S. Patent Application Serial No. 09/930,915 and the other parental applications.

### Inocula and Vaccines

A HBC chimer particle or HBc chimer particle conjugate with a hapten may be used as the immunogen of an inoculum or vaccine in an human patient or suitable animal host such as a chimpanzee, mouse, rat, horse, sheep or the like. An inoculum can induce a B cell or T cell response (stimulation) such as production of antibodies that immunoreact with the immunogenic epitope or hapten, or T cell activation, whereas a vaccine provides protection against the entity from which the immunogen has been derived via one or both of a B cell or T cell response.

T cell activation can be measured by a variety of techniques. In usual practice, a host animal is inoculated with a contemplated HBc chimer particle vaccine or inoculum, and peripheral mononuclear blood cells (PMBC) are thereafter collected. Those PMBC are then cultured *in vitro* in the presence of the T cell immunogen for a period of about three to five days. The cultured PMBC are then assayed for proliferation or secretion of a cytokine such as IL-2, GM-CSF of IFN-γ. Assays for T cell activation are well known in the art. See, for example; U. S. Patent No. 5,478,726 and the art cited therein.

Using antibody formation as exemplary, a contemplated inoculum or vaccine comprises an immunogenically effective amount of HBc chimer particles or HBc chimer particle conjugates that are dissolved or dispersed in a pharmaceutically acceptable diluent composition that typically also contains water. When administered to a host animal in need of immunization or in which antibodies are desired to be induced such as a mammal (e.g., a mouse, dog, goat, sheep, horse, bovine, monkey, ape, or human) or bird (e.g., a chicken, turkey, duck or goose), an inoculum induces antibodies that immunoreact with the genetically linked or conjugated (pendently-linked) hapten. Those antibodies also preferably bind to the protein or saccharide of the B cell immunogen.

The amount of recombinant HBc chimer immunogen utilized in each immunization is referred to as an immunogenically effective amount and can vary widely, depending *inter alia,* upon the recombinant HBc chimer immunogen, patient immunized, and the presence of an adjuvant in the vaccine, as discussed below. Immunogenically effective amounts for a vaccine and an inoculum provide the protection or antibody activity, respectively, discussed hereinbefore.

Vaccines or inocula typically contain a recombinant HBc chimer immunogen concentration of about 1 microgram to about 1 milligram per inoculation (unit dose), and preferably about 10 micrograms to about 50 micrograms per unit dose. The term "unit dose" as it pertains to a vaccine or inoculum of the present invention refers to physically discrete units suitable as unitary dosages for animals, each unit containing a predetermined quantity of active material calculated to individually or collectively produce the desired immunogenic effect in association with the required diluent; i.e., carrier, or vehicle.

vaccines or inocula are typically prepared from a recovered recombinant HBc chimer immunogen by dispersing the immunogen, preferably in particulate form, in a physiologically tolerable (acceptable) diluent vehicle such as water, saline, phosphate-buffered saline (PBS), acetate-buffered saline (ABS), Ringer's solution, or the like to form an aqueous composition. The diluent vehicle can also include oleaginous materials such as peanut oil, squalane, or squalene as is discussed hereinafter.

The preparation of inocula and vaccines that contain proteinaceous materials as active ingredients is also well understood in the art. Typically, such inocula or vaccines are prepared as parenterals, either as liquid solutions or suspensions; solid forms suitable for solution in, or suspension in, liquid prior to injection can also be prepared. The preparation can also be emulsified, which is particularly preferred.

The immunogenically active ingredient is often mixed with excipients that are pharmaceutically acceptable and compatible with the active ingredient. Suitable excipients are, for example, water, saline, dextrose, glycerol, ethanol, or the like and combinations thereof. In addition, if desired, an inoculum or vaccine can contain minor amounts of auxiliary substances such as wetting or emulsifying agents, pH buffering agents that enhance the immunogenic effectiveness of the composition.

A contemplated vaccine or inoculum advantageously also includes an adjuvant. Suitable adjuvants for vaccines and inocula of the present invention comprise those adjuvants that are capable of enhancing the antibody responses against B cell epitopes of the chimer, as well as adjuvants capable of enhancing cell mediated responses towards T cell epitopes contained in the chimer. Adjuvants are well known in the art (see, for example, Vaccine Design - The Subunit and Adjuvant Approach, 1995, Pharmaceutical Biotechnology, Volume 6, Eds. Powell, M.F., and Newman, M.J., Plenum Press, New York and London, ISBN 0-306-44867-X).

Exemplary adjuvants include complete Freund's adjuvants (CFA) that is not used in humans, incomplete Freund's adjuvant (IFA), squalene, squalane and alum [e.g., Alhydrogel™ (Superfos, Denmark)], which are materials well known in the art, and are available commercially from several sources.

Preferred adjuvants for use with immunogens include aluminum or calcium salts (for example hydroxide or phosphate salts). A particularly preferred adjuvant for use herein is an aluminum hydroxide gel such as Alhydrogel™. For aluminum hydroxide gels, the chimer protein is admixed with the adjuvant so that about 50 to about 800 micrograms of aluminum are present per dose, and preferably about 400 to about 600 micrograms are present. Another particularly preferred adjuvant is aluminum phosphate that is available under trademark Adju-Phos™ from Superfos Biosector, Denmark. Primary aluminum phosphate particles have a plate-like morphology and a diameter of about 50 to about 100 nm, with the final particle size in the product being about 0.5 to about 10 µ. Calcium phosphate nanoparticles (CAP) are an adjuvant being developed by Biosante, Inc (Lincolnshire, IL). The immunogen of interest can be either coated to the outside of particles, or encapsulated inside on the inside ([He et al., (Nov. 2000) Clin. Diagn. Lab. Immunol., 7(6) :899-903].

Another particularly preferred adjuvant for use with an immunogen is an emulsion. A contemplated emulsion can be an oil-in-water emulsion or a water-in-oil emulsion. In addition to the immunogenic chimer protein, such emulsions comprise an oil phase of squalene, squalane, peanut oil or the like as are well-known, and a dispersing agent. Non-ionic dispersing agents' are preferred and such materials include mono- and di-C₁₂-C₂₄-fatty acid esters of sorbitan and mannide such as sorbitan mono-stearate, sorbitan mono-oleate and mannide mono-oleate. An immunogen-contaiuing emulsion is administered as an emulsion.

Preferably, such emulsions are water-in-oil emulsions that comprise squalene and mannide mono-oleate (Arlacel^{™} A), optionally with squalane, emulsified with the chimer protein in an aqueous phase- Well-known examples of such emulsions include Montanide^{™} ISA-720, and Montanide^{™} ISA 703 (Seppic, Castres, France), each of which is understood to contain both squalene and squalane, with squalene predominating in each, but to a lesser extent in Montanide^{™} ISA 703. Most preferably, Montanide^{™} ISA-720 is used, and a ratio of oil-to-water of 7:3 (w/w) is used. Other preferred oil-in-water emulsion adjuvants include those disclosed in WO 95/17210 and EP 0 399 843.

The use of small molecule adjuvants is also contemplated herein. One type of small molecule adjuvant useful herein is a 7-substituted-8-oxo- or 8-sulfo-guanosine derivative described in U.S. Patents No. 4,539,205, No. 4,643,992, No. 5,011,828 and No. 5,093,318, whose disclosures are incorporated by reference. Of these materials, 7-allyl-8-oxoguanosine (loxoribine) is particularly preferred. That molecule has been shown to be particularly effective in inducing an antigen- (immunogen-) specific response.

A preferred useful adjuvant includes monophosphoryl lipid A (MPL), 3-deacyl monophosphoryl lipid A (3D-MPL), a well-known adjuvant manufactured by Ribi Immunochem, Hamilton, Montana. The adjuvant contains three components extracted from bacteria: monophosphoryl lipid (MPL) A, trehalose dimycolate (TDM) and cell wall skeleton (CWS) (MPL+TDM+CWS) in a 2% squalene/Tween^{®} 80 emulsion. This adjuvant can be prepared by the methods taught in GB 2122204B. A preferred form of 3-de-O-acylated monophosphoryl lipid A is in the form of an emulsion having a small particle size less than 0.2 µm in diameter (EP 0 689 454 B1). Most preferred are synthetic monosaccharide analogues of MPL called aminoalkyl glucosamide 4-phosphates (AGPs) such as sold under the designation RC-529 {2-[(R)-3-tetradecanoyloxy-tetradecanoyl-amino]-ethyl-2-deoxy-4-O-phosphono-3-O-[(R)-3-tetradecanoyl-oxytetradecanoyl]-2-[(R)-3-tetra-decanoyloxytetra-decanoylamino]-p-D-glucopyranoside triethylammonium salt}. RC-529 is available in a squalene emulsion sold as RC-529SE and in an aqueous formulation as RC-529AF available from Corixa Corp. (see U.S. Patents No. 4,987,237 and No. 6,113,918). These adjuvants can be used alone or in combination with one or more other adjuvants such as Alhydrogel^{™}.

Further contemplated adjuvants include synthetic oligonucleotide adjuvants containing the CpG nucleotide motif one or more times (plus flanking sequences) available from Coley Pharmaceutical Group. The adjuvant designated QS21, available from Aquila Biopharmaceuticals, Inc., is an immunologically active saponin fractions having adjuvant activity derived from the bark of the South American tree *Quillaja Saponaria Molina* (*e.g.* Quil^{™} A), and the method of its production is disclosed in U.S. Patent No. 5,057,540; semi-syntheic and synthetic derivatives of *Quillaja Saponaria Molina* saponins are also useful, such as those described in U.S. Patents No. 5,977,081 and No. 6,080,725. The adjuvant denominated MF59 available from Chiron Corp. is described in U.S. Patents No. 5,709,879 and No. 6,086,901.

Muramyl dipeptide adjuvants are also contemplated and include N-acetyl-muramyl-L-threonyl-D-isoglutamine (thur-MDP), N-acetyl-nor-muramyl-L-alanyl-D-isoglutamine [CGP 11637, referred to as nor-MDP], and N-acetylmuramyl-L-alanyl-D-isoglutaminyl-L-alanine-2-(1'-2'-dipalmityol-sn-glycero-3-hydroxyphosphoryloxy)ethylamine [(CGP) 1983A, referred to as MTP-PE]. The so-called muramyl dipeptide analogues are described in U.S. Patent No. 4,767,842.

Preferred adjuvant mixtures include combinations of 3D-MPL and QS21 (EP 0 671 948 B1), oil-in-water emulsions comprising 3D-MPL and QS21 (WO 95/17210, PCT/EP98/05714), 3D-MPL formulated with other carriers (EP 0 689 454 B1), QS21 formulated in cholesterol-containing liposomes (WO 96/33739), or immunostimulatory oligonucleotides (WO 96/02555). SBAS2 (now AS02) available from SKB (now Glaxo-SmithKline) contains QS21 and MPL in an oil-in-water emulsion. Alternative adjuvants include those described in WO 99/52549 and non-particulate suspensions of polyoxyethylene ether (UK Patent Application No. 9807805.8).

Adjuvants are utilized in an adjuvant amount, which can vary with the adjuvant, mammal and recombinant HBc chimer immunogen. Typical amounts can vary from about 1 µg to about 1 mg per immunization. Those skilled in the art know that appropriate concentrations or amounts can be readily determined.

Inocula and vaccines are conventionally administered parenterally, by injection, for example, either subcutaneously or intramuscularly. Additional formulations that are suitable for other modes of administration include suppositories and, in some cases, oral formulation. The use of a nasal spray for inoculation is also contemplated as discussed in Neirynck et al. (1999) Nature Med., 5(10):1157-1163. For suppositories, traditional binders and carriers can include, for example, polyalkalene glycols or triglycerides; such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate and the like.

An inoculum or vaccine composition takes the form of a solution, suspension, tablet, pill, capsule, sustained release formulation or powder, and contains an immunogenically effective amount of HBc chimer or HBc chimer conjugate, preferably as particles, as the active ingredient. In a typical composition, an immunogenically effective amount of preferred HBc chimer or HBc chimer conjugate particles is about 1 µg to about 1 mg of active ingredient per dose, and more preferably about 5 µg to about 50 µg per dose, as noted before.

A vaccine is typically formulated for parenteral administration. Exemplary immunizations are carried out sub-cutaneously (SC) intra-muscularly (IM), intravenously (IV), intraperitoneally (IP) or intra-dermally (ID). However, oral and nasal routes of vaccination are also contemplated.

The HBc chimer particles and HBc chimer particle conjugates can be formulated into the vaccine as neutral or salt forms. Pharmaceutically acceptable salts, include the acid addition salts (formed with the free amino groups of the protein or hapten) and are formed with inorganic acids such as, for example, hydrochloric or phosphoric acids, or such organic acids as acetic, oxalic, tartaric, mandelic, and the like. Salts formed with the free carboxyl groups can also be derived form inorganic bases such as, for example, sodium, potassium, ammonium, calcium, or ferric hydroxides, and such organic bases as isopropylamine, trimethylamine, 2-ethylamino ethanol, histidine, procaine, and the like.

A vaccine or inoculum is contemplated in which a gene encoding a contemplated HBc chimer is transfected into suitably attenuated enteric bacteria such as *s*. *typhi*, *S*. *typhimurium, s*. *typhimurium-E*. *coli* hybrids or *E*. *coli.* Exemplary attenuated or avirulent *S. typhi* and *S. typhimurium* and *S*. *typhimurium-E*. *coli* hybrids are discussed in the citations provided before. These vaccines and inocula are particularly contemplated for use against diseases that infect or are transmitter via mucosa of the nose, the gut and reproductive tract such as influenza, yeasts such as *Aspergiullus* and Candida, viruses such as polio, foot-and-mouth disease, hepatitis A, and bacteria such as *Cholera*, *Salmonella* and *E*. *coli* and where a mucosal IgA response is desired in addition to or instead of an IgG systemic response.

The enteric bacteria can be freeze dried, mixed with dry pharmaceutically acceptable diluents, made into tablets or capsules for ingestion and administered to or taken by the host animal as are usual solid phase medications. In addition, aqueous preparations of these bacterial vaccines are adapted for use in mucosal immunization as by oral, nasal, rectal or vaginal administration.

Oral immunization using plant matter containing contemplated chimeric molecule particles can be achieved by simple ingestion of the transgenic plant tissue such as a root, like a carrot, or seed, ouch as rice or corn. In this case, the water of the mouth or gastrointestinal tract provides the usually used aqueous medium used for immunizations and the surrounding plant tissue provides the pharmaceutically acceptable diluent.

The inocula or vaccines are administered in a manner compatible with the dosage formulation, and in such amounts as are therapeutically effective and immunogenic. The quantity to be administered depends on the subject to be treated, capacity of the subject's immune system to synthesize antibodies, and degree of protection desired. Precise amounts of active ingredient required to be administered depend on the judgment of the practitioner and are peculiar to each individual. However, suitable dosage ranges are of the order of tens of micrograms active ingredient per individual. Suitable regimes for initial administration and booster shots are also variable, but are typified by an initial administration followed in intervals (weeks or months) by a subsequent injection or other administration.

Once immunized, the mammal is maintained for a period of time sufficient for the recombinant HBc chimer immunogen to induce the production of a sufficient titer of antibodies that bind to an antigen of interest such as a sporozoite for a malarial vaccine. The maintenance time for the production of illustrative anti-sporozoite antibodies typically lasts for a period of about three to about twelve weeks, and can include a booster, or second immunizing administration of the vaccine. A third immunization is also contemplated, if desired, at a time 24 weeks to five years after the first immunization. It is particularly contemplated that once a protective level titer of antibodies is attained, the vaccinated mammal is preferably maintained at or near that antibody titer by periodic booster immunizations administered at intervals of about 1 to about 5 years.

The production of anti-sporozoite, in the case of a malaria vaccine, or other antibodies is readily ascertained by obtaining a plasma or serum sample from the immunized mammal and assaying the antibodies therein for their ability to bind to an appropriate antigen such as a synthetic circumsporozoite immunodominant antigen [e.g. the P. *falciparum* CS protein peptide (NANP)₅ used herein] in an ELISA assay as described hereinafter or by another immunoassay such as a Western blot, as is well known in the art.

It is noted that the induced antibodies such as anti-CS antibodies or anti-influenza antibodies can be isolated from the blood of an inoculated host mammal using well known techniques, and then reconstituted into a second vaccine for passive immunization as is also well known. Similar techniques are used for gamma-globulin immunizations of humans. For example, antiserum from one or a number of immunized hosts can be precipitated in aqueous ammonium sulfate (typically at 40-50 percent of saturation), and the precipitated antibodies purified chromatographically as by use of affinity chromatography in which (NANP)₅ or an influenza M2 polypeptide is utilized as the antigen immobilized on the chromatographic column. Thus, for example, an inoculum can be used in a horse or sheep to induce antibody production against a malarial species for use in a passive immunization in yet another animal such as humans.

The chimers of the invention may be used in a process for inducing antibodies, activated T cells or both in an animal host comprising the steps of inoculating said animal host with an inoculum. The inoculum used in the process comprises an immunogenic amount of a before-described HBc chimer particle or HBc chimer particle conjugate dissolved or dispersed in a pharmaceutically acceptable diluent. The animal host is maintained for a time sufficient for antibodies or activated T cells to be induced, as can be assayed by well-known techniques, which typically requires a time period of weeks to months, as is again well-known. A plurality of such immunizations is contemplated during this maintenance period.

### Computer Modeling to Predict Disulfide Bonds

Disulfide bond predictions were made using the program SS-BOND [Hazes et al., Protein Eng 2, 119-25 (1988)], which requires three-dimensional structure information about the protein of interest. The HBc structure coordinates were uploaded from the Protein Data Bank (this is the 3.3Å structure solved by Wynne et al.), and coordinates for the last three monomers were deleted, as this structure includes information for two dimers.

When run on a standard personal computer, the program automatically selects the most stringent requirements for the three parameters taken into account when predicting disulfide bonds: (1) the χ-3 angle, which is the rotation about the Sγ-Sγ bond (ideal is ±90° and the deviation from this angle is set at 0°), (2) the maximal energy required to allow bond formation (ideal is less than 10 keal/mol), and (3) the energy difference allowed above the lowest possible energy conformation for a given bond (maximum recommended is 5 kcal/mol). To obtain any hits for disulfide bond formation between immunodominant loop helix (ILH) 1 (amino acids 50-73) and ILH2 (amino acids 79-110), however, these requirements had to be relaxed. If running the program from a Linux operating system, these parameters can be edited.

Relaxation of the two latter parameters yielded no additional hits. Relaxation of the χ-3 angle, however, yielded five possible chimers when allowed 30° of rotation about the bond (Table). No additional hits were obtained between 29° and 72°, where maximum allowed deviation is 90°; this hit was, therefore, not included in the study. Side chain positions of the thus prepared outputs were then examined using the program GRASP [Nicholls et al., Proteins 30, 9686-97 (1991)] for visual examination of proximity of amino acids. All, except the 72° hit, appeared to be spatially proximal and these chimers were, therefore, cloned. The hit obtained at 29°, although not in either of the ILHs, was also included, as these residues are in the actual immunodominant loop. Epitope insertions were made between amino acids D78 and P79 so bridging would occur at the base of the epitope.

| SS-BOND Hits Bridging the Two Major HBc immunodominant Helices | | |
|---|---|---|
| χ-3 deviation (maximum 90°) | ILH1 amino acid (50-73) | ILH2 amino acid (79-110) |
| 4° | L55 | H104 |
| 6° | A58 | L100 |
| 17° | A69 | V89 |
| 24° | W62 | F97 |
| 29° | L76* | R82* |
| 72° | V72 | V85 |

| | | |
|---|---|---|
| *located in the immunodominant loop, rather than the immunodominant helices | | |

The invention is illustrated by the following non-limiting examples. Some of the examples are not in accordance with the claimed invention, but are included for reference.

### Example 1: Construction of Chimers Harboring Cysteine Mutations

### A. Plasmid Vector pKK223-3N, and Cysteine Substitutions

Zheng et al., J Biol Chem 267, 9422-9429 (1992) found that two of the four cysteines present in the HBc183 monomer sequence were not involved in disulfide bonding. Cysteine 48 was found to be partially covalently bound in a given sample and cysteine 107 was found to exist as a free thiol. Mutation of these cysteines was not found to affect particle assembly or stability and no sulfhydryl reactivity was detected, providing conclusive evidence that cysteine 51 and cysteine 183 are completely disulfide bonded.

Cysteine residues C48 and C107 were both mutated to serine residues to easily quantify sulfhydryl content of particles containing introduced cysteines. This is a conservative mutation, as serine is the amino acid most chemically similar to cysteine - they are identical, with the exception of a sulfhydryl, versus a hydroxyl R group. This chimer was used as a template for all subsequent chimers. Two versions of the template were made, one terminating at V149 [HBc149(C48S/C107S)], and the other harboring the additional cysteine at the C-terminus [HBc149 (C48S/C107S)+C].

The HBc gene was originally cloned into the pKK223-3 vector available from Pharmacia Biotech (Piscataway, NJ). The multi-cloning site was modified to include an NcoI site using PCR. All PCR reactions were performed using the Minicycler™ available from MJ Research (Waltham, MA). PCR reaction conditions for all studies were as follows: Step 1 - 94°C for 3 minutes
Step 2 - 92°C for 1 minute
Step 3 - 50°C for 1 minute
Step 4 - 72°C for 40 seconds
Step 5 - 14 times to Step 2
Step 6 - 72°C for 5 minutes
Step 7 - 4°C for 10 minutes
Step 8 - End

All oligonucleotide PCR primers were synthesized by Invitrogen (Carlsbad, CA) and used at a concentration of 1 µM, whereas approximately 0.1 µg of the requisite template was used in each reaction. Vent polymerase (New England BioLabs, Beverly, MA) was the amplification enzyme and no additional magnesium chloride was found to be necessary in any reaction. All primers referenced are listed in Table 2, hereinafter, and all restriction enzymes were purchased from New England BioLabs.

The DNA sequence of each chimer was verified by automated DNA sequencing outsourced to Sequi-Net (Fort Collins, CO). Plasmid stocks were created using the Qiagen (Valencia, CA) Midi Kit for plasmid isolation. Complete gene nucleotide sequences of all chimers made using PCR are provided in the sequence listing.

Primer sequences are shown in Table 2, below, and in which HBc amino acid residues are shown above the nucleotide sequence for forward primers and below for reverse primers. Bold italic amino acid residues and bases indicate substitutions. Restriction sites of interest are in bold. All sequences are written 5' to 3'.

**Table 2**

| # | Primer name | Sequence | SEQ ID NO |
|---|---|---|---|
| 1 | pKK-SphI-F | GGT GCA TGC AAG GAG ATG | 240 |
| 2 | pkk-Nco-Hind-R | | 241 |
| 3 | NcoI-F | | 411 242 |
| 4 | HindIII-R | | 243 412 |
| 5 | EarI (C107S)-R | | 244 413 |
| | | | |
| 6 | EarI-F | | 414 245 |
| 7 | C48S-HhaI-R | | 246 415 |
| 8 | A54-HhaI-F | | 416 247 |
| 9 | C-HindIII -R | | 248 417 |
| 10 | EarI-H104C-R | | 249 418 |
| | | | |
| 11 | Sau96I-V89C-R | | 250 419 |
| | | | |
| 12 | Cac8I-L55C-R | | 251 420 |
| | | | |
| 13 | Cac8I-F | | 421 252 |
| 14 | CacBI-A58C-R | | 253 422 |
| | | | |
| 15 | Sau96I-R | | 254 423 |
| 16 | Sau96I-L100C-F | | 424 255 |
| | | | |
| 17 | BstNI-A69C-R | | 256 425 |
| 18 | BstNI-F | | 426 257 |
| 19 | Sau96I-F | | 427 258 |
| 20 | Cac8I-W62C- | | 259 428 |
| | | | |
| 21 | Sau96I-F97C-F | | 429 260 |
| 22 | NheI-L76C-R | | 261 430 |
| 23 | NheI-R82C-F | | 431 262 |

### Chimers HBc149 and HBc149+C

The vector, pKK223-3 (Fig. 2), was amplified with primers 1 and 2. The PCR product and the parent vector were then digested with SphI and HindIII and purified from a 1.25% agarose gel (gel apparatus from Gibco BRL, Gaithersburg, MD) using the QIAEX II gel extraction system from Qiagen. (Note that all cloning was performed using these apparatuses and reagents). When digested, a fragment of 480 base pairs is cut from the parent vector - this fragment is discarded and the vector is ligated with the PCR product of 458 base pairs. This clone (pKK223-3N, Fig. 2) is now capable of accepting inserts flanked by NcoI and HindIII sites. The result of this modification to the pKK223-3 vector is that all restriction sites in the multi-cloning site have been deleted - only the NcoI-EcoRI-HindIII sequence from primer 2 remains between the tac promoter and the 5S rRNA region.

The HBV ayw subtype genome (sequence available from NCBI) was used to clone the HBc gene. Primers 3 and 4 were used to add flanking NcoI and HindIII sites, along with a stop codon following amino acid 149. The pKK223-3N vector and the PCR product were then digested with NcoI and HindIII. The 12 base pair fragment liberated from the vector was discarded. The vector was ligated with the roughly 450 base pair PCR fragment to yield the chimer HBc149. HBc149+C (CV-1123) was made the same way, except reverse primer 9 (SEQ ID NO:248) was used to insert a cysteine between valine 149 and the stop codon.

### Chimers HBc149(C48S/C107S) and HBc149(C48S/C107S)+C

To make these two chimers containing cysteine to serine mutations at positions 48 and 107 (C48S/C107S), several PCR reactions were necessary. The C107S mutation was made first, utilizing an endogenous EarI site. The 5' piece was produced using primer 3 and primer 5, which introduced the C107S mutation. The 3' piece was prepared using perfect match primers 6 and 4. These fragments (approximately 320 and 130 base pairs, respectively) were digested with EarI and ligated.

This ligation was then used as a template to introduce the C48S mutation. An HhaI site was introduced as a silent mutation into A54 to enable this mutation. Again, the PCR was done in two halves. The 5' piece introduced the C48S mutation using primers 3 and 7, whereas the 3', piece was made using primers 8 and 4. These products (about 165 and 295 base pairs, respectively) were then digested with HhaI and ligated. The ligation product was then amplified with primer 3 and either primer 4, which placed the stop codon after V149, or primer 9, which added a cysteine after V149 and then added the stop codon. Products, approximately 450 base pairs, were digested with NcoI and HindIII and ligated into the pKK223-3N vector prepared as above to yield the chimers HBc149(C48S/C107S) and HBc149(C48S/C107S)+C.

Construction of the panel of four of the five disulfide chimers was done in a similar manner to the C48S/C107S chimers - the first mutation was introduced by the PCR of two halves, then the second was introduced in two halves, using the first ligation as a template. All disulfide chimers were made with a C-terminal cysteine and all original PCRs were done using HBc149(C48S/C107S)+C as the template.

### Chimer HBc149(C48S/C107S)L55C/H104C+C

A silent Cac8I site was introduced around residue E64 of HBc to make the L55C mutation. Primers 3 and 12 were used to amplify the 5' half and primers 13 and 9 were used to amplify the 3' half. These PCR products were then digested with Cac8I and ligated. The ligation was then amplified in two halves. The 5' half, made using primers 3 and 10, introduced the H104C mutation and maintained the C107S mutation in the template, using the EarI site used previously in the construction of the C48S/C107S chimers. The 3' half used perfect match primers 6 and 9. After digestion with EarI, fragments were ligated and re-amplified with primers 3 and 9. This product was digested with NcoI and HindIII and cloned into pKK223-3N prepared as above.

### Chimer HBc149(C48S/C107S)A58C/L100C+C

The 5' half introducing the A58C mutation was made the same way as the L55C mutation above, using the introduced Cac8I site. Primers 3 and 14 were used to make the A58C mutation, whereas the same 3' PCR product made from primers 13 and 9 (see L55C/H104C above) was used in this ligation. To make the L100C mutation, however, an endogenous Sau96I site was utilized. The 5' half was made using perfect match primers 3 and 15, whereas the L100C mutation was introduced in the 3' half using primers 16 and 9. PCR products were digested with Sau96I and ligated, then re-amplified with primers 3 and 9 and cloned into pKK223-3N, as above.

### Chimer HBc149(C48S/C107S)A69C/V89C+C

The A69C mutation was introduced in the 5' PCR using primers 3 and 17 and an endogenous BstNI site. The 3' PCR used perfect match primers 18 and 9. Products were digested with BstNI and ligated. The ligation was then used to create the V89C mutation in the 5' PCR half, which again utilized the Sau961 site occurring naturally in the gene (primers 3 and 11). The 3' PCR does not introduce any mutations with perfect match primers 19 and 9. Products were digested Sau96I, ligated, re-amplified with primers 3 and 9, and cloned into pKK223-3, as above.

### Chimer HBc149(C48S/C107S)W62C/F97C+C

As with L55C and A58C, the W62C mutation was made from the Cac8I site. Primers 3 and 20 introduced the mutation in the 5' half, whereas the same 3' PCR product used to make the L55C and A58C mutations was used here. Products were digested with Cac8I, ligated and subjected to a second round of PCR. The Sau96I site was again utilized to make the 3' F97C mutation using primers 21 and 9, whereas the 5' primers 3 and 15 were perfect match. It is noted that the PCRs involved in making this chimer must be done in this order, as an additional Cac8I site is introduced when making the F97C mutation. After digestion with Sau96I, the products were ligated, re-amplified with primers 3 and 9, and cloned into pKK223-3, as above.

### Chimer HBc149(C48S/C107S)L76C/R82C+C

Making silent mutations in the A80-S81 sequence in the HBc loop made it possible to introduce an NheI site into the loop and make this chimer in only two rounds of PCR. The 5' piece introduced the L76C mutation using primers 3 and 22, and the 3' piece introduced the R82C mutation using primers 23 and 9. PCR products, roughly 245 and 222 base pairs, respectively, were digested with NheI and ligated. The ligation was then amplified using primers 3 and 9 to yield the standard 450 base pair fragment that was digested with NcoI and HindIII, and cloned in to pKK223-3N.

### B. Preparation of Loop Insertion Vectors

Four of the five cysteine-harboring engineered chimers successfully assembled into HBc particles. All of these were, therefore, assayed for their abilities to accept foreign epitopes into the immunodominant loop. Additionally, the chimer lacking any introduced cysteines was used as a control with which to compare abilities to assemble, and, later, stabilize. Epitopes were inserted between HBc amino acids aspartic acid 78 and proline 79. The novel restriction sites EcoRI and SacI were introduced so that all epitopes were flanked by Gly and Ile on the 5' end (encoded by the EcoRI restriction site) and Glu and Leu on the 3' end (encoded by the SacI restriction site). All cysteine chimers, except HBc149(C48S/C107S)L55C/H104C+C, which failed to form an assembled particle, were therefore first made into vectors able to accept heterologous epitopes between amino acid residues D78 and P79 of the HBc sequence.

Modified HBc149 (V2 and V16) or HBc183 (V8) genes, able to accept the directional insertion of synthetic dsDNA fragments into the immunodominant loop region, were constructed using PCR. (The plasmid accepting inserts between D78 and P79 and truncated to V149 was named V2, the same plasmid with an additional cysteine following V149 was named V16, and the plasmid accepting inserts between D78 and P79 and terminating at C183, was called V8). The HBc149 and HBc183 genes were amplified in two halves using two PCR primer pairs, one of which amplifies the amino terminus, the other amplifies the carboxyl terminus. For V2, the products of the PCR reactions are a 249 bp (N-terminus) and a 243 bp fragment (C-terminus); for V16, the products are a 249 bp (N-terminus) and a 246 bp fragment (C-terminus; for V8, the products are a 249 bp (N-terminus) and a 349 bp fragment (C-terminus).

The N-terminal fragments prepared were digested with NcoI and EcoRI, and the C-terminal fragments were digested with EcoRI and HindIII. The V2, V16, and V8 fragment pairs were then ligated together at the common EcoRI overhangs. The resultant NcoI-HindIII fragments were then ligated into the pKK223-3N vector, which had been prepared by digestion with NcoI and HindIII.

To insert B cell epitopes into the V2, V16, and V8 plasmids, the appropriate plasmid was digested with EcoRI and SacI restriction enzymes. Synthetic dsDNA fragments containing 5' EcoRI and 3' SacI overhangs were then inserted. In all cases, V2, V16, and V8, glycine-isoleucine (EcoRI) and glutamic acid-leucine (SacI) amino acid pairs, flank the inserted B cell epitopes. The inserted restriction sites are underlined in the primers below.

| | |
|---|---|
| V2 | |
| HBc149/NcoI-F 5'-TTGGGCCATGGACATCGACCCTTA | SEQ ID NO:263 |
| HBc-D78/EcoRI-R 5'-GCGGAATTCCATCTTCCAAATTAACACCCAC | SEQ ID NO:264 |
| HBc-P79/EcoRI-SacI-F 5'-CGCGAATTCAAAAAGAGCTCCCAGCGTCTAGAGACCTAG | SEQ ID NO:265 |
| HBc149/HindIII-R 5'-CGCAAGCTTAAACAACAGTAGTCTCCGGAAG | SEQ ID NO:266 |
| V16 | |
| HBc149/NcoI-F 5'-TTGGGCCATGGACATCGACCCTTA | SEQ ID NO:263 |
| HBc-D78/EcoRI-R 5'-GCGGAATTCCATCTTCCAAATTAACACCCAC | SEQ ID NO:264 |
| HBc-P79/EcoRI-SacI-F 5'-CGCGAATTCAAAAAGAGCTCCCAGCGTCTAGAGACCTAG | SEQ ID NO:265 |
| HBc149+C/HindIII-R 5'-CGCAAGCTTACTAGCAAACAACAGTAGTCTCCGGAAG | SEQ ID NO:267 |
| V8 | |
| HBc149/NcoI-F 5'-GGGCCATGGACATCGACCCTTA | SEQ ID NO:263 |
| HBc-D78/EcoRI-R 5'-GCGGAATTCCATCTTCCAAATTAACACCCAC | SEQ ID NO:264 |
| HBc-P79/EcoRI-SacI-F 5'-CGCGAATTCAAAAAGAGCTCCCAGCGTCTAGAGACCTAG | SEQ ID NO:265 |
| HBc183/HindIII-R 5'-GGAAAGCTTACTAACATTGAGATTCCCG | SEQ ID NO:268 |

Due to the proximity of the cysteine mutations to the loop, it was necessary to design a set of primers specifically for chimer HBc149(C48S/C107S)L76C/R82C+C, whereas all other chimers used the same sets of primers (Table 2 and Table 3, hereinafter). The 5' halves were made using primers 3 and 22 (23 in the case of HBc149(C48S/C107S)L76C/R82C+C), by adding an EcoRI site to the 3' end. The 3' halves were made using primers 24 (25 in the case of HBc149(C48S/C107S)-L76C/R82C+C) and 9, which added EcoRI sites and downstream SacI sites to these fragments. All PCR products were then digested with EcoRI, ligated with each corresponding half, and re-amplified with primers 3 and 9. These products were then digested with NcoI and HindIII and cloned into prepared plasmid pKK223-3N. The sequence of each clone was verified by automated DNA sequencing, as described before.

Epitope selection was based on past success or failure of an epitope in an equivalent vector that had no introduced cysteines (V16). Two epitopes that failed in V16 were selected - one from the β-amyloid protein (residues 1-32) implicated in Alzheimer's disease [Morgan et al., Nature 408, 982-985 (2000)], and residues 701-721 of the protective antigen from *B. anthracis* (Ba) [Little et al., Microbiology 142, 707-715 (1996)]. An epitope from the hookworm protein Asp-1 (residues 292-303) (provided by Dr. Peter Hotez, George Washington University) had yielded low, but detectable particle assembly in V16; this epitope was included to assess whether particle yields could be increased with the introduced disulfide bonds. A final epitope, which exhibited very good particle assembly in V16, was included in order to compare particle assembly and stability of this chimer to the engineered versions. That epitope is derived from the extracellular domain of the M2 protein of influenza A (residues 2-24) [Zebedee et al., J Virol 62, 2762-2772 (1988); and Neirynck et al., Nat Med 5, 1157-63 (1999)] with two modifications in the sequence that were found to be necessary for particle assembly in previous work with this epitope in our laboratory. All epitope sequences are given in Table 3, below.

Oligonucleotide sequences were designed as a top strand and a bottom strand that, when annealed, harbor single-stranded sticky ends that can be ligated into the cysteine-engineered vectors prepared above. Oligo annealing was performed using a Minicycler (MJ Research) by adding equimolar amounts of oligo (10 µM of each top and bottom oligo) and heating at 95°C for 5 minutes, then decreasing the temperature by 1° every minute for 75°. Annealed oligo-containing solution was then diluted 1:20 in TE buffer. This procedure was performed for the primer pairs 26 and 27, 28 and 29, 30 and 31, and 32 and 33. Each disulfide vector was prepared by digestion with EcoRI and SacI, followed by purifying the vector away from the small resultant fragment. One uL of each annealed oligo was then ligated into the prepared vector. Clone sequences were, again, verified by sequencing.

**Table 3**

| # | Primer name | Sequence | SEQ ID NO |
|---|---|---|---|
| 22 | EcoRI-D78-R | | 269 432 |
| 23 | EcoRI-D78 (L76C)-R | | 270 433 |
| 24 | EcoRI/SacI-P79-F | | 434 271 |
| 25 | EcoRI/SacI-P79(R82C)-F | | 435 272 |
| 26 27 | β-Am (1-32)-T β-Am(1-32)-B (3') | | 436 273 |
| | (continued-T) (continued-B) | Y | |
| | (continued-T) (continued-B) | | |
| 28 29 | Ba(701-721)-T Ba(701-721)-B (3') | | 437 274 |
| | (continued-T) (continued-B) | | |
| 30 31 | Asp-1(292-303)-T Asp-1(292-303) -B (3') | | 438 275 |
| | | | |
| 32 33 | IA(M2)2C/2S-T IA(M2)2C/2S-B (3') | | 439 276 |
| | (continued-T) (continued-B) | | |
| | (continued-T) (continued-B) | | |

### C. Expression and Purification of Chimers

After sequence verification, chimers were co-transformed with pREP4 (Qiagen), a plasmid expressing the lac repressor, into the BLR strain of E. coli (Novagen, Madison, WI). Cells were made competent by calcium chloride preparation [Sambrook et al., Molecular cloning: a laboratory manual - 2nd ed. Cold Spring Harbor Laboratory Press, 1989]. Plasmid pKK223-3 harbors the tac promoter, but does not encode the lac repressor, so this protein is provided in trans by pREP4 to repress expression of the HBc gene until it is induced by the addition of IPTG. Transformations are plated on LB plates supplemented with 50 µg/L ampicillin (pKK223-3 carries the ampicillin resistance marker), and 10 µg/L kanamycin (pREP4 carries the kanamycin resistance marker) to prevent loss of either plasmid.

Transformants were then picked into 3 mL starter cultures of TB Dry media (Doc Frugal, San Diego, CA) with the same concentrations of antibiotics and grown overnight. One mL of each starter culture was then used to inoculate 500 mL TB Dry media with same antibiotics and cultures were grown to an OD₆₀₀ of 0.6-1.0 and then induced with 25 µM IPTG. Cultures were grown 16-24 hours and the cells harvested by centrifugation for 10 minutes at 15,000xG.

Assembly of chimers was assayed using size exclusion chromatography. Assembled HBc elutes as a distinct peak (second in a profile of four peaks), which is absent when a particle fails to assemble - unassembled monomers elute in the later peaks. Cell pellets were resuspended in 50 mL of 50 mM Tris-HCl, pH 8.0 and 10 mM EDTA (lysis buffer) by stirring. The cell suspension was then lysed using a microfluidizer (Microfluidics, Newton, MA) and centrifuged for 20 minutes at 31,000xG to pellet the cell debris. HBc particles are typically localized in the soluble fraction, so the volume of the supernatant was measured and then diluted 1:1 with saturated ammonium sulfate to precipitate the protein. The resulting solution was stirred gently at 4°C for 15 minutes and then centrifuged a second time for 20 minutes at 31,000xG to pellet the ammonium sulfate precipitate.

The supernatant was discarded and the pellet resuspended in 5 mL of lysis buffer and then transferred to dialysis tubing (molecular weight cut off 8,000 daltons). Samples were dialyzed against 1L of lysis buffer for at least three hours, filtered through a 0.45 µm syringe filter, and then subjected to size exclusion chromatography using Sepharose CL-4B resin packed in a 2.5 x 100 cm XK 26 column and attached to an Äkta Prime FPLC (all Pharmacia Biotech).

The column was pre-equilibrated with 20 mM sodium phosphate, pH 6.8 and 0.02% sodium azide, and the same buffer was pumped through the column for approximately 800 mL at a flow rate of 1.5 mL/minute, while 6-8 mL fractions were collected. Particle production was assessed by examination of the elution profile for a characteristic peak following the initial narrow first peak of material traveling through the column.

If assembled particles were detected, the peak fractions were pooled and passed through a 20-25 mL column volume of coarse HYPATITE^{™} C resin (Clarkson, Williamsport, PA), packed in a 2.5 x 20 cm column (Kontes, Vineland, NJ). HBc particles typically exhibit minimal binding to this resin, so the protein was loaded onto the column and the entire eluent collected. The column was then washed with 50 mM sodium phosphate, pH 6.8 until the Abs₂₈₀ of the eluent was less than 0.1.

The pool was then passed through a 0.45 µm filter and loaded through the solvent line of an HPLC (BioCad from PerSeptive Biosystems, Framingham, MA) onto a MonoQ^{™} HR 10/10 anion exchange column (Pharmacia Biotech). The column was equilibrated in 25 mM Tris-HCl and 0.02% sodium azide and, once the sample was loaded, washed with the same buffer. Particles were eluted with a linear gradient of 0-3 M NaCl in 25 mM Tris-HCl. Peak fractions were pooled, diluted 1:1 with saturated ammonium sulfate to re-precipitate, stirred gently at 4°C for 1 hour, and then centrifuged for 30 minutes at 17,500xG. The resultant pellet was resuspended in a minimum volume of 20 mM sodium phosphate, pH 6.8, transferred to dialysis tubing, and dialyzed extensively against the same buffer. The final purified sample was then passed through a 0.45 µm filter and incubated (maintained) at 4°C for 24 hours before it was frozen. Protein concentration was determined by BCA assay (available from Sigma, St. Louis, MO).

### D. Characterization of

### Assembled Chimers

Purified proteins were characterized using two methods to examine particles in their native conformation (analytical size exclusion and Ellman's test), one that examined denatured particles under non-reducing conditions (non-reducing SDS-PAGE), and one that examined monomer integrity (reducing SDS-PAGE).

To assess particulate versus non-particulate material, purified protein samples were analyzed using analytical size exclusion chromatography. In that procedure, 50-90 µg of each sample were injected onto Superose 6 HR 10/30 columns (Pharmacia Biotech) and attached to either an HPLC (BioCad) or an Äkta Purifier (Pharmacia Biotech). 20 mM sodium phosphate, pH 6.8 was pumped through the column at a flow rate of 0.5 mL/min. Particle integrity was assessed by visualization of peak elution profiles, where the presence of one peak at an elution position of approximately 7 mL is fully formed particle. Later peaks represent non-particulate structures, such as dimers and monomers.

Disulfide bond formation was assessed using a method similar to that described in Zheng et al., J Biol Chem 267, 9422-9429 (1992). Thus, particles were diluted to a final concentration of 1 mg/mL in 20 mM sodium phosphate, pH 6.8 and 0.1% SDS in ELISA plate wells. A stock solution of Ellman's reagent (Sigma) was then made at a concentration of 200 mM in dimethyl sulfoxide (DMSO). This solution was then diluted 1:200 in 20 mM sodium phosphate, pH 6.8 and added to HBc samples for a final concentration of 0.1 mM. Reactions were permitted to proceed at room temperature for 15 minutes. The plate was read at 412 nm on a Spectramax^{™} (Molecular Devices, Sunnyvale, CA). Colorimetric analysis was then performed to determine disulfide bond formation, where reactivity of Ellman's reagent with free sulfhydryls yielded a yellow color and complete disulfide bonding in a sample resulted in no color.

The final characterization of particles was performed using non-reducing and reducing SDS-PAGE gels to examine disulfide bond induced cross-linking of monomers and dimers, as well as monomer integrity. All SDS-PAGE gels were run using the NuPAGE^{™} system from Invitrogen (Carlsbad, CA). NuPAGE^{™} 10% Bis-Tris 1.5mm x 10 well gels were run using MES buffer in XCell SureLock^{™} electrophoresis apparatuses. Samples were prepared by addition of LDS NuPAGE^{™} sample buffer and heating for 10 minutes at 50°C. To prepare reduced samples, β-mercaptoethanol was added prior to the heating step for a final concentration of 10%.

Gels were run for 30-40 minutes at 200V and then stained with SimplyBlue SafeStain^{™} (Invitrogen, Carlsbad, CA) following provided protocols. Cross-linking in non-reducing samples was established by the absence of monomeric and dimeric species and the presence of high molecular weight protein visible as a smear at the top of the gel. Monomeric integrity was established by the presence of one defined band of the same molecular weight as known standards (approximately 17 kDa).

Stability of particles was assessed using the above methods on samples incubated at various temperatures (4°C, room temperature, and 37°C) over time, collecting samples for analysis on days zero, 3, 7, and 14.

### E. Vectors to Express Chimer Particles Containing an N-Terminal Cysteine and the CS-Repeat Epitopes from P. falciparum in the Immunodominant Loop

Two expression vectors [V2.Pf1(N-MGCELDP) and V2.Pf1(N-MGCDIDP)] are prepared to determine the ability of N-terminal cysteine residues to stabilize chimer particles. To make the vector V2.Pf1(N-MGCELDP), the oligonucleotides HBc(MGCELDP)-NcoI-F and HBc149/HindIII-R are used to amplify the hybrid HBc gene from vector V2.Pf1. The resultant 528 bp fragment is cleaved with NcoI and HindIII and inserted into pKK-223-3N, which had been cleaved with the same two enzymes.

To make the vector V2.Pf1(N-MGCDIDP) the oligonucleotides HBc(MGCDIDP)-NcoI-F and HBc149/HindIII-R are used to amplify the hybrid HBc gene from vector V2.Pf1. The resultant 528 bp fragment is cleaved with NcoI and HindIII and inserted into pKK-223-3N, which has been cleaved with the same two enzymes.
HBc(MGCELDP)-NcoI-F
   M G C E L D P Y K E F G SEQ ID NO: 277
   5'-GCGCCATGGGGTGTGAGCTCGACCCTTATAAAGAATTTGG SEQ ID NO: 278
HBc(MGCDIDP)-NcoI-F ,
   M G C D I D P Y K E F G SEQ ID NO: 279
   5'-GCGCCATGGGGTGTGACATCGACCCTTATAAAGAATTTGG SEQ ID NO: 280

### F. Preparation of V7 Cloning Vector

To enable the fusion of T cell epitopes to the C terminus of a HBc chimer, a new vector was constructed. Unique EcoRI and SacI restriction sites were inserted between valine-149 and the HindIII site to facilitate directional insertion of synthetic dsDNAs into EcoRI-HindIII (or EcoRI-SacI) restriction sites. The pair of PCR primers below was used to amplify the HBc 149 gene with an NcoI restriction site at the amino-terminus and EcoRI, SacI and HindIII sites at the carboxyl-terminus. The product of the PCR reaction (479 bp) was digested with NcoI and HindIII and cloned into pKK223-3N to form V7.

To insert T cell epitopes, the plasmid (V7) was digested with EcoRI and HindIII (or EcoRI and SacI) and synthetic dsDNA fragments having EcoRI/HindIII (or EcoRI/SacI) overhangs, were ligated into V7. For all V7 constructs, the final amino acid of native HBc (valine-149) and the first amino acid of the inserted T cell epitope are separated by a glycine-isoleucine dipeptide sequence coded for by the nucleotides that form the EcoRI restriction site. For epitopes inserted at EcoRI/SacI, there are additional glutamic acid-leucine residues after the T cell epitope, prior to the termination codon, contributed by the SacI site. Restriction sites are again underlined in the primers shown.

| | |
|---|---|
| HBc149/NcoI-F 5'-TTGGGCCATGGACATCGACCCTTA | SEQ ID NO: 263 |
| HBc149/SacI-EcoRI-H3-R 5'-CGCAAGCTTAGAGCTCTTGAATTCCAACAACAGTAGTCTCCG | SEQ ID NO: 281 |

### G. Preparation of V12

### Expression Constructs

V12 vectors, which contain B cell epitopes between amino acids 78 and 79, as well as T cell epitopes downstream of valine-149, are constructed from V2 and V7 vectors. The carboxyl terminus of a V7 vector containing a T cell epitope inserted at EcoRI/HindIII is amplified using two PCR primers (HBc-P79/SacI-F and pKK223-3/4515-32R) to provide a dsDNA fragment corresponding to amino acids 79-149 plus the T cell epitope, flanked with SacI and HindIII restriction sites.

The PCR products are cut with SacI and HindIII and then cloned into the desired V2 vector prepared by cutting with the same two enzymes. The PCR primers are amenable for the amplification of the carboxyl terminus of all V7 genes, irrespective of the T cell epitope present after amino acid 149 of the HBc gene.

Restriction sites are underlined.

| | | |
|---|---|---|
| HBc-P79/SacI-F | 5'-CGCGAGCTCCCAGCGTCTAGAGACCTAG | SEQ ID NO: 282 |
| pKK223-3/4515-32R | 5'-GTATCAGGCTGAAAATC | SEQ ID NO: 283 |

### H. P.falciparum CS-repeat B cell

### Epitopes Inserted into V2

For V2 and V7 constructs, synthetic dsDNA fragments coding for the B (V2) or T cell epitope (V7) of interest are inserted into EcoRI/SacI restriction sites. Synthetic dsDNA fragments, encoding B and T cell epitopes of interest, are prepared by mixing complementary single stranded DNA oligonucleotides at equimolar concentrations, heating to 95°C for 5 minutes, and then cooling to room temperature at a rate of -1 °C per minute. This annealing reaction is performed in TE buffer. The double-stranded DNAs are shown below with the encoded epitope sequence shown above. The pound symbol, #, is used in some of the amino acid residue sequences that follow to indicate the presence of a stop codon.

| | |
|---|---|
| Pf1 | |
| | |
| N P E L | SEQ ID NO: 284 |
| ATCCGGAGCT | SEQ ID NO: 285 |
| TAGGCC | SEQ ID NO: 286 |
| Pf3 | |
| | |
| N A N P N V D P N A N P E L | SEQ ID NO:287 |
| ACGCTAATCCGAACGTTGACCCGAACGCTAATCCGGAGCT | SEQ ID NO:288 |
| TGCGATTAGGCTTGCAACTGGGCTTGCGATTAGGCCTCGAGG | SEQ ID NO: 289 |
| Pf3.1 | |
| | |
| N A N P E L | SEQ ID NO:290 |
| AAATGCGAACCCAGAGCT | SEQ ID NO:291 |
| TTTACGCTTGGGTC | SEQ ID NO:292 |
| Pf3.2 | |
| | |
| N A N P E L | SEQ ID NO: 293 |
| ATGCGAACCCAGAGCT | SEQ ID NO: 294 |
| TACGCTTGGGTC | SEQ ID NO: 295 |
| Pf3.3 | |
| | |
| N A N P N V D P N A N P E L | SEQ ID NO: 296 |
| ACGCCAACCCGAATGTTGACCCCAATGCCAATCCGGAGCT | SEQ ID NO: 297 |
| TGCGGTTGGGCTTACAACTGGGGTTACGGTTAGGCC | SEQ ID NO: 298 |
| Pf3.4 | |
| | |
| N P N V E L | SEQ ID NO: 299 |
| ACCCGAATGTTGAGCT | SEQ ID NO: 300 |
| TGGGCTTACAAC | SEQ ID NO: 301 |
| Pf3.5 | |
| | |
| N P N V D P E L | SEQ ID NO: 302 |
| ACCCGAATGTTGACCCTGAGCT | SEQ ID NO: 303 |
| TGGGCTTACAACTGGGAC | SEQ ID NO: 304 |
| Pf3.6 | |
| | |
| N P N V D P N A E L | SEQ ID NO: 305 |
| ACCCGAATGTTGACCCTAATGCTGAGCT | SEQ ID NO: 306 |
| TGGGCTTACAACTGGGATTACGAC | SEQ ID NO: 307 |
| Pf3 . 7 | |
| | |
| N V E L | SEQ ID NO: 308 |
| ATGTTGAGCT | SEQ ID NO: 309 |
| TACAAC | SEQ ID NO: 310 |
| Pf3.8 | |
| | |
| N V D P E L | SEQ ID NO: 311 |
| ATGTTGACCCTGAGCT | SEQ ID NO: 312 |
| TACAACTGGGAC | SEQ ID NO: 313 |
| Pf3.9 | |
| | |
| N V D P N A E L | SEQ ID NO: 314 |
| ATGTTGACCCTAATGCTGAGCT | SEQ ID NO: 315 |
| TACAACTGGGATTACGAC | SEQ ID NO: 316 |
| Pf3.10 | |
| | |
| N V E L | SEQ ID NO: 317 |
| CGAATGTTGAGCT | SEQ ID NO: 318 |
| GCTTACAAC | SEQ ID NO: 319 |
| Pf3.11 | |
| | |
| D P E L | SEQ ID NO: 320 |
| ACCCTGAGCT | SEQ ID NO: 321 |
| TGGGAC | SEQ ID NO: 322 |
| Pf 3.12 | |
| | |
| D P N A E L | SEQ ID NO: 323 |
| ACCCTAATGCCGAGCT | SEQ ID NO: 324 |
| TGGGATTACGGC | SEQ ID NO: 325 |
| Pf-UTC (PF/CS326-345) | |
| | |
| C S V T # # | SEQ ID NO: 326 |
| GCTCCGTTACCTAGTA | SEQ ID NO: 327 |
| CGAGGCAATGGATCATTCGA I. P.vivax CS-repeat B cell | SEQ ID NO: 328 epitopes |
| I. P. *vivax* CS-repeat B cell epitopes | |
| Pv-T1A | |
| | |
| G Q P A G E L | SEQ ID NO: 329 |
| GCCAGCCGGCTGGCGAGCT | SEQ ID NO: 330 |
| CGGTCGGCCGACCGC | SEQ ID NO: 331 |
| Pv-T1B | |
| | |
| A G E L | SEQ ID NO: 332 |
| CAGGGGAGCT | SEQ ID NO: 333 |
| GTCCCC | SEQ ID NO: 334. |
| Pv-T2A | |
| | |
| P G E L | SEQ ID NO: 335 |
| CAGGGGAGCT | SEQ ID NO: 336 |
| GTCCCC | SEQ ID NO: 337 |
| Pv-T2B | |
| | |
| P G E L | SEQ ID NO: 338 |
| CAGGCGAGCT | SEQ ID NO: 339. |
| GTCCGC | SEQ ID NO: 340 |
| Pv-T2C | |
| | |
| | |
| Q P G E L | SEQ ID NO: 341 |
| AACCCGGCGAGCT | SEQ ID NO: 342 |
| TTGGGCCGC | SEQ ID NO: 343 |
| PV-T3 | |
| | |
| Q E G G A A E L | SEQ ID NO: 344 |
| AAGAAGGCGGTGCAGCGGAGCT | SEQ ID NO: 345 |
| TTCTTCCGCCACGTCGCC | SEQ ID NO: 346 |

### Example 2: Preparation of Chimers Containing Influenza A M2 Polypeptide Sequences

### A. Insertion of Influenza A M2 N-terminal Domain into V2, V7, V8, V16, V34, V47, V48, V54, and V55 Cloning Vectors

For V2, V7, V8, V16, V34 and V55 constructs, synthetic dsDNA fragments coding for an M2 epitope (residues 2-24 of the influenza A M2 protein; SEQ ID NO:9) were inserted into EcoRI/SacI restriction sites, whereas for V47, V48, and V54 constructs, residues 1-24 of the same were inserted into NcoI/SacI restriction sites. Synthetic dsDNA fragments were prepared by mixing complementary single stranded DNA oligonucleotides at equimolar concentrations, heating to 95°C for 5 minutes, and then cooling to room temperature at a rate of -1 °C per minute. This annealing reaction was performed in TE buffer. The double-stranded DNAs are shown below with the encoded epitope sequence shown above.

| | |
|---|---|
| V2/V7/V8/V16/V34/V55 | |
| M2(2-24) | |
| | |
| C N D S S D E L | SEQ ID NO:347 |
| CTGTAATGATTCTTCCGACGAGCT | SEQ ID NO:348 |
| GACATTACTAAGAAGGCTGC | SEQ ID NO:349 |
| V47/V48/V54 | |
| M2(1-24) | |
| | |
| C N D S S D E L | SEQ ID NO:350 |
| TGTAACGATTCAAGTGATGAGCT | SEQ ID NO:351 |
| ACATTGCTAAGTTCACTAC | SEQ ID NO:352 |

### B. Insertion of individual cysteine-mutated Influenza A M2 N-terminal domains [M2 (1-24/C17S), M2 (1-24/C19S)] into V47

### Expression Vector

Annealed DNA fragments encoding residues 1-24 of the M2 protein with the cysteine at either position 17 or 19 mutated to serine are shown below. They were inserted into the NcoI/SacI restriction sites of V47 as described in part A above.

| | |
|---|---|
| V4 7 | |
| M2(1-24/C17S) | |
| | |
| C N D S S D E L | SEQ ID NO:353 |
| TGTAACGATTCAAGTGATGAGCT | SEQ ID NO:354 |
| ACATTGCTAAGTTCACTAC | SEQ ID NO:355 |
| M2(1-24/C19S) | |
| | |
| S N D S S D E L | SEQ ID NO:356 |
| TCGAACGATTCAAGTGATGAGCT | SEQ ID NO:357 |
| AGCTTGCTAAGTTCACTAC | SEQ ID NO:358 |

### C. Insertion of cysteine-mutated Influenza A M2 N-terminal domain [M2(2-24/C17S,C19S)] into Expression Vectors V8, V16, V47, V48, and V54

For V8 and V16 constructs, synthetic dsDNA fragments harboring two cysteine to serine mutations and coding for the M2 epitope (residues 2-24 of the influenza A M2 protein) were inserted into EcoRI/SacI restriction sites, whereas for V47, V48, and V54 constructs, residues 1-24 of the same were inserted into NcoI/SacI restriction sites. Synthetic dsDNA fragments were prepared as described in part A above.

| | |
|---|---|
| V8 , V16 | |
| M2 (2-24/C17S, C19S) | |
| | |
| S N D S S D E L | SEQ ID NO:359 |
| TCTAATGATAGCTCTGACGAGCT | SEQ ID NO:360 |
| AGATTACTATCGAGACTGC | SEQ ID NO:361 |
| M2(1-24/C17S,C19S) | |
| | |
| S N D S S D E L | SEQ ID NO:362 |
| TCGAACGATTCAAGTGATGAGCT | SEQ ID NO:363 |
| AGCTTGCTAAGTTCACTAC | SEQ ID NO:364 |

### D. Insertion of Additional Copies of the Influenza A M2 N-terminal domain onto the N-terminus of the

### Expression Vector V54.M2(1-24)

One additional copy of either native M2 sequence [M2 (1-24)] or mutated M2 sequence [M2 (1-24/C17S, C19S) was cloned N-terminally to the existing M2(1-24) sequence. In constructing these clones, the original methionine is removed, such that the added copy supplies only one initiator methionine. PCR was used to make the constructs in two fragments. To make the clone containing two native M2 copies [M2 (1-24)/V54.M2 (2-24)], the template V54.M2(1-24) was used to amplify first the N-terminal fragment, which inserts an XhoI site (and, therefore, amino acids leucine, followed by glutamic acid) after D24 of the M2 sequence (resultant fragment is 353 bp), then the C-terminal fragment, which inserts an XhoI site N-terminal to S2 of the M2 sequence, thereby removing the methionine (resultant fragment is 538 bp). To make the clone containing a mutant, followed by a native copy of M2 [M2(1-24/C17S,C19S/V54.M2(2-24)]), the template V54.M2(1-24/C17S,C19S) was used to generate the N-terminal fragment, while the C-terminal fragment is identical to that above (resultant fragment sizes are also identical).

The N-terminal fragments prepared were digested with BamHI and XhoI, and the C-terminal fragment was digested with XhoI and HindIII. Fragment pairs were then ligated together at the common XhoI overhangs. The resultant BamHI-HindIII fragments were then ligated into the pKK223-3N vector, which had been prepared by digestion with BamHI and HindIII.

Two additional copies of mutated M2 sequence were cloned N-terminally to existing M2(1-24) sequence. Again, only one initiator methionine was preserved at position one of the gene to yield the construct M2(1-24/C17S,C19S/M2(2-24/C17S,C19S/V54.M2(2-24). Again, the clone was produced in two PCR fragments. The template V54.M2(1-24/C17S,C19S) was used to generate the N-terminal fragment, which inserts a PstI site (and, therefore, amino acids leucine, followed by glutamine) after D24 of the mutant M2 sequence (resultant fragment is 353 bp). The template M2(1-24/C17S,C19S/V54.M2(2-24) from above was used to generate the C-terminal fragment, which inserts a PstI site N-terminal to S2 of the mutant M2 sequence, thereby removing the methionine (resultant fragment is 613 bp).

The N-terminal fragment prepared was digested with BamHI and PstI, and the C-terminal fragment was digested with PstI and HindIII. Fragment pairs were then ligated together at the common PstI overhangs. The resultant BamHI-HindIII fragment was then ligated into the pKK223-3N vector, which had been prepared by digestion with BamHI and HindIII.

M2(1-24)/V54.M2(2-24); M2(1-24/C17S,C19S/V54.M2(2-24)

| | |
|---|---|
| pKK-BamHI-F 5'-CGTAGAGGATCCGGAGCTTATCGACTGCACGG | SEQ ID NO:365 |
| M2-D24/XhoI-R 5'-GCGCTCGAGATCACTTGAATCGTT | SEQ ID NO:366 |
| M2-XhoI/S2-F 5'-GCGCTCGAGAGCTTATTGACCGAAGTTGAAACC | SEQ ID NO:367 |
| HBc149+C/HindIII-R 5'-CGCAAGCTTACTAGCAAACAACAGTAGTCTCCGGAAG | SEQ ID NO:267 |
| M2(1-24/C17S,C19S)/M2(2-24/C17S,C19S)/V54.M2(2-24) | |
| pKK-BamHI-F 5'-CGTAGAGGATCCGGAGCTTATCGACTGCACGG | SEQ ID NO:365 |
| M2-D24/PstI-R 5'-GCGCTGCAGATCACTTGAATCGTT | SEQ ID NO:368 |
| M2-PstI/S2-F 5'-GCGCTGCAGTCTCTGCTGACCGAAG | SEQ ID NO:369 |
| HBc149+C/HindIII-R 5'-CGCAAGCTTACTAGCAAACAACAGTAGTCTCCGGAAG | SEQ ID NO:267 |

### E. Construction of Truncated

### Version of Native M2-HBc

The original M2-HBc construct [Neirynck et al., (1999) Nature Med. , 5 (10) :1157-1163 : WO 99/07839] that contained the 183-residue, full length HBc sequence was truncated to V149, and the entire gene was moved into the pKK223-3 expression vector. To achieve this, the plasmid 3453, which was provided by the University of Gent, was used as a template for a PCR reaction that yielded a product of 523 bp. This product was digested with restriction enzymes AflIII and HindIII, and then ligated into the pKK223-3N vector, which had been prepared by digestion with NcoI and HindIII.

| | |
|---|---|
| AflIII-M2-F 5'-CGCGACATGTCTCTGCTGACCG | SEQ ID NO:370 |
| HBc-HindIII-R 5'-CGCAAGCTTAAACAACAGTAGTCTCCGGAAG | SEQ ID NO:371 |

| Clone Number | Clone Name |
|---|---|
| CV-1438 | V34.M2(2-24) |
| CV-1440 | V2.M2(2-24) |
| CV-1475 | V16.M2(2-24) |
| CV-1492 | V52.M2(2-24) |
| CV-1560 | 3453/149 |
| CV-1569 | V16.M2(1-24/C17S,C19S) |
| CV-1586 | V8.M2(2-24) |
| CV-1587 | V8.M2(1-24/C17S,C19S) |
| CV-1588 | V7.M2(2-24) |
| CV-1590 | V47.M2(1-24) |
| CV-1603 | V47.M2 (1-24/C17S,C19S) |
| CV-1604 | V54.M2(1-24) |
| CV-1605 | V54.M2(1-24/C17S,C19S) |
| CV-1606 | V48.M2(1-24) |
| CV-1607 | V48.M2(1-24/C17S,C19S) |
| CV-1671 | V47.M2(1-24/C17S) |
| CV-1672 | V47.M2(1-24/C19S) |
| CV-1816 | M2(1-24)/V54.M2(2-24) |
| CV-1817 | M2(1-24/C17S,C19S/V54.M2(2-24) |
| CV-1818 | M2(1-24/C17S,C19S)/M2(2-24/C17S,C19S)/V54.M2(2-24) |

### Example 3: Synthesis of Expression Vectors for

### Expressing Partially Truncated Particles

To prepare expression plasmids for expressing partially truncated HBc particles, a single amino terminal oligonucleotide PCR primer (HBc149/NcoI-F) was used in combination with a unique C-terminal primer. For example, to prepare the HBc156 (E.cR; CV-1600 particles) expression plasmid, the primers HBc149/NcoI-F and HBc156(E.cR)-H3-R are used. Primers HBc149/NcoI-F and HBc156C(E.cR)-H3-R were used to prepare the HBc156(E.cR)+C (CV-1601 particles) expression plasmids. The sequences of all primers used are displayed below.

In addition to truncating the particles, and in some cases incorporating a C-terminal cysteine residue, codons that are optimal for expression in *E. coli* were also used. It is known that several arginine codons, particularly AGA and AGG are rarely used by *E.coli* and are believed to.be problematic for efficient expression of proteins in *E. coli* by leading to stalling of polypeptide synthesis during translation and resulting in premature termination. Of the 16 arginine codons between 150 and 183 of HBc, 7 are encoded by the rare AGA codon and 2 are encoded by the very rare AGG codon. Therefore, in this study, all AGA and AGG codons were replaced with codons that are more frequently used by *E.coli.*

To enable sequential replacement of the rare arginine codons, HBc156 genes were synthesized first (CV-1600 and CV-1601 particles), and then used as a template for the HBc163 constructs (CV-1634 and CV-1632 particles); the HBc163 constructs were thereafter used as a template for the HBc171 constructs (CV-1642 and CV-1643 particles); finally, the HBc 171 constructs are used as a templates for the arginine codon optimized HBc182 and HBc183 constructs. A non-optimized HBc182 construct (CV-1575) was also prepared for control purposes. All PCR products were cleaved with the restriction enzymes NcoI and HindIII and cloned into the expression vector pKK223-3N, which had been cut with the same enzymes as discussed before.

Amino Terminal Primer Sequence (NcoI restriction site is underlined):

| | |
|---|---|
| HBc149/NcoI-F 5'-TTGGGCCATGGACATCGACCCTTA | SEQ ID NO:263 |
| Carboxyl-Terminal Primer Sequences (HindIII restriction sites are underlined): | |
| HBc156(E.cR)-H3-R 5'-GCGAAGCTTACTAAGGGGAGCGGCCTCGTCGACGAACAACAGTAGTCTCCGG | SEQ ID NO:372 |
| HBc156C(E.cR)-H3-R 5'-GCGAAGCTTACTAACAAGGGGAGCGGCCTCGTCGACGAACAACAGTAGT-CTCCGG | SEQ ID NO:373 |
| HBc163(E.cR)-H3-R 5'-GCGAAGCTTACTAAGGCGAGGGAGTGCGCCGACGAGGGGAGCGGCCTCG | SEQ ID NO:374 |
| HBc163C (E.cR) -H3-R 5'-GCGAAGCTTACTAACAAGGCGAGGGAGTGCGCCGACGAGGGGAGCGGCCTCG | SEQ ID NO:375 |
| HBc171(E.cR)-H3-R 5'-GCGAAGCTTACTACGGCGATTGAGAGCGTCGACGGCGAGGCGAGGGAGT | SEQ ID NO:376 |
| HBc171C(E.cR)-H3-R 5'-GCGAAGCTTACTAACACGGCGATTGAGAGCGTCGACGGCGAGGCGAGGGAGT | SEQ ID NO:377 |
| HBc183 (E.cR) -H3 -R 5'-GCGAAGCTTACTAACATTGAGATTCCCGAGATTGAGATCGCCGGCGACGCGG-CGATTGAGAGCGTC | SEQ ID NO:378 |
| HBc182-H3-R 5'-GCGAAGCTTACTATTGAGATTCCCGAGATTGA | SEQ ID NO:379 |
| HBc183-H3-R 5' -GGAAAGCTTACTAACATTGAGATTCCCG | SEQ ID NO:380 |
| HBc149/HindIII-R 5'-CGCAAGCTTAAACAACAGTAGTCTCCGGAAG | SEQ ID NO:266 |
| HBc149+C/HindIII-R 5'-CGCAAGCTTACTAGCAAACAACAGTAGTCTCCGGAAG | SEQ ID NO:267 |

| Clone Number | Clone Name |
|---|---|
| CV-1600 | HBc156 (E.cR) |
| CV-1601 | HBc156(E.cR)+C |
| CV-1632 | HBc163 (E.cR) +C |
| CV-1634 | HBc163 (E.cR) |
| CV-1642 | HBc171 (E.cR) |
| CV-1643 | HBc171 (E.cR)+C |

The table, below, shows an alignment that illustrates the configuration of the C-termini of the full-length HBcAg (HBc183), and all particles harboring C-terminal truncations. Sequences are aligned according to amino acid residue position 149 from the N-terminus of HBc of SEQ ID NO:1 that is shared by all constructs. C-terminal cysteine residues, when present, are underlined.

**Table**

| Construct Name | Sequence | SEQ ID NO |
|---|---|---|
| HBc183 | VRRRGRSPRRRTPSPRRRRSQSPRRRRSQSRESQC | 381 |
| HBc182 | VRRRGRSPRRRTPSPRRRRSQSPRRRRSQSRESQ | 382 |
| HBc171(E.cR)+C | VRRRGRSPRRRTPSPRRRRSQSPC | 383 |
| HBc171(E.cR) | VRRRGRSPRRRTPSPRRRRSQSP | 384 |
| HBc163(E.cR)+C | VRRRGRSPRRRTPSPC | 385 |
| HBc163(E.cR) | VRRRGRSPRRRTPSP | 386 |
| HBc156(E.cR)+C | VRRRGRSPC | 387 |
| HBc156(E.cR) | VRRRGRSP | 388 |

### Example 4: Assay Procedures

### A. Antigenicity

### 1. Particle ELISA

Purified particles were diluted to a concentration of 2 µg/mL in coating buffer (50 mM sodium bicarbonate, pH 9.6) and coated onto the wells of ELISA strips (100 µL/well). The ELISA strips are incubated at room temperature overnight (about 18 hours). Next morning, the wells are washed with ELISA wash buffer (EWB) [phosphate buffered saline (PBS), pH 7.4, 0.05% Tween^{®}-20] and blocked with 3% BSA in PBS for 1 hour (200 µL/well). ELISA strips were stored, dry, at -20°C until needed.

To determine the antigenicity of particles, antisera are diluted using 1% BSA in EWB and 100 µL/well added to antigen-coated ELISA wells. Sera are incubated for 1 hour, washed with ELISA wash buffer (above) and probed using an anti-mouse (IgG)-HRP (The Binding Site, San Diego, CA; HRP = horseradish peroxidase) conjugate (100 µL/well) or other appropriate antibody for 1 hour. After washing with ELISA wash buffer the reaction was visualized by the addition of TM Blue substrate (100 µL/well). After 10 minutes, the reaction is stopped by the addition of 1N H₂SO₄ (100 µL/well) and read on an ELISA plate reader set at 450 nm.

### 2. Synthetic Peptide ELISA

A 20 amino acid residue synthetic peptide (NANP)₅ or 24 amino acid residue synthetic peptide M2 is diluted to a concentration of 1 µg/mL in coating buffer (50 mM sodium bicarbonate, pH 9.6) and coated onto the wells of ELISA strips (100 µL/well). Peptides are dried onto the wells by incubating overnight (about 18 hours), in a37°C incubator. Next morning, the wells are washed with ELISA wash buffer (phosphate buffered saline, pH 7.4, 0.05% Tween^{®}-20) and blocked with 3% BSA in PBS (200 µL/well) for 1 hour. ELISA strips are stored, dry, at -20°C until needed.

To determine antibody antigenicity of particles, antisera (monoclonal or polyclonal) are diluted using 1% BSA in EWB, and 100 µL/well added to peptide-coated ELISA wells. Sera are incubated for 1 hour, washed with ELISA wash buffer, and probed using an anti-mouse(IgG)-HRP conjugate (as above at 100 µL/well) or other appropriate antibody for 1 hour, washed again with ELISA wash buffer, and then visualized by the addition of TM Blue substrate (100 µL/well). After 10 minutes, the reaction is stopped by the addition of 1N H₂SO₄ (100 µL/well) and read on an ELISA plate reader set at 450 nm.

### B. Immunogenicity of Particles

To assay the immunogenicity of particles, mice are immunized, IP, with 10 or 20 µg of particles in an adjuvant of choice, and then boosted at 4 weeks with 10 µg in the same adjuvant. Mice are bled at 2, 4, 6, and 8 weeks.

### Example 5: Determination of 280:260 Absorbance Ratios

To determine the 280:260 absorbance ratio of purified particles, the particles were diluted to a concentration of approximately 0.2 mg/mL in 20 mM sodium phosphate buffer, pH 6.8, and absorbance values determined at wavelengths of 260 and 280 nm. The absorbance measured at 280 nm was divided by the value at 260 nm to determine the 280:260 ratio. The ratios were obtained for several samples, including native particles (HBc183), HBc particles truncated after residue position 149 (HBc149), and several HBc chimers that are identified elsewhere herein, are shown below in the Table. Full length particle CV-1559 is a preparation of the particles first reported in Neirynck et al., (1999) Nature Med., 5(10):1157-1163, whereas full length particle CV-1607 is a similar particle, in which the M2 polypeptide cysteines at polypeptide positions 17 and 19, (X₁₇ and X₁₉ of SEQ ID NO:9) were mutated to serine residues.

**Table**

| Particle Number | Full Length, (F) or C-Terminal Truncated, (T) | 280:260 Absorbance Ratio |
|---|---|---|
| HBc183 CV-1532 | F | 0.84 |
| HBc149 | T | 1.59 |
| CV-1438 | T | 1.57 |
| CV-1440 | T | NT |
| CV-1475 | T | 1.04 |
| CV-1492 | T | 1.33 |
| *CV-1559 | F | 0.68 |
| CV-1560 | T | 1.36 |
| CV-1569 | T | 1.38 |
| CV-1588 | T | 1.16 |
| CV-1590 | T | 1.51 |
| CV-1603 | T | 1.68 |
| CV-1604 | T | 1.40 |
| CV-1605 | T | 1.26 |
| CV-1607 | F | 0.73 |
| CV-1600 | T | 1.23 |
| CV-1601 | T | 1.12 |
| CV-1634 | T | 0.92 |
| CV-1632 | T | 0.96 |
| CV-1642 | T | 0.79 |
| CV-1643 | T | 0.77 |
| CV-1671 | T | NT |
| CV-1672 | T | 1.27 |

| | | |
|---|---|---|
| NT, not tested. *CV-1159 is identical to IM2-HBc described by Neirynck, 1999. | | |

### Example 6: Thermal Stability Protocol

Purified particles were diluted to a concentration of 0.5-1 mg/mL using 50 mM NaPO₄, pH 6.8 and allowed to incubate at 4°C, room temperature, or 37°C. Samples were taken at various time points and mixed with SDS-PAGE sample buffer (reducing) and run on 10% SDS-PAGE gels. Gels were stained using SimplyBlue SafeStain (Invitrogen, Carlsbad, CA), and then analyzed.

### Example 7: Analytical Gel Filtration

### Analysis of Hybrid particles

Analytical gel filtration analysis of purified hybrid HBc particles was performed using a 25 mL Superose^{®} 6 HR 10/30 chromatographic column (Amersham Pharmacia # 17-0537-01) and a BioCAD^{™} SPRINT Perfusion Chromatography System. The UV detector is set to monitor a wavelength of 280 nm. The column was equilibrated with 3 column volumes (CV; about 75 mL) of buffer (20 mM NaPO₄, pH 6.8) at a flow rate of 0.50 mL/minute.

The particles to be analyzed were diluted to a concentration of 1 mg/mL using 20 mM NaPO₄, pH 6.8. 200 Microliters (µL) of the sample were then loaded onto a 200 µL loop and injected onto the column. The sample was eluted from the column with 20 mM NaPO₄, pH 6.8 at a flow rate of 0.50 mL/minute.

Particles containing N-terminal cysteine residues or similar particles free of such cysteines were analyzed using the above procedure. Integration of the 280 nm trace was carried out using BioCAD^{™} software (PerSeptive^{™}).

### Example 8: Influenza M2 Constructs

Recently, Neirynck et al., (1999) Nature Med., 5(10):1157-1163 and WO 99/07839 reported the fusion of the 24 amino acid extracellular domain of M2 to the N-terminus of full-length HBc particles (HBc183), lacking amino acid residues 1-4. A schematic representation of that construct referred to herein as IM2HBc is shown below in which the 24-mer is linked to the N-terminus of HBc.

| | |
|---|---|
| IM2HBc MSLLTEVETPIRNEWGCRCNDSSD-HBc(5-183) | SEQ ID NO: 389 |

In one illustrative preparation, the M2 epitope was inserted into the immunodominant loop of hepatitis B core and particles referred to as CV-1475 were successfully expressed and purified using techniques discussed previously for such insertions and purifications. A mutated version of the M2 epitope, in which two cysteine residues at M2 native positions 17 and 19 were substituted by alanine residues, was also expressed in the immunodominant loop (CV-1473 particles) and the resulting particles purified. These two particles are illustrated schematically below.

| | |
|---|---|
| CV-1475 HBc(1-78)-GI-SLLTEVETPIRNEWGCRCNDSSD-EL-HBc(79-149) | SEQ ID NO: 390 |
| CV-1473 HBc(1-78)-GI-SLLTEVETPIRNEWGARANDSSD-EL-HBc(79-149)-C | SEQ ID NO: 391 |

The CV-1473 particle construct yielded approximately 7-fold more purified particles when compared with the native sequence (CV-1475). It remains to be determined if the mutation of the cysteine residues alters protective potential of the particles. However, epitopes delivered on the immunodominant loops of HBc are usually significantly more immunogenic as compared to when the same are fused to other regions (including the N-terminus), and resulting particles exhibit reduced anti-HBc immunogenicity.

Particles have also been prepared in which the M2 N-terminal 24-mer epitope was fused to the N-terminus of C-terminal truncated hepatitis B core particles. That construct (CV-1438) also contained the N-terminal pre-core sequence (SEQ ID NO:259). A similar construct was prepared that contained a single cysteine residue at the end of the hybrid protein (CV-1492), in this case immediately after Val-149 of the HBc gene. These constructs are shown schematically below.

| | |
|---|---|
| CV-1438 MGISLLTEVETPIRNEWGCRCNDSSDELLGWLWGI-HBc(2-149) | SEQ ID NO:392 |
| CV-1492 MGISLLTEVETPIRNEWGCRCNDSSDELLGWLWGI-HBc(2-149)-C | SEQ ID NO:393 |

It should be noted that to guard against translation initiation from the natural HBc initiator methionine, the codon for that residue was omitted. Residues contributed by EcoRI (GI) and SacI (EL) restriction sites are underlined. The pre-core sequence is recited between the underlined EL residues and "-HBc(2-149)".

Analysis by SDS-PAGE, showed that upon preparation, the CV-1438 monomer construct was unstable as compared to CV-1492, with HBc149, CV-1475 and CV-1473 serving as additional molecular weight controls on the SDS-PAGE gel. The instability of the CV-1438 monomers was not evident using analytical gel filtration of particles.

Both CV-1475 were expected to have slightly lower molecular weights than CV-1438 and CV-1492, because the former two contain the M2 epitope inserted directly into the immunodominant loop and therefore lack the pre-core sequence (SEQ ID NO:259) present in CV-1438 and CV-1492. As expected, CV-1492 was larger than CV-1475 and CV-1473; however, CV-1438, which is identical to CV-1492 save the C-terminal cysteine residue, is clearly not larger than CV-1475 and CV-1473 due to an apparent cleavage.

A construct containing an M2 N-terminal extracellular sequence as discussed before linked to the HBc N-terminus (Domain I) or loop (Domain II) and also containing an M2 protein C-terminal sequence such as that of SEQ ID NO: 11 (see Table A) linked to the loop (Domain II) or at the C-terminus (Domain IV) of HBc is also contemplated. Such a contemplated construct also contains at least one stabilizing C-terminal cysteine residue as discussed elsewhere herein.

To modify the amino-terminus of hybrid HBc particles containing immunodominant loop fusions to incorporate a cysteine residue, and minimal M2-derived sequence, a series of synthetic oligonucleotides are synthesized. To make V2.Pf1(N-M2(17-24/C17S), the oligonucleotides M2(17-24/C17S)-NcoI-F and HBc149/HindIII-R are used to amplify the hybrid HBc gene from vector V2.Pf1. The resultant 546 bp fragment is cleaved with NcoI and HindIII and inserted into pKK-223-3N, which has been cleaved with the same two enzymes.

To make V2.Pf1[N-M2(17-24/C19S)], the oligonucleotides M2(17-24/C19S)-NcoI-F and HBc149/HindIII-R are used to amplify the hybrid HBc gene in vector V2.Pf1. The resultant 540 bp fragment is cleaved with NcoI and HindIII and inserted into pKK-223-3N, which had been cleaved with the same two enzymes.

| | |
|---|---|
| M2(17-24/C17S)-NcoI-F | |
| | |
| F G | SEQ ID NO: 394 |
| ATTTCG | SEQ ID NO: 395 |
| M2(17-24/C19S)-NcoI-F | |
| | |
| | SEQ ID NO: 396 |
| | SEQ ID NO: 397 |

### Example 9: HBc Chimer Molecules With and Without

### Both N- and C-Terminal Cysteine Residues

A series of HBc chimer molecule-containing particles was prepared that contained residues 1-24 of the influenza A, M2 protein peptide-bonded at or near the N-terminus of HBc whose C-terminus was truncated at residue 149. The component chimeric protein molecules contained different N-terminal sequences that included an M2 sequence or variant, and some contained a C-terminal cysteine residue.

All purified particles listed in Table 9A-9C, hereinafter, were analyzed by analytical size exclusion chromatography to assess the retention of particulate structure following purification. Particles designated CV-1603, which contain no N-terminal cysteine residues, displayed evidence of disassembly back to sub-particulate structures because the protein eluted in the 1500 second range (particles elute at approximately 1000 seconds).

Similar analysis of particles CV-1590, which are similar to CV-1603 particles except for the mutation of two serine residues to cysteine residues in the native N-terminal M2 sequence, revealed that that construct remained particulate following purification, with elution occurring at around 1000 seconds, which is typical for a hybrid particle (Fig. 4). There was no evidence of disassembly for CV-1590 particles.

Analysis of CV-1560 particles, whose chimer protein also has two N-terminal cysteine residues, revealed that it too was particulate following purification, although it did exhibit some degree of disassembly, suggesting that the stabilization was not quite as robust as it was for CV-1590 particles. Comparison of the N-terminal configurations of CV-1590 and CV-1560 particles (Table 11, hereinafter), shows that the relative position of the two cysteine residues in CV-1560 particles is shifted by 3 amino acid residues relative to CV-1590 particles via the deletion of three amino acid residues (DEL), indicating that the cysteine residues may be required to be a minimal distance from the start of the core gene to enable optimal cross-linking.

### Example 10: Particles with an M2 or M2 Variant

### Sequence and a C-Terminal Cysteine. Residue

CV-1603 particles were shown in Fig. 4 to rapidly disassemble following purification. The HBc chimer molecules that comprise CV-1605 particles are similar to those of CV-1603 particles, except that the CV-1605 component chimer molecules have a single C-terminal stabilizing cysteine. A plasmid was made to direct the expression of CV-1605 particles to determine if the addition of a C-terminal cysteine residue to CV-1603 particles could impart greater stability on the particle. Following purification, CV-1605 particles were analyzed using analytical size exclusion chromatography.

The results of this study demonstrated that particle stabilization was more complete than for the CV-1603 particles, but incomplete compared to CV-1590 particles, which contain two amino-terminal cysteine residues and no C-terminal stabilizing cysteine. Although a significant amount of CV-1605 remained particulate, there was evidence of a heterogeneous mixture of sub-particulate structures that eluted over a broad range. These observations suggest that for this hybrid particle (CV-1603), C-terminal stabilization as found in CV-1605 particles was less complete than for the N-terminal stabilization found in CV-1590 particles.

To investigate the compatibility of combined amino and carboxyl-terminal cysteine stabilization of hybrid particles, an expression plasmid was constructed to direct the expression of CV-1604 particles. The component chimer molecules of CV-1604 particles contain both the two amino-terminal stabilizing cysteine residues present in a native M2 polypeptide sequence (as in CV-1590) as well as a C-terminal stabilizing cysteine (as in CV-1605 particles). Analysis of CV-1604 particles showed that they retained a homogeneous particulate state following purification, indicating that the two stabilizing methods are complementary and can be used in concert with each other.

Alternative linker sequences between the N-terminus of HBc and the N-terminal cysteine residues were investigated using particles CV-1438 and CV-1492. Both of these particles contain the amino acid sequence ELLGWLWGIDI (SEQ ID NO:398) between the M2 fusion and amino acid D4 of HBc. Amino acid residues LGWLWGIDI are derived from amino acids -6 of pre-core to amino acid 13 of HBc, with the initiator codon of HBc deleted to prevent translation initiation from this position, which would compromise the study. The HB pre-core sequence includes a cysteine at position -7.

These particles differed only in the fact that the CV-1438 component chimer molecule terminated at position 149 of HBc, whereas the CV-1492 component chimer molecule terminated at 149 of HBc and contained a terminal cysteine at position 150 relative to the HBc of SEQ ID NO:1. When analyzed by analytical gel filtration, using an alternative but similar method to that discussed before, whereby particles elute at approximately 10 minutes, both constructs were shown to be particulate following purification. This study demonstrated the compatibility of amino- and carboxyl-terminal cysteine stabilization of truncated particles, and the tolerance of substantial variability in the amino acid sequence and distance between the N-terminal cysteine residues and start of the HBc gene.

**Table 9A**

| Construct Number | N-terminal Fusion | HBc N-term Start | Residues Between M2 and HBc | C-term End | Bound Nucleic Acid | C-term Cysteine Stab |
|---|---|---|---|---|---|---|
| CV-1438 | M2 (2-24) | D2 | ELLGWLWGI | 149 | No | No |
| CV-1492 | M2(2-24) | D2 | ELLGWLWGI | 149 | No | Yes (C150) |
| CV-1560 | M2 (1-24) | D4 | None | 149 | No | No |
| CV-1590 | M2 (1-24) | D4 | EL | 149 | No | No |
| CV-1603 | M2 (1-24) (2C>2S) | D4 | EL | 149 | No | No |
| CV-1604 | M2 (1-24) | D4 | EL | 149 | No | Yes (C150) |
| CV-1605 | M2 (1-24) (2C>2S) | D4 | EL | 149 | No | Yes (C150) |
| CV-1606 | M2 (1-24) | D4 | EL | 183 | Yes | Yes (C183) |
| CV-1607 | M2 (1-24) (2C>2S) | D4 | EL | 183 | Yes | Yes (C183) |
| CV-1671 | M2 (1-24) (C17S) | D4 | EL | 149 | No | No |
| CV-1672 | M2 (1-24) (C19S) | D4 | EL | 149 | No | No |
| CV-1816 | M2 (1-24)/ LE/ M2 (2-24) | D4 | EL | 149 | No | Yes (C150) |
| CV-1817 | M2 (1-24) (2C>2S)/ LE/ M2 (2-24) | D4 | EL | 149 | No | Yes (C150) |
| CV-1818 | M2 (1-24) (2C>2S)/ LQ/M2 (2-24). (2C>2S)/ LE/ M2 (2-24) | D4 | EL | 149 | No | Yes (C150) |

**Table 9B**

| Construct Number | Loop Fusion | 5'/3' Fusion Flanking Sequence | C-term End | Bound Nucleic Acid | C-term Cysteine Stab |
|---|---|---|---|---|---|
| CV-1440 | M2 (2-24) | GI/EL | 149 | No | No |
| CV-1475 | M2(2-24) | GI/EL | 149 | No | Yes (C150) |
| CV-1569 | M2 (2-24) (2C>2S) | GI/EL | 149 | No | Yes (C150) |
| CV-1586 | M2 (2-24) | GI/EL | 183 | Yes | Yes (C183) |
| CV-1587 | M2 (2-24) (2C>2S) | GI/EL | 183 | Yes | Yes (C183) |

**Table 9C**

| Construct Number | C-terminal Fusion | HBc C-term End | Residues Between M2 and HBc | C-term End | Bound Nucleic Acid | C-term Cysteine Stab |
|---|---|---|---|---|---|---|
| CV-1588 | M2 (2-24) | V149 | GI | (M2)EL | No | No |

Table 10, below, shows an alignment that illustrates the configuration of the N-termini of HBeAg, and particles harboring N-terminal fusions. Sequences are aligned according to amino acid residue position 4 from the N-terminus of HBc of SEQ ID NO:1 that is shared by all constructs. N-terminal cysteine residues, when present, are underlined.

**Table 10**

| Construct Name | Sequence | SEQ ID NO |
|---|---|---|
| HBeAg | SKLCLGWLWGMDID | 399 |
| CV-1438/CV-1492 | MGISLLTEVETPIRNEWGCRCNDSSDELLGWLWGIDID | 400 |
| CV-1560 | MSLLTEVETPIRNEWGCRCNDSSD | 401 |
| CV-1590/CV-1604/CV-1606 | MSLLTEVETPIRNEWGCRCNDSSDELD | 402 |
| CV-1603/CV-1605/CV-1607 | MSLLTEVETPIRNEWGSRSNDSSDELD | 403 |
| CV-1671 | MSLLTEVETPIRNEWGSRCNDSSDELD | 404 |
| CV-1672 | MSLLTEVETPIRNEWGCRSNDSSDELD | 405 |
| CV-1816 | | 406 |
| CV-1817 | | 407 |
| CV-1818 | | |
| | | 408 |

Table 11, below, provides a tabulation of the results in which stability was assessed for particles containing an N-terminal influenza A M2 sequence or variant contemplated herein. As is seen, stable particles have been prepared from HBc chimer molecules that contain an N-terminal cysteine residue at a position of minus 14 (-14) relative to the N-terminus of the HBc sequence of SEQ ID NO:1 to about the N-terminus itself.

**Table 11**

| Construct Name | Amino Acids HBc D4 and Cysteine Between N-terminal Residues | | C-terminal Cysteine Stabilization | Stable Particle Formed |
|---|---|---|---|---|
| | Cys 1 | Cys 2 | | |
| HBeAg | - | 9 | No | No |
| CV-1438 | 18 | 16 | No | Yes |
| CV-1492 | 18 | 16 | Yes | Yes |
| CV-1560 | 6 | 4 | No | Yes |
| CV-1590 | 9 | 7 | No | Yes |
| CV-1603 | - | - | No | No |
| CV-1604 | 9 | 7 | Yes | Yes |
| CV-1605 | - | - | Yes | Yes/No |
| CV-1607 | - | - | Yes | Yes |
| CV-1671 | - | 7 | No | Yes |
| CV-1672 | 9 | - | No | Yes |
| CV-1817 | 9 | 7 | Yes | Yes |
| CV-1818 | 9 | 7 | Yes | Yes |
| CV-1901^{a} | 34^{b}/9 | 32^{b}/7 | Yes | Yes |

| | | | | |
|---|---|---|---|---|
| ^{a} A particle whose cysteines at 48 and 107 were changed to serines. ^{b} Counted from the second N-terminal M2 copy. | | | | |

### Example 11: Antigenicity of Various

### M2-Containing Particles

The antigenicity of the various particles to the monoclonal antibody 14C2 was examined using an ELISA. To ensure retention of particles in their native conformation, ELISA plates were first coated with a polyclonal antibody (rabbit) to capture the particles, which were then probed with various dilutions of either the 14C2 monoclonal antibody, or anti-HBc monoclonal antibodies with specificity for the immunodominant loop region of HBc particles. The data, presented in the table below, demonstrate that presentation of M2e in the immunodominant loop of HBc does not significantly alter the accessibility of the M2e epitope for the 14C2 monoclonal antibody, relative to presentation at the N-terminus (IM2HBc/CV-1559 and CV-1604). These observations were not surprising because it has previously been shown that 14C2 binds to an internal region of M2e (amino acids 8, 10, 11, and 14 of M2, as opposed to binding the N-terminus [Zebedee et al., (1988) J. Virol., 62 (8) :2762-2772] .

In addition, all particles, with the exception of CV-1569, retained antigenicity to anti-HBc monoclonal antibodies 3105. The loss of recognition by 3105 is a previously observed phenomenon for particles with sequences inserted into the immunodominant loop, and this typically translates to reduced anti-HBc responses for these particles following immunization. Monoclonal antibodies 3105 were purchased from the Institute of Immunology, Tokyo, Japan.

| Particle | Monoclonal | Antibody |
|---|---|---|
| | 14C2 | 3105 |
| CV-1123 | - | + |
| IM2HBc/ CV-1559 | + | + |
| CV-1604 | + | + |
| CV-1569 | + | - |

### Example 12: Antibody Subclass and Protection

A summary of several studies in which various M2e-HBc constructs (10 µg/mouse) and various adjuvants were assayed. About one-half were i.p. administration and about one-half i.n. For each group (14 mice) the sera were pooled and the titer of anti-M2e IgG subclass antibodies was determined. The results are from sera taken one week after the second boost. For mice where the IgG2a titer was more than 10⁴, the IgG2A titer was 10⁴ (*).

| IgG2a | Number of Groups | Percent Protection |
|---|---|---|
| 10⁴ | 8 | 100 |
| < 10^{4*} | 4 | 70-95 |

Adjuvants are increasingly being investigated for their ability to enhance the magnitude and persistence of immune responses to vaccines, as well as modulate the Th1/Th2 bias of the immune response. Although many experimental adjuvants are under investigation, alum remains the only adjuvant that is a component of FDA-approved vaccines in the US. Typically, alum biases immune responses towards a Th2 type, which is manifested by the production of high levels of IgG1 antibody in mice.

It is found that alum-formulated M2e-HBc particles do elicit a significant IgG1 response; however, IgG2a and IgG2b antibodies, which are Th1 indicators, are also elicited. In an attempt to enhance the production of Th1-type IgG subclasses, the immunogenicity of Alhydrogel™-formulated particles supplemented with RC529, a synthetic derivative of MPL developed by Corixa Corporation, was tested in mice. These studies revealed that inclusion of RC529 in the Alhydrogel™ formulation resulted in a dramatic enhancement of anti-M2e IgG2a titers, increasing the anti-M2e IgG2a:IgG1 ratio by approximately 10-fold. All mice in both groups were completely protected from lethal challenge; however, there was an indication of reduced morbidity (temperature decrease and weight loss) in mice immunized with CV-1569 formulated with Alhydrogel™ + RC529, versus Alhydrogel™ alone.

### Example 13: Particle Assembly of Chosen Chimers

Analysis of size exclusion chromatography elution profiles revealed that four of the five predicted cysteine mutation chimers successfully assembled into particles. To confirm that mutation of C48 and C107 to serines had no affect on particle assembly, HBc149 and HBc149(C48S/C107S) were included, as well as the C-terminal cysteine harboring pair, HBc149+C and HBc149(C48S/C107S)+C. Inclusion of these constructs was also necessary for comparison of stability of the particles, as well as for determining the extent of disulfide bonding in the mutated particles.

All expressed particles were purified and final yields tabulated, hereinafter. Particle yields were generally very high, relative to non-engineered HBc149, which suggests that the introduced mutations did not greatly impact particle assembly. Due to the high success in producing the first round of particles, all of the chimers were similarly modified to accept heterologous loop inserts, with the exception of the failed particle HBc149(C48S/C107S) L55C/H104C+C.

**Table 12**

| Purified Protein Yields of Chimers Harboring Cysteine Mutations | | |
|---|---|---|
| Chimer Name | Particle Yield Following CL-4B Treatment* (total AU) | Final Yield (mg) |
| HBc149 | 109 | 19 |
| HBc149(C48S/C107S) | 94 | 12 |
| HBc149+C | 117 | 13 |
| HBc149(C48S/C107S)+C | 105 | 14 |
| HBc149(C48S/C107S)L55C/H104C+C | NA | NA |
| HBc149(C48S/C107S)A58C/L100C+C | 87 | 15 |
| HBc149(C48S/C107S)A69C/V89C+C | 150 | 28 |
| HBc149(C48S/C107S)W62C/F97C+C | 59 | 13 |
| HBc149(C48S/C107S)L76C/R82C+C | 100 | 10 |

| | | |
|---|---|---|
| *Particles are typically more than 75% pure at this stage. NA = No assembly. | | |

It was determined that approximately 2 mg of each chimer were required for characterization studies, so, in most cases, a maximum of about 50 AU (25 mg of semi-pure protein) were applied to the anion exchange column to avoid overloading. The final purified protein yields, therefore, represent predicted yields based upon the amount of material discarded following the HA chromatography step.

### Example 14: Characterization of Assembled Particles

Disulfide bond formation was found to be a function of time and temperature. Analysis of each engineered particle in its native conformation by analytical size exclusion chromatography revealed no difference between particle integrity over a two week stability study conducted at 4°C, room temperature, and 37°C and analyzed on days zero, 3, 7, and 14. For simplicity, only profiles for days zero and 14 at 37°C (the most severe conditions) are noted, as all elution profiles for a given particle were substantially identical. The two control particles lacking C-terminal cysteines, HBc149 and HBc149(C48S/C107S), did not entirely retain their particulate structure, their elution profiles revealing distinct populations of lower order structures.

Further analysis of the cross-linking of particles was assessed by determining the ability of the particles/protein to migrate into non-reducing SDS-PAGE gels. Disulfide bonding was shown to be incomplete in most particles, including controls, at day zero and remained incomplete at 4°C for the duration of the study. Incubation at room temperature enhanced disulfide bonding, as determined by the reduction in intensity of monomer and dimer bands with a corresponding increase in the appearance of higher order multimers.

Significantly, all particles (except non-C-terminally stabilized controls) that were incubated at 37°C were entirely disulfide bonded after a period of 7 days. Of particular note was the fact that the C-terminally stabilized C48S/C107S chimer appeared to be entirely disulfide bonded at day zero, whereas its C48/C107 counterpart was not and did not reach the same level of cross-linking achieved by the C48S/C107S chimers during the period of study. Additionally, the engineered particle HBc149(C48S/C107S)W62C/F97C+C exhibited the same high level of cross-linking at day zero.

Reducing SDS-PAGE gels of all samples revealed no significant change in the intensity of monomers from the start to the finish of the stability study, indicating that all monomers remained intact over all temperatures, as illustrated by comparison of samples taken on day zero and day 14 at 37°C gels. Importantly, these observations confirm that the reduction in monomer and dimer intensity over time for selected chimers analyzed under non-reducing conditions was not due to degradation of the monomers.

The presence of free thiols was measured for all particles at all time points in the stability study to establish the degree of disulfide bond formation. Although free thiols react with Ellman's reagent, those involved in disulfide bonds do not. The results of these studies demonstrate that the presence of free thiols decreased over time and was accelerated for the samples incubated at 37°C, relative to those incubated at room temperature and 4°C. These observations are consistent with those made for the analysis of particles using non-reducing SDS-PAGE gels, whereby the migration of constructs decreased commensurate with decreased levels of free thiols.

Consistent with the observations of Zheng *et al*, above, the two control particles containing wild type C48 and C107 exhibited the presence of free thiols at all times and temperatures, confirming that these cysteine residues remain largely in the reduced state. In corresponding particles where those cysteines were mutated to serines, free thiol reactivity was abolished, again attesting to the fact that C61 and C150 are entirely disulfide bonded. Although engineered particles were not entirely cross-linked at the end of the purification regimen, as evidenced by the presence of free monomers and dimers on non-reducing SDS-PAGE gels, all had barely detectable levels of free thiols by day 7 at 37°C.

These data were encouraging, and the computer modeling used to predict putative disulfide bond-forming residues had been very successful, but not completely predictive. Loop stability, however, was difficult to predict in this manner and so the suite of epitope-carrying vectors was created.

It should be noted, first, that all C48S/C107S epitope-carrying particles behaved in a manner similar to their wild type counterparts, signifying that these mutations did not affect particle assembly. The β-amyloid epitope, which failed to assemble into a stable particle in non-disulfide engineered particles, also failed in all four cysteine-engineered vectors, indicating an inability of the cysteines bridging the immunodominant loop to retain the monomer in its desired conformation. The ASP-1 epitope behaved similarly in engineered vectors, although the control vectors with no added cysteines produced low levels of assembled particle. The anthrax epitope, PA, which failed in control particles, actually did produce low levels of assembled particle in the A58C/L100C vector, but no others. Finally, the influenza epitope, which yields high levels of assembled HBc in control vectors, produced moderate levels of assembled vectors in two of the four engineered particles, the L76C/R82C chimer and the W62C/F97C chimer. A summary of the particle yields of all chimers is shown in Table 13 below. Successfully assembled particles are in bold.

**Table 13**

| Purified Protein Yields of Epitope-Carrying Chimers* | | | |
|---|---|---|---|
| Chimer Number | Chimer Name | Particle Yield After CL-4B Treatment* (total AU) | Final Yield (mg) |
| 1510 | V16.β-Am | No assembly | No assembly |
| 1794 | V16(C48S/C107S).β-Am | No assembly | No assembly |
| 1797 | A58C/L100C.β-Am | No assembly | No assembly |
| 1796 | A69C/V89C.β-Am | No assembly | No assembly |
| 1798 | W62C/F97C.β-Am | No assembly | No assembly |
| 1795 | L76C/R82C.β-Am | No assembly | No assembly |
| 1546 | V16.ASP-1 | 48 | 4.9 |
| 1775 | V16(C48S/C107S).ASP-1 | 50 | 5.1 |
| 1778 | A58C/L100C.ASP-1 | No assembly | No assembly |
| 1777 | A69C/V89C.ASP-1 | No assembly | No assembly |
| 1779 | W62C/F97C.ASP-1 | No assembly | No assembly |
| 1776 | L76C/R82C.ASP-1 | No assembly | No assembly |
| 1629 | V16.PA | No assembly | No assembly |
| 1780 | V16(C48S/C107S).PA | No assembly | No assembly |
| 1783 | A58C/L100C.PA | 52 | 5.0 |
| 1782 | A69C/V89C.PA | No assembly | No assembly |
| 1784 | W62C/F97C.PA | No assembly | No assembly |
| 1781 | L76C/R82C.PA | No assembly | No assembly |

| | | | |
|---|---|---|---|
| *Particles are typically more than 75% pure at this stage. "HBc149(C48S/C107S)+C" has been omitted from disulfide-engineered particle names for simplicity. | | | |

No particle expressed as well as the original V16.IA(M2)2C/2S clone (chimer CV-1569), although the C48S/C107S version exhibited similar expression levels. When purified, however, the C48S/C107S version yielded less than a milligram of material, a tenth of the purified material recovered from the original clone, indicating that a different method of purification may be necessary for this type of chimer. In purification, the particles seemed to bind tightly to the hypatite column and recovery from this column was only 10% of what was loaded, where other particles made in this study were recovered at 60%, or higher.

Because the influenza set of particles was the most complete, this was the only set of particles set up for stability studies. Although the purifications did not appear to be optimal (recovery of two of the four particles was very low), the material was incubated at 37°C (to facilitate disulfide bonding, as found previously) and the same analytical methods used for the initial stability study were employed to characterize these particles. Originally, the study was set up to run for two weeks, as described previously, but samples collected at zero, 3, and 7 days were sufficient to see a pattern.

Although particles exhibited typical disulfide bonding on non-reducing gels at day zero, analysis of the four particles revealed a distinct cleaved monomer band in all engineered particles. The control particle, V16.2A(M2)2C/2S, exhibited no cleavage, although this was the only lot to remain intact - all others (five lots) showed the same cleaving pattern (data not shown). Incubation for three days revealed complete degradation of the monomer in the V16(C48S/C107S) chimer and the L76C/R82C chimer and a definite increase of cleaved product in the W62C/F97C chimer, although these native particles remained intact. Native particle analysis via analytical size exclusion chromatography, did, however, show that the purified V16(C48S/C107S) chimer was not an assembled particle, perhaps due to the extremely low purified yield - it is likely that formed particles were not efficiently eluted off the purification columns. The degradation trend of all particles continued through day 7, when the stability study was terminated.

In recent work with the IA(M2)2C/2S epitope, it has been found that, when displayed in the loop, this epitope is prone to cleavage. Thus, N-terminal sequencing revealed fragments cut roughly in half, cleaving at a site within the inserted epitope. It is therefore thought that monomers would cleave, regardless of loop stabilization simply due to the nature of the particular epitope. Further exploration of loop stability with other epitopes is underway.

### Example 15: HBc Proteins CV-1843 and CV-1842 Control

An HBc carrier was designed and constructed to permit one or two conjugation events per protein monomer. Conjugation chemistries particularly contemplated herein link a saccharide, peptide, lipid or glycolipid to a primary amine. Carrier protein CV-1843 contains only a single lysine residue and that residue is an added lysine that is present at HBc position 77 in the HBc immunodominant loop. Studies with a lysine minus mutant, CV-1842, have shown that certain haptens (particularly peptides) can be conjugated to the N-terminus whereas an oligosaccharide, diacyl-LOS, cannot. The difference is probably due to the difference in geometry and/or hydrophilicity of the hapten types. This particular synthesis does not involve the addition of a helper T cell epitope, although a helper T cell epitope can be conjugated to the carrier or expressed therewith as a fusion protein.

Thus, the CV-1843 protein contains four mutations of amino acids on HBc core at position 7 by replacing lysine codon AAA with arginine codon (CGC), position 97 by replacing lysine codon AAG with arginine codon AGG and at positions 48 and 107 by replacing cysteine codons (TGT) with serine codons TCT. One insertion of a lysine codon was made between amino acids L76 and E77 of the HBc gene. The above site-directed mutagenesis was completed by using the starting plasmid CV-1123 with QuickChange™ XL Site-Directed Mutagenesis Kit from Stratagene.

Two oligonucleotide primers were used for each mutation or insertion. One primer is the complementary strand of the other. Oligonucleotide primers for all mutagenesis reactions were designed in such a way that a mutation codon was present with 16 to 20 nucleotides of flanking sequence on either side of the codon, with G or C as the proximal nucleotide [closest to the site of the mutation]. Pfu Turbo™ DNA polymerase was used in mutagenic PCR and Dpn I was used to digest the parental DNA plasmid.

The primer sets used are set out below, wherein the original residue at a given position is noted first in single letter designation, followed by the position number of the replacement, and then the single letter designation for the replacement residue. The primers for insertion of a lysine (K) at position 77 contained no original or replacement residues as a single residue was inserted at that position.
Primers for Position 7
   K7R-Forward
      CCATGGACATCGACCCTTATCGCGAATTTGGAGCTACTGTGGAG SEQ ID NO:440
   K7R-Rerverse
      CTCCACAGTAGCTCCAAATTCGCGATAAGGGTCGATGTCCATGG SEQ ID No:441
Primers for Position 97
   K97R-Forward
      CACTAATATGGGCCTAAGGTTCAGGCAACTCTTGTGG SEQ ID No:442
   K97R-Reverse
      CCACAAGAGTTGCCTGAACCTTAGGCCCATATTAGTG SEQ ID No:443
Primers for Position 48
   C48S-Forward
      GCCTTAGAGTCTCCTGAGCATTGTTCACCTCACCATACTGC SEQ ID No:444
   C48S-Reverse
      GCAGTATGGTGAGGTGAAGAATGCTCAGGAGACTCTAAGGC SEQ ID No:445
Primers for Position 107
   C107S-Forward
      GGCAACTCTTGTGGTTTCACATTTCTTGTCTCACTTTTGGAAGAG SEQ ID No:446
   C107S-Reverse
      CTCTTCCAAAAGTGAGAGAAGAAATGTGAAACCACAAGAGTTGCC SEQ ID No:447
Primers for Lysine Insertion at Position 77
   K77-Forward
      CCTGGGTGGGTGTTAATTTGAAAGAAGATCCAGCGTCTAGAG SEQ ID No:448
   K77-Reverse
      CTCTAGACGCTGGATCTTCTTTCAAATTAACACCCACCCAGG SEQ ID No:449

The CV-1842 protein particle vector was constructed in the same way as the vector for formation of CV-1843 protein particles. The CV-1842 protein contains the same four mutations as CV-1843 at position 7, 97, 48 and 107. However, the CV-1842 protein does not contain the lysine insertion at position 77. The CV-1842 protein particles serve as a control particle for CV-1843 in conjugation when using lysine as the functional group.

The recombinant plasmids were grown in E. coli BLR, induced with 0.5 mM IPTG, and grown overnight (about 18 hours). The cells were harvested by centrifugation and lysed by microfluidization. The soluble material was ammonium sulfate precipitated twice and loaded on a Sepharose CL-4B column (Amersham). The second peak eluted from the column was loaded on a ceramic hydroxyapatite column, eluted and reprecipitated in ammonium sulfate. The protein was resuspended in water and dialyzed into 50 mM sodium phosphate buffer (pH 6.8) for analysis and conjugation.

### Example 16: dLOS Conjugation to CV-1843 and CV-1123 Proteins

The CV-1843 protein and CV-1123 protein were used for the following study. Adipic acid dihydrazide- (ADH-) derivitized 9274 dLOS was provided by Dr. Xin-Xing Gu of the National Institutes of Health, Bethesda MD and was separately conjugated to each type of HBc by each of two methods. The first chemistry was direct, carbodiimide linkage to acidic residues on CV-1123. The second was linkage to the lysines at positions 7, 77 and 97 using ADH-derivitized-succinimidyl 3- [2-pyridyldithio] propionamido (SPDP) -N- [γ-maleimidobutyryloxy]succinimide ester (GMBS) prepared according to the distributor's instructions.

Thus, ADH-derivitized dLOS was reacted with N-succinimidyl-3-[2-pyridyldithio]propionate (SPDP) to form the mixed disulfide, and N-[γ-maleimido-butyryloxy]succinimide ester (GMBS) was reacted with the N-terminal amine or ε-amino lysine of the HBc protein to form an HBc-derivatized succinimido ester. Reaction of the SPDP mixed disulfide with dithiothreitol (DTT) followed by dialysis provided the 3-thiopropionhydrazido-dLOS derivative that was reacted with the HBc-derivatized succinimido ester to form desired conjugate.

The conjugates prepared above were used to immunize mice (5 per group) either using a saline solution or saline plus a pre-adsorption of the conjugate on to alum. Mice were immunized three times, three weeks apart, and the titers shown are two weeks after the final boost. Each dose contained 5 µg of carbohydrate. ELISA conditions were taken from [Sun. et. al. (2000) Vaccine 2000 18 (13) :1264-1272] The only antibody class to show preexisting titers were IgM. Titers were boosted one log with all of the constructs.

IgG2b titers were significantly lower when the vaccine was adsorbed to alum. This can be due to the deacylation or hydrolysis of the carbohydrates [Sturgess et. al. (1999) Vaccine, 17 (9-10) :1169-1178]. None of the mice showed measurable titers to IgG2a. IgG1 induction, characteristic of a Th2 response, was slowed with immunization by the longer cross-linker on HBc, but it eventually rose to the same level as the rest of the animals. IgG3 levels were comparable.

### Example 17: Peptide Conjugation to Particles Prepared from CV-1843 and CV-1842 Proteins

A peptide was conjugated to particles prepared from the CV-1843and CV-1842 proteins. The peptide had the amino acid sequence SLLTEVETPIRNEWGCRCNDSSD (SEQ ID NO:450) and was conjugated using the linker N-[y-maleimidobutyryloxy]-sulfosuccinimide ester (sulfo-GMBS, Pierce Chemical Co.). After optimization, the results of the conjugation showed binding to the desired lysine and also to the amino-terminus as discussed below.

Thus, silver-stained reducing SDS PAGE analysis for particles prepared from CV-1843 protein showed a monomer band of completely reduced HBc polypeptide chain at about 22,000 daltons, a one repeat (one peptide addition) band above it and a two repeat (two peptide additions) band above the one repeat band. At the region of approximately 44,000 daltons, there was again a primary band and two conjugate bands. For particles prepared from CV-1842 proteins, monomer and dimer bands were present as were bands for monomer and dimer plus a single polypeptide. Thus, the peptide was conjugated to both the added lysine at position 77 and the N-terminus for the particles prepared from the CV-1843 protein, but the peptide was only conjugated to the N-terminus for the particles prepared from the CV-1842 particles that had no lysines residues. Monomer and dimer refer to the inability to completely reduce and separate the two 22,000 dalton monomers after conjugation. These results show clearly that the purified particles are capable of having peptides chemically conjugated to them.

The use of the article "a" or "an" is intended to include one or more.

The foregoing description and the examples are intended an illustrative and are not to be taken as limiting. Still other variations within the scope of the claims are possible and will readily present themselves to those skilled in the art.

## Claims

1. A recombinant chimer hepatitis B core (HBc) protein molecule up to 380 amino acid residues in length that
(a) includes the HBc sequence of residue positions 4 through 75 and 85 through 140 in which one or both cysteine residues at positions 48 and 107 is replaced by another residue;
(b) has up to all of the residues in the sequence of HBc positions 76 through 85 present and peptide-bonded to a heterologous sequence of up to 75 amino acid residues that constitute an Immunogen, or a sequence of one to 40 amino acid residues that constitute a chemically-reactive linker residue for a conjugated hapten;
(c) contains one to three cysteine residues present (I) at an amino acid position of the chimer molecule corresponding to amino acid position-20 to +1 from the N-terminus of the HBc sequence of SEQ ID NO: 1 in a sequence other than that of the HBc precore sequence, and, optionally, (ii) toward the C-terminus of the molecule from the C-terminal residue of the HBc sequence and within 30 residues from the C-terminus of the chimer molecule ("C-terminal cysteine residue(s)"): (d) contains up to 20 percent substituted amino acid residues in the HBc sequence, and (e) self-assembles into particles after expression that upon collection, purification and dissolution, exhibit a ratio of absorbance at 280 nm to 260 nm of 0.9 to 1.7.

2. The recombinant chimer hepatitis B core protein molecule according to claim 1 that contains one to three C-terminal cysteine residue(s).

3. The recombinant chimer hepatitis B core protein molecule according to claim 1 that contains at least 135 of the N-terminal 163 amino acid residues of HBc.

4. The recombinant chimer hepatitis B core protein molecule according to claim 3 that contains an HBc sequence of at least 135 of the N-terminal 156 amino acid residues of the HBc molecule.

5. The recombinant HBc chimer protein molecule according to claim 1 wherein said peptide-bonded sequence of up to 75 residues is present.

6. The recombinant HBc chimer protein molecule according to claim 5 that contains a second peptide-bonded sequence of up to 75 residues present bonded to the N-terminus or to the C-terminus of the chimer.

7. The recombinant HBc chimer protein molecule according to claim 6 wherein said first-named sequence of up to 75 residues contains a B call epitope.

8. The recombinant HBc chimer protein molecule according to claim 6 wherein said second-named sequence of up to 75 residues contains a T cell epitope.

9. The recombinant HBc chimer protein molecule according to claim 1 wherein both cysteine residues at positions 48 and 107 are replaced by another residue.

10. The recombinant HBc chimer protein molecule according to claim 9 wherein the replacement residue for each cysteine Is selected from the group consisting of glutamine, asparagine, serine, alanine, threonine and lysine.

## Patentansprüche

1. Rekombinantes chimäres Hepatitis-B-Core-(HBc-)Proteinmolekül mit einer Länge von bis zu 380 Aminosäureresten, bei dem
(a) die HBc-Sequenz der Restpositionen 4 bis 75 und 85 bis 140 enthalten ist, worin einer der oder beide Cysteinreste an Positionen 48 und 107 durch einen anderen Rest ersetzt ist bzw. sind;
(b) die Reste in der Sequenz der HBc-Positionen 76 bis 85 bis zur Gänze vorliegen und an eine heterologe Sequenz von bis zu 75 Aminosäureresten, die ein Immunogen darstellen, oder eine Sequenz von bis zu 40 Aminosäureresten, die einen chemisch reaktiven Linker-Rest für ein konjugiertes Hapten darstellen, peptidgebunden sind;
(c) ein bis drei Cysteinreste enthalten sind, die (i) an einer Aminosäureposition -20 bis +1 vom N-Terminus der HBc-Sequenz gemäß SEQ ID NO: 1 entsprechenden Aminosäureposition des chimären Moleküls in einer anderen Sequenz als der der HBc-Precore-Sequenz und gegebenenfalls (ii) nach dem C-Terminus des Moleküls von dem C-terminalen Rest der HBc-Sequenz und innerhalb von 30 Resten vom C-Terminus des chimären Moleküls ("C-terminaler Cysteinrest" bzw. "C-terminale Cysteinreste") liegen; (d) bis zu 20 Prozent substituierte Aminosäurereste in der HBc-Sequenz enthalten sind und (e) nach der Expression eine Selbstorganisation zu Partikeln erfolgt, die nach Sammeln, Aufreinigen und Lösen ein Verhältnis der Absorption bei 280 nm zu der bei 260 nm von 0,9 bis 1,7 zeigen.

2. Rekombinantes chimäres Hepatitis-B-Core-Proteinmolekül nach Anspruch 1, das einen C-terminalen Cysteinrest bis drei C-terminale Cysteinreste enthält.

3. Rekombinantes chimäres Hepatitis-B-Core-Proteinmolekül nach Anspruch 1, das wenigstens 135 der N-terminalen 163 Aminosäurereste von HBc enthält.

4. Rekombinantes chimäres Hepatitis-B-Core-Proteinmolekül nach Anspruch 3, das eine HBc-Sequenz von wenigstens 135 der N-terminalen 156 Aminosäurereste des HBc-Moleküls enthält.

5. Rekombinantes chimäres HBc-Proteinmolekül nach Anspruch 1, wobei die peptidgebundene Sequenz von bis zu 75 Resten vorliegt.

6. Rekombinantes chimäres HBc-Proteinmolekül nach Anspruch 5, das eine zweite peptidgebundene Sequenz von bis zu 75 vorliegenden Resten enthält, und zwar gebunden an den N-Terminus oder den C-Terminus des Chimären.

7. Rekombinantes chimären HBc-Proteinmolekül nach Anspruch 6, wobei die erstgenannte Sequenz von bis zu 75 Resten ein B-Zellen-Epitop enthält.

8. Rekombinantes chimäres HBc-Proteinmolekül nach Anspruch 6, wobei die zweitgenannte Sequenz von bis zu 75 Resten ein T-Zellen-Epitop enthält.

9. Rekombinantes chimäres HBc-Proteinmolekül nach Anspruch 1, wobei beide Cysteinreste an Positionen 48 und 107 durch einen anderen Rest ersetzt sind.

10. Rekombinantes chimäres HBc-Proteinmolekül nach Anspruch 9, wobei der Ersatzrest für jedes Cystein ausgewählt ist aus der Gruppe Glutamin, Asparagin, Serin, Alanin, Threonin und Lysin.

## Revendications

1. Molécule de protéine de noyau d'hépatite B (HBc) chimérique recombinante de jusqu'à 380 résidus d'acide aminé de longueur qui
(a) comprend la séquence HBc des positions de résidu 4 à 75 et 85 à 140 dans laquelle l'un ou les deux résidus cystéine aux positions 48 et 107 sont remplacés par un autre résidu ;
(b) a jusqu'à tous les résidus dans la séquence de HBc des positions 76 à 85 présents et liés par liaison peptidique à une séquence hétérologue de jusqu'à 75 résidus d'acide aminé qui constituent un immunogène, ou une séquence d'un à 40 résidus d'acide aminé qui constituent un résidu lieur chimiquement réactif pour un haptène conjugué ;
(c) contient un à trois résidus cystéine présents (I) à une position d'acide aminé de la molécule chimérique correspondant à la position d'acide aminé -20 à +1 de l'extrémité N-terminale de la séquence HBc de SEQ ID NO: 1 dans une séquence autre que celle de la séquence de prénoyau de HBc, et, facultativement, (ii) vers l'extrémité C-terminale de la molécule depuis le résidu C-terminal de la séquence HBc et à 30 résidus de l'extrémité C-terminale de la molécule chimérique (« résidu(s) cystéine C-terminal(terminaux) ») ; (d) contient jusqu'à 20 pour cent de résidus d'acide aminé substitués dans la séquence HBc, et (e) s'auto-assemble en particules après expression qui, après collecte, purification et dissolution, présentent un rapport d'absorbance à 280 nm à 260 nm de 0,9 à 1,7.

2. Molécule de protéine de noyau d'hépatite B chimérique recombinante selon la revendication 1 qui contient un à trois résidu(s) cystéine C-terminal(terminaux).

3. Molécule de protéine de noyau d'hépatite B chimérique recombinante selon la revendication 1 qui contient au moins 135 des 163 résidus d'acide aminé N-terminaux de HBc.

4. Molécule de protéine de noyau d'hépatite B chimérique recombinante selon la revendication 3 qui contient une séquence HBc d'au moins 135 des 156 résidus d'acide aminé N-terminaux de la molécule HBc.

5. Molécule de protéine chimérique HBc recombinante selon la revendication 1 dans laquelle ladite séquence liée par liaison peptidique de jusqu'à 75 résidus est présente.

6. Molécule de protéine chimérique HBc recombinante selon la revendication 5 qui contient une deuxième séquence liée par liaison peptidique de jusqu'à 75 résidus présents liés à l'extrémité N-terminale ou C-terminale de la chimère.

7. Molécule de protéine chimérique HBc recombinante selon la revendication 6 dans laquelle ladite première séquence nommée de jusqu'à 75 résidus contient un épitope de lymphocyte B.

8. Molécule de protéine chimérique HBc recombinante selon la revendication 6 dans laquelle ladite deuxième séquence nommée de jusqu'à 75 résidus contient un épitope de lymphocyte T.

9. Molécule de protéine chimérique HBc recombinante selon la revendication 1 dans laquelle les résidus cystéine aux positions 48 et 107 sont remplacés par un autre résidu.

10. Molécule de protéine chimérique HBc recombinante selon la revendication 9 dans laquelle le résidu de remplacement pour chaque cystéine est choisi dans le groupe constitué de la glutamine, l'asparagine, la sérine, l'alanine, la thréonine et la lysine.
